(19) Europäisches Patentamt / European Patent Office / Office européen des brevets

(11) **EP 4 375 287 A1**

(12) **EUROPEAN PATENT APPLICATION**
published in accordance with Art. 153(4) EPC


(43) Date of publication:
**29.05.2024 Bulletin 2024/22**

(21) Application number: **22845234.8**

(22) Date of filing: **15.07.2022**

(51) International Patent Classification (IPC):
***C07D 498/22*** *(2006.01)* ***A61K 31/496*** *(2006.01)*
***A61P 35/00*** *(2006.01)*

(52) Cooperative Patent Classification (CPC):
**A61K 31/496; A61P 35/00; C07D 498/22**

(86) International application number:
**PCT/CN2022/105959**

(87) International publication number:
**WO 2023/001069 (26.01.2023 Gazette 2023/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**23.07.2021 CN 202110839425**
**20.08.2021 CN 202110960699**
**18.09.2021 CN 202111127366**
**12.10.2021 CN 202111187955**
**26.01.2022 CN 202210093818**
**24.06.2022 CN 202210731479**



(71) Applicant: **Medshine Discovery Inc.**
**Nanjing, Jiangsu 210032 (CN)**

(72) Inventors:
- **WANG, Jianfei**
  **Shanghai 200131 (CN)**
- **CHEN, Shulun**
  **Shanghai 200131 (CN)**
- **YANG, Guangwen**
  **Shanghai 200131 (CN)**
- **ZHANG, Yang**
  **Shanghai 200131 (CN)**
- **CHEN, Shuhui**
  **Shanghai 200131 (CN)**

(74) Representative: **Cabinet Becker et Associés**
**25, rue Louis le Grand**
**75002 Paris (FR)**


(54) **MACROCYCLIC AMIDE COMPOUNDS AND APPLICATION THEREOF**


(57) A series of macrocyclic amide compounds and an application thereof. Specifically disclosed are a compound as represented by formula (V) or a pharmaceutically acceptable salt thereof and an application thereof.

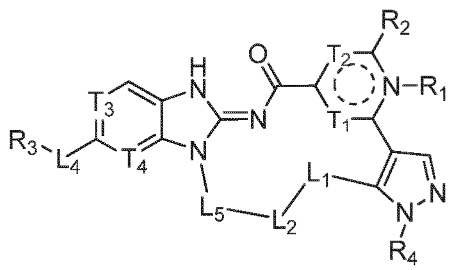

(V)


EP 4 375 287 A1

## Description

**This application claims the priority of:**

CN202110839425.1, filed on July 23, 2021;
CN202110960699.6, filed on August 20, 2021;
CN202111127366.1, filed on September 18, 2021;
CN202111187955.9, filed on October 12, 2021;
CN202210093818.7, filed on January 26, 2022;
CN202210731479.0, filed on June 24, 2022.

## TECHNICAL FIELD

The present disclosure relates to a series of macrocyclic amide compounds and applications thereof, specifically to a compound represented by formula (V) and a pharmaceutically acceptable salt thereof.

## BACKGROUND

Epidermal growth factor receptor (EGFR) is a receptor tyrosine kinase that transduces mitotic signals and is widely expressed in normal tissues. At the same time, abnormal activating mutations of EGFR exist in 10-15% of Caucasians and up to 50% of patients with non-small cell lung cancer in East Asian. These activating mutations promote survival, proliferation, and metastasis of tumor cells. Among them, deletion of exon 19 (del 19) and the L858R point mutation of exon 21 are the most common mutations. Therefore, tyrosine kinase inhibitors (TKIs) targeting EGFR were developed to competitively bind to EGFR with endogenous ligands, inhibit the abnormal activation of EGFR, and block the EGFR signaling pathway to treat lung cancer.

The first-generation EGFR inhibitors, such as gefitinib and erlotinib, were first developed and showed good efficacy in patients. However, after a period of treatment, the patient developed multiple drug-resistant mutations, especially the T790M mutation. Therefore, the second-generation EGFR inhibitors afatinib and dacomitinib were developed to alleviate the drug resistance caused by T790M-related mutations. Subsequently, the third-generation EGFR inhibitor osimertinib solved the drug resistance problem of T790M and was highly selective for wild-type EGFR.

However, recent data suggest that 20-40% of second-line patients who relapse on osimertinib acquire novel EGFR mutations at a cysteine residue (C797S) required for covalent inhibitor binding. More importantly, the EGFR del19/L858R T790M C797S cis mutated kinase variant that emerged in second-line patients who progressed after osimertinib treatment is no longer inhibited by any approved first, second, or third generation EGFR TKI inhibitors. Therefore, developing safer and more effective fourth-generation EGFR inhibitors targeting the C797S mutation has important research significance and strong clinical demand.

In "J. Med. Chem. 2019, 62, 22, 10272-10293" in 2019, the German pharmaceutical company Boehringer Ingelheim reported a new generation of EGFR TKI-BI-4020. BI-4020 is a non-covalent macrocyclic TKI that not only inhibits the EGFR del19/T790M/C797S triple mutant, but also inhibits the double mutant EGFR del19/T790M and single mutant EGFR del19, while having good selectivity for EGFR wild type. In addition, BI-4020 also shows good inhibitory activity against EGFR mutant cells, superior kinase selectivity and pharmacokinetic properties in vivo. In the PC-9 (EGFR del19/T790M /C797S) NSCLC xenograft model, BI-4020 also showed good anti-tumor efficacy. However, BI-4020 ultimately did not enter clinical research.

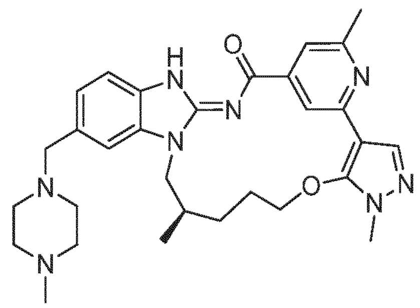

BI-4020

## SUMMARY

The present disclosure provides a compound represented by formula (V) or a pharmaceutically acceptable salt thereof,

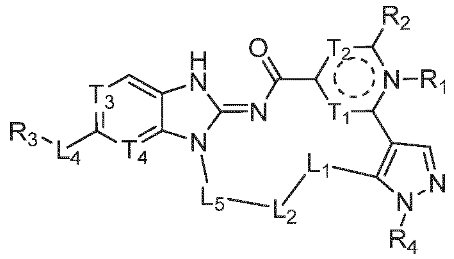

(V)

wherein

$T_1$ and $T_2$ are each independently selected from N and CH;
$T_3$ is selected from N and $CR_e$;
$T_4$ is selected from N and CRf;
$L_1$ is selected from -O- and -NH-;
$L_2$ is selected from $-CH_2-$ and -C(=O)-;
$L_3$ is selected from -O-, -NH- and $-CH_2-$;
$L_4$ is selected from $-C_{1-3}$ alkyl-, $-P(=O)(R_a)-$, $-S(=O)_2-$, $-S(=O)(=NR_c)-$ and $-N=S(=O)(R_b)-$;
$L_5$ is selected from

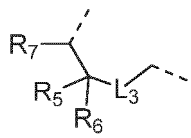

,

$-C_{3-5}$ cycloalkyl-, -4- to 5-membered heterocycloalkyl-, $-CH_2-C_{5-6}$ cycloalkyl-, $-CH_2-C_{5-6}$ cycloalkyl-$CH_2$-, $-CH_2$-5- to 6-membered heterocycloalkyl- and $-CH_2$-5- to 6-membered heterocycloalkyl-$CH_2$-;
$R_1$ is absent, $R_2$ is selected from $CH_3$ and cyclopropyl;
alternatively, $R_1$ and $R_2$ are taken together with the atoms to which they are attached to form a 5-membered heteroaryl group;
$R_3$ is selected from $C_{1-3}$ alkyl, $C_{3-5}$ cycloalkyl and -piperazinyl-methyl;
$R_4$ is selected from $C_{1-3}$ alkyl, wherein the $C_{1-3}$ alkyl is optionally substituted with 1, 2 or 3 $R_d$;
$R_5$ is selected from $CH_3$, wherein the $CH_3$ is optionally substituted with 1, 2 or 3 halogens;
$R_6$ is selected from H and $CH_3$, wherein the $CH_3$ is optionally substituted with 1, 2 or 3 halogens;
$R_7$ is selected from H and $CH_3$, wherein the $CH_3$ is optionally substituted with 1, 2 or 3 halogens;
alternatively, $R_5$ and $R_6$ are taken together with the atom to which they are attached to form a cyclopropyl;
$R_a$ is selected from $C_{1-3}$ alkyl and $C_{3-5}$ cycloalkyl;
$R_b$ is selected from $C_{1-3}$ alkyl;
$R_c$ is selected from H and $C_{1-3}$ alkyl;
$R_d$ is selected from halogen and $C_{1-3}$ alkylamino;
alternatively, $R_a$ and $R_3$ are taken together with the atom to which they are attached to form a 5- to 6-membered heterocycloalkyl, wherein the 5- to 6-membered heterocycloalkyl is optionally substituted with 1 or 2 $CH_3$;
alternatively, $R_b$ and $R_3$ are taken together with the atoms to which they are attached to form a 5- to 11-membered heterocycloalkyl, wherein the 5- to 11-membered heterocycloalkyl is optionally substituted with 1 or 2 $R_g$;
alternatively, $R_c$ and $R_3$ are taken together with the atoms to which they are attached to form a 5- to 6-membered heterocycloalkyl, wherein the 5- to 6-membered heterocycloalkyl is optionally substituted with 1 or 2 $CH_3$;
$R_e$ is selected from H, F, Cl, Br, I and CN;
$R_f$ is selected from H, F, Cl, Br, I and CN;
$R_g$ is selected from $CH_3$ and 4- to 6-membered heterocycloalkyl, wherein the 4- to 6-membered heterocycloalkyl is optionally substituted with 1 or 2 $CH_3$;

provided that, when $L_1$ is selected from -O-, $L_2$ is selected from $-CH_2-$, $L_3$ is selected from $-CH_2-$, $L_4$ is selected from $-CH_2-$, and $R_3$ is selected from -piperazinyl-methyl, then at least one of $T_1$ and $T_2$ is selected from N, alternatively, $R_1$ and $R_2$ are taken together with the atoms to which they are attached to form a 5-membered heteroaryl group.

In some embodiments of the present disclosure, the $T_1$ is selected from N, and other variables are as defined in the present disclosure.

In some embodiments of the present disclosure, the $T_1$ is selected from CH, and other variables are as defined in the present disclosure.

In some embodiments of the present disclosure, the $T_2$ is selected from N, and other variables are as defined in the present disclosure.

In some embodiments of the present disclosure, the $T_2$ is selected from CH, and other variables are as defined in the present disclosure.

In some embodiments of the present disclosure, the $R_g$ is selected from $CH_3$, oxetanyl, azetidinyl, tetrahydropyranyl, morpholinyl, piperidinyl, piperazinyl and N-methylpiperidinyl, and other variables are as defined in the present disclosure.

In some embodiments of the present disclosure, the $R_a$ is selected from $CH_3$, $CH_2CH_3$, $CH(CH_3)_2$ and cyclopropyl, and other variables are as defined in the present disclosure.

In some embodiments of the present disclosure, the $R_a$ and $R_3$ are taken together with the atom to which they are attached to form a

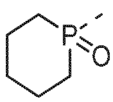, 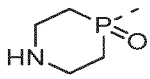 and 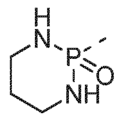,

wherein the

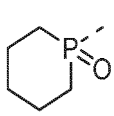, 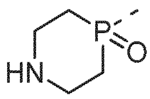 and 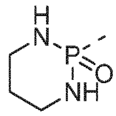

are optionally substituted with 1 or 2 $CH_3$, and other variables are as defined in the present disclosure.

In some embodiments of the present disclosure, the $R_b$ is selected from $CH_3$, $CH_2CH_3$ and $CH(CH_3)_2$, and other variables are as defined in the present disclosure.

In some embodiments of the present disclosure, the $R_b$ and $R_3$ are taken together with the atoms to which they are attached to form a 5- to 6-membered monocyclic heterocycloalkyl and 7-to 11-membered bicyclic heterocycloalkyl, wherein the 5- to 6-membered monocyclic heterocycloalkyl and 7- to 11-membered bicyclic heterocycloalkyl are optionally substituted with 1 or 2 $R_g$, and other variables are as defined in the present disclosure.

In some embodiments of the present disclosure, the $R_b$ and $R_3$ are taken together with the atoms to which they are attached to form a

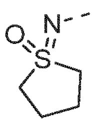, 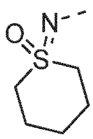, 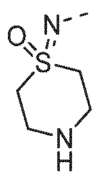, 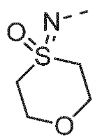, 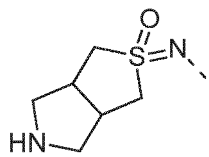,

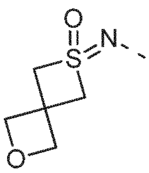, 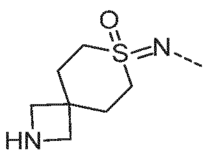, 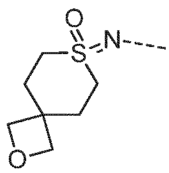, 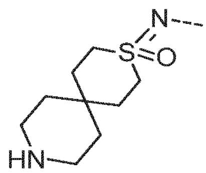 and 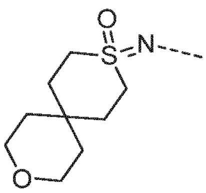,

wherein the

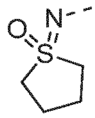, 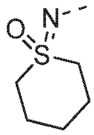, 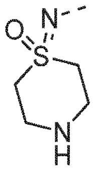, 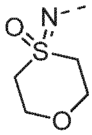, 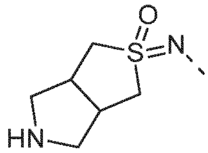, 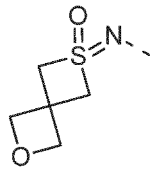, 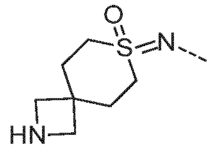, 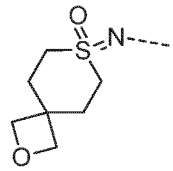,

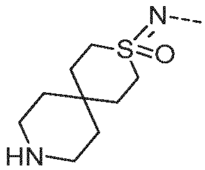 and 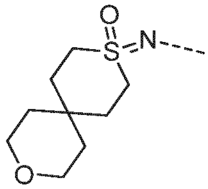

are optionally substituted with 1 or 2 $R_g$, and other variables are as defined in the present disclosure.

In some embodiments of the present disclosure, the $R_c$ and $R_3$ are taken together with the atoms to which they are attached to form a

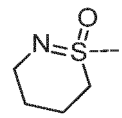,

wherein the

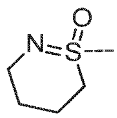

is optionally substituted with 1 or 2 $CH_3$, and other variables are as defined in the present disclosure.

In some embodiments of the present disclosure, the $L_4$ is selected from $-CH_2-$, $-P(=O)(CH_3)-$, $-P(=O)(CH_2CH_3)-$,

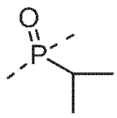, 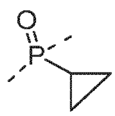,

$-S(=O)_2-$, $-S(=O)(=NH)-$, $-S(=O)(=NCH_3)-$, $-S(=O)(=NCH_2CH_3)-$, $-N=S(=O)(CH_3)-$, $-N=S(=O)(CH_2CH_3)-$, and other variables are as defined in the present disclosure.

In some embodiments of the present disclosure, the $R_3$ is selected from $CH_3$, $CH_2CH_3$, $CH(CH_3)_2$, cyclopropyl and

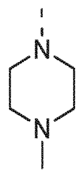,

and other variables are as defined in the present disclosure.

In some embodiments of the present disclosure, the $R_4$ is selected from $CH_3$, $CH_2CH_2N(CH_3)_2$ and $CH_2CH_2NHCH(CH_3)_2$, and other variables are as defined in the present disclosure.

In some embodiments of the present disclosure, the structural moiety $-L_4-R_3$ is selected from

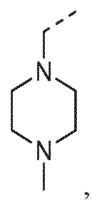, 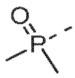, 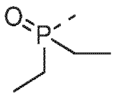, 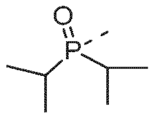, 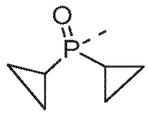, 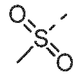, 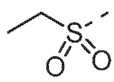, 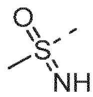, 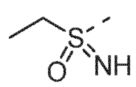,

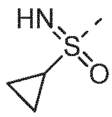, 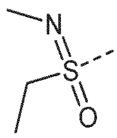, 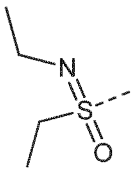, 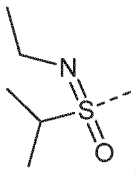, 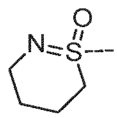, 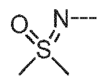, 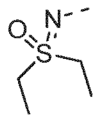, 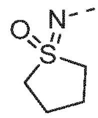, 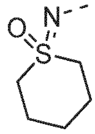, 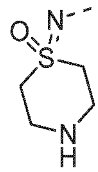,

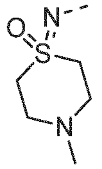, 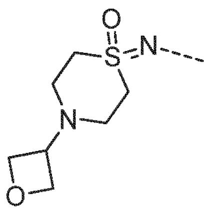, 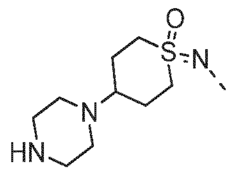, 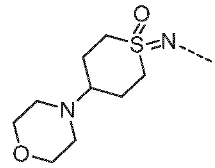, 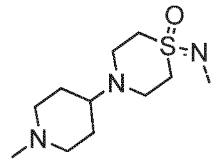,

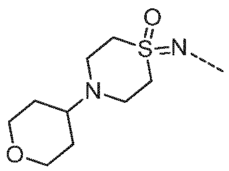, 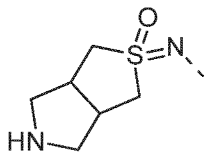, 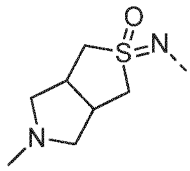, 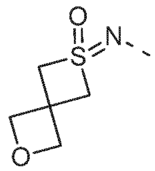, 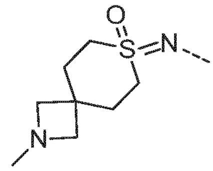,

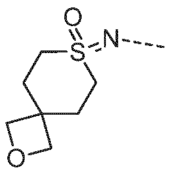, 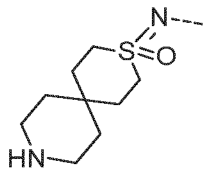, 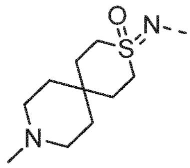, 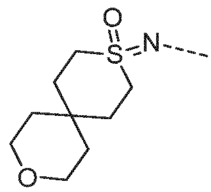, 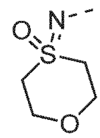, 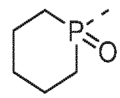,

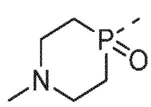 and 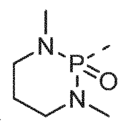,

and other variables are as defined in the present disclosure.

In some embodiments of the present disclosure, the $L_5$ is selected from $-CH_2CH(CH_3)CH_2CH_2-$, $-CH_2CH(CH_3)OCH_2-$, $-CH_2C(CH_3)_2CH_2CH_2-$, $-CH(CH_3)CH(CH_3)CH_2CH_2-$, $-CH_2CH(CF_3)CH_2CH_2-$, cyclopentyl, pyrrolidinyl, $-CH_2$-cyclopentyl-, $-CH_2$-cyclopentyl-$CH_2-$, $-CH_2$-cyclohexyl-, $-CH_2$-pyrrolidinyl-, $-CH_2$-tetrahydrofuranyl- and

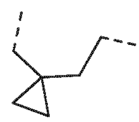,

and other variables are as defined in the present disclosure.

In some embodiments of the present disclosure, the structural moiety

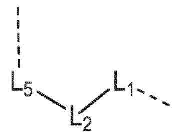

is selected from

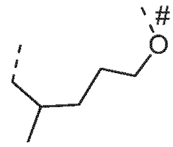, 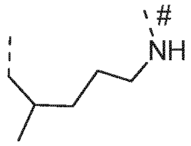, 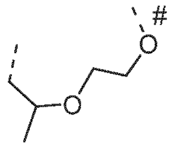, 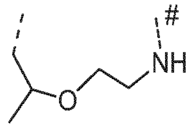, 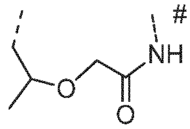,

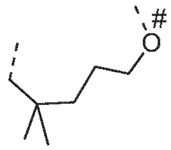, 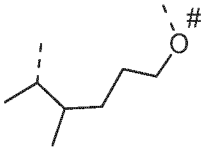, 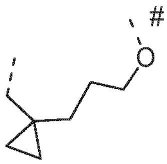, 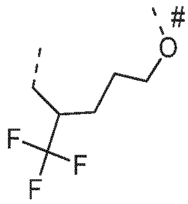, 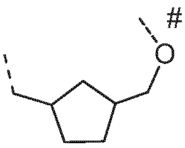, 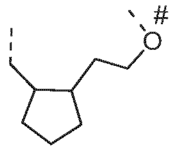,

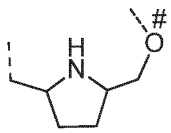, 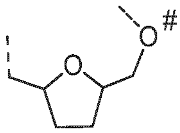 and 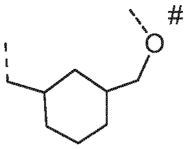,

wherein the "#" end is connected to the pyrazolyl group, and other variables are as defined in the present disclosure.

In some embodiments of the present disclosure, the structural moiety

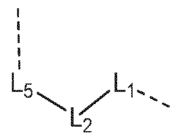

is selected from

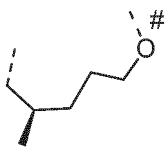, 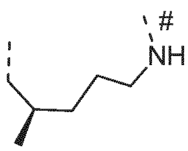, 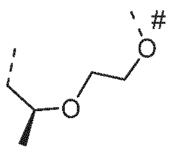, 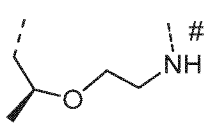, 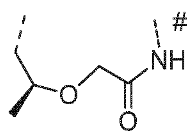,

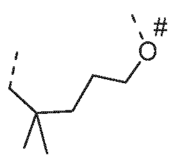, 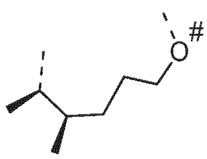, 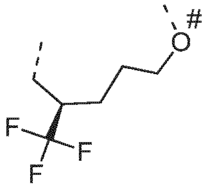, 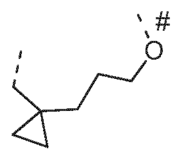, 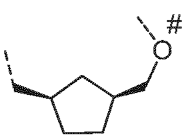, 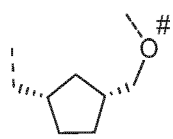,

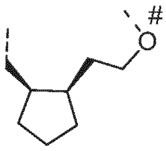, 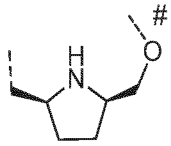, 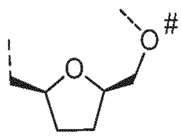 and 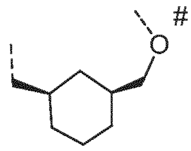,

wherein the "#" end is connected to the pyrazolyl group, and other variables are as defined in the present disclosure.

In some embodiments of the present disclosure, the structural moiety

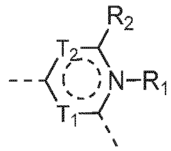

is selected from

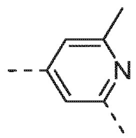, 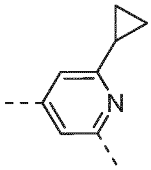, 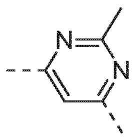, 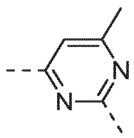, 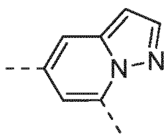, 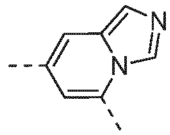,

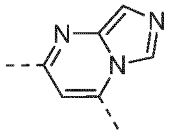 and 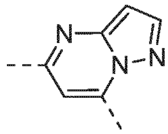,

and other variables are as defined in the present disclosure.

The present disclosure provides a compound represented by formula (V) or a pharmaceutically acceptable salt thereof,

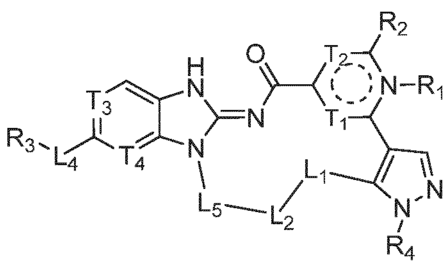

(V)

wherein

- $T_1$ and $T_2$ are each independently selected from N and CH;
- $T_3$ is selected from N and $CR_e$;
- $T_4$ is selected from N and CRf;
- $L_1$ is selected from -O- and -NH-;
- $L_2$ is selected from $-CH_2-$ and -C(=O)-;
- $L_3$ is selected from -O-, -NH- and $-CH_2-$;
- $L_4$ is selected from $-C_{1-3}$ alkyl-, $-P(=O)(R_a)-$, $-S(=O)_2-$, -S(=O)(=NH)- and $-N=S(=O)(R_b)-$;
- $L_5$ is selected from

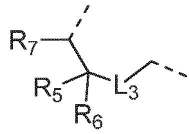

,

$C_{3-5}$ cycloalkyl and 4- to 5-membered heterocycloalkyl;
$R_1$ is absent;
$R_2$ is selected from $CH_3$;
alternatively, $R_1$ and $R_2$ are taken together with the atoms to which they are attached to form a 5-membered heteroaryl group;
$R_3$ is selected from $C_{1-3}$ alkyl, $C_{3-5}$ cycloalkyl and -piperazinyl-methyl;
$R_4$ is selected from $C_{1-3}$ alkyl, wherein the $C_{1-3}$ alkyl is optionally substituted with 1, 2 or 3 $R_d$;
$R_5$ is selected from $CH_3$, wherein the $CH_3$ is optionally substituted with 1, 2 or 3 halogens;
$R_6$ is selected from H and $CH_3$, wherein the $CH_3$ is optionally substituted with 1, 2 or 3 halogens;
$R_7$ is selected from H and $CH_3$, wherein the $CH_3$ is optionally substituted with 1, 2 or 3 halogens; alternatively, $R_5$ and $R_6$ are taken together with the atom to which they are attached to form a cyclopropyl;
$R_a$ is selected from $C_{1-3}$ alkyl and $C_{3-5}$ cycloalkyl;
$R_b$ is selected from $C_{1-3}$ alkyl;
$R_c$ is selected from H and $C_{1-3}$ alkyl;
$R_d$ is selected from halogen and $C_{1-3}$ alkylamino;
alternatively, $R_a$ and $R_3$ are taken together with the atom to which they are attached to form a 5- to 6-membered heterocycloalkyl, wherein the 5- to 6-membered heterocycloalkyl is optionally substituted with 1 or 2 $CH_3$;
alternatively, $R_b$ and $R_3$ are taken together with the atoms to which they are attached to form a 5- to 11-membered heterocycloalkyl, wherein the 5- to 11-membered heterocycloalkyl is optionally substituted with 1 or 2 $R_g$;
alternatively, $R_c$ and $R_3$ are taken together with the atoms to which they are attached to form a 5- to 6-membered heterocycloalkyl, wherein the 5- to 6-membered heterocycloalkyl is optionally substituted with 1 or 2 $CH_3$;
$R_e$ is selected from H, F, Cl, Br, I and CN;
$R_f$ is selected from H, F, Cl, Br, I and CN;
$R_g$ is selected from $CH_3$, piperidinyl, piperazinyl and N-methyl piperidinyl;
provided that, when $L_1$ is selected from -O-, $L_2$ is selected from $-CH_2-$, $L_3$ is selected from $-CH_2-$, $L_4$ is selected from $-CH_2-$, $R_3$ is selected from -piperazinyl-methyl, then at least one of $T_2$ and $T_3$ is selected from N, alternatively, $R_1$ and $R_2$ are taken together with the atoms to which they are attached to form a 5-membered heteroaryl group.

In some embodiments of the present disclosure, the $R_a$ is selected from $CH_3$, $CH_2CH_3$, $CH(CH_3)_2$ and cyclopropyl, and other variables are as defined in the present disclosure.

In some embodiments of the present disclosure, the $R_b$ is selected from $CH_3$, $CH_2CH_3$ and $CH(CH_3)_2$, and other variables are as defined in the present disclosure.

In some embodiments of the present disclosure, the $L_4$ is selected from $-CH_2-$, $-P(=O)(CH_3)-$, $-P(=O)(CH_2CH_3)-$,

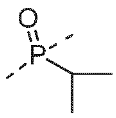

,

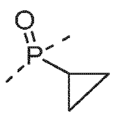

,

$-S(=O)_2-$, $-S(=O)(=NH)-$, $-S(=O)(=NCH_3)-$, $-S(=O)(=NCH_2CH_3)-$, $-N=S(=O)(CH_3)-$, $-N=S(=O)(CH_2CH_3)-$, and other variables are as defined in the present disclosure.

In some embodiments of the present disclosure, the $R_3$ is selected from $CH_3$, $CH_2CH_3$, $CH(CH_3)_2$, cyclopropyl and

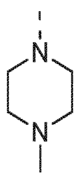

,

and other variables are as defined in the present disclosure.

In some embodiments of the present disclosure, the $R_4$ is selected from $CH_3$, $CH_2CH_2N(CH_3)_2$ and $CH_2CH_2NHCH(CH_3)_2$, and other variables are as defined in the present disclosure.

In some embodiments of the present disclosure, the structural moiety $-L_4-R_3$ is selected from

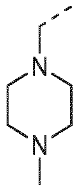 , 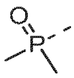 , 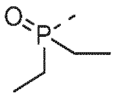 , 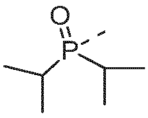 , 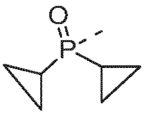 , 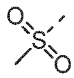 , 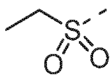 , 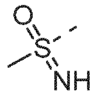 , 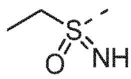 ,

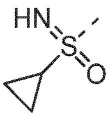 , 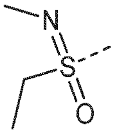 , 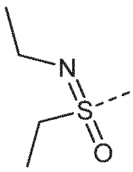 , 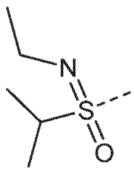 , 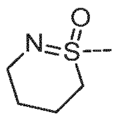 , 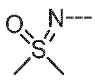 , 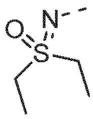 , 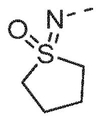 , 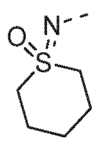 , 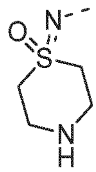 ,

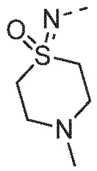 , 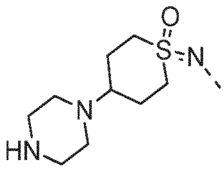 , 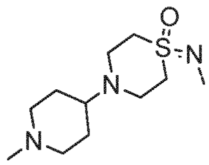 , 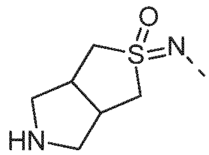 , 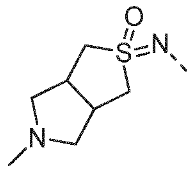 , 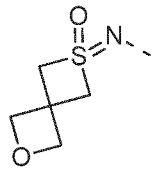 ,

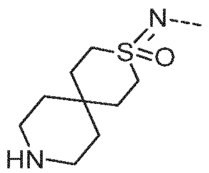 , 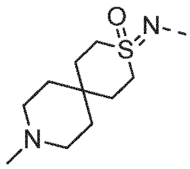 , 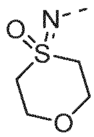 , 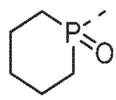 , 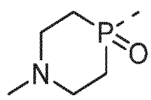 and 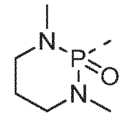 ,

and other variables are as defined in the present disclosure.

In some embodiments of the present disclosure, the $L_5$ is selected from $-CH_2CH(CH_3)CH_2CH_2-$, $-CH_2CH(CH_3)OCH_2-$, $-CH_2C(CH_3)_2CH_2CH_2-$, $-CH(CH_3)CH(CH_3)CH_2CH_2-$, $-CH_2CH(CF_3)CH_2CH_2-$, cyclopentyl, pyrrolidinyl and

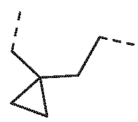 ,

and other variables are as defined in the present disclosure.

In some embodiments of the present disclosure, the structural moiety

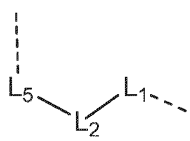

is selected from

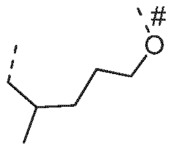, 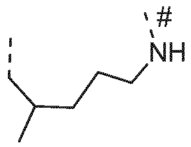, 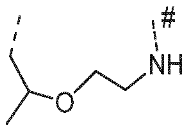, 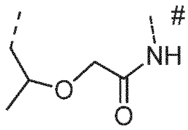, 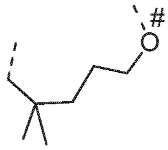,

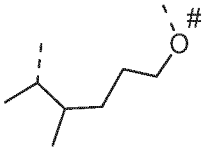, 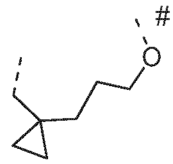, 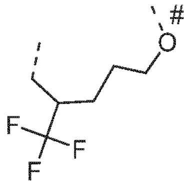, 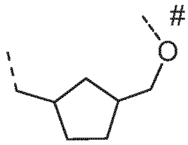 and 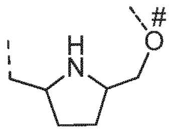,

wherein the "#" end is connected to the pyrazolyl group, and other variables are as defined in the present disclosure.

In some embodiments of the present disclosure, the structural moiety

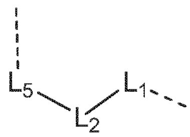

is selected from

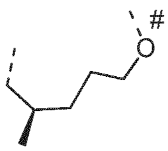, 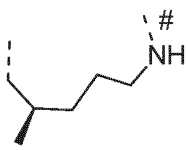, 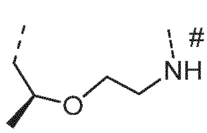, 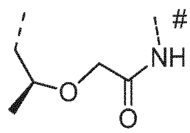, 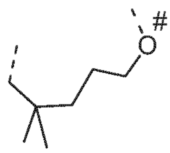,

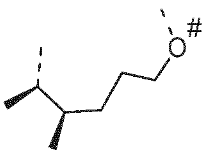, 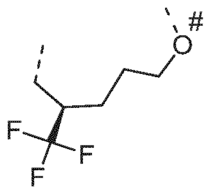, 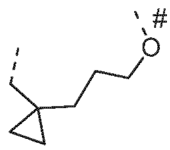, 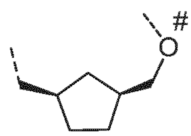 and 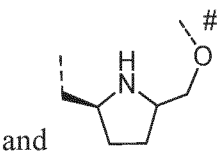,

wherein the "#" end is connected to the pyrazolyl group, and other variables are as defined in the present disclosure.

In some embodiments of the present disclosure, the structural moiety

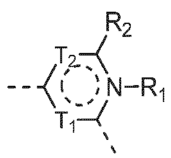

is selected from

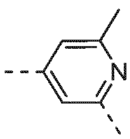, 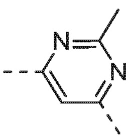, 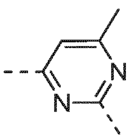, 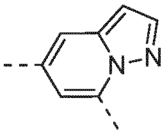, 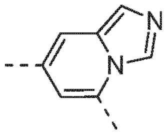, 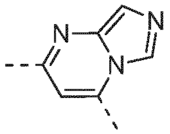

and

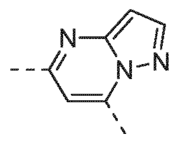

,

and other variables are as defined in the present disclosure.

The present disclosure provides a compound represented by formula (IV) or a pharmaceutically acceptable salt thereof,

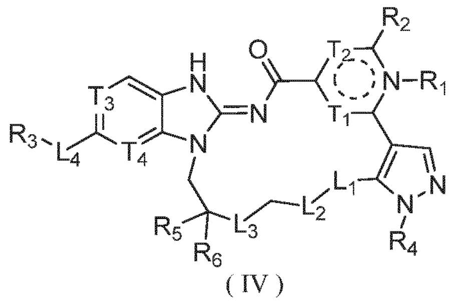

(IV)

wherein

$T_1$ and $T_2$ are each independently selected from N and CH;
$T_3$ is selected from N, CF and CH;
$T_4$ is selected from N and CH;
$L_1$ is selected from -O- and -NH-;
$L_2$ is selected from $-CH_2-$ and -C(=O)-;
$L_3$ is selected from -O-, -NH- and $-CH_2-$;
$L_4$ is selected from $-C_{1-3}$ alkyl-, $-P(=O)(R_a)-$, $-S(=O)_2-$, -S(=O)(=NH)- and $-N=S(=O)(R_b)-$;
$R_1$ is absent;
$R_2$ is selected from $CH_3$;
alternatively, $R_1$ and $R_2$ are taken together with the atoms to which they are attached to form a 5-membered heteroaryl group;
$R_3$ is selected from $C_{1-3}$ alkyl, $C_{3-5}$ cycloalkyl and -piperazinyl-methyl;
$R_4$ is selected from $C_{1-3}$ alkyl, wherein the $C_{1-3}$ alkyl is optionally substituted with 1, 2 or 3 $R_d$;
$R_5$ is selected from $CH_3$;
$R_6$ is selected from H and $CH_3$;
alternatively, $R_5$ and $R_6$ are taken together with the atom to which they are attached to form a cyclopropyl;
$R_a$ is selected from $C_{1-3}$ alkyl and $C_{3-5}$ cycloalkyl;
$R_b$ is selected from $C_{1-3}$ alkyl;
$R_c$ is selected from H and $C_{1-3}$ alkyl;
$R_d$ is selected from halogen and $C_{1-3}$ alkylamino;
alternatively, $R_a$ and $R_3$ are taken together with the atom to which they are attached to form a 5- to 6-membered heterocycloalkyl, wherein the 5- to 6-membered heterocycloalkyl is optionally substituted with 1 or 2 $CH_3$;
alternatively, $R_b$ and $R_3$ are taken together with the atoms to which they are attached to form a 5- to 6-membered heterocycloalkyl, wherein the 5- to 6-membered heterocycloalkyl is optionally substituted with 1 or 2 $CH_3$;
alternatively, $R_c$ and $R_3$ are taken together with the atoms to which they are attached to form a 5- to 6-membered heterocycloalkyl, wherein the 5- to 6-membered heterocycloalkyl is optionally substituted with 1 or 2 $CH_3$;
provided that, when $L_1$ is selected from -O-, $L_2$ is selected from $-CH_2-$, $L_3$ is selected from $-CH_2-$, $L_4$ is selected from $-CH_2-$, $R_3$ is selected from -piperazinyl-methyl, then at least one of $T_2$ and $T_3$ is selected from N, alternatively, $R_1$ and $R_2$ are taken together with the atoms to which they are attached to form a 5-membered heteroaryl group.

In some embodiments of the present disclosure, the $R_a$ is selected from $CH_3$, $CH_2CH_3$, $CH(CH_3)_2$ and cyclopropyl, and other variables are as defined in the present disclosure.

In some embodiments of the present disclosure, the $R_b$ is selected from $CH_3$, $CH_2CH_3$ and $CH(CH_3)_2$, and

other variables are as defined in the present disclosure.

In some embodiments of the present disclosure, the $L_4$ is selected from $-CH_2-$, $-P(=O)(CH_3)-$, $-P(=O)(CH_2CH_3)-$,

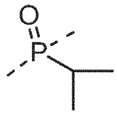, 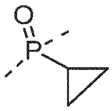,

$-S(=O)_2-$, $-S(=O)(=NH)-$, $-S(=O)(=NCH_3)-$, $-S(=O)(=NCH_2CH_3)-$, $-N=S(=O)(CH_3)-$, $-N=S(=O)(CH_2CH_3)-$, and other variables are as defined in the present disclosure.

In some embodiments of the present disclosure, the $R_3$ is selected from $CH_3$, $CH_2CH_3$, $CH(CH_3)_2$, cyclopropyl and

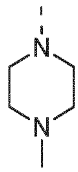,

and other variables are as defined in the present disclosure.

In some embodiments of the present disclosure, the $R_4$ is selected from $CH_3$, $CH_2CH_2N(CH_3)_2$ and $CH_2CH_2NHCH(CH_3)_2$, and other variables are as defined in the present disclosure.

In some embodiments of the present disclosure, the structural moiety $-L_4-R_3$ is selected from

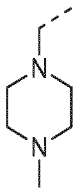, 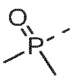, 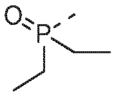, 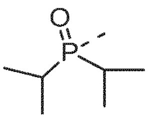, 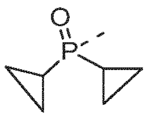, 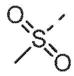, 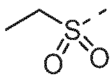, 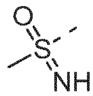, 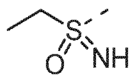,

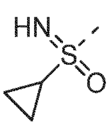, 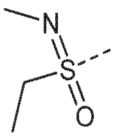, 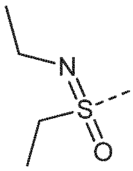, 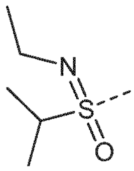, 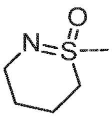, 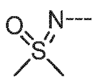, 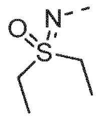, 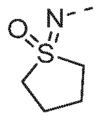, 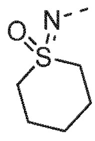, 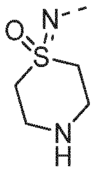,

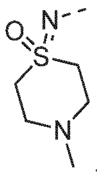, 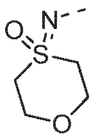, 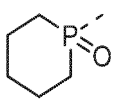, 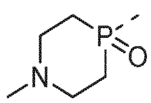 and 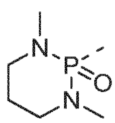,

and other variables are as defined in the present disclosure.

In some embodiments of the present disclosure, the structural moiety $-L_3-CH_2-L_2-L_1-$ is selected from $-(CH_2)_3-O-\#$, $-(CH_2)_3-NH-\#$, $-O-CH_2CH_2-NH-\#$, $-O-CH_2-C(=O)-NH-\#$ and $-O-CH_2CH_2-O-\#$, wherein the "#" end is connected to the pyrazolyl group, and other variables are as defined in the present disclosure.

In some embodiments of the present disclosure, the structural moiety

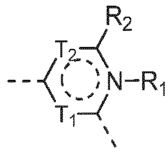

is selected from

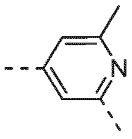, 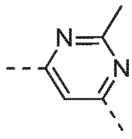, 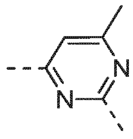, 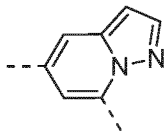, 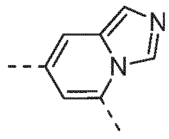, 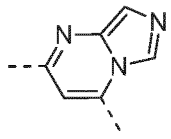

and

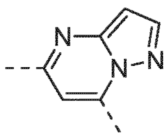,

and other variables are as defined in the present disclosure.

The present disclosure provides a compound represented by formula (I) or a pharmaceutically acceptable salt thereof,

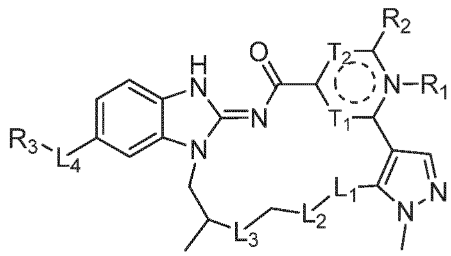

(I)

wherein

$T_1$ and $T_2$ are each independently selected from N and CH;
$L_1$ is selected from -O- and -NH-;
$L_2$ is selected from $-CH_2-$ and -C(=O)-;
$L_3$ is selected from -O-, -NH- and $-CH_2-$;
$L_4$ is selected from $-C_{1-3}$ alkyl-, $-P(=O)(R_a)-$, $-S(=O)_2-$, -S(=O)(=NH)- and $-N=S(=O)(R_b)-$;
$R_1$ is absent;
$R_2$ is selected from $CH_3$;
alternatively, $R_1$ and $R_2$ are taken together with the atoms to which they are attached to form a 5-membered heteroaryl group;
$R_3$ is selected from $C_{1-3}$ alkyl, $C_{3-5}$ cycloalkyl and -piperazinyl-methyl;
$R_a$ is selected from $C_{1-3}$ alkyl and $C_{3-5}$ cycloalkyl;
$R_b$ is selected from $C_{1-3}$ alkyl;
$R_c$ is selected from H and $C_{1-3}$ alkyl;
alternatively, $R_a$ and $R_3$ are taken together with the atom to which they are attached to form a 5- to 6-membered heterocycloalkyl, wherein the 5- to 6-membered heterocycloalkyl is optionally substituted with 1 or 2 $CH_3$;
alternatively, $R_b$ and $R_3$ are taken together with the atoms to which they are attached to form a 5- to 6-membered heterocycloalkyl, wherein the 5- to 6-membered heterocycloalkyl is optionally substituted with 1 or 2 $CH_3$;
alternatively, $R_c$ and $R_3$ are taken together with the atoms to which they are attached to form a 5- to 6-membered heterocycloalkyl, wherein the 5- to 6-membered heterocycloalkyl is optionally substituted with 1 or 2 $CH_3$;

provided that, when $L_1$ is selected from -O-, $L_2$ is selected from $-CH_2-$, $L_3$ is selected from $-CH_2-$, $L_4$ is selected from $-CH_2-$, $R_3$ is selected from -piperazinyl-methyl, then at least one of $T_2$ and $T_3$ is selected from N, alternatively, $R_1$ and $R_2$ are taken together with the atoms to which they are attached to form a 5-membered heteroaryl group.

In some embodiments of the present disclosure, provided herein are compounds or pharmaceutically acceptable salts thereof, wherein the compound is selected from

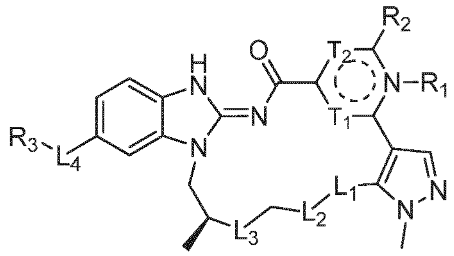

( I-1 )

wherein

$T_1$, $T_2$, $L_1$, $L_2$, $L_3$, $L_4$, $R_1$, $R_2$ and $R_3$ are as defined in the present disclosure.

In some embodiments of the present disclosure, the $R_a$ is selected from $CH_3$, $CH_2CH_3$, $CH(CH_3)_2$ and cyclopropyl, and other variables are as defined in the present disclosure.

In some embodiments of the present disclosure, the $R_b$ is selected from $CH_3$, $CH_2CH_3$ and $CH(CH_3)_2$, and other variables are as defined in the present disclosure.

In some embodiments of the present disclosure, the $L_4$ is selected from $-CH_2-$, $-P(=O)(CH_3)-$, $-P(=O)(CH_2CH_3)-$,

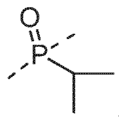 , 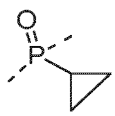 ,

$-S(=O)_2-$, $-S(=O)(=NH)-$, $-S(=O)(=NCH_3)-$, $-S(=O)(=NCH_2CH_3)-$, $-N=S(=O)(CH_3)-$, $-N=S(=O)(CH_2CH_3)-$, and other variables are as defined in the present disclosure.

In some embodiments of the present disclosure, the $R_3$ is selected from $CH_3$, $CH_2CH_3$, $CH(CH_3)_2$, cyclopropyl and

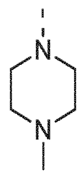 ,

and other variables are as defined in the present disclosure.

In some embodiments of the present disclosure, the structural moiety $-L_4-R_3$ is selected from

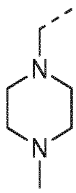 , 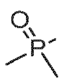 , 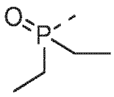 , 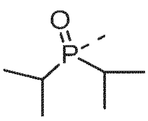 , 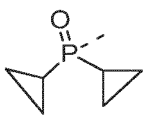 , 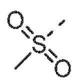 , 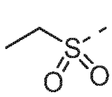 , 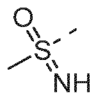 , 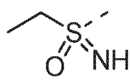 ,

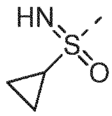, 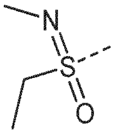, 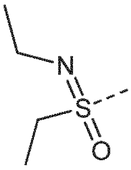, 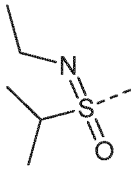, 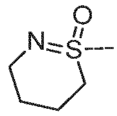, 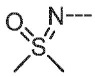, 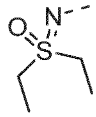, 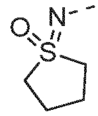, 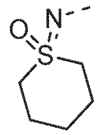, 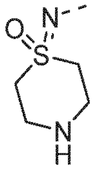,

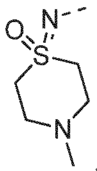, 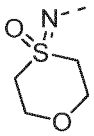, 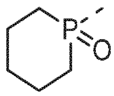, 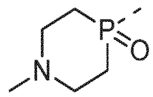 and 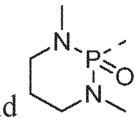,

and other variables are as defined in the present disclosure.

In some embodiments of the present disclosure, the structural moiety $-L_3-CH_2-L_2-L_1-$ is selected from $-(CH_2)_3-O-\#$, $-(CH_2)_3-NH-\#$, $-O-CH_2CH_2-NH-\#$, $-O-CH_2-C(=O)-NH-\#$ and $-O-CH_2CH_2-O-\#$, wherein the "#" end is connected to the pyrazolyl group, and other variables are as defined in the present disclosure.

In some embodiments of the present disclosure, the structural moiety

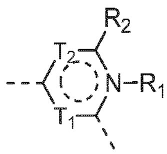

is selected from

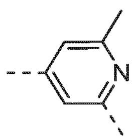, 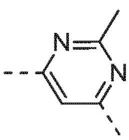, 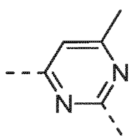, 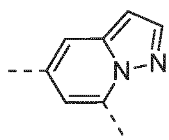, 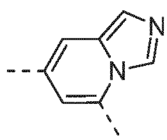, 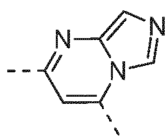

and

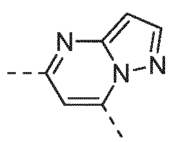,

and other variables are as defined in the present disclosure.

The present disclosure provides a compound represented by formula (I) or a pharmaceutically acceptable salt thereof,

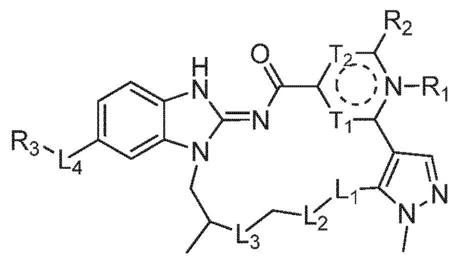

(I)

wherein

$T_1$ and $T_2$ are each independently selected from N and CH;
$L_1$ is selected from -O- and -NH-;
$L_2$ is selected from $-CH_2-$ and -C(=O)-;
$L_3$ is selected from -O-, -NH- and $-CH_2-$;
$L_4$ is selected from $-C_{1-3}$ alkyl-, $-P(=O)(R_a)-$, $-S(=O)_2-$, -S(=O)(=NH)- and $-N=S(=O)(R_b)-$;
$R_1$ is absent;
$R_2$ is selected from $CH_3$;
alternatively, $R_1$ and $R_2$ are taken together with the atoms to which they are attached to form a 5-membered heteroaryl group;
$R_3$ is selected from $C_{1-3}$ alkyl, $C_{3-5}$ cycloalkyl and -piperazinyl-methyl;
$R_a$ is selected from $C_{1-3}$ alkyl and $C_{3-5}$ cycloalkyl;
$R_b$ is selected from $C_{1-3}$ alkyl;
$R_c$ is selected from H and $C_{1-3}$ alkyl;
alternatively, $R_a$ and $R_3$ are taken together with the atom to which they are attached to form a 5- to 6-membered heterocycloalkyl, wherein the 5- to 6-membered heterocycloalkyl is optionally substituted with 1 $CH_3$;
alternatively, $R_b$ and $R_3$ are taken together with the atoms to which they are attached to form a 5- to 6-membered heterocycloalkyl, wherein the 5- to 6-membered heterocycloalkyl is optionally substituted with 1 $CH_3$;
provided that, when $L_1$ is selected from -O-, $L_2$ is selected from $-CH_2-$, $L_3$ is selected from $-CH_2-$, $L_4$ is selected from $-CH_2-$, $R_3$ is selected from -piperazinyl-methyl, then at least one of $T_2$ and $T_3$ is selected from N, alternatively, $R_1$ and $R_2$ are taken together with the atoms to which they are attached to form a 5-membered heteroaryl group.

In some embodiments of the present disclosure, provided herein are compounds or pharmaceutically acceptable salts thereof, wherein the compound is selected from

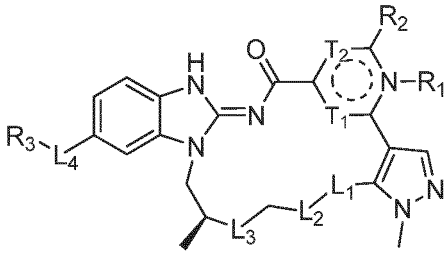

( I-1 )

wherein
$T_1$, $T_2$, $L_1$, $L_2$, $L_3$, $L_4$, $R_1$, $R_2$ and $R_3$ are as defined in the present disclosure.
In some embodiments of the present disclosure, the $R_a$ is selected from $CH_3$, $CH_2CH_3$, $CH(CH_3)_2$ and cyclopropyl, and other variables are as defined in the present disclosure.
In some embodiments of the present disclosure, the $R_b$ is selected from $CH_3$, $CH_2CH_3$ and $CH(CH_3)_2$, and other variables are as defined in the present disclosure.
In some embodiments of the present disclosure, the $L_4$ is selected from $-CH_2-$, $-P(=O)(CH_3)-$, $-P(=O)(CH_2CH_3)-$,

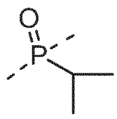
,
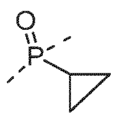
,

$-S(=O)_2-$, -S(=O)(=NH)-, $-S(=O)(=NCH_3)-$, $-S(=O)(=NCH_2CH_3)-$, $-N=S(=O)(CH_3)-$, $-N=S(=O)(CH_2CH_3)-$, and other variables are as defined in the present disclosure.
In some embodiments of the present disclosure, the $R_3$ is selected from $CH_3$, $CH_2CH_3$, $CH(CH_3)_2$, cyclopropyl and

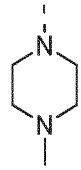,

and other variables are as defined in the present disclosure. In some embodiments of the present disclosure, the structural moiety $-L_4-R_3$ is selected from

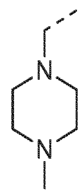,

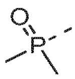, 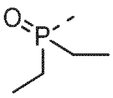, 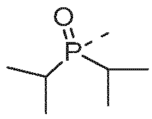, 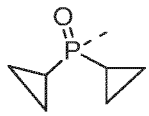, 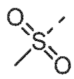, 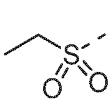, 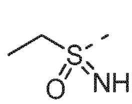, 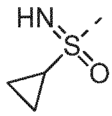, 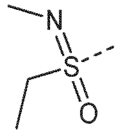,

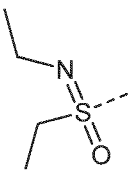, 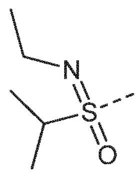 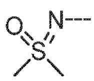, 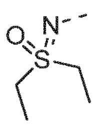, 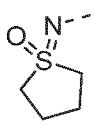, 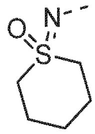, 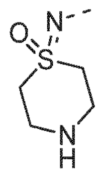, 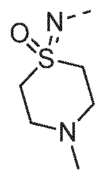, 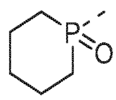, 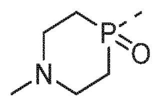

and

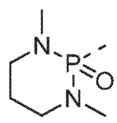,

and other variables are as defined in the present disclosure.

In some embodiments of the present disclosure, the structural moiety $-L_3-CH_2-L_2-L_1-$ is selected from $-(CH_2)_3-O-\#$, $-(CH_2)_3-NH-\#$, $-O-CH_2CH_2-NH-\#$ and -O-CH2-C(=O)-NH-#, wherein the "#" end is connected to the pyrazolyl group, and other variables are as defined in the present disclosure.

In some embodiments of the present disclosure, the structural moiety

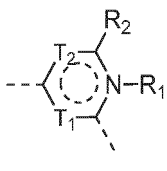

is selected from

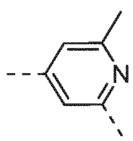, 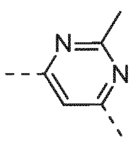, 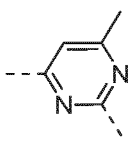, 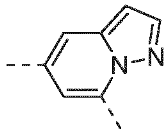, 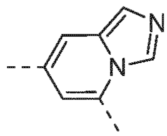, 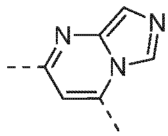

and

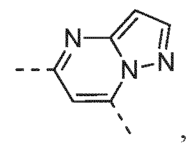

,

and other variables are as defined in the present disclosure.

The present disclosure provides a compound represented by formula (I) or a pharmaceutically acceptable salt thereof,

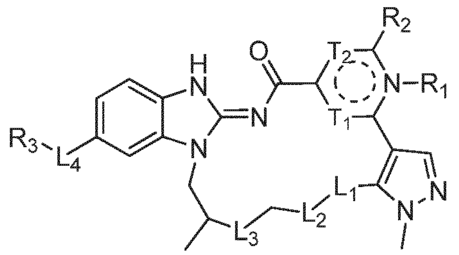

(I)

wherein

$T_1$ and $T_2$ are each independently selected from N and CH;
$L_1$ is selected from -O- and -NH-;
$L_2$ is selected from $-CH_2-$ and -C(=O)-;
$L_3$ is selected from -O-, -NH- and $-CH_2-$;
$L_4$ is selected from $-C_{1-3}$ alkyl-, $-P(=O)(R_a)-$, $-S(=O)_2-$, -S(=O)(=NH)- and $-N=S(=O)(R_b)-$;
$R_1$ is absent;
$R_2$ is selected from $CH_3$;
alternatively, $R_1$ and $R_2$ are taken together with the atoms to which they are attached to form a 5-membered heteroaryl group;
$R_3$ is selected from $C_{1-3}$ alkyl, $C_{3-5}$ cycloalkyl and -piperazinyl-methyl;
$R_a$ is selected from $C_{1-3}$ alkyl and $C_{3-5}$ cycloalkyl;
$R_b$ is selected from $C_{1-3}$ alkyl;
provided that, when $L_1$ is selected from -O-, $L_2$ is selected from $-CH_2-$, $L_3$ is selected from $-CH_2-$, $L_4$ is selected from $-CH_2-$, $R_3$ is selected from -piperazinyl-methyl, then at least one of $T_2$ and $T_3$ is selected from N, alternatively, $R_1$ and $R_2$ are taken together with the atoms to which they are attached to form a 5-membered heteroaryl group.

In some embodiments of the present disclosure, provided herein are compounds or pharmaceutically acceptable salts thereof, wherein the compound is selected from

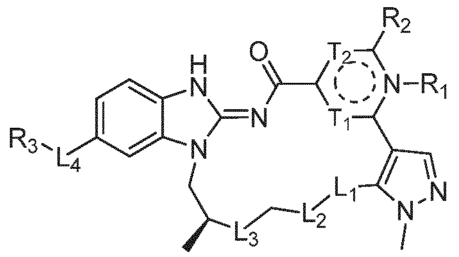

(I-1)

wherein

$T_1$, $T_2$, $L_1$, $L_2$, $L_3$, $L_4$, $R_1$, $R_2$ and $R_3$ are as defined in the present disclosure.

In some embodiments of the present disclosure, the $R_a$ is selected from $CH_3$, $CH_2CH_3$, $CH(CH_3)_2$ and cyclo-

propyl, and other variables are as defined in the present disclosure.

In some embodiments of the present disclosure, the $R_b$ is selected from $CH_3$, $CH_2CH_3$ and $CH(CH_3)_2$, and other variables are as defined in the present disclosure.

In some embodiments of the present disclosure, the $L_4$ is selected from $-CH_2-$, $-P(=O)(CH_3)-$, $-P(=O)(CH_2CH_3)-$,

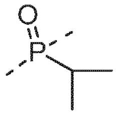,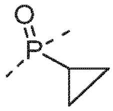,

$-S(=O)_2-$, $-S(=O)(=NH)-$ and $-N=S(=O)(CH_3)-$, and other variables are as defined in the present disclosure.

In some embodiments of the present disclosure, the $R_3$ is selected from $CH_3$, $CH_2CH_3$, $CH(CH_3)_2$, cyclopropyl and -piperazinyl-methyl, and other variables are as defined in the present disclosure. In some embodiments of the present disclosure, the structural moiety $-L_4-R_3$ is selected from

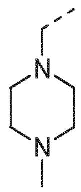, 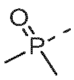, 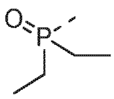, 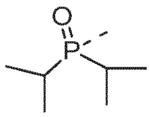, 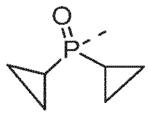, 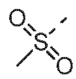, 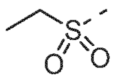, 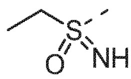, 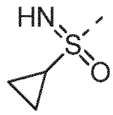

and

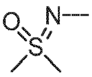,

and other variables are as defined in the present disclosure.

In some embodiments of the present disclosure, the structural moiety $-L_3-CH_2-L_2-L_1-$ is selected from $-(CH_2)_3-O-\#$, $-(CH_2)_3-NH_2-\#$, $-O-CH_2CH_2-NH-\#$ and $-O-CH_2-C(=O)-NH-\#$, wherein the "#" end is connected to the pyrazolyl group, and other variables are as defined in the present disclosure.

In some embodiments of the present disclosure, the structural moiety

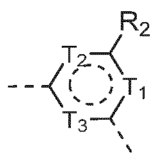

is selected from

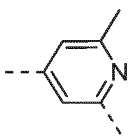, 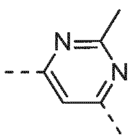, 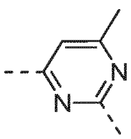, 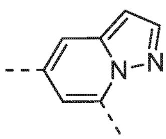, 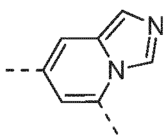, 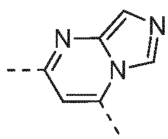

and

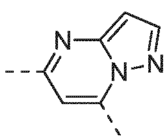,

and other variables are as defined in the present disclosure.

There are also embodiments of the present disclosure derived from any combination of the above variables.

In some embodiments of the present disclosure, provided herein are compounds or pharmaceutically acceptable salts thereof, wherein the compound is selected from

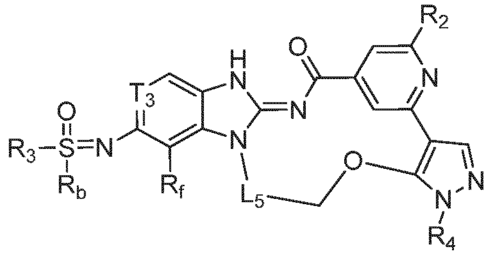

( VI-1 )

,

wherein $T_3$, $L_5$, $R_2$, $R_3$, $R_4$, $R_b$ and $R_f$ are as defined in the present disclosure.

The present disclosure also provides the following compounds or pharmaceutically acceptable salts thereof,

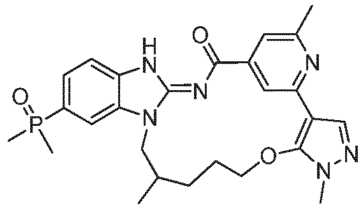
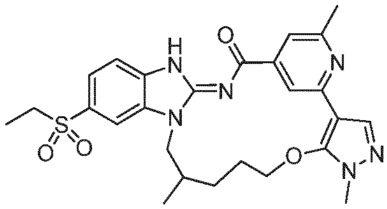
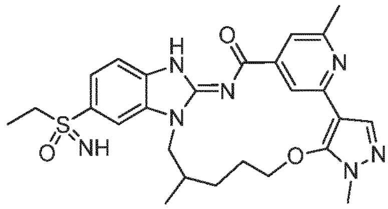
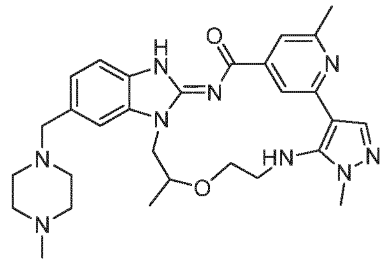
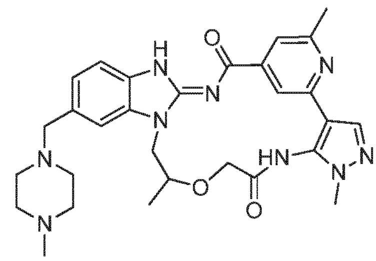
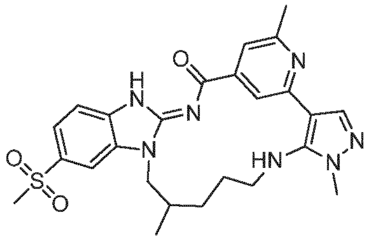
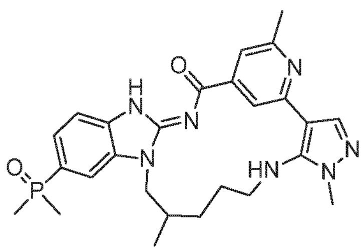
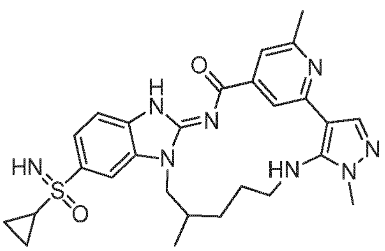
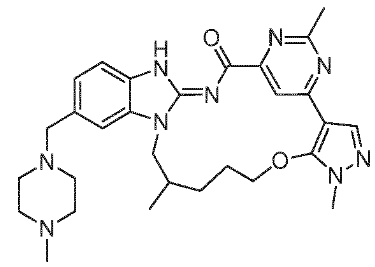
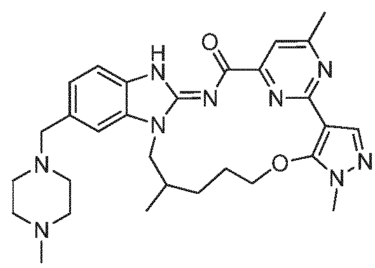
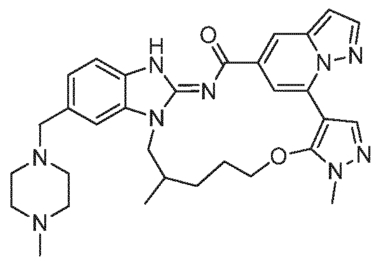
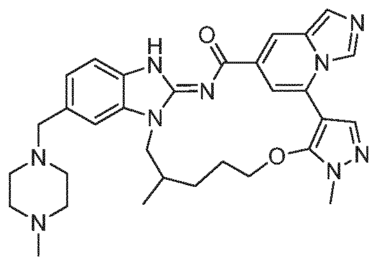

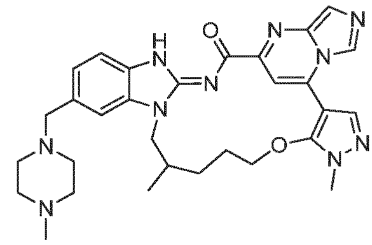
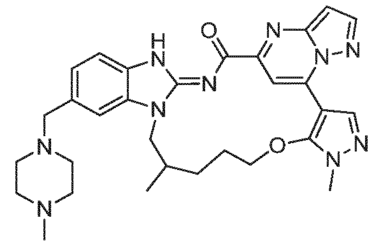
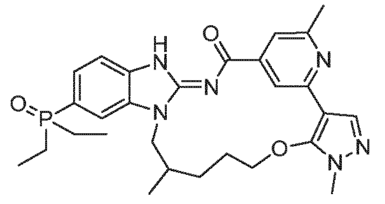
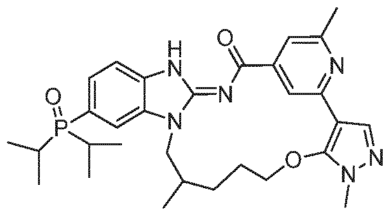
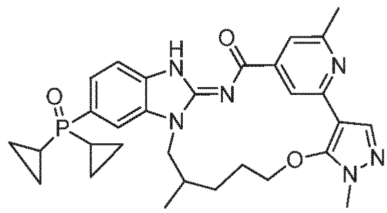
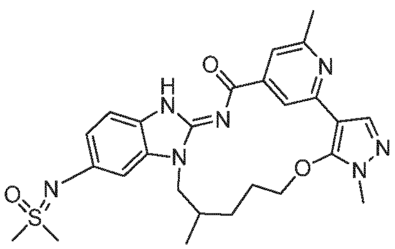
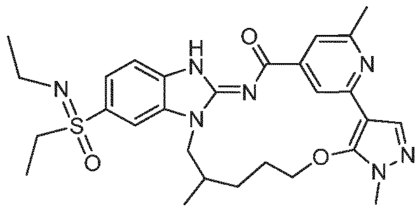
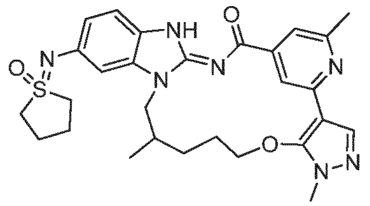
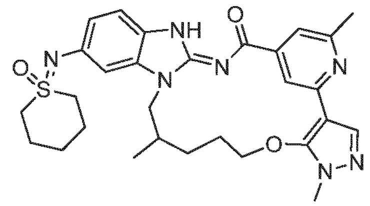
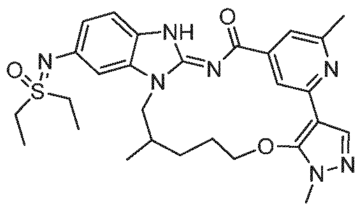
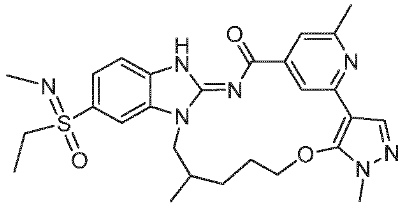
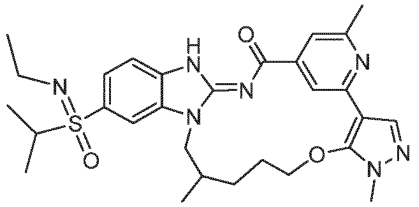
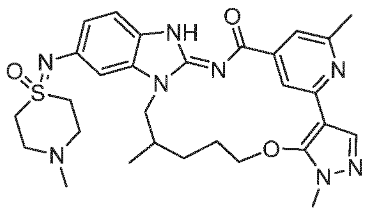
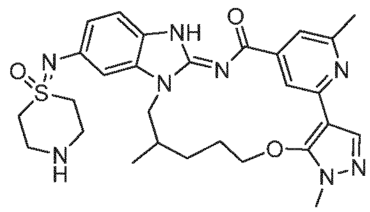
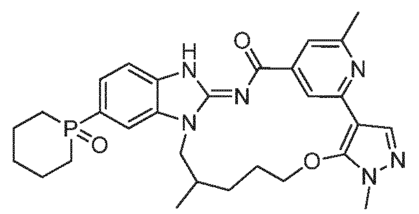
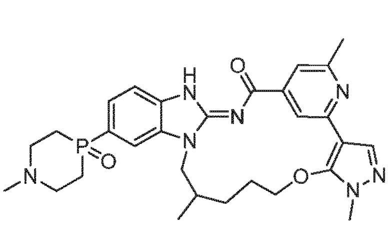
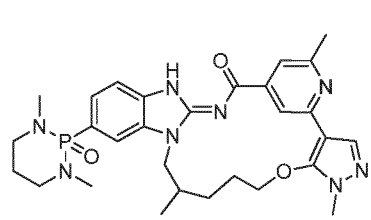
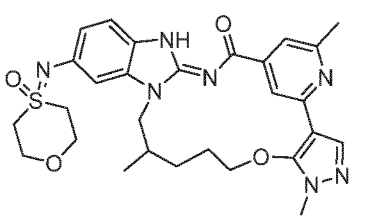
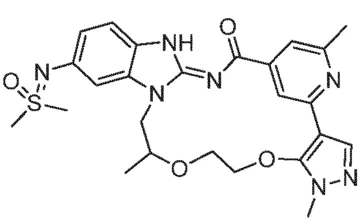
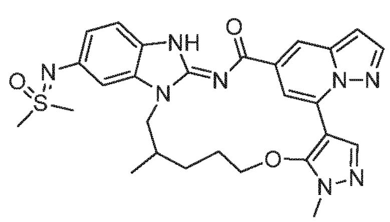
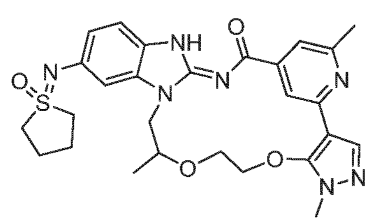

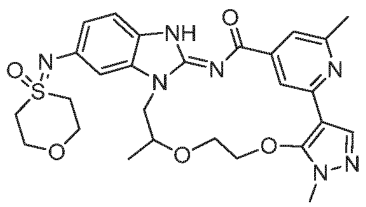
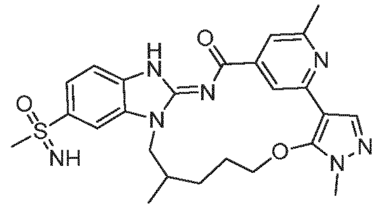
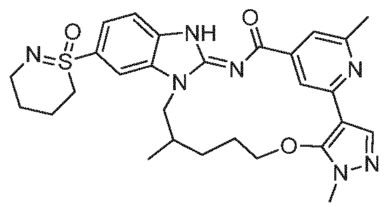

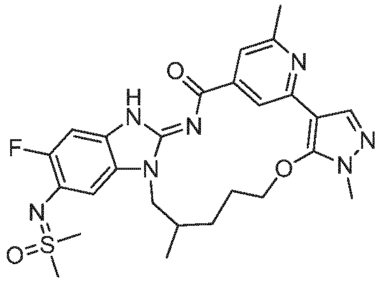
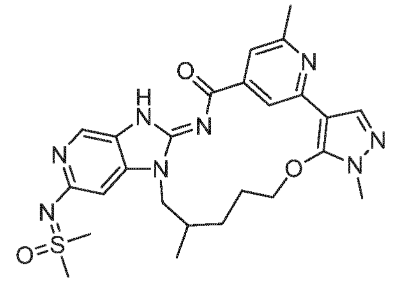
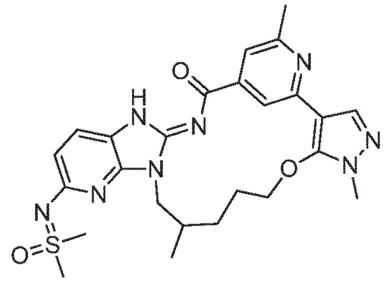

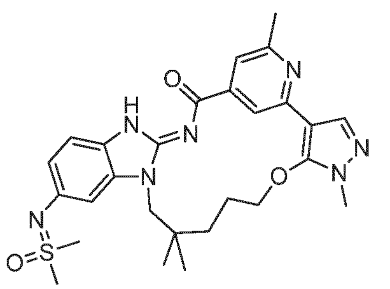
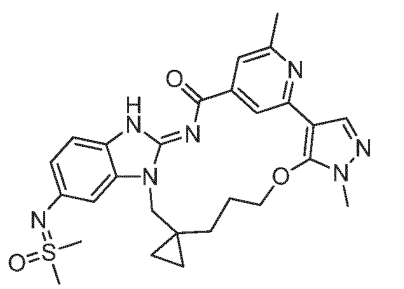
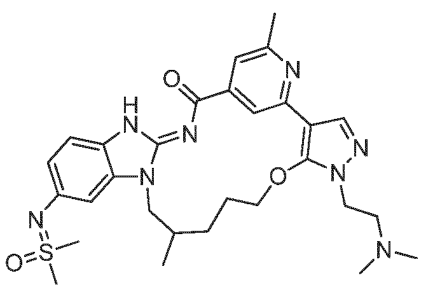

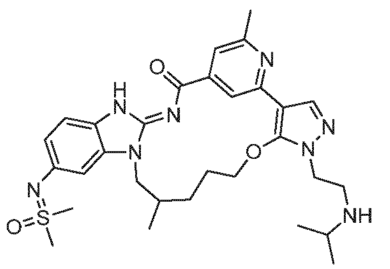
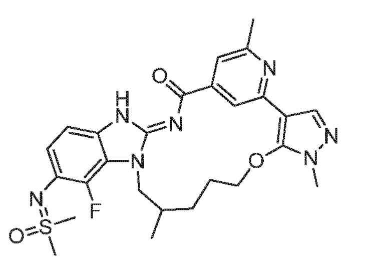
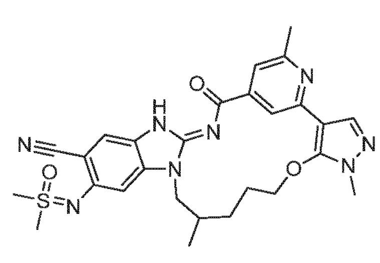

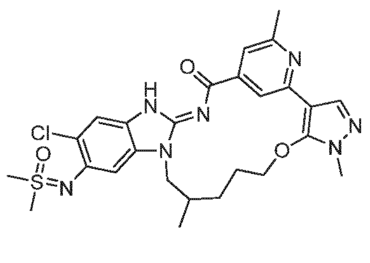
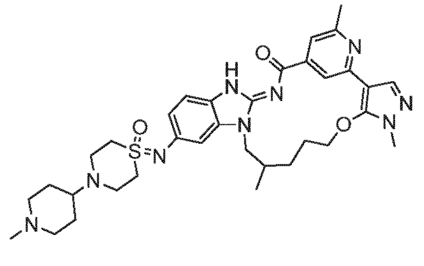
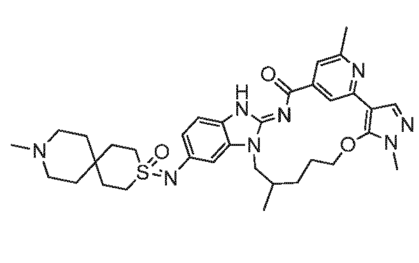

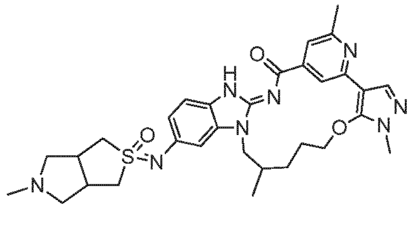
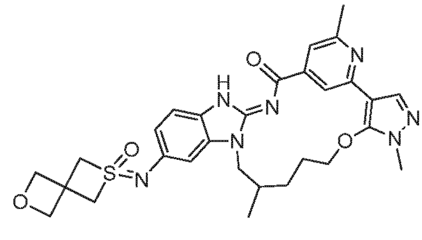
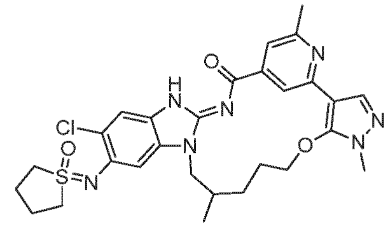

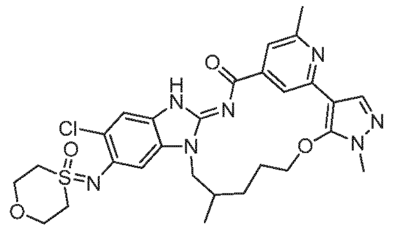

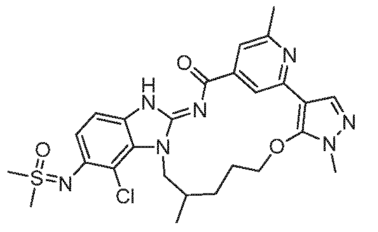

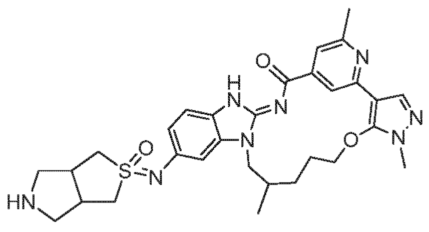

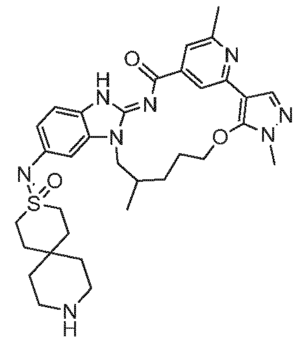

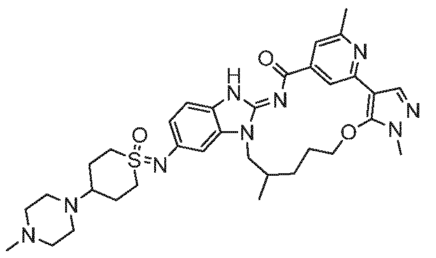

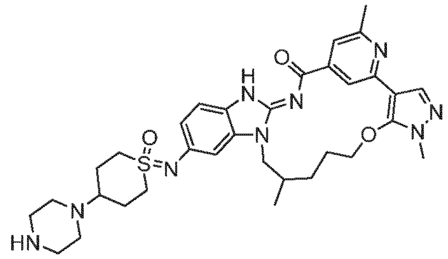

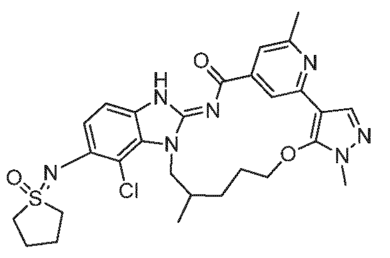

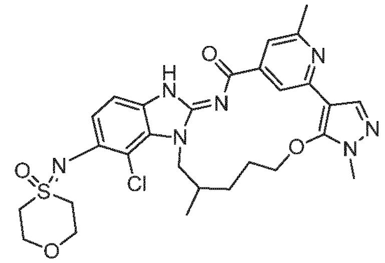

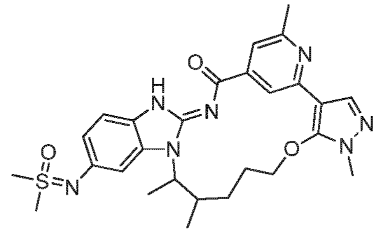

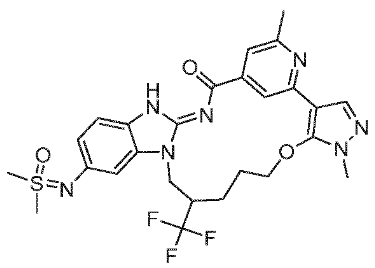

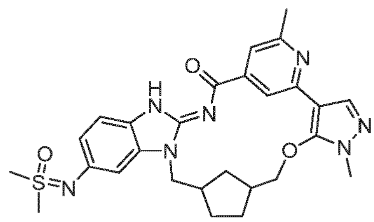

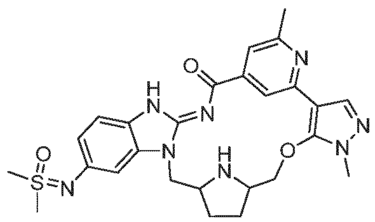

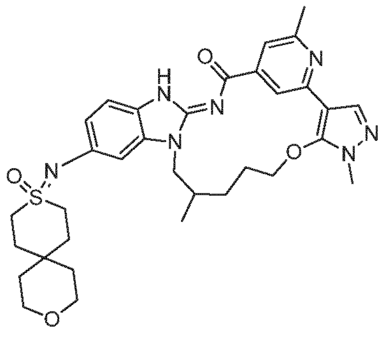

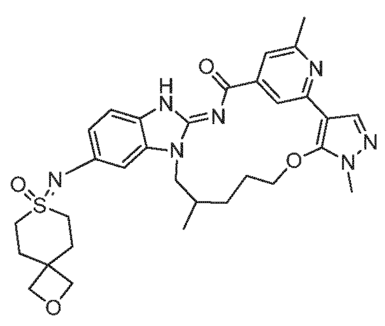

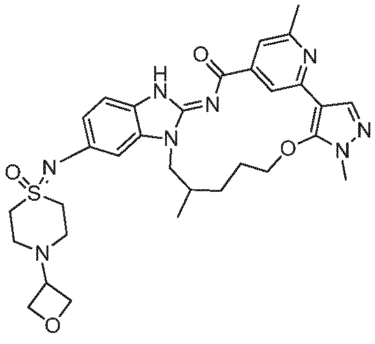

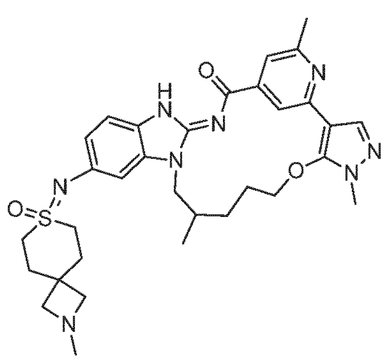
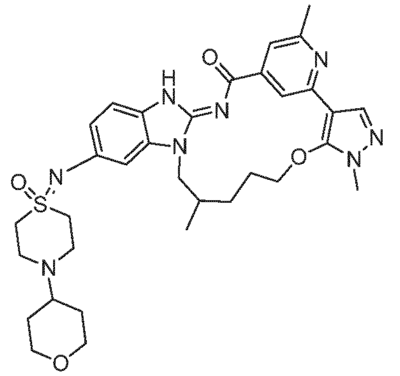
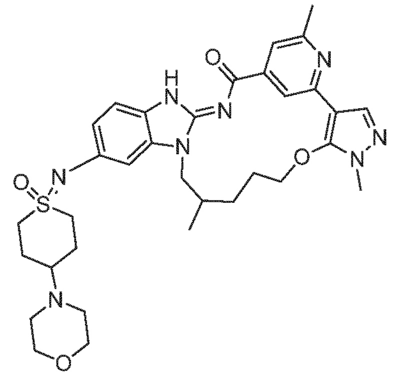

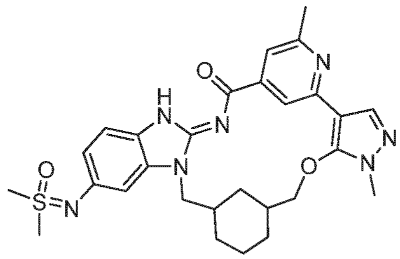
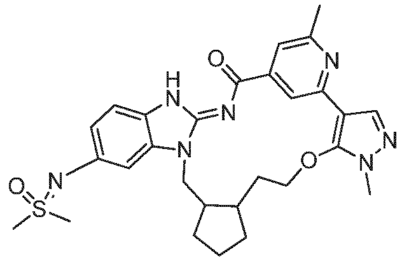
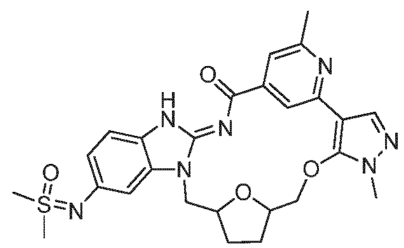

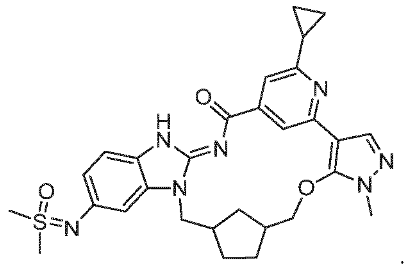

In some embodiments of the present disclosure, provided herein are compounds or pharmaceutically acceptable salts thereof, wherein the compound is selected from

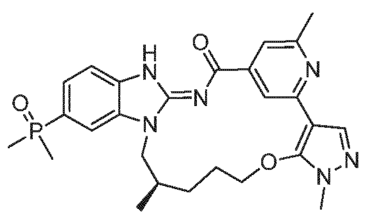
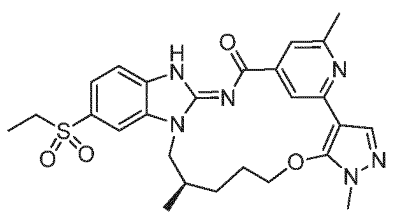
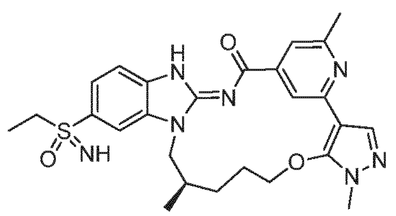

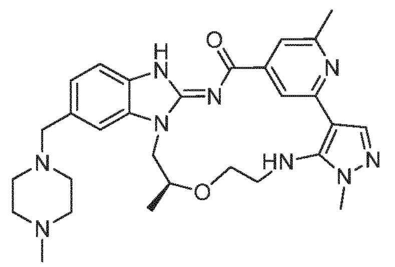
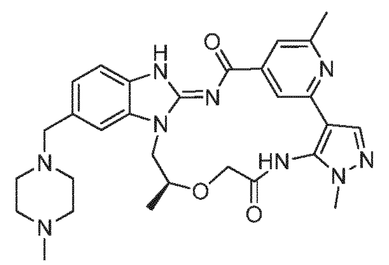
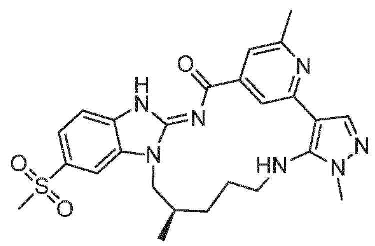

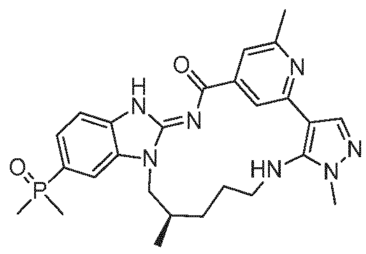
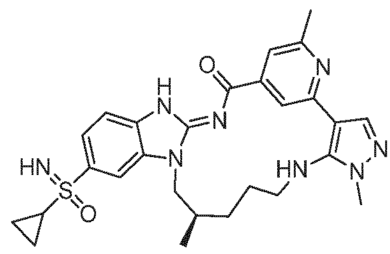
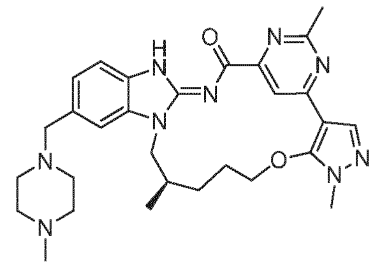

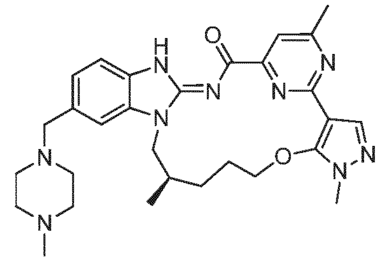
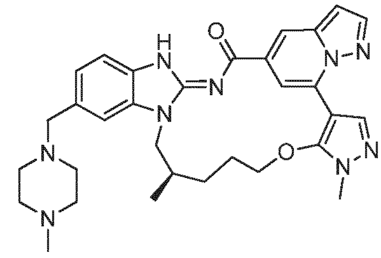
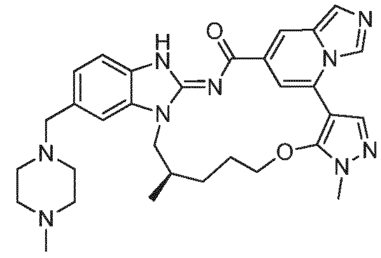

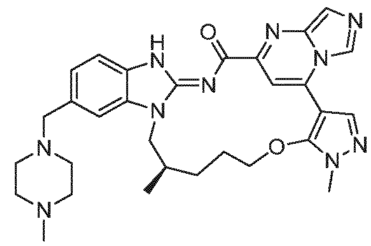
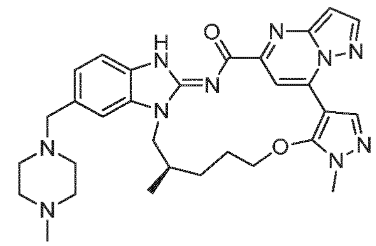
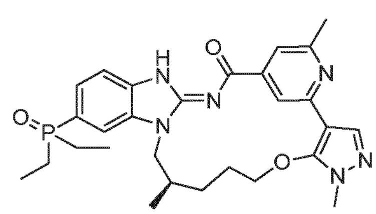

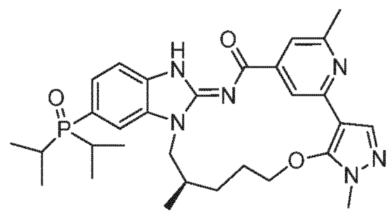
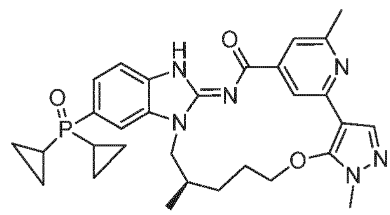
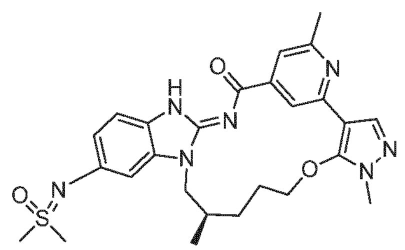

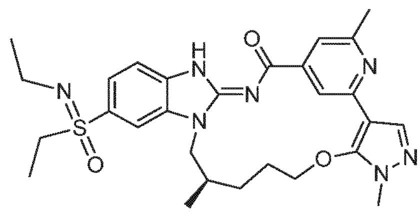
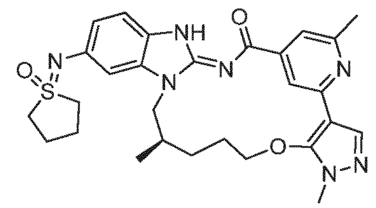
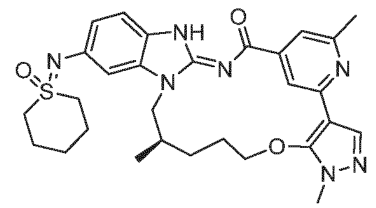

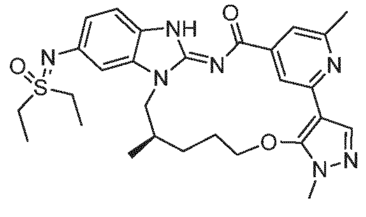
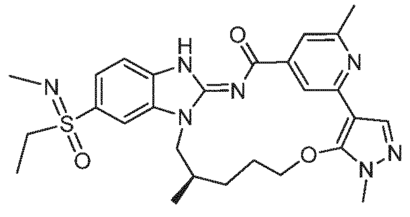
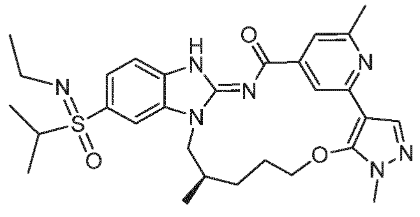

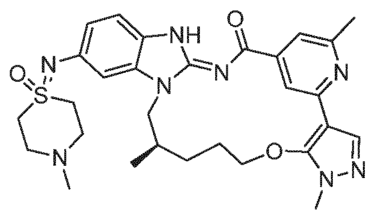
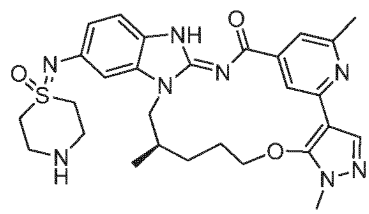
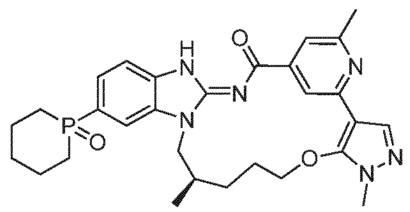

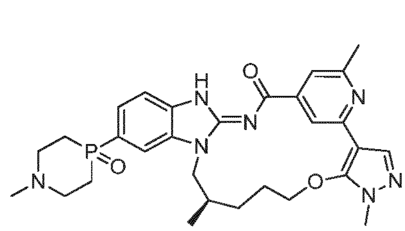
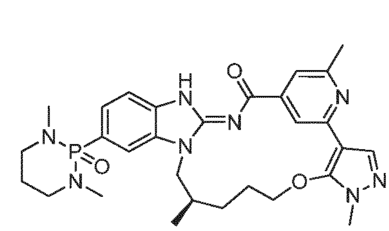
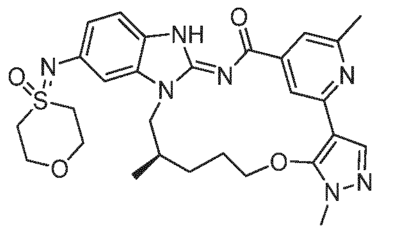

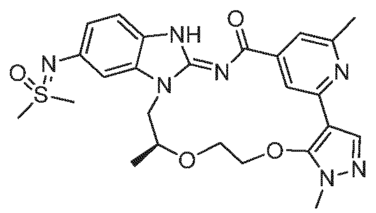
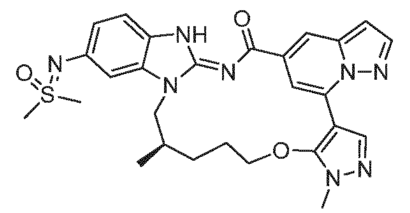
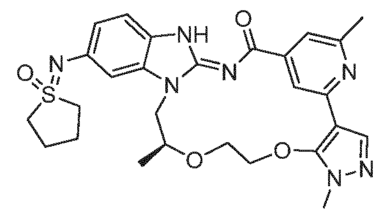

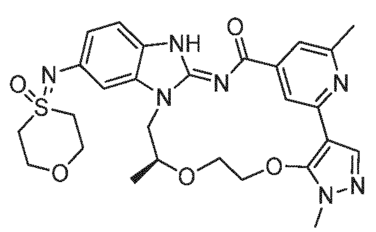
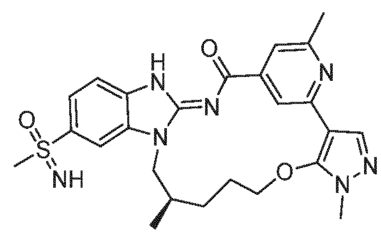
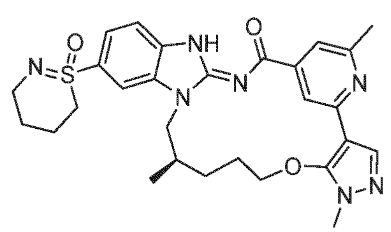

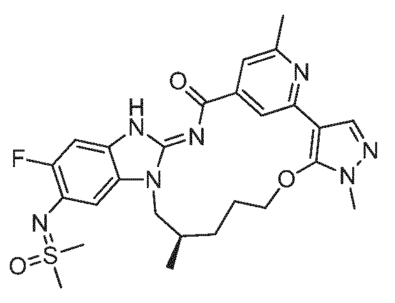
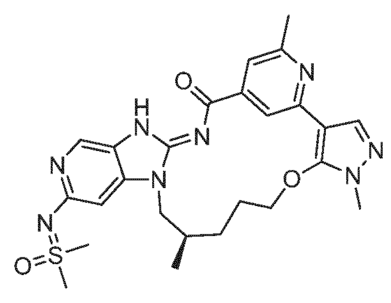
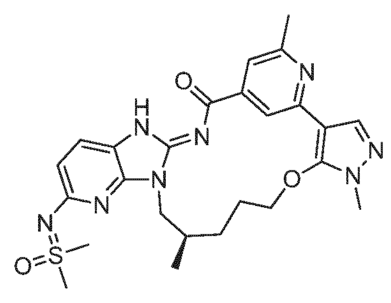

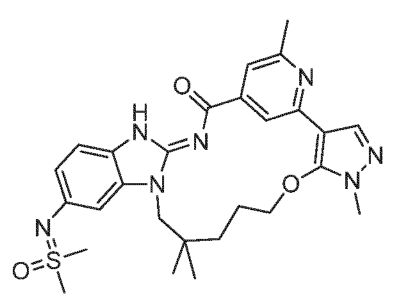
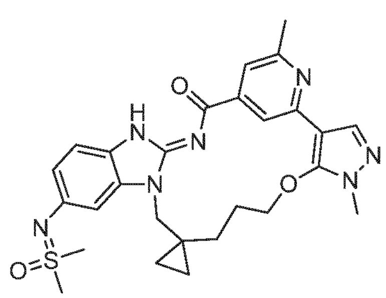

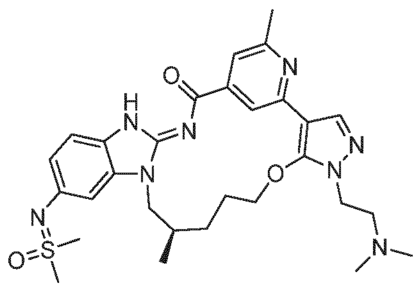

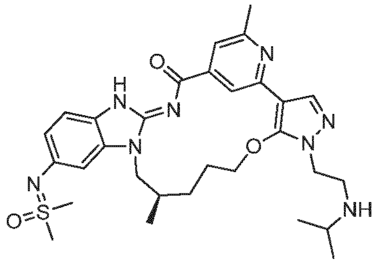

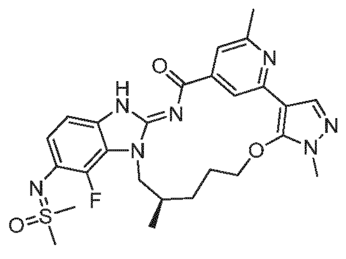

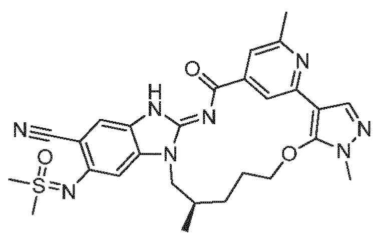

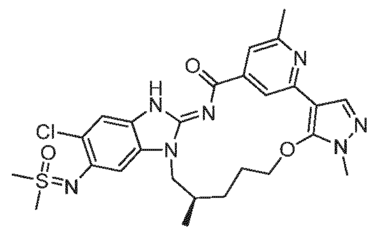

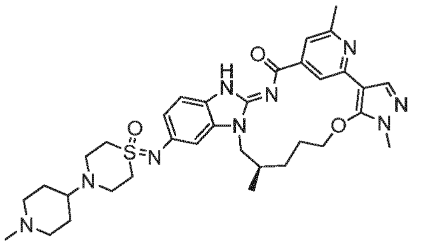

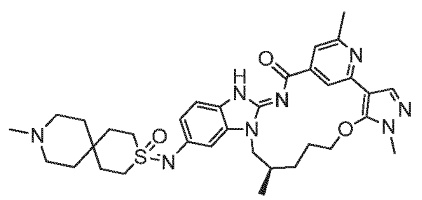

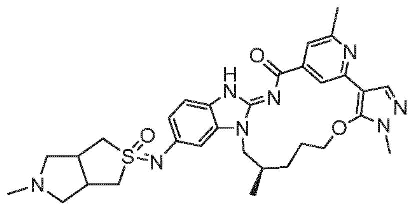

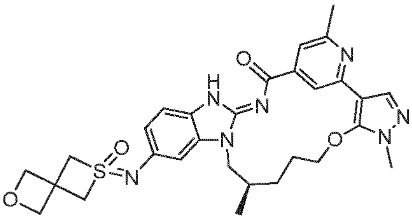

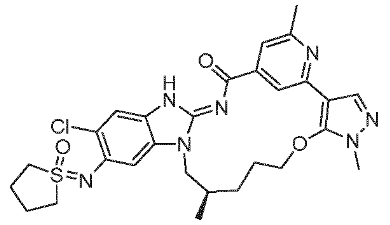

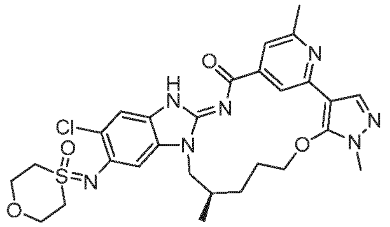

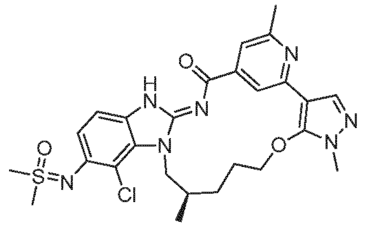

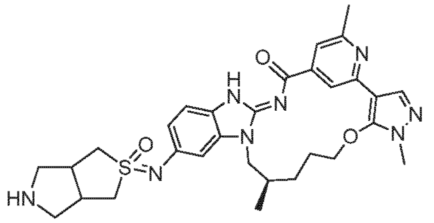

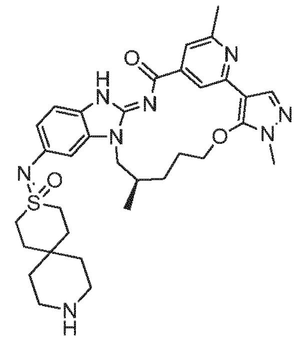
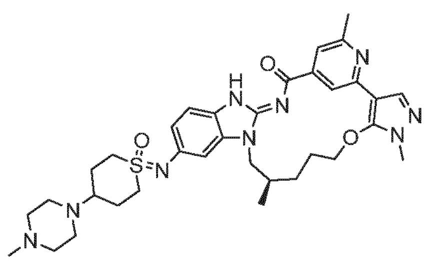
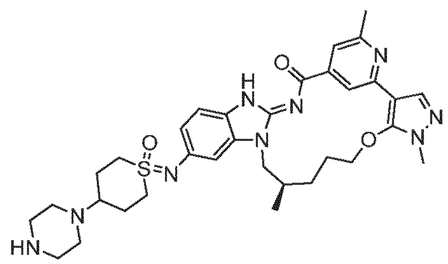

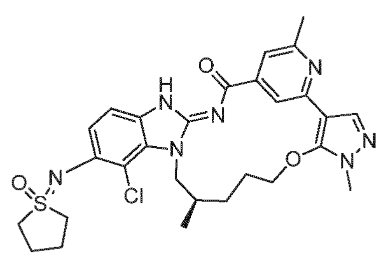
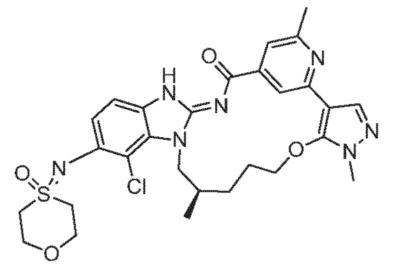
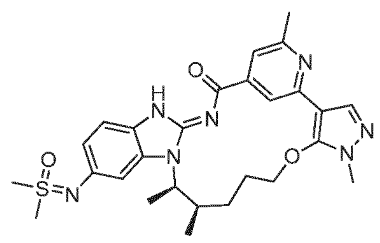

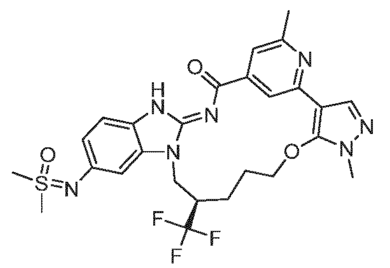
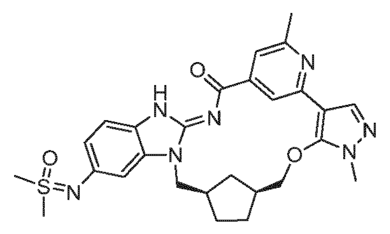
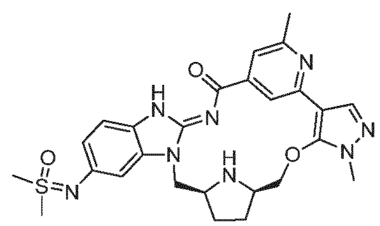

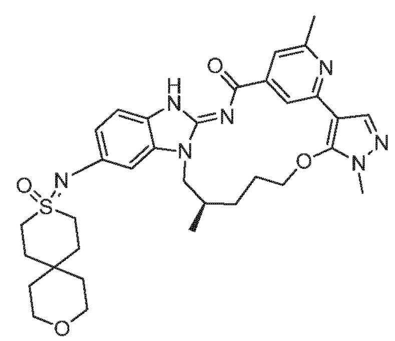
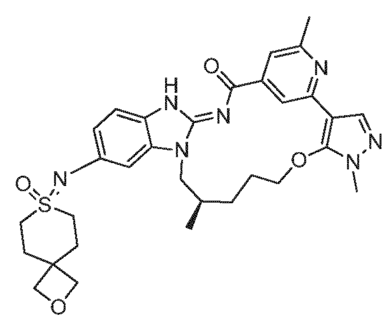
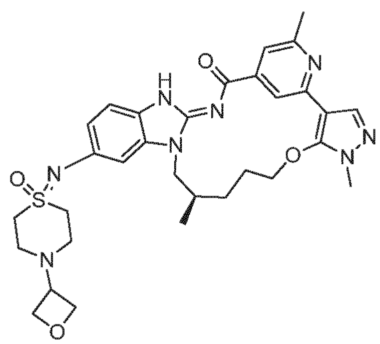

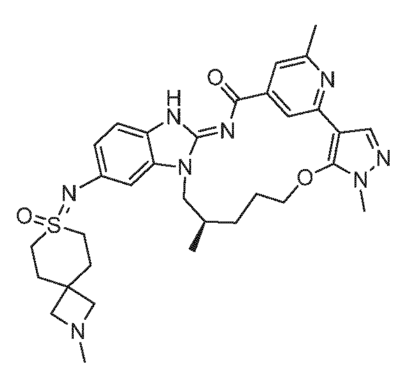
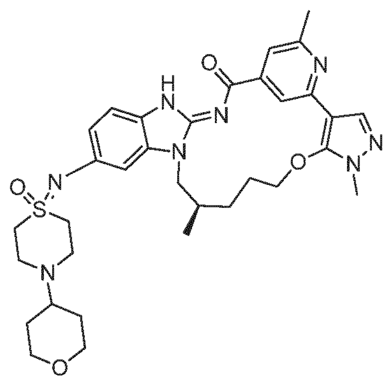
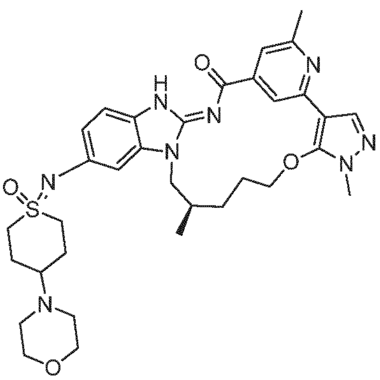

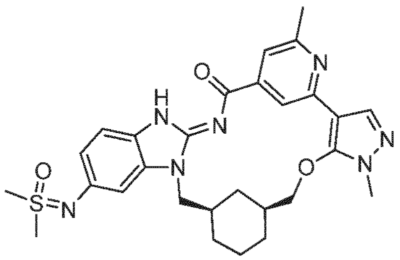
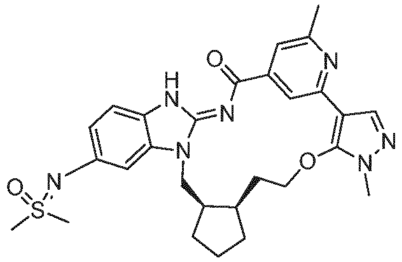
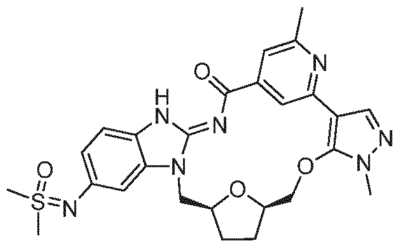

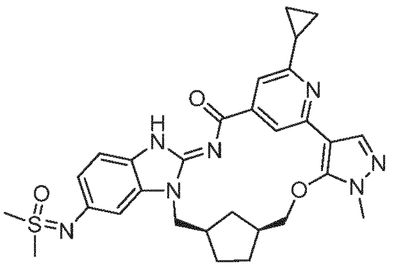
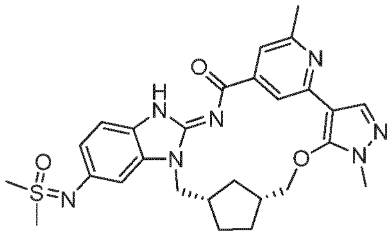

## Related Definitions

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. When compounds disclosed herein contain a relatively basic functional group, an acid addition salt can be obtained by bringing the compound into contact with a sufficient amount of acid in a pure solution or a suitable inert solvent. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (*R*)- and (*S*)-enantiomers, diastereoisomer, (*D*)-isomer, (*L*)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored relationship with each other.

Unless otherwise specified, the term "cis-trans isomer" or "geometric isomer" is produced by the inability of a double bond or a single bond between ring-forming carbon atoms to rotate freely.

Unless otherwise specified, the term "diastereomer" means a stereoisomer in which two or more chiral centers of are contained in a molecule and is in a non-mirrored relationship between molecules.

Unless otherwise specified, "(+)" means dextroisomer, "(-)" means levoisomer, and "(±)" means racemate.

Unless otherwise specified, a wedged solid bond (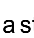) and a wedged dashed bond () indicate the absolute configuration of a stereocenter; a straight solid bond (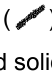) and a straight dashed bond () indicate the relative configuration of a stereocenter; a wavy line () indicates a wedged solid bond (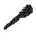) or a wedged dashed bond (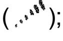); or a wavy line ()

indicates a straight solid bond (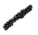) and a straight dashed bond (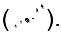).

Unless otherwise specified, the terms "tautomer" or "tautomeric form" means that different functional groups are in dynamic equilibrium at room temperature and can be rapidly converted into each other. If tautomers are possible (as in solution), a chemical equilibrium of tautomers can be achieved. For example, proton tautomers (also known as prototropic tautomers) include interconversions by proton transfer, such as keto-enol isomerization and imine-enamine isomerization. Valence tautomers include interconversions by recombination of some bonding electrons. A specific example of keto-enol tautomerization is interconversion between two tautomers pentane-2,4-dione and 4-hydroxypent-3-en-2-one.

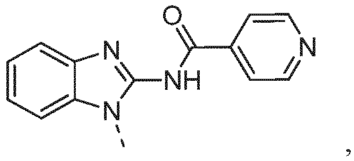,

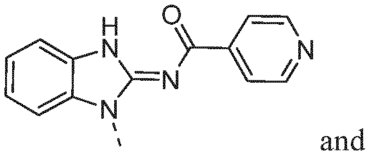 and 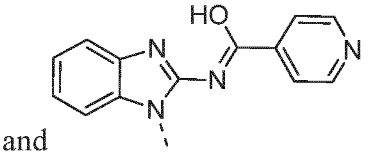

are tautomers.

Unless otherwise specified, the term "enriched in one isomer", "isomer enriched", "enriched in one enantiomer" or "enantiomeric enriched" means that the content of one isomer or enantiomer is less than 100%, and the content of the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the term "isomer excess" or "enantiomeric excess" means the difference between the relative percentages of two isomers or two enantiomers. For example, if one isomer or enantiomer is present in an amount of 90% and the other isomer or enantiomer is present in an amount of 10%, the isomer or enantiomeric excess (ee value) is 80%.

Compounds disclosed herein may contain an unnatural proportion of atomic isotopes at one or more of the atoms that make up the compounds. For example, a compound may be labeled with a radioisotope such as tritium ($^3H$), iodine-125 ($^{125}I$) or C-14($^{14}C$). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite, that the term includes the instance in which the event or condition occurs and the instance in which the event or condition does not occur.

The term "substituted" means that one or more than one hydrogen atoms on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo.

The term "optionally substituted" means an atom can be substituted by a substituent or not, unless otherwise specified, the species and number of the substituent may be arbitrary so long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of variables is a single bond, it means that the two groups linked by the single bond are connected directly. For example, when L in A-L-Z represents a single bond, the structure of A-L-Z is actually A-Z.

When an enumerated linking group does not indicate its linking direction, its linking direction is arbitrary. For

example, when the linking group L in

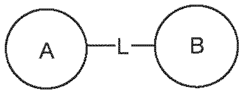

is -M-W-, the -M-W- can be linked to the ring A and the ring B in the same direction as the reading order from left to right to constitute

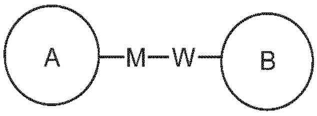,

or can be linked to the ring A and the ring B in the reverse direction as the reading order from left to right to constitute

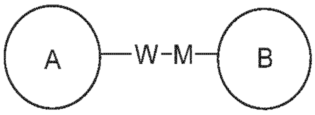.

A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. The chemical bond between the site and other groups can be represented by a straight solid bond (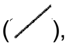), a straight dashed bond (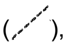), or a wavy line (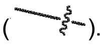). For example, the straight solid bond in $-OCH_3$ indicates that the group is connected to other groups through the oxygen atom in the group; the straight dashed bond in

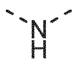

indicates that the group is connected to other groups through two ends of the nitrogen atom in the group; the wavy line in

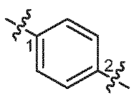

indicates that the group is connected to other groups through the 1- and 2-carbon atoms in the phenyl group;

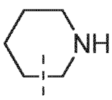

indicates that any connectable site on the piperidinyl group can be connected to other groups through one chemical bond, including at least four connection ways,

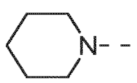, 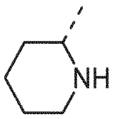, 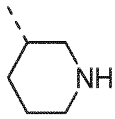

and

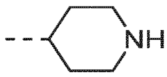;

even if a H atom is drawn on -N-,

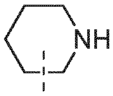

still includes the connection way of

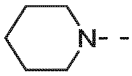;

it's just that when one chemical bond is connected, the H at this site will be reduced by one, and the group will become the corresponding monovalent piperidinyl group;

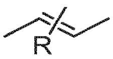

indicates that R can be connected at both ends of the double bond arbitrarily, that is,

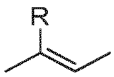

and

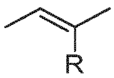.

Unless otherwise specified, the term "halo" or "halogen" by itself or as part of another substituent means a fluorine (F), chlorine (Cl), bromine (Br) or iodine (I) atom.

Unless otherwise specified, the term "$C_{1-3}$ alkyl" is used to represent a linear or branched saturated hydrocarbon group composed of 1 to 3 carbon atoms. The $C_{1-3}$ alkyl includes $C_{1-2}$ alkyl, $C_{2-3}$ alkyl, etc. It may be monovalent (such as methyl), divalent (such as methylene) or multivalent (such as methenyl). Examples of the $C_{1-3}$ alkyl include, but are not limited to, methyl (Me), ethyl (Et), propyl (including n-propyl and isopropyl), and the like.

Unless otherwise specified, the term "$C_{1-3}$ alkylamino" means alkyl groups containing 1 to 3 carbon atoms and attached to the remainder of a molecule by an amino group. The $C_{1-3}$ alkylamino group includes $C_{1-2}$, $C_3$ and $C_2$ alkylamino groups and the like. Examples of $C_{1-3}$ alkylamino groups include, but are not limited to $-NHCH_3$, $-N(CH_3)_2$, $-NHCH_2CH_3$, $-N(CH_3)CH_2CH_3$, $-NHCH_2CH_2CH_3$, $-NHCH_2(CH_3)_2$, and the like.

Unless otherwise specified, "$C_{3-5}$ cycloalkyl" refer to a saturated cyclic hydrocarbon group composed of 3 to 5 carbon atoms, which is a monocyclic ring system. The $C_{3-5}$ cycloalkyl includes $C_{3-4}$ and $C_{4-5}$ cycloalkyl and the like. It may be monovalent, divalent or multivalent. Examples of $C_{3-5}$ cycloalkyl groups include, but are not limited to, cyclopropyl, cyclobutyl, cyclopentyl, etc.

Unless otherwise specified, "$C_{5-6}$ cycloalkyl" refer to a saturated cyclic hydrocarbon group composed of 5 to 6

carbon atoms, which comprises monocyclic and bicyclic ring systems. The $C_{5-6}$ cycloalkyl includes $C_5$ and $C_6$ cycloalkyl and the like. It may be monovalent, divalent or multivalent. Examples of $C_{5-6}$ cycloalkyl groups include, but are not limited to, cyclopentyl, cyclohexyl, etc.

Unless otherwise specified, the terms "5-membered heteroaromatic ring" and "5-membered heteroaryl" may be used interchangeably. The term "5-membered heteroaryl" means a monocyclic group having a conjugated pi electron system and composed of 5 ring atoms, in which 1, 2, 3 or 4 ring atoms are heteroatoms independently selected from O, S and N, and the remainder is carbon atoms, wherein the nitrogen atom is optionally quaternized and the nitrogen and sulfur heteroatoms are optionally oxidized (i.e., NO and $S(O)_p$, p is 1 or 2). A 5-membered heteroaryl can be attached to the remainder of the molecule through a heteroatom or a carbon atom. Examples of the 5-membered heteroaryl include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like)..

Unless otherwise specified, the term "4- to 5-membered heterocycloalkyl" alone or in combination with other terms respectively represents a saturated monocyclic group composed of 4 to 5 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and $S(O)_p$, p is 1 or 2). In addition, with respect to the "4- to 5-membered heterocycloalkyl", the heteroatom may be present on the position of attachment of the heterocycloalkyl group to the remainder of a molecule. The 4- to 5-membered heterocycloalkyl includes 4 membered and 5 membered heterocycloalkyl. Examples of the 4- to 5-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), and tetrahydrofuranyl (including tetrahydrofuran-2-yl and the like), etc.

Unless otherwise specified, the term "4- to 6-membered heterocycloalkyl" alone or in combination with other terms respectively represents a saturated cyclic group composed of 4 to 6 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and $S(O)_p$, p is 1 or 2). The ring comprises monocyclic and bicyclic ring systems, wherein the bicyclic ring systems comprise spiro, fused, and bridged cyclic rings. In addition, with respect to the "4- to 6-membered heterocycloalkyl", the heteroatom may be present on the position of attachment of the heterocycloalkyl group to the remainder of a molecule. The 4- to 6-membered heterocycloalkyl includes 5 to 6 membered, 4 membered, 5 membered, and 6 membered heterocycloalkyl, etc. Examples of the 4- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, or hexahydropyridazinyl, and the like.

Unless otherwise specified, the term "5- to 6-membered heterocycloalkyl" alone or in combination with other terms respectively represents a saturated or partially unsaturated cyclic group composed of 5 to 6 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and $S(O)_p$, p is 1 or 2). The ring comprises monocyclic and bicyclic ring systems, wherein the bicyclic ring systems comprise spiro, fused, and bridged cyclic rings. In addition, with respect to the "5- to 6-membered heterocycloalkyl", the heteroatom may be present on the position of attachment of the heterocycloalkyl group to the remainder of a molecule. The 5- to 6-membered heterocycloalkyl includes 5 membered and 6 membered heterocycloalkyl. Examples of the 5- to 6-membered heterocycloalkyl include, but are not limited to, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, hexahydropyridazinyl, homopiperazinyl, or homopiperidinyl, and the like.

Unless otherwise specified, the term "5- to 6-membered monocyclic heterocycloalkyl" alone or in combination with other terms respectively represents a saturated or partially unsaturated cyclic group composed of 5 to 6 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and $S(O)_p$, p is 1 or 2), which is only a monocyclic ring.

Unless otherwise specified, the term "7- to 11-membered heterocycloalkyl" alone or in combination with other terms respectively represents a saturated cyclic group composed of 7 to 11 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and $S(O)_p$, p is 1 or 2). The ring comprises monocyclic, bicyclic and tricyclic ring systems, wherein the bicyclic and tricyclic ring systems comprise spiro, fused, and bridged cyclic rings. In addition, with respect to the "7- to 11-membered heterocycloalkyl", the heteroatom may be present on the position of attachment of the heterocycloalkyl group to the remainder of a molecule. The 7- to 11-membered heterocycloalkyl includes 7 membered, 8 membered, 9 membered, 10 membered, and 11 membered heterocycloalkyl, etc. Examples of the 7- to 11-membered heterocycloalkyl include, but are not limited to, homopiperazinyl, homopiperidinyl or dioxepanyl, and the like.

Unless otherwise specified, the term "7- to 11-membered bicyclic heterocycloalkyl" alone or in combination with other terms respectively represents a saturated cyclic group composed of 7 to 11 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and $S(O)_p$, p is 1 or 2). The bicyclic ring systems comprise spiro, fused, and bridged cyclic rings.

Unless otherwise specified, the term "5- to 11-membered heterocycloalkyl" alone or in combination with other terms respectively represents a saturated cyclic group composed of 5 to 11 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and $S(O)_p$, p is 1 or 2). The ring comprises monocyclic, bicyclic and tricyclic ring systems, wherein the bicyclic and tricyclic ring systems comprise spiro, fused, and bridged cyclic rings. In addition, with respect to the "5- to 11-membered heterocycloalkyl", the heteroatom may be present on the position of attachment of the heterocycloalkyl group to the remainder of a molecule. The 5- to 11-membered heterocycloalkyl includes 5 to 10 membered, 5 to 9 membered, 5 to 8 membered, 5 to 7 membered, 5 to 6 membered, 5 membered, 6 membered, 7 membered, 8 membered, 9 membered, 10 membered, and 11 membered heterocycloalkyl, etc. Examples of the 5- to 11-membered heterocycloalkyl include, but are not limited to, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, and the like.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$ and $C_{12}$, also includes any range from n to n+m, for example, $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$ and $C_{9-12}$, etc.; similarly, n membered to n+m membered indicates that the number of atoms on a ring is n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, and the like.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to the examples disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuK$\alpha$ radiation in a scanning mode of $\varphi/\omega$ scan; after collecting the relevant data, the crystal structure is further analyzed by the direct method (Shelxs97) to confirm the absolute configuration.

Solvents used in the present disclosure are commercially available.

Compounds are named according to general naming principles in the art or by ChemDraw® software, and commercially available compounds are named with their vendor directory names.

## Technical effect

The compounds of the present disclosure exhibit high inhibitory activity against kinases carrying triple mutations of EGFR (L858R/T790M/C797S) and EGFR (del19-E746-A750/T790M/C797S); show extremely strong cell proliferation inhibitory activity against cell lines carrying triple mutations of EGFR (L858R/T790M/C797S), EGFR(del19-E746-A750/T790M/C797S) and also show high cell proliferation inhibitory activity against cell lines carrying double mutations of EGFR(L858R/T790M), EGFR(del19-E746-A750/T790M), EGFR(del19-E746-A750/C797S) and EGFR(L858R/C797S); and have good inhibitory effects on EGFR phosphorylation of cell lines carrying triple mutations of EGFR(L858R/T790M/C797S), EGFR(del19/T790M/C797S). Moreover, the compounds have weak inhibition on EGFR (WT) kinase and cell lines, and thus have good selectivity. The compounds of the present disclosure have weak inhibitory effect on P450 isoenzyme. The compounds of the present disclosure also have excellent pharmacokinetic properties and excellent tumor inhibitory effect.

## DETAILED DESCRIPTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Reference example 1

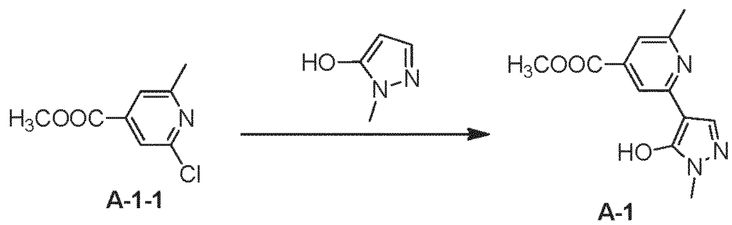

Step 1: Synthesis of compound **A-1**

**A-1-1**(2.11 g, 21.55 mmol, 2 *eq*), sodium bicarbonate (3.43 g, 32.33 mmol, 3 *eq*) and [1,1'-bis(diphenylphosphino)ferrocene]palladium dichloride dichloromethane complex (8.80 g, 10.78 mmol, 1 *eq*) were added to anisole (40 mL), and the reaction solution was stirred at 130°C for 16 hours. After the reaction was completed, the reaction solution was filtered through diatomaceous earth to obtain a filtrate, and the filter cake was washed 3 times with toluene (30 mL). The filtrates were combined to obtain a black filtrate. 20 mL of methanol was added to the black filtrate, and 4M hydrogen chloride/1,4-dioxane (30 mL) was dropwise added with stirring. After the addition was completed, the mixture was stirred at room temperature for another 1 hour, and a solid was precipitated. Then the mixture was filtered to obtain a filter cake. The filter cake was washed with toluene (60 mL) and n-heptane (60 mL) in sequence, and then rotary-evaporated to dryness under vacuum to obtain **A-1.** LCMS m/z = 248.0 $[M+H]^+$.

### Reference example 2

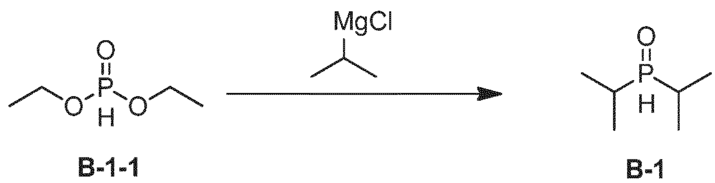

Step 1: Synthesis of compound **B-1**

**B-1-1**(1.38 g, 10.00 mmol, 1.29 mL, 1 *eq*) was dissolved in THF (30 mL), and the mixture was cooled to -70°C. A solution of isopropyl magnesium chloride in THF (2 M, 3.09 g, 30 mmol, 15 mL, 3 eq) was added, and the mixture was stirred for 2 hours, then slowly warmed to 25 °C. The mixture was stirred for 3 hours. Then the reaction mixture was cooled to 0 °C, and quenched with 0.5 M aqueous hydrochloric acid solution (40 mL). The mixture was extracted

three times with 30 mL of ethyl acetate. The organic phases were combined, washed once with saturated sodium chloride solution (20 mL), and dried over anhydrous sodium sulfate, filtered and concentrated under reduced pressure. $^1H$ NMR (400MHz, $CDCl_3$) δ = 6.27 (dt, 1H, *J* = 432, 3.0 Hz), 2.02 - 1.83 (m, 2H), 1.24 - 1.08 (m, 12H).

**Reference example 3**

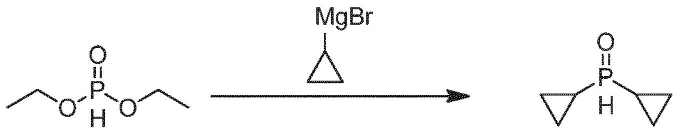

B-1-1 C-1

Step 1: Synthesis of compound **C-1**

**B-1-1** (1.38 g, 10.00 mmol, 1.29 mL, 1 *eq*) was dissolved in THF (30 mL), and the solution was cooled to -70°C. A solution of cyclopropylmagnesium bromide in THF (0.5 M, 3.09 g, 30 mmol, 60 mL, 3 eq) was added dropwise, and the mixture was stirred for 2 hours. The mixture was then slowly warmed to 25 °C and stirred for 3 hours. Then the reaction mixture was cooled to 0 °C, and quenched with 0.5 M hydrochloric acid aqueous solution (40 mL). The mixture was extracted three times with 30 mL of ethyl acetate. The organic phases were combined, washed once with saturated sodium chloride solution (20 mL), and dried over anhydrous sodium sulfate, filtered and concentrated under reduced pressure to obtain compound **C-1.** LCMS m/z = 131.2 $[M+H]^+$.

**Reference example 4**

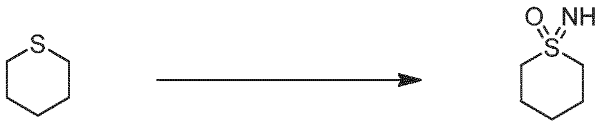

D-1-1 D-1

Step 1: Synthesis of compound **D-1**

**D-1-1** (200 mg, 1.96 mmol, 1 *eq*) was dissolved in methanol (20 mL). Iodosobenzene diacetate (1.89 g, 5.87 mmol, 3 *eq*) and ammonium acetate (603.40 mg, 7.83 mmol, 4 *eq*) were added, and the mixture was stirred at 25 °C for 4 hours. The reaction solution was concentrated in vacuo to obtain a crude product. The crude product was separated and purified by silica gel column chromatography (gradient elution: dichloromethane/methanol = 100:0~100:5) to obtain **D-1**. $^1H$ NMR (400MHz, $CDCl_3$) δ = 3.04 - 2.96 (m, 4H), 2.06 - 1.95 (m, 4H), 1.63 - 1.52 (m, 2H); LCMS m/z = 134.1 $[M+H]^+$.

**Reference example 5**

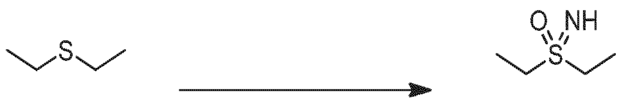

E-1-1 E-1

Step 1: Synthesis of compound **E-1**

**E-1-1** (200 mg, 2.22 mmol, 1 *eq*) was dissolved in methanol (20 mL). Iodosobenzene diacetate (2.14 g, 6.65 mmol, 3 *eq*) and ammonium acetate (683.76 mg, 8.87 mmol, 4 *eq*) were added, and the mixture was stirred at 25 °C

for 4 hours. The reaction solution was concentrated in vacuo to obtain a crude product. The crude product was separated and purified by silica gel column chromatography (gradient elution: dichloromethane/methanol = 100:0~100:5) to obtain **E-1**. $^1$H NMR (400MHz, $CDCl_3$) δ = 3.09 - 2.94 (m, 4H), 1.34 (t, J = 7.5 Hz, 6H); LCMS m/z = 122.1 $[M+H]^+$.

**Reference example 6**

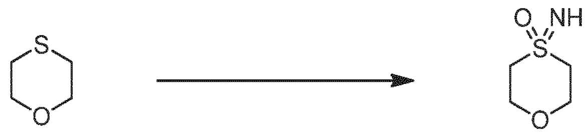

F-1-1 F-1

Step 1: Synthesis of compound **F-1**

**F-1-1** (200 mg, 1.92 mmol, 1 *eq*) was dissolved in methanol (10 mL). Iodosobenzene diacetate (1.86 g, 5.76 mmol, 3 *eq*) and ammonium acetate (591.97 mg, 7.68 mmol, 4 *eq*) were added, and the mixture was stirred at 25 °C for 4 hours. The reaction solution was concentrated in vacuo to obtain a crude product. The crude product was separated and purified by silica gel column chromatography (gradient elution: dichloromethane/methanol = 100:0~100:5) to obtain **F-1**. $^1$H NMR (400MHz, $CDCl_3$) δ = 4.05 (br s, 4H), 3.09 (br s, 4H), 1.97 (s, 1H); LCMS m/z = 136.2 $[M+H]^+$.

**Reference example 7**

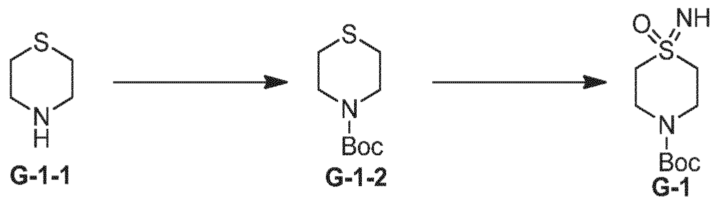

G-1-1 G-1-2 G-1

Step 1: Synthesis of compound **G-1-2**

**G-1-1** (200 mg, 1.94 mmol, 1 *eq*) was dissolved in dichloromethane (10 mL). Di-tert-butyl dicarbonate (846 mg, 3.88 mmol, 2 *eq*), triethylamine (0.81 mL, 5.81 mmol, 3 *eq*) and dimethylaminopyridine (118 mg, 0.97 mmol, 0.5 *eq*) were added, and the mixture was stirred at 25 °C for 16 hours. The reaction solution was concentrated under reduced pressure to obtain a crude product. The crude product was separated and purified by silica gel column chromatography (gradient elution: dichloromethane/methanol = 100:0-100:10) to obtain **G-1-2.** $^1$H NMR (400MHz, $CDCl_3$) δ = 3.51 - 3.72 (m, 4 H), 2.41 - 2.63 (m, 4 H), 1.39 (s, 9 H).

Step 2: Synthesis of compound **G-1**

**G-1-2** (400 mg,1.97 mmol, 1 *eq*) was dissolved in methanol (5 mL). Iodosobenzene diacetate (1.90 g, 5.90 mmol, 3 *eq*) and ammonium acetate (606.65 mg, 7.87 mmol, 4 *eq*) were added, and the mixture was stirred at 25 °C for 2 hours. The reaction solution was concentrated in vacuo to obtain a crude product. The crude product was separated and purified by silica gel column chromatography (gradient elution: dichloromethane/methanol = 100:0~100:3) to obtain **G-1.** $^1$H NMR (400MHz, $CDCl_3$) δ = 3.56 - 4.08 (m, 4 H), 2.93 - 3.09 (m, 4 H), 1.31 - 1.53 (s, 9 H).

**Reference example 8**

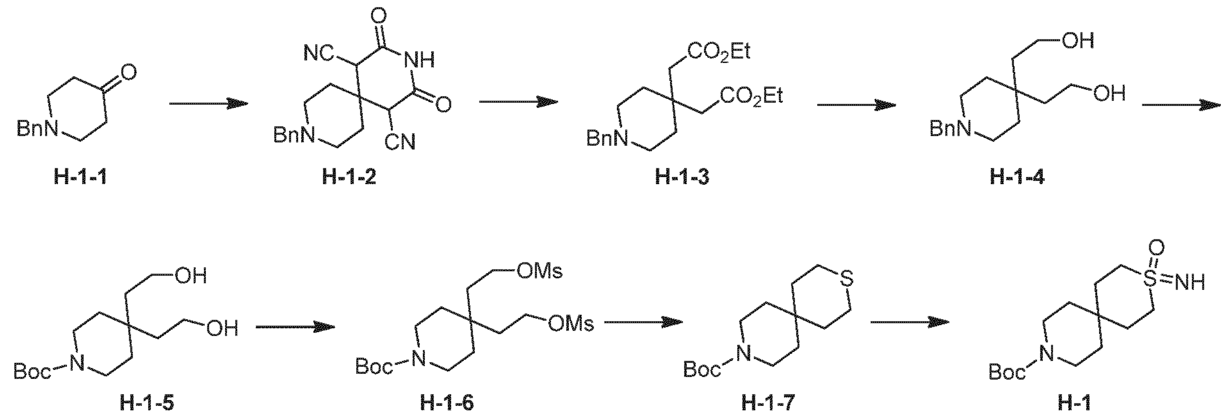

H-1-1 H-1-2 H-1-3 H-1-4

H-1-5 H-1-6 H-1-7 H-1

Step 1: Synthesis of compound **H-1-2**

Ethyl cyanoacetate (1.78 g, 15.77 mmol, 3 *eq*) was dissolved in methanol (5 mL). Ammonium acetate (40.51 mg, 525.59 μmol, 0.1 *eq*) was added, then **H-1-1** (994.70 mg, 5.26 mmol, 974.24 μL, 1 *eq*) was added dropwise, and ammonia water (0.75 mL) was added finally. The mixture was stirred at 25 °C for 24 hours. After the reaction was completed, 1 mL of concentrated hydrochloric acid was added and a solid was precipitated. The mixture was filtered by suction, and the filter cake was washed with ethyl acetate to obtain **H-1-2.** LCMS m/z = 323.1 $[M+1]^+$.

Step 2: Synthesis of compound **H-1-3**

Sulfuric acid (60 mL) was mixed with water (40 mL). **H-1-2** (10 g, 31.02 mmol, 1 *eq*) was added in batches, and the mixture was gradually heated to 130°C and stirred for 24 hours. After the reaction was completed, the mixture was concentrated under pressure, then the residue was dissolved in ethanol (110 mL). The mixture was reacted at 120 °C for 20 hours. After the reaction was completed again, the mixture was cooled. Then, sodium bicarbonate (40 g) was added under an ice bath, followed by water (100 mL) and the mixture was extracted with dichloromethane (200 mL). The organic phase was dried over anhydrous sodium sulfate, filtered and concentrated to obtain **H-1-3.** LCMS m/z = 348.3 $[M+1]^+$.

Step 3: Synthesis of compound **H-1-4**

Compound **H-1-3** (3.1 g, 8.92 mmol, 1 *eq*) was dissolved in tetrahydrofuran (3 mL). Lithium aluminum hydride (677.27 mg, 17.84 mmol, 2 *eq*) was added in batches at 0 °C under nitrogen, and the mixture was stirred at 25 °C overnight. The reaction mixture was quenched by slowly adding sodium sulfate decahydrate with stirring. The mixture was filtered, washed with tetrahydrofuran, and concentrated to obtain compound **H-1-4.** LCMS m/z = 264.2 $[M+1]^+$.

Step 4: Synthesis of compound **H-1-5**

Compound **H-1-4** (3.98 g, 15.13 mmol, 1 *eq*) was dissolved in methanol (70 mL). Di-tert-butyl dicarbonate (3.30 g, 15.13 mmol, 3.48 mL, 1 *eq*) and palladium hydroxide (640 mg, 4.56 mmol, 0.301 *eq*) were added, and the mixture was stirred at 40 °C for 12 hours under hydrogen. The reaction solution was then filtered through diatomaceous earth, and the filtrate was concentrated under reduced pressure to obtain compound **H-1-5.** $^1H$ NMR (400MHz, $CDCl_3$) δ = 3.77 (t, J = 6.7 Hz, 4H), 3.50 (s, 2H), 3.44 - 3.38 (m, 4H), 1.72 (t, J = 6.7 Hz, 4H), 1.49 - 1.42 (m, 13H).

Step 5: Synthesis of compound **H-1-6**

Compound **H-1-5**(1.76 g, 6.45 mmol, 1 *eq*) was dissolved in dichloromethane (25 mL), and the solution was cooled to -20°C. Methylsulfonyl chloride (2.43 g, 21.21 mmol, 3.29 *eq*) and triethylamine (2.61 g, 25.82 mmol, 4 *eq*) were added, and the mixture was stirred for 30 minutes. The reaction solution was quenched by slowly adding 30 mL of water, and the mixture was extracted with 40 mL of dichloromethane. The organic phase was collected, dried, and concentrated. The crude product was separated and purified by silica gel column chromatography to obtain compound **H-1-6.** $^1H$ NMR (400MHz, $CDCl_3$) δ = 4.25 (t, J = 6.9 Hz, 4H), 3.38 - 3.32 (m, 4H), 2.97 (s, 6H), 1.82 (t, J = 7.0 Hz, 4H), 1.41 (br d, J = 5.8 Hz, 4H), 1.38 (s, 9H).

Step 6: Synthesis of compound **H-1-7**

Compound **H-1-6** (0.5 g, 1.16 mmol, 1 *eq*) was dissolved in *N,N*-dimethylformamide (4 mL), and sodium sulfide nonahydrate (335.49 mg, 1.40 mmol 1.2 *eq*) was added. The mixture was stirred at 50 °C for 12 hours, and extracted with 20 mL of ethyl acetate and 20 mL of water. The organic phase was collected, dried and concentrated. The crude product was separated and purified by silica gel column chromatography to obtain compound **H-1-7.** $^1$H NMR (400MHz, $CDCl_3$) $\delta$ = 3.44 - 3.36 (m, 4H), 2.66 - 2.59 (m, 4H), 1.78 - 1.72 (m, 4H), 1.49 - 1.42 (m, 13H).

Step 7: Synthesis of compound **H-1**

**H-1-7** (0.25 g, 921.09 μmol, 1 *eq*) was dissolved in methanol (5 mL). Iodosobenzene diacetate (890.04 mg, 2.76 mmol, 3 *eq*) and ammonium acetate (284.00 mg, 3.68 mmol, 4 *eq*) were added, and the mixture was stirred at 25 °C for 2 hours. The reaction solution was concentrated in vacuo to obtain a crude product. The crude product was separated and purified by silica gel column chromatography, to obtain compound **H-1.** $^1$H NMR (400MHz, $CDCl_3$) $\delta$ = 3.42 (br d, J = 4.1 Hz, 4H), 3.12 - 3.03 (m, 4H), 2.01 (br t, J = 5.9 Hz, 4H), 1.55 - 1.51 (m, 4H), 1.47 (s, 9H).

**Reference example 9**

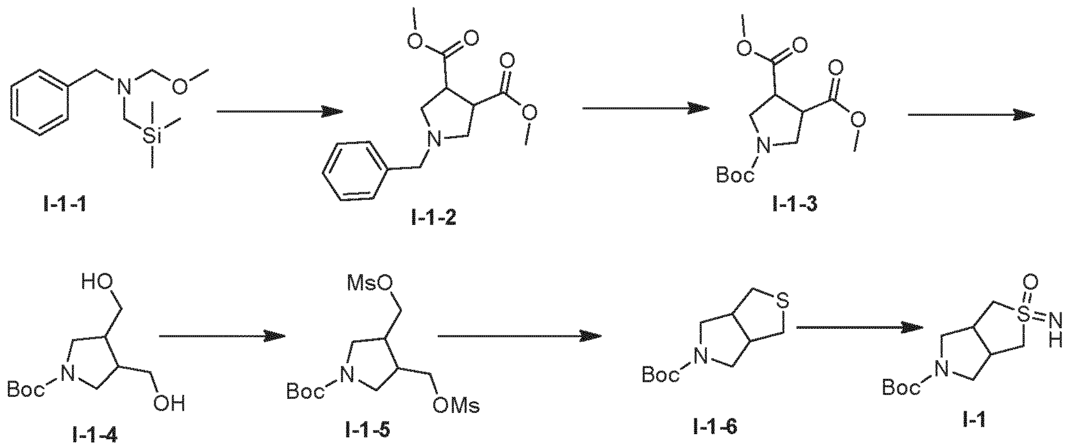

I-1-1 I-1-2 I-1-3

I-1-4 I-1-5 I-1-6 I-1

Step 1: Synthesis of compound **I-1-2**

Compound **I-1-1** (5.93 g, 24.98 mmol, 1.2 *eq*) and dimethyl maleate (3 g, 20.82 mmol, 1 *eq*) were dissolved in dichloromethane (20 mL). Trifluoroacetic acid (237.34 mg, 2.08 mmol, 0.1 *eq*) was added, and the mixture was stirred at 25 °C for 12 hours. 20 mL of water was added, and the mixture was extracted with 50 mL of dichloromethane. The organic phase was washed with saturated brine, dried over anhydrous sodium sulfate, filtered and concentrated. The crude product was separated and purified by silica gel column chromatography to obtain **I-1-2.** $^1$H NMR (400MHz, $CDCl_3$) $\delta$ = 7.31 - 7.16 (m, 5H), 3.67 - 3.56 (m, 8H), 3.29 - 3.21 (m, 2H), 3.13 - 3.03 (m, 2H), 2.72 - 2.61 (m, 2H).

Step 2: Synthesis of compound **I-1-3**

**I-1-2** was dissolved in methanol (100 mL). Di-tert-butyl dicarbonate (4.72 g, 21.64 mmol, 2 *eq*) and palladium hydroxide (1.52 g, 2.16 mmol, 0.2 *eq*) were added, and the mixture was stirred at 25 °C under hydrogen for 48 hours. The reaction solution was filtered through diatomaceous earth and concentrated to obtain **I-1-3.** $^1$H NMR (400MHz, $CDCl_3$) $\delta$ = 3.73 - 3.62 (m, 8H), 3.62 - 3.54 (m, 2H), 3.29 - 3.15 (m, 2H), 1.46 - 1.36 (m, 9H).

Step 3: Synthesis of compound **I-1-4**

**I-1-3** (1.6 g, 5.57 mmol, 1 *eq*) was dissolved in tetrahydrofuran (20 mL). Lithium aluminum hydride (634.02 mg, 16.71 mmol, 3 *eq*) was added at -5 °C, and the mixture was stirred at -5 °C for 2 hours. Subsequently, the mixture was quenched by slowly adding sodium sulfate decahydrate. The mixture was stirred for 10 minutes, filtered, and concentrated to obtain **I-1-4**. $^1$H NMR (400MHz, $CDCl_3$) $\delta$ = 4.19 - 3.86 (m, 2H), 3.70 - 3.55 (m, 4H), 3.45 - 3.29 (m, 2H), 3.18 - 2.97

(m, 2H), 2.44 (br d, J = 3.6 Hz, 2H), 1.37 (s, 9H).

Step 4: Synthesis of compound **I-1-5**

Compound **I-1-4** (580 mg, 2.51 mmol, 1 *eq*) was dissolved in dichloromethane (12 mL). The mixture was cooled to 0°C. Triethylamine (1.27 g, 12.54 mmol, 5 *eq*), methylsulfonyl chloride (890 mg, 7.77 mmol, 3.10 eq) were added, and the mixture was stirred at 0 °C for 4 hours. The raw materials were completely consumed by monitoring, and stirring was stopped. 10 mL of water and 15 mL of dichloromethane were slowly added to the reaction solution for liquid separation and extraction. The organic phase was collected, dried over anhydrous sodium sulfate, filtered and concentrated. The crude product was separated and purified by silica gel column chromatography to obtain compound **I-1-5.** $^1H$ NMR (400MHz, $CDCl_3$) $\delta$ = 4.25 (br dd, J = 5.8, 12.8 Hz, 2H), 4.21 - 4.15 (m, 2H), 3.54 - 3.46 (m, 2H), 3.30 - 3.22 (m, 2H), 2.99 (s, 6H), 2.76 - 2.68 (m, 2H), 1.40 (s, 9H).

Step 5: Synthesis of compound **I-1-6**

Compound **I-1-5** (500 mg, 1.29 mmol, 1 *eq*) was dissolved in *N,N*-dimethylformamide (5 mL). Sodium sulfide nonahydrate (371.92 mg, 1.55 mmol,1.2 eq) was added, and the mixture was stirred at 50 °C under nitrogen for 16 hours. The reaction solution was extracted with 15 mL of water and 30 mL of ethyl acetate. The organic phase was washed once with saturated sodium chloride solution and dried over anhydrous sodium sulfate. The crude product was separated and purified by silica gel column chromatography to obtain compound **I-1-6.** $^1H$ NMR (400MHz, $CDCl_3$) $\delta$ = 3.56 (br d, J = 6.9 Hz, 2H), 3.32 - 3.11 (m, 2H), 3.07 - 2.98 (m, 2H), 2.98 - 2.95 (m, 2H), 2.71 (br dd, J = 3.8, 10.8 Hz, 2H), 1.43 (s, 9H).

Step 6: Synthesis of compound **I-1**

Compound **I-1-6** (300 mg, 1.31 mmol, 1 *eq*), ammonium acetate (403.33 mg, 5.23 mmol, 4 *eq*), and iodosobenzene diacetate (1.26 g, 3.92 mmol, 3 *eq*) were dissolved in ethanol (6 mL), and the mixture was stirred at 25 °C for 2 hours. The raw materials were completely consumed, and the reaction solution was concentrated. The crude product was separated and purified by silica gel column chromatography to obtain compound **I-1.** $^1H$ NMR (400MHz, $CDCl_3$) $\delta$ = 3.77 - 3.70 (m, 2H), 3.50 - 3.38 (m, 4H), 3.20 - 3.10 (m, 4H), 1.49 (s, 9H).

**Reference example 10**

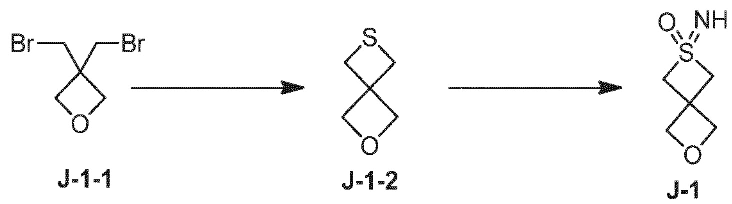

J-1-1 J-1-2 J-1

Step 1: Synthesis of compound **J-1-2**

**J-1-1** was dissolved in *N,N*-dimethylformamide (25 mL). Sodium sulfide nonahydrate (1.62 g, 6.76 mmol, 1.1 *eq*) was added in batches, and the mixture was reacted at 50 °C for 7 hours. The mixture was extracted with 50 mL of water and 100 mL of ethyl acetate, washed with saturated sodium chloride solution, dried over anhydrous sodium sulfate, filtered and concentrated. The residue was subjected to silica gel column chromatography to obtain compound **J-1-2**. $^1H$ NMR (400MHz, $CDCl_3$) $\delta$ = 4.81 - 4.62 (m, 4H), 3.50 - 3.27 (m, 4H).

Step 2: Synthesis of compound **J-1**

**J-1-2** (0.2 g, 1.72 mmol, 1 *eq*), ammonium acetate (530.77 mg, 6.89 mmol, 4 *eq*), iodosobenzene diacetate (1.66 g, 5.16 mmol, 3 *eq*) were dissolved in methanol (5 mL), and the mixture was stirred at 25 °C for 2 hours. The reaction solution was concentrated and subjected to silica gel column chromatography to obtain compound **J-1.** $^1H$ NMR (400MHz, $CDCl_3$) $\delta$ = 4.24 (s, 4H), 3.62 (d, J = 3.0 Hz, 4H).

**Reference example 11**

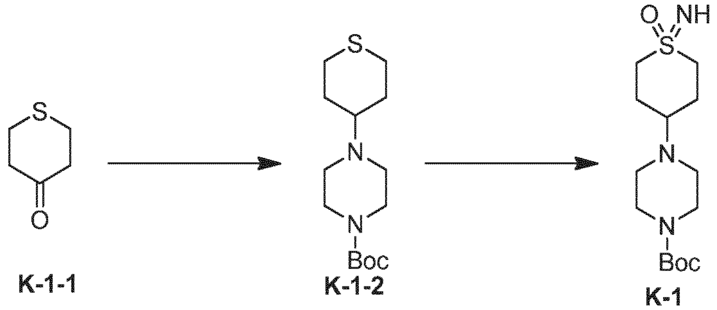

**K-1-1** **K-1-2** **K-1**

Step 1: Synthesis of compound **K-1-2**

1-Tert-butoxycarbonylpiperazine (961.86 mg, 5.16 mmol, 1.2 *eq*), **K-1-1**(500 mg, 4.30 mmol, 1 *eq*) were dissolved in ethanol (9 mL). Acetic acid (0.3 mL) and sodium triacetoxyborohydride (1.82 g, 8.61 mmol, 2 *eq*) were added, and the mixture was stirred at 25 °C for 16 hours. Then the mixture was quenched with 10 mL of water, extracted with 50 mL of ethyl acetate, washed with saturated sodium chloride solution, dried over anhydrous sodium sulfate, filtered, and concentrated. The crude product was separated by silica gel column chromatography to obtain compound **K-1-2.** $^1$H NMR (400MHz, $CDCl_3$) $\delta$ = 3.50 - 3.35 (m, 4H), 2.75 - 2.67 (m, 4H), 2.58 - 2.47 (m, 4H), 2.43 - 2.32 (m, 1H), 2.12 (br dd, J = 2.9, 12.7 Hz, 2H), 1.78 - 1.64 (m, 2H), 1.51 - 1.41 (m, 9H).

Step 2: Synthesis of compound **K-1**

**K-1-2** (90 mg, 314.21 $\mu$mol, 1 *eq*) was dissolved in methanol (2 mL). Ammonium acetate (96.88 mg, 1.26 mmol, 4 *eq*) and iodosobenzene diacetate (303.62 mg, 942.63 $\mu$mol, 3 *eq*) were added, and the mixture was stirred at 25 °C for 2 hours. The reaction solution was directly concentrated and separated by silica gel column chromatography to obtain compound **K-1**. $^1$H NMR (400MHz, $CDCl_3$) $\delta$ = 3.40 - 3.32 (m, 4H), 3.20 (ddd, J = 3.0, 7.3, 11.0 Hz, 2H), 3.02 - 2.90 (m, 2H), 2.54 - 2.47 (m, 1H), 2.45 - 2.37 (m, 4H), 2.23 - 2.01 (m, 4H), 1.42 - 1.36 (m, 9H).

**Reference example 12**

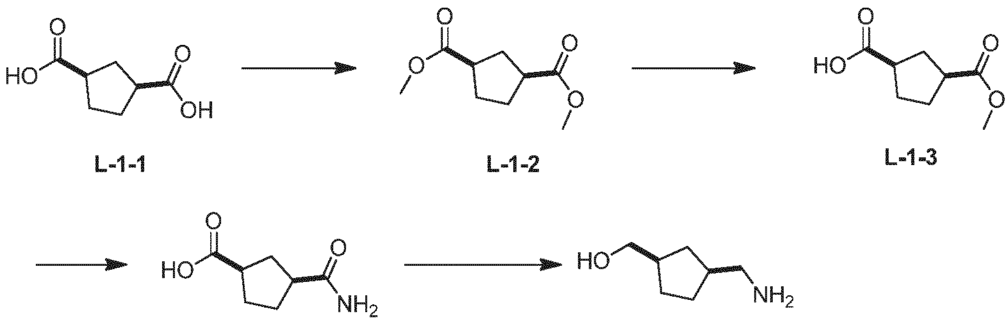

**L-1-1** **L-1-2** **L-1-3**

**L-1-4** L-1

Step 1: Synthesis of compound **L-1-2**

**L-1-1**(21.00 g, 132.78 mmol, 1 *eq*) was dissolved in methanol (200 mL). Concentrated sulfuric acid (13.02 g, 132.78 mmol, 7.08 mL, 1 *eq*) was added, and the mixture was stirred at 80 °C for 16 hours. The reaction solution was concentrated, and the residue was dissolved in 200 mL of ethyl acetate. 200 mL of water was added. The organic phase was washed with 100 mL of saturated sodium chloride aqueous solution, dried over anhydrous sodium sulfate, and filtered. The filtrate was concentrated to obtain compound **L-1-2.** LCMS *m*/*z*: 187.1 [M+H]$^+$.

Step 2: Synthesis of compound **L-1-3**

**L-1-2** (24.67 g, 132.49 mmol, 1 *eq*) and sodium hydroxide (5.30 g, 132.49 mmol, 1 *eq*) were added to MeOH (200 mL), and the mixture was reacted at 25 °C for 12 hours. The reaction solution was filtered. The filtrate was concentrated, and the residue was dissolved in 200 mL of water. The solution was adjusted to pH of 2, and extracted with 200 mL of ethyl acetate. The organic phase was washed with 100 mL of aqueous saturated sodium chloride solution, dried over anhydrous sodium sulfate, and filtered. The filtrate was concentrated to obtain **L-1-3.** $^1$H NMR (400MHz, DMSO-$d_6$) $\delta$ = 12.12 (br s, 1H), 3.59 (s, 3H), 2.94 - 2.62 (m, 2H), 2.03 - 1.64 (m, 6H). LCMS *m/z*: 173.1 [M+H]$^+$.

Step 3: Synthesis of compound **L-1-4**

**L-1-3** was dissolved in ammonia water (136.50 g, 1.09 mol, 9.48 *eq*), and the mixture was stirred at 25 °C for 16 hours. The reaction solution was directly concentrated to obtain **L-1-4.** $^1$H NMR (400MHz, DMSO-$d_6$) $\delta$ = 7.53 (br s, 1H), 6.68 (br s, 1H), 2.66 - 2.55 (m, 1H), 1.98 - 1.83 (m, 1H), 1.83 - 1.49 (m, 6H). LCMS m/z = 158.1 [M+1]$^+$.

Step 5: Synthesis of compound **L-1-5**

Compound **L-1-4** (19 g, 120.89 mmol, 1 *eq*) was dissolved in tetrahydrofuran (100 mL), and the mixture was cooled to 0 °C. Borane dimethyl sulfide (10 M, 54.40 mL, 4.5 *eq*) was added dropwise under nitrogen, the internal temperature was controlled to be less than 20 °C. After the addition, the mixture was slowly warmed to room temperature 20°C, and stirred for 2 hours. The mixture was then heated to 80°C and reacted for 16 hours. The reaction mixture was cooled to 0 °C, and quenched by slowly adding dropwise a solution of methanol (50 mL) in tetrahydrofuran (100 mL) into the reaction solution. The internal temperature was detected, and kept below 25 °C. The mixture was stirred for 2 hours, and then directly concentrated to obtain **L-1.** LCMS m/z = 130.2 [M+1]$^+$

**Reference example 13**

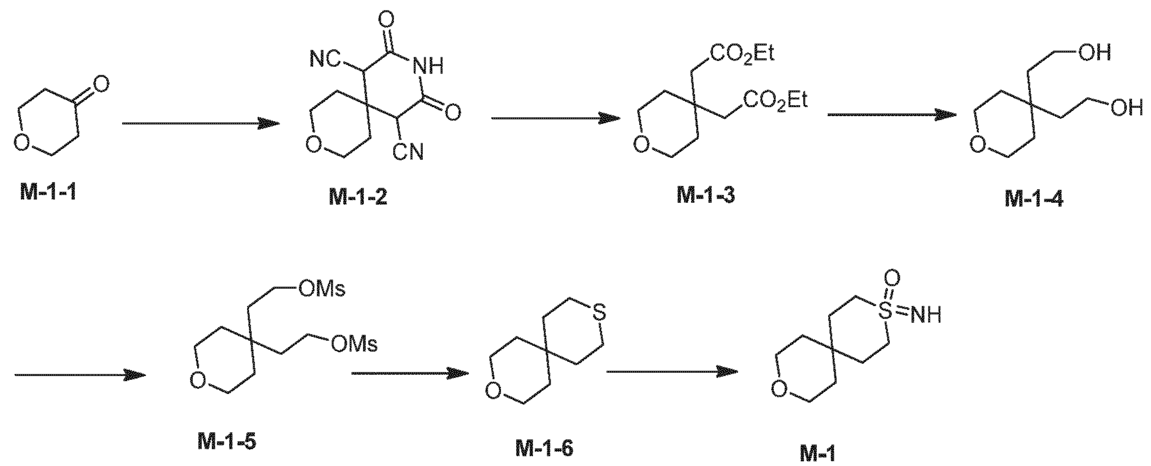

M-1-1 M-1-2 M-1-3 M-1-4

M-1-5 M-1-6 M-1

Step 1: Synthesis of compound **M-1-2**

Ethyl cyanoacetate (33.90 g, 299.65 mmol, 31.98 mL, 3 *eq*) was dissolved in methanol (40 mL). Ammonia water (15.38 mL) was added, then compound **M-1-1** (10 g, 99.88 mmol 1 *eq*) was added, and ammonium acetate (769.94 mg, 9.99 mmol, 0.1 eq) was added. The mixture was stirred at 25 °C for 16 hours. After the reaction was completed, 15 mL of concentrated hydrochloric acid was added, and a solid was precipitated. The mixture was filtered by suction, and the filter cake was washed with ethanol to obtain compound **M-1-2.** $^1$H NMR (400MHz, DMSO-$d_6$) $\delta$ = 12.28 (s, 1H), 5.09 (s, 2H), 3.75 - 3.60 (m, 4H), 1.86 - 1.63 (m, 4H).

Step 2: Synthesis of compound **M-1-3**

**M-1-2** (14 g, 60.03 mmol, 1 *eq*) was added to a mixed solution of sulfuric acid (60 mL) and water (40 mL), and the mixture was heated to 130°C and stirred for 16 hours. After the reaction was completed, the mixture was concentrated

under reduced pressure. Then ethanol (60 mL) was added, and the mixture was reacted at 120 °C for 12 hours. After the reaction was completed, the mixture was cooled. Then sodium bicarbonate (50 g) was added in an ice bath. The mixture was extracted with ethyl acetate (300 mL), and washed once with saturated brine. The organic phase was dried over anhydrous sodium sulfate, filtered and concentrated to obtain **M-1-3**. $^1$H NMR (400MHz, $CDCl_3$) δ = 4.14 - 4.00 (m, 4H), 3.68 - 3.59 (m, 4H), 2.57 - 2.50 (m, 4H), 1.67 - 1.56 (m, 4H), 1.23 - 1.14 (m, 6H).

Step 3: Synthesis of compound **M-1-4**

Compound **M-1-3** (200 mg, 774.26 μmol, 1 *eq*) was dissolved in tetrahydrofuran (5 mL). Lithium aluminum hydride (705.28 mg, 18.58 mmol, 3 *eq*) was added in batches at 0 °C under nitrogen, and the mixture was stirred at 25 °C overnight. The mixture was quenched by slowly adding sodium sulfate decahydrate with stirring. The mixture was filtered, washed with tetrahydrofuran, and concentrated to obtain compound **M-1-4.** $^1$H NMR (400MHz, $CDCl_3$) δ = 3.81 - 3.73 (m, 4H), 3.72 - 3.64 (m, 4H), 1.77 (t, J = 6.7 Hz, 4H), 1.56 - 1.48 (m, 4H).

Step 5: Synthesis of compound **M-1-5**

Compound **M-1-4**(115 mg, 660.02 μmol, 1 *eq*) was dissolved in dichloromethane (5 mL), and the solution was cooled to 0°C. Methylsulfonyl chloride (260 mg, 2.27 mmol, 3.44 *eq*), triethylamine (267.15 mg, 2.64 mmol, 4 *eq*) were added, and the mixture was stirred for 30 minutes. The reaction solution was quenched by slowly adding 30 mL of water, extracted with 40 mL of dichloromethane, washed once with saturated brine. The organic phase was collected, dried and concentrated to obtain compound **M-1-5.** $^1$H NMR (400MHz, $CDCl_3$) δ = 4.25 (t, J = 6.9 Hz, 4H), 3.38 - 3.32 (m, 4H), 2.97 (s, 6H), 1.82 (t, J = 7.0 Hz, 4H), 1.47 (t, J = 5.3 Hz, 4H).

Step 6: Synthesis of compound **M-1-6**

Compound **M-1-5** (210 mg, 635.56 μmol, 1 *eq*) was dissolved in *N,N*-dimethylformamide (4 mL). Sodium sulfide (59.52 mg, 762.67 μmol, 1.2 *eq*) was added, and the mixture was stirred at 50 °C for 12 hours. The mixture was extracted with 50 mL of ethyl acetate and 20 mL of water. The organic phase was collected, dried and concentrated. The crude product was separated and purified by silica gel column chromatography to obtain compound **M-1-6.** $^1$H NMR (400MHz, $CDCl_3$) δ = 3.71 - 3.64 (m, 4H), 2.68 - 2.59 (m, 4H), 1.86 - 1.77 (m, 4H), 1.55 - 1.47 (m, 4H).

Step 7: Synthesis of compound **M-1**

**M-1-6** (250 mg, 1.45 mmol, 1 *eq*) was dissolved in methanol (5 mL). Iodosobenzene diacetate (1.40 g, 4.35 mmol, 3 *eq*) and ammonium acetate (447.41 mg, 5.80 mmol, 4 *eq*) were added, and the mixture was stirred at 25 °C for 2 hours. The reaction solution was concentrated in vacuo to obtain a crude product. The crude product was separated and purified by silica gel column chromatography, to obtain compound **M-1.** $^1$H NMR (400MHz, $CDCl_3$) δ = 3.88 - 3.71 (m, 1H), 3.67 - 3.57 (m, 4H), 3.02 (br d, J = 5.0 Hz, 4H), 1.99 (br d, J = 4.3 Hz, 4H), 1.53 (br d, J = 4.3 Hz, 4H).

**Reference example 14**

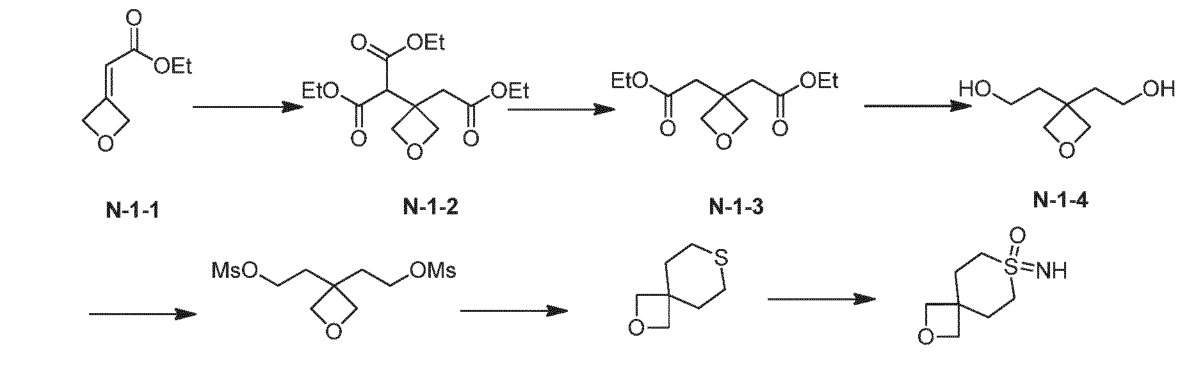

**N-1-1** **N-1-2** **N-1-3** **N-1-4**

**N-1-5** **N-1-6** **N-1**

Step 1: Synthesis of compound **N-1-2**

Sodium hydride (844.09 mg, 21.10 mmol, 3 *eq*) was added into a reaction flask, and the atmosphere was replaced with nitrogen. Tetrahydrofuran (8 mL) was slowly added at 0°C, and the mixture was stirred for 10 minutes. Diethyl malonate (3.94 g, 24.62 mmol, 3.5 *eq*) was slowly added, and the mixture was heated to 25 °C and stirred for 20 minutes. Tetrabutylammonium bromide (907.10 mg, 2.81 mmol, 0.4 *eq*) and a solution of compound **N-1-1** (1 g, 7.03 mmol, 1 *eq*) in tetrahydrofuran (1.65 mL) were added, and the mixture was stirred at 25 °C for 16 hours. Subsequently, acetic acid (1.5 mL) was added to quench the reaction, and 40 mL of ethyl acetate was added. The organic phase was washed three times with aqueous saturated sodium chloride solution, dried over anhydrous sodium sulfate, filtered, and concentrated. The crude product was separated by silica gel column chromatography to obtain compound **N-1-2.** $^1$H NMR (400MHz, $CDCl_3$) $\delta$ = 4.70 (d, J = 7.0 Hz, 2H), 4.52 (d, J = 7.0 Hz, 2H), 4.16 (q, J = 7.0 Hz, 4H), 4.07 (q, J = 7.3 Hz, 2H), 3.90 (s, 1H), 2.92 (s, 2H), 1.28 - 1.17 (m, 9H).

Step 2: Synthesis of compound **N-1-3**

Compound **N-1-2**(0.5 g, 1.65 mmol, 1 *eq*) was dissolved in dimethyl sulfoxide (5 mL). Sodium chloride (193.31 mg, 3.31 mmol, 2 *eq*) and water (0.1 mL) were added, and the mixture was gradually heated to 160 °C and stirred for 3.5 hours. Then the mixture was cooled to room temperature. 40 mL of ethyl acetate was added, the mixture was washed once with 20 mL of saturated brine, and the aqueous layer was washed once with 20 mL of ethyl acetate. The two organic phases were combined, and washed twice with 20 mL of saturated brine. The organic phase was dried over anhydrous sodium sulfate and concentrated. The crude product was separated by silica gel column chromatography to obtain compound **N-1-3.** $^1$H NMR (400MHz, $CDCl_3$) $\delta$ = 4.56 (s, 4H), 4.14 (q, J = 7.1 Hz, 4H), 2.92 (s, 4H), 1.26 (t, J = 7.1 Hz, 6H).

Step 3: Synthesis of compound **N-1-4**

Lithium aluminum tetrahydride (219.21 mg, 5.78 mmol, 3.8 *eq*) was added into a reaction flask, and the atmosphere was replaced with nitrogen. Tetrahydrofuran (10 mL) was slowly added at 0 °C, and the mixture was stirred for 10 minutes. Then a solution of **N-1-3** (0.35 g, 1.52 mmol, 1 *eq*) in tetrahydrofuran (0.5 mL) was slowly added, and the mixture was stirred for 2 hours. 1 mL of water and 1 .5 mL of 15% aqueous sodium hydroxide solution were slowly added under nitrogen flow, then 20 mL of tetrahydrofuran was added. The mixture was dried over anhydrous sodium sulfate and filtered. The filter cake was washed twice with tetrahydrofuran. The filtrate and the washing liquid were combined and concentrated under reduced pressure to obtain compound **N-1-4.** $^1$H NMR (400MHz, $CDCl_3$) $\delta$ = 4.51 - 4.37 (m, 4H), 3.81 - 3.63 (m, 4H), 2.05 - 1.95 (m, 4H).

Step 4: Synthesis of compound **N-1-5**

Compound **N-1-4**(0.2 g, 1.37 mmol, 1 *eq*) was dissolved in dichloromethane (8 mL). The mixture was cooled to 0°C. Methylsulfonyl chloride (0.510 g, 4.45 mmol, 3.25 *eq*) and triethylamine (692.20 mg, 6.84 mmol, 5 *eq*) were added. The mixture was stirred at 0 °C for 4 hours. Subsequently, the reaction solution was quenched by slowly adding 10 mL of ice water, and extracted twice with 20 mL of dichloromethane. The organic phases were combined, dried and concentrated to obtain compound **N-1-5.** $^1$H NMR (400MHz, $CDCl_3$) $\delta$ = 4.42 (s, 4H), 4.28 (t, J = 6.4 Hz, 4H), 2.96 (s, 6H), 2.20 (t, J = 6.4 Hz, 4H).

Step 5: Synthesis of compound **N-1-6**

Compound **N-1-5**(0.35 g, 1.16 mmol, 1 *eq*) was dissolved in *N,N*-dimethylformamide (5 mL). Sodium sulfide (333.63 mg, 1.39 mmol, 1.2 *eq*) was added, and the mixture was stirred at 50 °C for 12 hours. 10 mL of water was added, and the mixture was extracted three times with 20 mL of ethyl acetate. The organic phase was collected, washed twice with 20 mL of saturated brine, dried over anhydrous sodium sulfate and concentrated. The residue was separated by silica gel column chromatography to obtain compound **N-1-6.** $^1$H NMR (400MHz, $CDCl_3$) $\delta$ = 4.30 (s, 4H), 2.53 - 2.43 (m, 4H), 2.08 - 1.98 (m, 4H).

Step 6: Synthesis of compound **N-1**

Compound **N-1-6**(0.1 g, 693.32 $\mu$mol, 1 *eq*), ammonium acetate (213.76 mg, 2.77 mmol, 4 *eq*) and iodosobenzene diacetate (669.95 mg, 2.08 mmol, 3 *eq*) were dissolved in methanol (4 mL), and the mixture was stirred at 25 °C for 2 hours. The reaction solution was then concentrated, and the crude product was separated and purified by silica

gel column chromatography to obtain compound **N-1.** $^1$H NMR (400MHz, $CDCl_3$) δ = 4.46 - 4.29 (m, 4H), 2.96 (br d, J = 4.3 Hz, 4H), 2.33 (br s, 4H).

**Reference example 15**

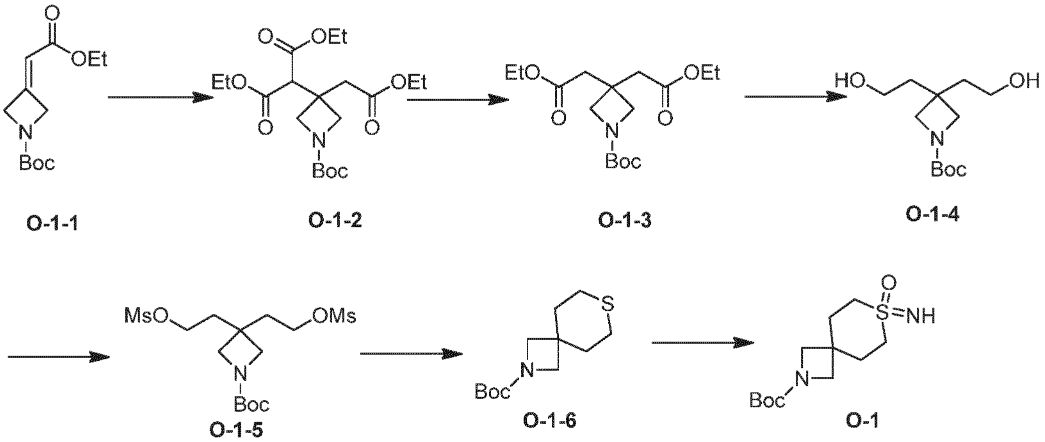

O-1-1 O-1-2 O-1-3 O-1-4

O-1-5 O-1-6 O-1

Step 1: Synthesis of compound **O-1-2**

Sodium hydride (497.34 mg, 12.43 mmol, 3 *eq*) was added into a reaction flask, and the atmosphere was replaced with nitrogen. Tetrahydrofuran (8 mL) was slowly added at 0°C, and the mixture was stirred for 10 minutes. Diethyl malonate (2.32 g, 14.51 mmol, 3.5 *eq*) was slowly added, and the mixture was heated to 25 °C and stirred for 20 minutes. Tetrabutylammonium bromide (534.42 mg, 1.66 mmol, 0.4 *eq*) and a solution of compound **O -1-1** (1 g, 4.14 mmol, 1 *eq*) in tetrahydrofuran (1.65 mL) were added, and the mixture was stirred at 25 °C for 18 hours. Subsequently, acetic acid (1.5 mL) was added to quench the reaction, and 40 mL of ethyl acetate was added. The organic phase was washed three times with aqueous saturated sodium chloride solution, dried over anhydrous sodium sulfate, filtered, and concentrated. The crude product was separated by silica gel column chromatography to obtain compound **O-1-2.** $^1$H NMR (400MHz, $CDCl_3$) δ = 4.26 - 4.18 (m, 4H), 4.17 - 4.07 (m, 4H), 3.91 (s, 1H), 3.84 (d, J = 9.5 Hz, 2H), 2.88 (s, 2H), 1.43 (s, 9H), 1.27 (q, J = 7.1 Hz, 9H).

Step 2: Synthesis of compound **O-1-3**

Compound **O-1-2**(6.5 g, 16.19 mmol, 1 *eq*) was dissolved in dimethyl sulfoxide (65 mL). Sodium chloride (1.89 g, 32.38 mmol, 2 *eq*) and water (0.9 mL) were added. The mixture was gradually heated to 160 °C and stirred for 3.5 hours. Then the mixture was cooled to room temperature. 300 mL of ethyl acetate was added, the mixture was washed once with 150 mL of saturated brine, and the aqueous layer was washed once with 200 mL of ethyl acetate. The two organic phases were combined, and washed twice with 200 mL of saturated brine. The organic phase was dried over anhydrous sodium sulfate and concentrated. The crude product was separated by silica gel column chromatography to obtain compound **O-1-3.** $^1$H NMR (400MHz, $CDCl_3$) δ = 4.18 - 4.13 (m, 4H), 3.85 - 3.82 (m, 4H), 2.82 (s, 4H), 1.49 - 1.41 (m, 9H), 1.30 - 1.26 (m, 6H).

Step 3: Synthesis of compound **O-1-4**

Lithium aluminum tetrahydride (831.84 mg, 21.92 mmol, 3.8 *eq*) was added into a reaction flask, and the atmosphere was replaced with nitrogen. Tetrahydrofuran (20 mL) was slowly added at 0 °C, and the mixture was stirred for 10 minutes. Then **O-1-3** (1.9 g, 5.77 mmol, 1 *eq*) in tetrahydrofuran (4 mL) was slowly added, and the mixture was stirred for 2 hours. 2 mL of water and 3 mL of 15% aqueous sodium hydroxide solution were slowly added under nitrogen flow, and then 40 mL of tetrahydrofuran was added. The mixture was dried over sodium sulfate and filtered. The filter cake was washed twice with tetrahydrofuran. The filtrate and the washing liquid were combined and concentrated under reduced pressure to obtain compound **O-1-4**. $^1$H NMR (400MHz, $CDCl_3$) δ = 3.79 - 3.73 (m, 4H), 3.68 (s, 4H), 2.55 (br s, 2H), 1.96 (t, J = 6.3 Hz, 4H), 1.44 (s, 9H).

Step 4: Synthesis of compound **O-1-5**

Compound **O-1-4**(1.11 g, 4.52 mmol, 1 *eq*) was dissolved in dichloromethane (25 mL). The mixture was cooled to 0°C. Methylsulfonyl chloride (1.750 g, 15.28 mmol, 3.25 *eq*) and triethylamine (2.29 g, 22.62 mmol, 5 *eq*) were added. The mixture was stirred at 0 °C for 4 hours. Subsequently, the reaction solution was quenched by slowly adding 20 ml of ice water, and extracted twice with 40 mL of dichloromethane. The organic phases were combined, dried and concentrated to obtain compound **O-1-5.** $^1$H NMR (400MHz, $CDCl_3$) $\delta$ = 4.25 (t, J = 6.5 Hz, 4H), 3.66 (s, 4H), 2.97 (s, 6H), 2.09 (t, J = 6.4 Hz, 4H), 1.37 (s, 9H).

Step 5: Synthesis of compound **O-1-6**

Compound **O-1-5**(1.62 g, 4.03 mmol, 1 *eq)* was dissolved in *N,N*-dimethylformamide (20 mL). Sodium sulfide (1.16 g, 4.84 mmol, 1.2 *eq)* was added, and the mixture was stirred at 50 °C for 12 hours. 20 mL of water was added, and the mixture was extracted three times with 50 mL of ethyl acetate. The organic phase was collected, washed twice with 30 mL of saturated brine, dried over anhydrous sodium sulfate and concentrated. The residue was separated by silica gel column chromatography to obtain compound **0-1-6.** $^1$H NMR (400MHz, $CDCl_3$) $\delta$ = 3.50 (s, 4H), 2.53 - 2.44 (m, 4H), 1.94 - 1.86 (m, 4H), 1.37 (s, 9H).

Step 6: Synthesis of compound **O-1**

Compound **O-1-6**(0.3 g, 1.23 mmol, 1 *eq*), ammonium acetate (380.07 mg, 4.93 mmol, 4 *eq)* and iodosobenzene diacetate (1.19 g, 3.70 mmol, 3 *eq*) were dissolved in methanol (4 mL), and the mixture was stirred at 25 °C for 2 hours. The reaction solution was then concentrated, and the crude product was separated and purified by silica gel column chromatography to obtain compound **O-1.** $^1$H NMR (400MHz, $CDCl_3$) $\delta$ = 3.73 (d, J = 4.5 Hz, 4H), 3.13 - 2.96 (m, 4H), 2.40 - 2.21 (m, 4H), 1.47 (s, 9H).

**Example 1**

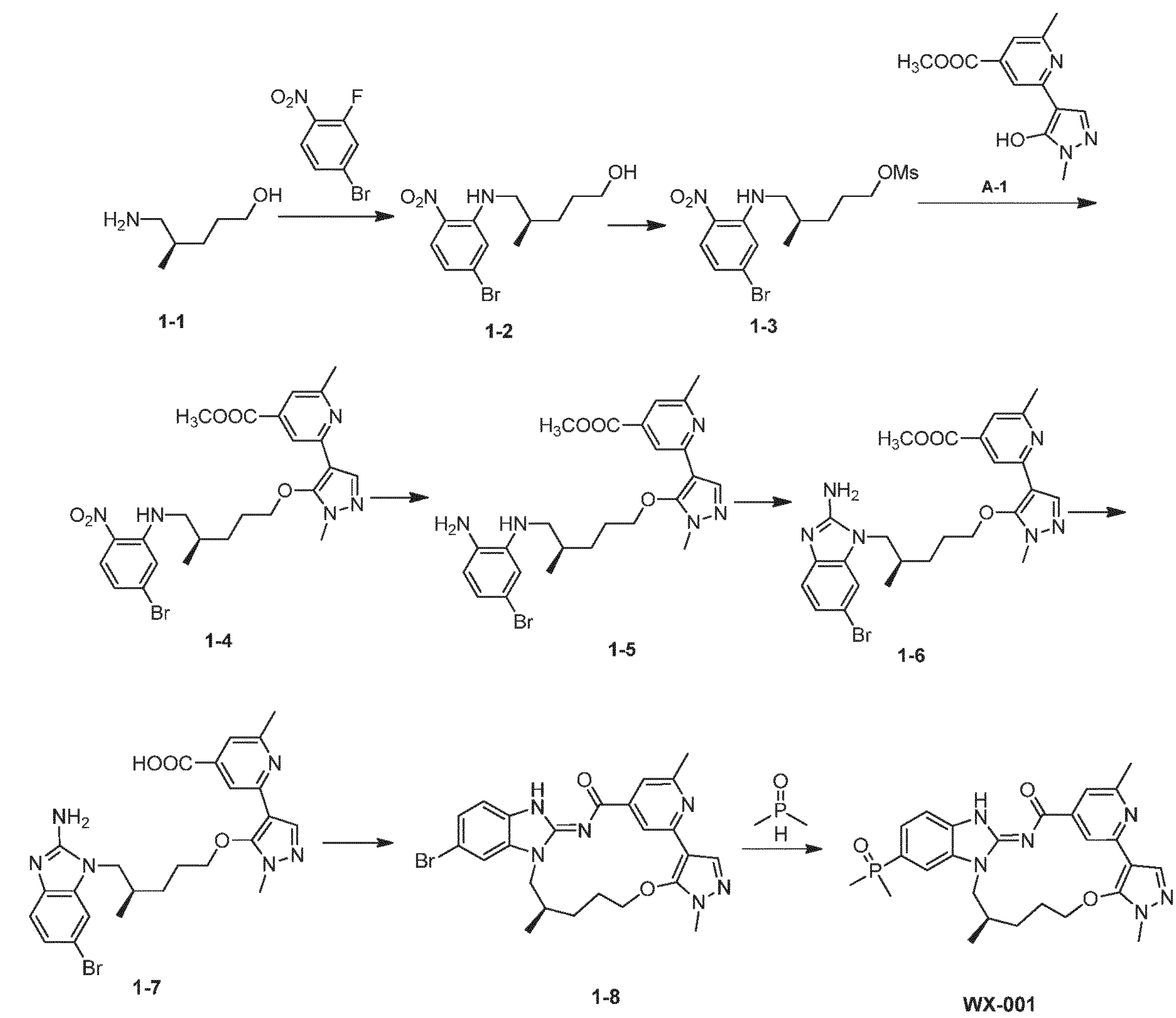


1-1 1-2 1-3 1-4 1-5 1-6 1-7 1-8 WX-001

Step 1: Synthesis of compound **1-2**

To a solution of 2-fluoro-4-bromonitrobenzene (5 g, 22.73 mmol, 1 *eq*) and compound **1-1** (hydrochloride) (3.49 g, 22.73 mmol, 1 *eq*) in ethanol (50 mL) was added triethylamine (9.20 g, 90.91 mmol, 12.65 mL, 4 *eq*), and the mixture was stirred at 80 °C for 16 hours. Water (200 mL) was slowly added to the reaction solution, then the mixture was extract three times with 150 mL of ethyl acetate. The organic phases were combined and dried over anhydrous sodium sulfate. Water (200 mL) was added and the mixture was washed with saturated brine (200 mL). The organic phase was dried over anhydrous sodium sulfate and concentrated under reduced pressure to obtain crude product **1-2.** LCMS m/z = 316.7/318.7 $[M+H]^+$.

Step 2: Synthesis of compound **1-3**

**1-2** (2.50 g, 7.88 mmol, 1 *eq*) was dissolved in dichloromethane (20 mL), and the solution was cooled to 0°C. Triethylamine (957.10 mg, 9.46 mmol, 1.32 mL, 1.2 *eq*) and methylsulfonyl chloride (1.36 g, 11.90 mmol, 921.19 μL, 1.51 *eq*) were added. Then, the mixture was heated to 25°C and stirred to react for 3 hours. After the reaction was completed, 50 mL of dichloromethane was added to the reaction solution, and the mixture was washed twice with aqueous saturated sodium bicarbonate solution (50 mL). The layers were separated. The organic phase was collected, dried over anhydrous sodium sulfate and concentrated in vacuo to obtain a crude product. The crude product was separated and purified by silica gel column chromatography (petroleum ether:ethyl acetate = 10:1~3:1) to obtain compound **1-3.** LCMS m/z = 394.8/396.8 $[M+H]^+$.

Step 3: Synthesis of compound **1-4**

**1-3** (9.00 g, 22.77 mmol, 1 *eq*), **A-1**(7.32 g, 29.60 mmol, 1.3 *eq*), and potassium carbonate (9.44 g, 68.31 mmol,

3 *eq*) were dissolved in *N,N*-dimethylformamide (100 mL), and the mixture was heated to 60 °C and stirred to react for 16 hours. Subsequently, the reaction solution was directly concentrated under vacuum to obtain a crude product, which was then separated and purified by silica gel column chromatography to obtain product **1-4.** $^1$H NMR (400MHz, $CD_3OD$) $\delta$ = 7.97 (dd, J = 2.4, 9.2 Hz, 1H), 7.90 (s, 1H), 7.87 (d, J= 1.0 Hz, 1H), 7.49 (s, 1H), 7.13 (s, 1H), 6.74 (td, J = 1.9, 9.2 Hz, 1H), 5.49(s, 1H), 4.12 (d, J= 2.3 Hz, 1H), 3.91 (s, 3H), 3.73 (s, 3H), 3.30 - 3.23 (m, 1H), 3.22 - 3.14 (m, 1H), 2.56 (s, 3H), 2.00 - 1.79 (m, 3H), 1.79 - 1.65 (m, 1H), 1.54 - 1.40 (m, 1H), 1.06 (d, J = 6.8 Hz, 3H); LCMS m/z = 546.1/548.1 $[M+H]^+$.

Step 4: Synthesis of compound **1-5**

**1-4** (8.50 g, 15.56 mmol, 1 *eq*), ferric chloride hexahydrate (630.71 mg, 2.33 mmol, 0.15 *eq*), and activated carbon (1.12 g, 93.34 mmol, 6 *eq*) were dissolved in tetrahydrofuran (100 mL). 50% hydrazine hydrate (46.72 g, 466.68 mmol, 45.36 mL, 30 *eq*) was added, and the mixture was stirred and reacted at 65 °C for 6 hours. The reaction solution was filtered through diatomaceous earth to obtain a filtrate. The filter cake was washed twice with 30 mL of ethyl acetate. The filtrate was concentrated in vacuum to obtain a crude product. The crude product was purified by silica gel column chromatography (dichloromethane : methanol = 20:1) to obtain compound **1-5.** $^1$H NMR (400MHz, $CD_3OD$) $\delta$ = 7.87 (s, 1H), 7.79 (s, 1H), 7.37 (s, 1H), 6.58 (s, 3H), 3.92 (s, 1H), 3.73 (br s, 1H), 3.72 (s, 3H), 3.05 - 2.97 (m, 1H), 2.92 (br d, J = 6.5 Hz, 1H), 2.58 (br s, 1H), 2.57 (s, 3H), 2.10 (s, 3H), 2.00 (s, 1H), 1.70 (br d, J = 5.3 Hz, 1H), 1.44 - 1.30 (m, 2H), 1.00 (d, J = 6.5 Hz, 3H); LCMS m/z = 516.2/518.2 $[M+H]^+$.

Step 5: Synthesis of compound **1-6**

**1-5** (8 g, 15.49 mmol, 1 *eq*) was dissolved in dichloromethane (150 mL) and tert-butanol (30 mL). Cyanogen bromide (13.42 g, 126.72 mmol, 9.32 mL, 8.18 *eq*) was then added, and the mixture was stirred to react at 25 °C for 18 hours. 30 mL of aqueous saturated sodium bicarbonate solution was added to the reaction solution, and the mixture was stirred for 10 minutes. The organic layer was separated, washed twice with saturated sodium bicarbonate (30 mL) and once with saturated brine (30 mL). The organic layer was dried over anhydrous sodium sulfate and concentrated in vacuo to obtain a crude product. The crude product was separated and purified using silica gel column chromatography (dichloromethane : methanol = 20:1) to obtain compound **1-6.** $^1$H NMR (400MHz, $CD_3OD$) $\delta$ = 7.89 (s, 1H), 7.87 (s, 1H), 7.53 (s, 1H), 7.27 (d, J = 1.5 Hz, 1H), 7.25 - 7.20 (m, 1H), 7.08 (d, J = 8.3 Hz, 1H), 4.09 - 4.01 (m, 1H), 3.94 (s, 3H), 3.72 (d, J = 3.5 Hz, 1H), 3.70 (s, 3H), 3.65 (s, 1H), 2.58 (s, 3H), 2.57 (br s, 1H), 2.18 - 2.10 (m, 1H), 2.02 - 1.96 (m, 1H), 1.84 - 1.78 (m, 1H), 1.68 - 1.59 (m, 1H), 1.50 - 1.43 (m, 1H), 0.99 (d, J = 6.5 Hz, 3H); LCMS m/z = 541.1/543.1 $[M+H]^+$.

Step 6: Synthesis of compound **1-7**

**1-6** (4 g, 7.39 mmol, 1 *eq*) was dissolved in tetrahydrofuran (50 mL). Sodium hydroxide (1.18 g, 29.55 mmol, 4 *eq*) was added, and the mixture was stirred at room temperature 25°C for 1 hour. After the reaction was completed, the reaction solution was concentrated in vacuum, adjusted to pH of 4-5 with 3M aqueous hydrochloric acid solution, and then concentrated in vacuum to remove the solvent. The residue was dissolved in a mixed solvent of dichloromethane (30 mL) and methanol (5 mL), dried over anhydrous sodium sulfate, and filtered. The filtrate was concentrated in vacuum to obtain crude product **1-7.** $^1$H NMR (400MHz, DMSO-$d_6$) $\delta$ = 7.86 - 7.85 (m, 1H), 7.87 - 7.82 (m, 1H), 7.44 (s, 1H), 7.39 - 7.30 (m, 1H), 7.05 (s, 1H), 6.72 (br s, 1H), 4.08 (br t, J = 6.3 Hz, 2H), 3.96 - 3.85 (m, 2H), 3.84 - 3.77 (m, 1H), 3.62 (s, 1H), 3.63 - 3.60 (m, 1H), 3.64 (br d, J = 4.3 Hz, 1H), 3.17 (s, 3H), 2.52 (br s, 3H), 2.03 (br s, 1H), 1.98 - 1.88 (m, 1H), 1.79 - 1.68 (m, 1H), 1.64 - 1.51 (m, 1H), 0.83 (d, J = 6.5 Hz, 3H); LCMS m/z = 527.1/529.1 $[M+H]^+$.

Step 7: Synthesis of compound **1-8**

1-7 (4 g, 6.45 mmol, 1 *eq*) was dissolved in dichloromethane (50 mL). Triethylamine (3.26 g, 32.23 mmol, 4.49 mL, 5 *eq*) and *O*-benzotriazol-*N,N,N',N'*-tetramethyluronium tetrafluoroborate (6.21 g, 19.34 mmol, 3 *eq)* were added, and the mixture was stirred and reacted at 25 °C for 16 hours. Dichloromethane (30 mL) was added, and the mixture was washed 3 times with saturated brine (30 mL). The organic layer was dried over anhydrous sodium sulfate, filtered, and concentrated in vacuo to obtain a crude product. The crude product was separated by column chromatography to obtain compound **1-8.** $^1$H NMR (400MHz, $CDCl_3$) $\delta$ = 12.28 - 11.75 (m, 1H), 8.54 (s, 1H), 8.42 - 8.22 (m, 1H), 7.69 (s, 1H), 7.44 (d, J = 1.5 Hz, 1H), 7.39 (d, J = 8.6 Hz, 1H), 7.24 (d, J = 8.5 Hz, 1H), 4.46 (br d, J = 4.8 Hz, 1H), 4.35 (dd, J = 3.1, 13.7 Hz, 1H), 3.96 - 3.88 (m, 1H), 3.80 (s, 3H), 3.71 - 3.63 (m, 1H), 2.83 (br d, J = 14.3 Hz, 1H), 2.72 (br s, 3H), 2.34 - 2.22 (m, 1H), 2.19 - 2.09 (m, 1H), 2.02 - 1.92 (m, 1H), 1.54 (br d, J = 6.8 Hz, 1H), 0.94 (d, J = 6.5 Hz, 3H); LCMS m/z = 509.1/511.1 $[M+H]^+$.

Step 8: Synthesis of compound **WX-001** trifluoroacetate

To a solution of **1-8** (50 mg, 98.16 μmol, 1 *eq*), dimethylphosphine oxide (76.61 mg, 981.55 μmol, 10 *eq*) in 1,4-dioxane (3 mL) were added 4,5-bisdiphenylphosphino-9,9-dimethylxanthene (34.08 mg, 58.89 μmol, 0.6 *eq*), *N,N*-diisopropylethylamine (63.43 mg, 490.78 μmol, 85.48 μL, 5 *eq*). The atmosphere was replaced with nitrogen, and tris(dibenzylideneacetone)dipalladium (26.96 mg, 29.45 μmol, 0.3 *eq*) was added. The mixture was stirred at 120 °C for 36 hours under nitrogen. The reaction solution was concentrated under reduced pressure to obtain a crude product. The crude product was separated by preparative HPLC, column: Welch Xtimate C18 100*40mm*3μm; mobile phase: [water (trifluoroacetic acid)-acetonitrile]; gradient: acetonitrile%: 15%-45%,8 minutes, to obtain **WX-001** trifluoroacetate. $^1H$ NMR (400MHz, $CD_3OD$) δ = 8.82 (s, 1H), 8.19 (s, 1H), 8.02 (s, 1H), 7.94 (br d, J = 12.3 Hz, 1H), 7.72 - 7.57 (m, 2H), 4.50 (td, J = 4.7, 9.2 Hz, 1H), 4.37 (br dd, J = 2.4, 13.7 Hz, 1H), 4.10 (br d, J = 3.5 Hz, 1H), 4.00 (br dd, J = 10.7, 13.4 Hz, 1H), 3.80 (s, 3H), 2.80 (s, 4H), 2.32 (br d, J= 7.0 Hz, 1H), 2.16 - 1.96 (m, 2H), 1.87 (dd, J = 3.3, 13.3 Hz, 6H), 1.66 - 1.49 (m, 1H), 0.89 (br d, J = 6.5 Hz, 3H); LCMS m/z = 507.3 $[M+H]^+$.

**Example 2**

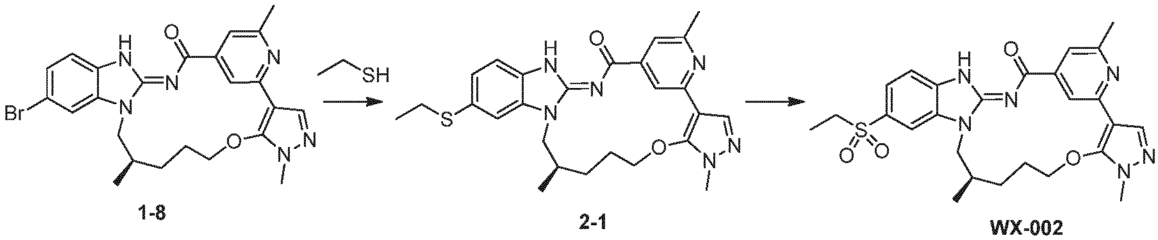


1-8 2-1 WX-002

Step 1: Synthesis of compound **2-1**

Compound **1-8** (150.00 mg, 294.47 μmol, 1 *eq*), tris(dibenzylideneacetone)dipalladium (80.89 mg, 88.34 μmol, 0.3 *eq*), 4,5-bisdiphenylphosphino-9,9-dimethylxanthene (102.23 mg, 176.68 μmol, 0.6 *eq*) and *N,N*-diisopropylethylamine (114.17 mg, 883.40 μmol, 153.87 μsL, 3 *eq*) were added to dioxane (13 mL). Ethanethiol (2.71 g, 43.62 mmol, 3.23 mL, 148.12 *eq*) was added, and the mixture was stirred under nitrogen to react at 120 °C for 2.5 hours. Then, 50 mL of dichloromethane was added to the reaction solution, and the mixture was washed three times with saturated brine (50 mL). 50 mL of aqueous sodium hypochlorite solution was added to the aqueous layer, and the aqueous layer was stirred for 5 min. The organic layer was filtered to obtain a filtrate. The filtrate was dried over anhydrous sodium sulfate, and concentrated in vacuum to obtain a crude product. The crude product was separated and purified by silica gel column chromatography (gradient elution: dichloromethane/methanol = 100:0~100:10) to obtain compound **2-1.** $^1H$ NMR (400MHz, $CDCl_3$) δ = 8.52 (br s, 1H), 8.22 (br s, 1H), 7.69 (br s, 1H), 7.33 (d, J = 9.8 Hz, 2H), 7.10 - 6.98 (m, 1H), 4.55 - 4.44 (m, 1H), 4.41 - 4.32 (m, 1H), 3.97 - 3.88 (m, 1H), 3.81 (s, 3H), 3.71 (br dd, J = 9.9, 13.8 Hz, 1H), 3.03 - 2.81 (m, 3H), 2.69 (br s, 3H), 2.28 (br s, 1H), 2.20 - 2.12 (m, 1H), 1.98 (dt, J = 4.9, 9.3 Hz, 1H), 1.55 (br d, J = 6.6 Hz, 1H), 1.34 (t, J = 7.3 Hz, 3H), 0.95 (br d, J = 6.5 Hz, 3H); LCMS m/z = 491.2 $[M+H]^+$.

Step 2: Synthesis of compound **WX-002** formate

Compound **2-1**(20 mg, 40.76 μmol, 1 *eq*) was dissolved in a mixed solvent of water (1 mL) and methanol (1 mL). Potassium monopersulfate (75.18 mg, 122.29 μmol, 3 *eq*) was added, and the mixture was stirred at 30 °C for 1 hour. Subsequently, the mixture was quenched by adding water (20 mL), extracted three times with 10 mL of ethyl acetate, washed with saturated sodium chloride solution (10 mL), dried over anhydrous sodium sulfate, and filtered. The organic phase was concentrated to obtain a crude product. The crude product was separated by preparative HPLC, column: Phenomenex C18 150*40mm*5μm; mobile phase: [water (formic acid)-acetonitrile]; gradient: acetonitrile%: 15%-45%,10 minutes, freeze-dried, and concentrated under reduced pressure to obtain **WX-002** formate. $^1H$ NMR (400MHz, $CDCl_3$) δ = 8.46 (br s, 1H), 7.87 - 7.72 (m, 2H), 7.61 (br s, 1H), 7.46 (br d, J = 8.5 Hz, 1H), 7.22-7.14 (m, 1H), 4.48 - 4.27 (m, 3H), 3.85 (br s, 1H), 3.77 - 3.68 (m, 3H), 3.12 (q, J = 7.4 Hz, 2H), 2.86 - 2.52 (m, 4H), 2.27 - 1.86 (m, 4H), 1.25 (t, J = 7.3 Hz, 3H), 0.86 (d, J = 6.5 Hz, 3H); LCMS m/z = 523.2 $[M+H]^+$.

## Example 3

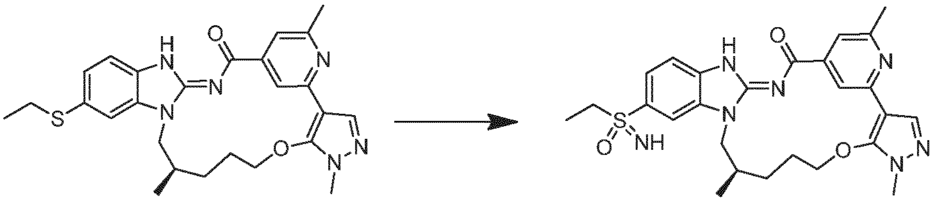

2-1 WX-003

Step 1: Synthesis of compound **WX-003** formate

Compound **2-1**(20 mg, 40.76μmol, 1 *eq*) was added to methanol (0.5 mL). Iodosobenzene Diacetate (39.39 mg, 122.29 μmol, 3 *eq*) and ammonium acetate (12.57 mg, 163.06 μmol, 4 *eq*) were then added, and the mixture was reacted at 30 °C for 2 hours. The reaction solution was filtered through a filter membrane and separated by preparative HPLC, column: Phenomenex C18 150*40mm*5μm; mobile phase: [water (formic acid)-acetonitrile]; gradient: acetonitrile%: 10%-40%, 10 minutes, to obtain **WX-003** formate. $^1H$ NMR (400MHz, $CDCl_3$) δ = 8.51 (s, 1H), 8.22 (br s, 1H), 8.08 (s, 1H), 7.96 (s, 1H), 7.92 (d, J = 8.5 Hz, 1H), 7.68 (s, 1H), 7.53 (d, J = 8.5 Hz, 1H), 4.52 - 4.37 (m, 3H), 3.98 - 3.77 (m, 5H), 3.33 - 3.21 (m, 2H), 2.90 (br s, 1H), 2.69 (s, 3H), 2.36 - 2.12 (m, 2H), 2.07 - 2.02 (m, 1H), 1.32 (t, J = 7.5 Hz, 3H), 0.95 (br d, J = 6.5 Hz, 3H); LCMS m/z = 522.3 $[M+H]^+$.

## Example 4

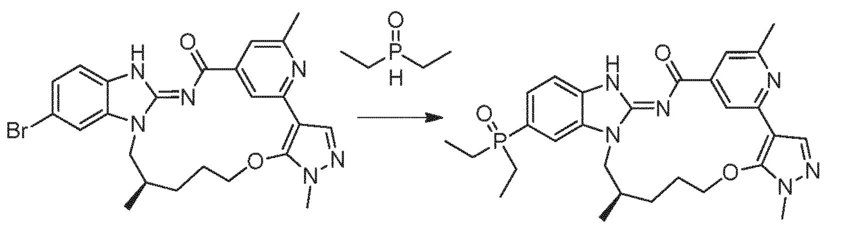

1-8 WX-004

Step 1: Synthesis of compound **WX-004** trifluoroacetate

To a solution of compound **1-8** (50 mg, 98.16 μmol, 1 *eq*), diethylphosphine oxide (104.15 mg, 981.55 μmol, 10 *eq*) in 1,4-dioxane (3 mL) were added 4,5-bisdiphenylphosphino-9,9-dimethylxanthene (34.08 mg, 58.89 μmol, 0.6 *eq*), *N,N*-diisopropylethylamine (63.43 mg, 490.78 μmol, 85.48 μL, 5 *eq*). The atmosphere was replaced with nitrogen, and tris(dibenzylideneacetone)dipalladium (26.96 mg, 29.45 μmol, 0.3 *eq*) was added. The mixture was stirred at 120 °C under nitrogen for 20 hours. The reaction solution was concentrated under reduced pressure to obtain a crude product. The crude product was separated by preparative HPLC, column: Welch Xtimate C18 100*40mm*3μm; mobile phase: [water (trifluoroacetic acid)-acetonitrile]; gradient: acetonitrile%: 20%-50%,8 minutes, to obtain **WX-004** trifluoroacetate. $^1H$ NMR (400MHz, $CD_3OD$) δ = 9.02 - 8.84 (m, 1H), 8.38 - 8.23 (m, 1H), 8.15 - 8.03 (m, 1H), 8.04 - 7.89 (m, 1H), 7.79 - 7.58 (m, 2H), 4.64 - 4.49 (m, 1H), 4.47 - 4.37 (m, 1H), 4.23 - 4.01 (m, 2H), 3.91 - 3.77 (m, 3H), 2.98 - 2.75 (m, 4H), 2.47 - 2.30 (m, 1H), 2.28 - 1.98 (m, 6H), 1.70 - 1.52 (m, 1H), 1.18 - 1.03 (m, 6H), 0.98 - 0.85 (m, 3H); LCMS m/z = 535.3 $[M+H]^+$.

## Example 5

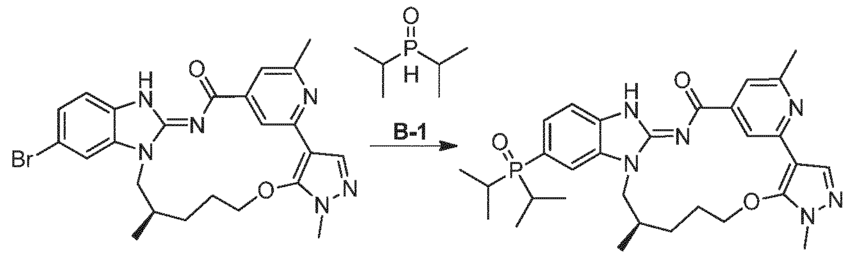


1-8 WX-005

Step 1: Synthesis of compound **WX-005** trifluoroacetate

To a solution of compound **1-8** (50 mg, 98.16 μmol, 1 *eq*), **B-1** (131.69 mg, 981.55 μmol, 10 *eq)* in 1,4-dioxane (3 mL) were added 4,5-bisdiphenylphosphino-9,9-dimethylxanthene (34.08 mg, 58.89 μmol, 0.6 *eq*), *N,N*-diisopropylethylamine (63.43 mg, 490.78 μmol, 85.48 μL, 5 *eq*). The atmosphere was replaced with nitrogen, and tris(dibenzylideneacetone)dipalladium (26.96 mg, 29.45 μmol, 0.3 *eq*) was added. The mixture was stirred at 120 °C under nitrogen for 20 hours. The reaction solution was concentrated under reduced pressure to obtain a crude product. The crude product was separated by preparative HPLC, column: Welch Xtimate C18 100*40mm*3μm; mobile phase: [water (trifluoroacetic acid)-acetonitrile]; gradient: acetonitrile%: 20%-50%,8 minutes, to obtain **WX-005** trifluoroacetate. $^1$H NMR (400MHz, $CD_3OD$) δ = 8.97 (s, 1H), 8.28 (s, 1H), 8.10 (s, 1H), 7.92 (br d, J = 10.4 Hz, 1H), 7.84 - 7.60 (m, 2H), 4.66 - 4.55 (m, 1H), 4.41 (br d, J = 13.4 Hz, 1H), 4.28 - 4.01 (m, 2H), 3.85 (s, 3H), 2.84 (s, 4H), 2.68 - 2.00 (m, 5H), 1.62 (br s, 1H), 1.21 (br dd, J = 6.9, 15.1 Hz, 6H), 1.15 - 1.03 (m, 6H), 0.92 (br d, J = 6.3 Hz, 3H); LCMS m/z = 563.1 $[M+H]^+$.

**Example 6**

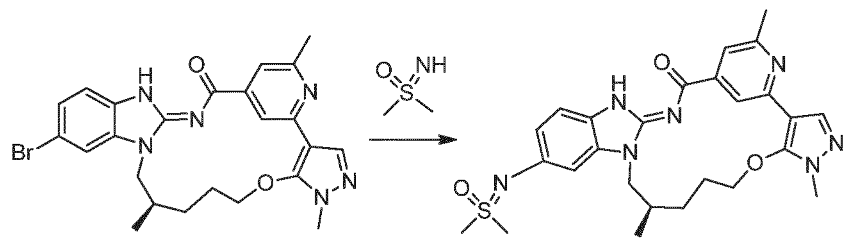

1-8 WX-006

Synthetic route 1

Step 1: Synthesis of compound **WX006** trifluoroacetate

To a solution of compound **1-8** (30 mg, 58.89 μmol, 1 *eq*), S,S-dimethyl sulfoximine (6.86 mg, 73.62 μmol, 1.25*eq*) in 1,4-dioxane (3 mL) were added 2-(di-tert-butylphosphino)biphenyl (5.27 mg, 17.67 μmol, 0.3 *eq*), sodium tert-butoxide (8.49 mg, 88.34 μmol, 1.5 *eq*). The atmosphere was replaced with nitrogen, and tris(dibenzylideneacetone)dipalladium (16.18 mg, 17.67 μmol, 0.3 *eq*) was added. The mixture was stirred at 100 °C under nitrogen for 5 hours. The reaction solution was concentrated under reduced pressure to obtain a crude product. The crude product was separated by preparative HPLC, column: Welch Xtimate C18 100*40mm*3μm; mobile phase: [water (trifluoroacetic acid)-acetonitrile]; gradient: acetonitrile%: 20%-50%,8 minutes, and concentrated under reduced pressure to obtain **WX-006** trifluoroacetate. LCMS m/z = 522.1 $[M+H]^+$.

Synthetic route 2

Step 1: Synthesis of compound **WX-006**

To a solution of compound **1-8** (30 mg, 58.89 μmol, 1 *eq*), S,S-dimethyl sulfoximine (6.86 mg, 73.62 μmol, 1.25*eq*) in 1,4-dioxane (3 mL) were added 2-(di-tert-butylphosphino)biphenyl (5.27 mg, 17.67 μmol, 0.3 *eq*), sodium tert-butoxide in tetrahydrofuran (2 M, 88.34 μmol, 44.17 μL, 1.5 *eq*). The atmosphere was replaced with nitrogen, and tris(dibenzylideneacetone)dipalladium (16.18 mg, 17.67 μmol, 0.3 *eq*) was added. The mixture was stirred at 80 °C under nitrogen for 12 hours. The mixture was cooled to room temperature, and 10 mL of water was added. The mixture was extracted with 50 mL of dichloromethane, and then washed once with 10 mL of saturated sodium chloride solution. The organic phase was dried over anhydrous sodium sulfate, filtered, and concentrated in vacuo to obtain a crude product. The crude product was separated and purified by silica gel column chromatography (gradient elution: dichloromethane/methanol = 100:0~100:3) to obtain **WX-006**. $^1H$ NMR (400MHz, $CDCl_3$) δ = 11.85 (br s, 1H), 8.40 (s, 1H), 8.08 (s, 1H), 7.59 (s, 1H), 7.14 (d, *J* = 8.5 Hz, 1H), 6.98 (d, *J* = 1.5 Hz, 1H), 6.94 - 6.90 (m, 1H), 4.40 (dt, *J* = 4.9, 9.1 Hz, 1H), 4.25 (dd, *J* = 3.0, 13.8 Hz, 1H), 3.86 - 3.78 (m, 1H), 3.71 (s, 3H), 3.59 (dd, *J* = 9.8, 13.6 Hz, 1H), 3.11 (d, *J* = 1.0 Hz, 6H), 2.77 (br s, 1H), 2.57 (s, 3H), 2.24 - 2.12 (m, 1H), 2.10 - 1.99 (m, 1H), 1.92 - 1.82 (m, 1H), 1.48 - 1.38 (m, 1H), 0.84 (d, *J* = 6.5 Hz, 3H); LCMS m/z = 522.1 $[M+H]^+$.

**Example 7**

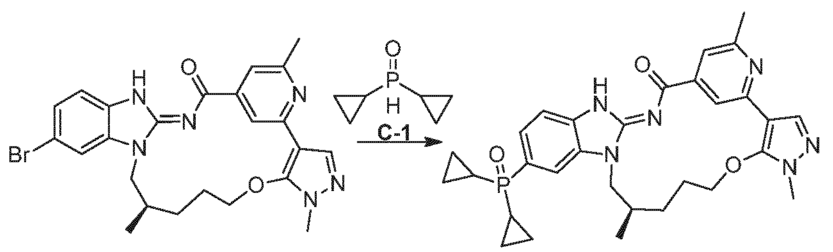


1-8

WX-007

Step 1: Synthesis of compound **WX-007** trifluoroacetate

To a solution of compound **1-8** (40 mg, 78.52 μmol, 1 *eq*), **C-1** (102.18 mg, 785.24 μmol, 10 *eq)* in 1,4-dioxane (3 mL) were added 4,5-bisdiphenylphosphino-9,9-dimethylxanthene (27.26 mg, 47.11 μmol, 0.6 *eq*), *N,N*-diisopropylethylamine (50.74 mg, 392.62 μmol, 5 *eq*). The atmosphere was replaced with nitrogen, and tris(dibenzylideneacetone)dipalladium (21.57 mg, 23.56 μmol, 0.3 *eq*) was added. The mixture was stirred at 120 °C under nitrogen for 20 hours. The reaction solution was concentrated under reduced pressure to obtain a crude product. The crude product was separated by preparative HPLC, column: Welch Xtimate C18 100*40mm*3μm; mobile phase: [water (trifluoroacetic acid)-acetonitrile]; gradient: acetonitrile%: 16%-46%,8 minutes, to obtain **WX-007** trifluoroacetate. $^1H$ NMR (400MHz, $CD_3OD$) δ = 8.80 (s, 1H), 8.23 (s, 1H), 8.05 - 7.94 (m, 2H), 7.76 (dd, J = 8.7, 10.7 Hz, 1H), 7.61 (dd, J = 2.0, 8.0 Hz, 1H), 4.51 (td, J = 4.7, 9.0 Hz, 1H), 4.40 (br d, J = 12.0 Hz, 1H), 4.11 (br s, 1H), 4.01 (br dd, J = 10.8, 13.3 Hz, 1H), 3.81 (s, 3H), 2.78 (s, 4H), 2.33 (br d, J = 7.0 Hz, 1H), 2.11 - 1.97 (m, 2H), 1.66 - 1.53 (m, 1H), 1.49 - 1.27 (m, 2H), 1.17 - 0.78 (m, 11H); LCMS m/z = 559.1 $[M+H]^+$.

**Example 8**

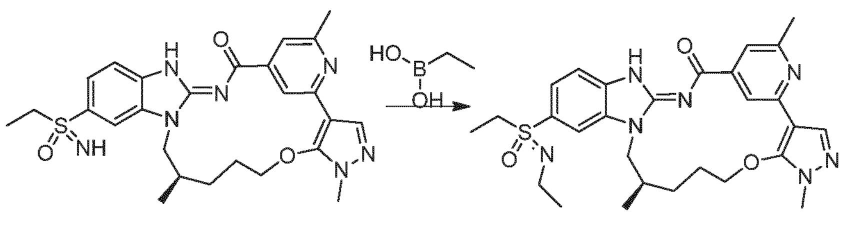

WX-003 WX-008

Step 1: Synthesis of compound **WX-008** trifluoroacetate

To a solution of **WX-003**(20.00 mg, 38.34 μmol, 1 *eq)* in 1,4-dioxane (3 mL) were added copper acetate (10.45 mg, 57.51 μmol, 1.5 *eq*), pyridine (7.28 mg, 92.02 μmol, 7.43 μL, 2.4 *eq*) and ethylboronic acid (8.50 mg, 115.02 μmol, 3 *eq*), and the mixture was stirred at 100 °C for 4 hours under nitrogen. The reaction solution was concentrated under reduced pressure to obtain a crude product. The crude product was separated by preparative HPLC, column: Welch Xtimate C18 100*40mm*3μm; mobile phase: [water (trifluoroacetic acid)-acetonitrile]; gradient: acetonitrile%: 17%-47%,8 minutes, to obtain **WX-008** trifluoroacetate. $^1H$ NMR (400MHz, $CD_3OD$) δ = 9.01 - 8.91 (m, 1H), 8.38 - 8.22 (m, 2H), 8.14 - 8.07 (m, 1H), 8.01 - 7.86 (m, 2H), 4.60 - 4.52 (m, 1H), 4.48 - 4.39 (m, 1H), 4.22 - 4.07 (m, 2H), 4.05 - 3.92 (m, 2H), 3.91 - 3.81 (m, 3H), 3.78 - 3.60 (m, 2H), 2.98 - 2.79 (m, 4H), 2.43 - 2.34 (m, 1H), 2.14 - 2.02 (m, 2H), 1.68 - 1.59 (m, 1H), 1.39 - 1.30 (m, 6H), 1.00 - 0.91 (m, 3H); LCMS m/z = 550.1 $[M+H]^+$.

**Example 9**

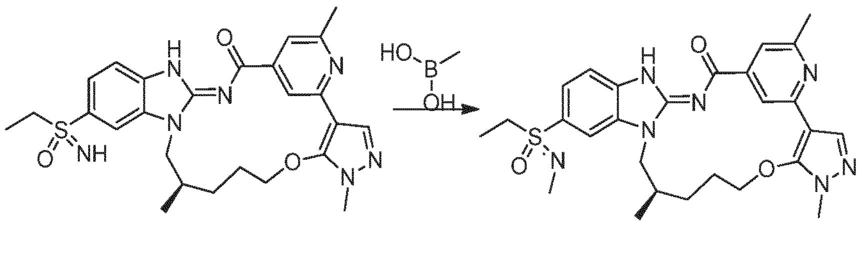

WX-003 WX-009

Step 1: Synthesis of compound **WX-009** trifluoroacetate

To a solution of **WX-003** (30.00 mg, 57.51 μmol, 1 *eq*) in 1,4-dioxane (3 mL) were added copper acetate (15.67 mg, 86.27 μmol, 1.5 *eq*), pyridine (10.92 mg, 138.03 μmol, 11.14 μL, 2.4 *eq)* and methylboronic acid (10.33 mg, 172.54 μmol, 3 *eq*), and the mixture was stirred at 100 °C for 4 hours under nitrogen. The reaction solution was concentrated under reduced pressure to obtain a crude product. The crude product was separated by preparative HPLC, column: Welch Xtimate C18 100*40mm*3μm; mobile phase: [water (trifluoroacetic acid)-acetonitrile]; gradient: acetonitrile%: 9%-39%,10minutes, to obtain **WX-009** trifluoroacetate. $^1H$ NMR (400MHz, $CD_3OD$) δ = 8.91 (br s, 1H), 8.28 (br s, 1H), 8.17 (s, 1H), 8.06 (br s, 1H), 7.94 - 7.84 (m, 2H), 4.56 (br s, 1H), 4.43 (br d, J = 9.5 Hz, 1H), 4.16 (br s, 1H), 4.24 - 4.03 (m, 1H), 3.92 - 3.76 (m, 5H), 2.91 (br s, 1H), 2.86 - 2.78 (m, 1H), 2.78 (br s, 1H), 2.97 - 2.77 (m, 3H), 2.94 - 2.75 (m, 1H), 2.43 - 2.32 (m, 1H), 2.17 - 2.01 (m, 2H), 1.68 - 1.58 (m, 1H), 1.36 - 1.33 (m, 3H), 0.95 (d, J = 6.5 Hz, 3H); LCMS m/z = 536.1 $[M+H]^+$.

**Example 10**

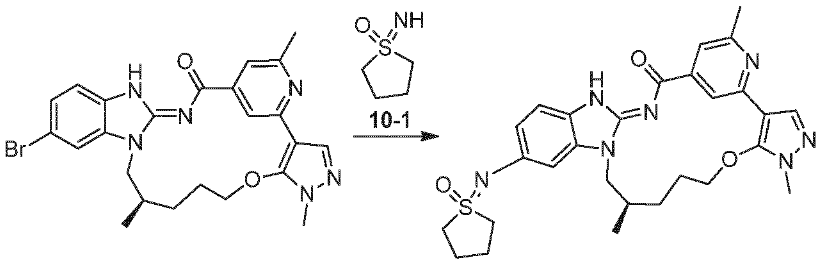


1-8 WX-010

Step 1: Synthesis of compound **WX-010** trifluoroacetate

To a solution of compound **1-8** (50.00 mg, 98.16 μmol, 1 *eq*), **10-1** (23.40 mg, 196.31 μmol, *2eq)* in 1,4-dioxane (3 mL) were added 2-(di-tert-butylphosphino)biphenyl (8.79 mg, 29.45 μmol, 0.3 *eq*), and a solution of sodium tert-butoxide in tetrahydrofuran (2 M, 196.31 μmol, 98.16 μl, 2 *eq).* The atmosphere was replaced with nitrogen, and tris(dibenzylideneacetone)dipalladium (26.96 mg, 29.45 μmol, 0.3 *eq*) was added. The mixture was stirred at 80 °C under nitrogen for 12 hours. The reaction solution was concentrated under reduced pressure to obtain a crude product. The crude product was separated by preparative HPLC, column: Welch Xtimate C18 100*40mm*3μm; mobile phase: [water (trifluoroacetic acid)-acetonitrile]; gradient: acetonitrile%: 26%-56%,8minutes, to obtain **WX-010** trifluoroacetate. $^1$H NMR (400MHz, $CD_3OD$) δ = 8.75 (s, 1H), 8.24 (s, 1H), 7.87 (s, 1H), 7.31 (br d, J = 8.3 Hz, 1H), 7.03 - 6.90 (m, 2H), 4.53 - 4.41 (m, 1H), 4.30 (br d, J = 13.3 Hz, 1H), 4.08 (br s, 1H), 3.82 (s, 3H), 3.73 (br t, J = 11.8 Hz, 1H), 3.50 - 3.37 (m, 2H), 3.32 - 3.20 (m, 2H), 2.87 - 2.64 (m, 4H), 2.46 - 2.20 (m, 5H), 2.04 (br s, 2H), 1.62 - 1.48 (m, 1H), 0.87 (br d, J = 6.5 Hz, 3H); LCMS m/z = 548.1 $[M+H]^+$.

**Example 11**

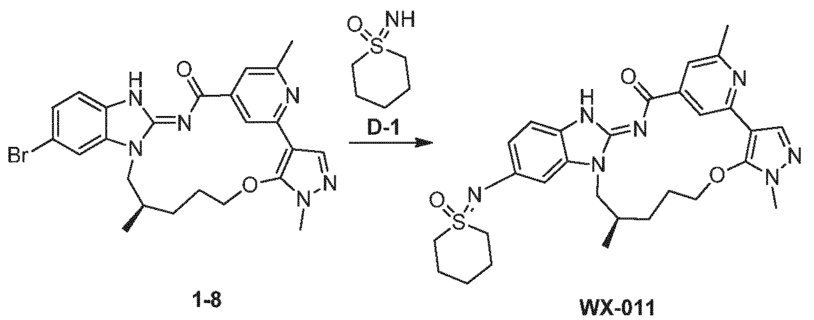


1-8 WX-011

Step 1: Synthesis of compound **WX-011**

To a solution of compound **1-8** (50 mg, 98.16 μmol, 1 *eq*), **D-1** (26.15 mg, 196.32 μmol, 2 *eq)* in 1,4-dioxane (3 mL) were added 2-(di-tert-butylphosphino)biphenyl (17.57 mg, 58.90 μmol, 0.6 *eq),* and a solution of sodium tert-butoxide in tetrahydrofuran (2 M, 147.24 μmol, 73.62 μl, 1.5 *eq).* The atmosphere was replaced with nitrogen, and tris(dibenzylideneacetone)dipalladium (26.96 mg, 29.45 umol, 0.3 *eq)* was added. The mixture was stirred at 80 °C under nitrogen for 12 hours. The mixture was cooled to room temperature, and 10 mL of water was added. The mixture was extracted with 50 mL of dichloromethane, and then washed once with 10 mL of saturated sodium chloride solution. The organic phase was dried over anhydrous sodium sulfate, filtered, and concentrated in vacuo to obtain a crude product. The crude product was separated and purified by silica gel column chromatography (gradient elution: dichloromethane/methanol = 100:0~100:3) to obtain **WX-011.** $^1$H NMR (400MHz, $CDCl_3$) δ = 11.89 (br s, 1H), 8.50 (s, 1H), 8.18 (s, 1H), 7.68 (s, 1H), 7.22 (d, J = 8.4 Hz, 1H), 7.11 (d, J = 1.6 Hz, 1H), 7.06 (dd, J = 1.8, 8.4 Hz, 1H), 4.81 (br s, 1H), 4.54 - 4.45 (m, 1H), 4.34 (dd, J = 3.0, 14.1 Hz, 1H), 3.96 - 3.86 (m, 1H), 3.81 (s, 3H), 3.74 - 3.63 (m, 1H), 3.38 (br s, 2H), 3.20 - 3.07 (m, 2H), 2.86 (br s, 1H), 2.67 (s, 3H), 2.28 (br s, 2H), 2.18 - 2.03 (m, 5H), 1.97 (br d, J = 5.0 Hz, 1H), 1.82 (br d, J = 14.6 Hz, 1H), 0.94 (d, J = 6.6 Hz, 3H); LCMS m/z = 562.1 $[M+H]^+$.

**Example 12**

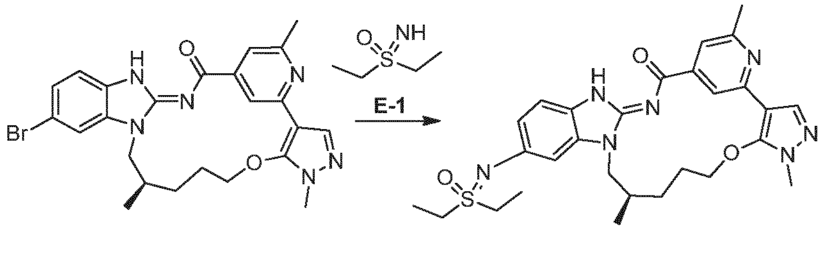


1-8 WX-012

Step 1: Synthesis of compound **WX-012** hydrochloride

To a solution of compound **1-8** (30.00 mg, 58.89 μmol, 1 *eq*), **E-1** (14.28 mg, 117.79 pmol, *2 eq*) in 1,4-dioxane (3 mL) were added 2-(di-tert-butylphosphino)biphenyl (5.27 mg, 17.67 μmol, 0.3 *eq*), a solution of sodium tert-butoxide in tetrahydrofuran (2 M, 88.34 μmol, 44.17 μl, 1.5 *eq*). The atmosphere was replaced with nitrogen, and tris(dibenzylideneacetone)dipalladium (16.18 mg, 17.67 μmol, 0.3 *eq*) was added. The mixture was stirred at 80 °C under nitrogen for 12 hours. The reaction solution was concentrated under reduced pressure to obtain a crude product. The crude product was separated by preparative HPLC, column: Welch Xtimate C18 150*40mm*5 μm; mobile phase: [water (hydrochloric acid)-acetonitrile]; gradient: acetonitrile%: 10%-40%,10minutes, to obtain **WX-012** hydrochloride. $^1$H NMR (400MHz, $CD_3OD$) δ = 8.85 (s, 1H), 8.32 (s, 1H), 7.99 (s, 1H), 7.35 (d, J = 8.5 Hz, 1H), 7.16 (d, J = 1.8 Hz, 1H), 7.05 (dd, J = 1.8, 8.5 Hz, 1H), 4.57 - 4.47 (m, 1H), 4.32 (dd, J = 2.6, 13.7 Hz, 1H), 4.18 - 4.10 (m, 1H), 3.86 - 3.76 (m, 4H), 3.41 (quin, J = 7.2 Hz, 4H), 2.86 (s, 3H), 2.80 - 2.70 (m, 1H), 2.43 - 2.27 (m, 1H), 2.13 - 2.00 (m, 2H), 1.64 - 1.51 (m, 1H), 1.43 (q, J = 7.3 Hz, 6H), 0.94 - 0.84 (m, 3H); LCMS m/z = 550.2 $[M+H]^+$.

**Example 13**

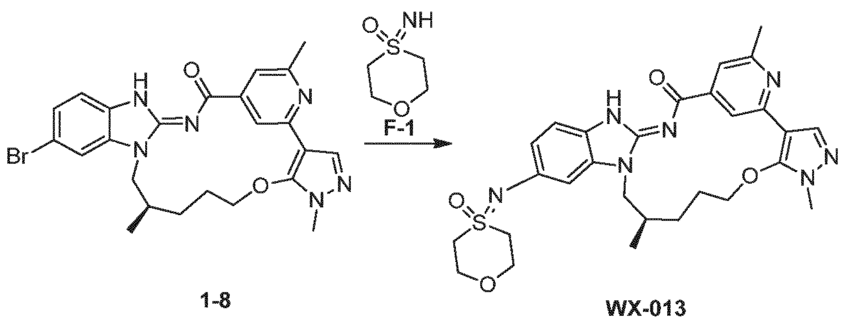


1-8 WX-013

Step 1: Synthesis of compound **WX-013**

To a solution of compound **1-8** (50 mg, 98.16 μmol, 1, 1 *eq*), **F-1** (26.54 mg, 196.31 μmol, *2 eq*) in 1,4-dioxane (3 mL) were added 2-(di-tert-butylphosphino)biphenyl (17.57 mg, 58.89 μmol, 0.6 *eq*), a solution of sodium tert-butoxide in tetrahydrofuran (2 M, 147.24 μmol, 73.62 μl, 1.5 *eq*). The atmosphere was replaced with nitrogen, and tris(dibenzylideneacetone)dipalladium (26.96 mg, 29.45 μmol, 0.3 *eq*) was added. The mixture was stirred at 80 °C under nitrogen for 12 hours. The mixture was cooled to room temperature, and 10 mL of water was added. The mixture was extracted with 50 mL of dichloromethane, and then washed once with 10 mL of saturated sodium chloride solution. The organic phase was dried over anhydrous sodium sulfate, filtered, and concentrated in vacuo to obtain a crude product. The crude product was separated and purified by silica gel column chromatography (gradient elution: dichloromethane/methanol = 100:0~100:3) to obtain **WX-013.** $^1$H NMR (400MHz, $CDCl_3$) δ = 11.84 (br s, 1H), 8.40 (s, 1H), 8.08 (s, 1H), 7.59 (s, 1H), 7.14 (d, J = 8.3 Hz, 1H), 7.01 (d, J = 1.5 Hz, 1H), 6.95 (dd, J = 1.8, 8.3 Hz, 1H), 4.40 (dt, J = 5.1, 9.1 Hz, 1H), 4.25 (dd, J = 2.8, 13.6 Hz, 1H), 4.18 - 4.10 (m, 2H), 4.09 - 4.01 (m, 2H), 3.87 - 3.78 (m, 1H), 3.71 (s, 3H), 3.60 (dd, J = 9.8,

13.6 Hz, 1H), 3.39 - 3.30 (m, 2H), 3.27 - 3.17 (m, 2H), 2.77 (br s, 1H), 2.57 (s, 3H), 2.26 - 2.11 (m, 1H), 2.05 (dt, J = 4.9, 9.5 Hz, 1H), 1.92 - 1.82 (m, 1H), 1.49 - 1.39 (m, 1H), 0.84 (d, J = 6.5 Hz, 3H); LCMS m/z = 564.1 $[M+H]^+$.

**Example 14**

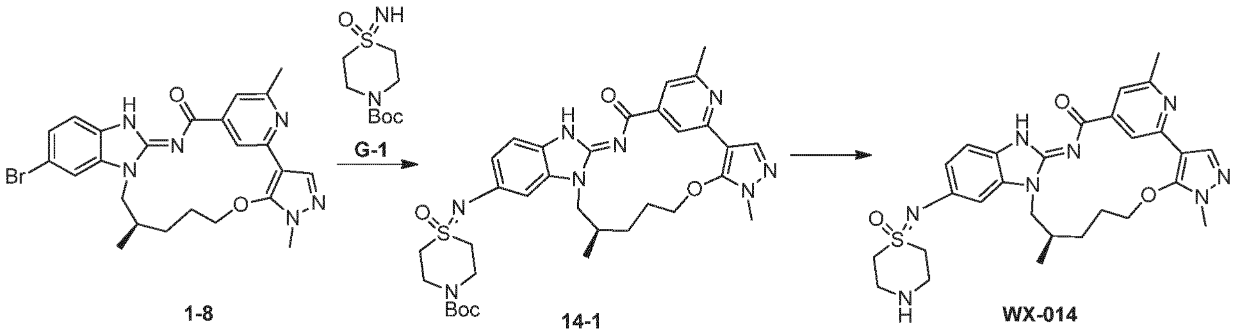


1-8 14-1 WX-014

Step 1: Synthesis of compound **14-1**

To a solution of compound **1-8** (100.00 mg,196.31 μmol, 1 *eq*), **G-1** (92.00 mg, 392.62 μmol, 2 *eq*) in 1,4-dioxane (5 mL) were added 2-(di-tert-butylphosphino)biphenyl (70.30 mg, 235.57 μmol, 1.2 *eq*), a solution of sodium tert-butoxide in tetrahydrofuran (2 M, 500 μmol, 250 μl, 1.5 *eq*). The atmosphere was replaced with nitrogen, and tris(dibenzylideneacetone)dipalladium (71.91 mg, 78.52 μmol, 0.4 *eq*) was added. The mixture was stirred at 80 °C under nitrogen for 16 hours. The mixture was cooled to room temperature, and 10 mL of water was added. The mixture was extracted with 50 mL of dichloromethane, and then washed once with 10 mL of saturated sodium chloride solution. The organic phase was dried over anhydrous sodium sulfate, filtered, and concentrated in vacuo to obtain a crude product. The crude product was separated and purified by silica gel column chromatography (gradient elution: dichloromethane/methanol = 100:0-100:3) to obtain compound **14-1.** LCMS m/z = 663.2 $[M+H]^+$.

Step 2: Synthesis of compound **WX-014**

Compound **14-1** (40.00 mg, 60.35 μmol, 1 *eq*) was dissolved in ethyl acetate (1 mL). Hydrochloric acid/ethyl acetate solution (4 M, 1 mL, 66.28 *eq*) was added, and the mixture was stirred at 25 °C for 1 hour. The reaction solution was concentrated, and the residue was dissolved in 10 mL of water. The solution was adjusted to pH of 8 with aqueous saturated sodium bicarbonate solution. The mixture was extracted three times with 15 mL of dichloromethane. The organic phase was separated, dried over anhydrous sodium sulfate, filtered, and concentrated in vacuum to obtain compound **WX-014.** $^1$H NMR (400MHz, $CDCl_3$) δ = 11.86 (br s, 1 H), 8.40 (s, 1 H), 8.08 (s, 1 H), 7.59 (s, 1 H), 7.12 (d, J = 8.53 Hz, 1 H), 7.01 (d, J = 1.51 Hz, 1 H), 6.95 (dd, J = 8.41, 1.63 Hz, 1 H), 4.40 (td, J = 9.16, 5.02 Hz, 1 H), 4.24 (dd, J= 13.55, 3.01 Hz, 1 H), 3.78 - 3.84 (m, 1 H), 3.71 (s, 3 H), 3.59 (dd, J = 13.55, 9.79 Hz, 1 H), 3.23 - 3.34 (m, 6 H), 3.05 - 3.15 (m, 2 H), 2.72 - 2.83 (m, 1 H), 2.56 (s, 3 H), 2.13 - 2.21 (m, 1 H), 2.01 - 2.09 (m, 1 H), 1.90 - 1.80 (m, 1 H), 1.37 - 1.48 (m, 1 H), 0.83 (s, 3 H); LCMS m/z = 563.2 $[M+H]^+$.

**Example 15**

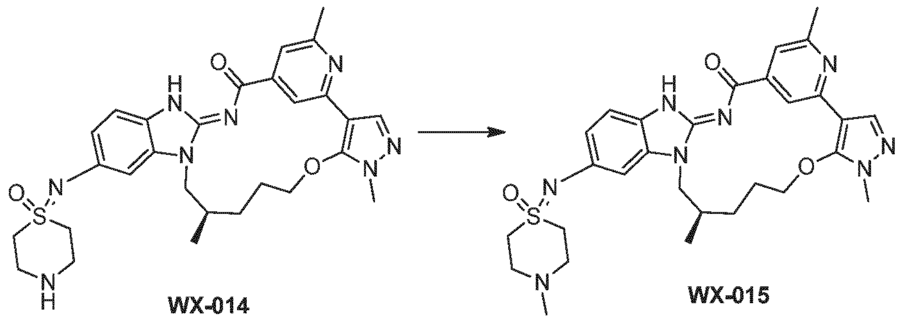

Step 1: Synthesis of compound **WX-015**

**WX-014** (20 mg, 35.54 μmol, 1 *eq*) was dissolved in anhydrous methanol (2 mL) and acetic acid (0.06 mL) was added. Aqueous formaldehyde solution (5.77 mg, 71.09 μmol, 37%, 2 eq) and sodium cyanoborohydride (5.58 mg, 88.86 μmol, 2.5 eq) were added, and the mixture was stirred at 25 °C for 3 hours. The reaction solution was separated by preparative HPLC, column: Welch Xtimate C18 150*40mm*5 μm; mobile phase: [water (ammonium bicarbonate)-acetonitrile]; gradient: acetonitrile%: 25%-55%,10minutes, to obtain **WX-015**. $^1$H NMR (400MHz, $CD_3OD$) δ = 8.99 (s, 1H), 8.34 (s, 1H), 8.12 (s, 1H), 7.45 (d, J = 8.5 Hz, 1H), 7.30 (s, 1H), 7.14 (br d, J = 7.0 Hz, 1H), 4.57 (d, J = 4.8 Hz, 1H), 4.35 (br d, J = 11.0 Hz, 1H), 4.19 (br s, 1H), 3.98 - 3.80 (m, 12H), 3.10 (s, 3H), 2.89 (s, 4H), 2.42 - 2.29 (m, 1H), 2.15 - 2.05 (m, 2H), 1.62 (br s, 1H), 0.99 - 0.92 (m, 3H); LCMS m/z = 577.2 [M+H]$^+$.

**Example 16**

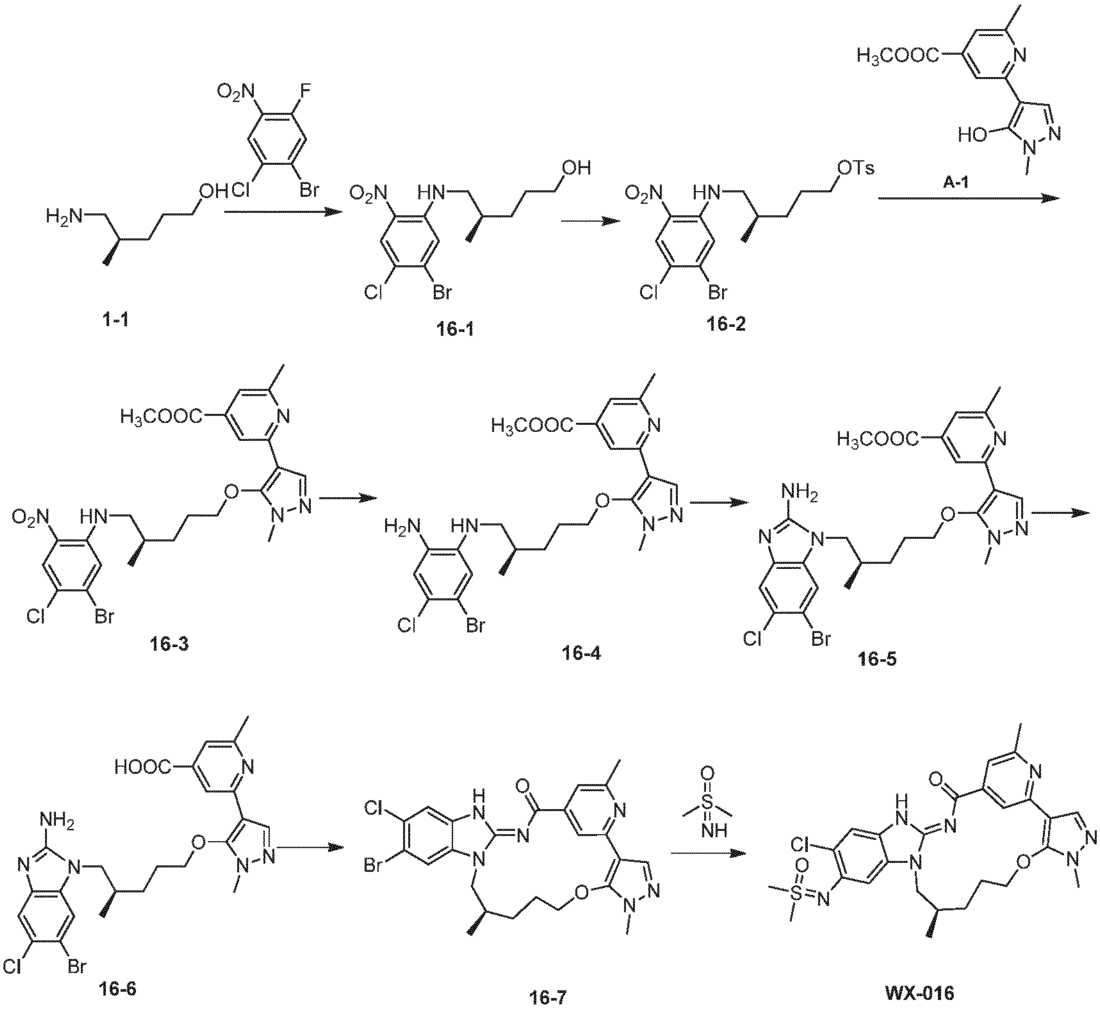


1-1 16-1 16-2 16-3 16-4 16-5 16-6 16-7 WX-016

Step 1: Synthesis of compound **16-1**

Compound 1-bromo-2-chloro-5-fluoro-4-nitrobenzene (4 g, 15.72 mmol, 1 *eq)* was dissolved in absolute ethanol (40 mL). Compound **1-1** (2.42 g, 15.72 mmol, 1 *eq)* and N,N-diisopropylethylamine (6.10 g, 47.16 mmol, 3 *eq*) were added at 25°C, and the mixture was stirred at 60°C for 12 hours. The reaction solution was concentrated under reduced pressure, then dissolved in 60 mL of dichloromethane. The solution was washed twice with saturated brine. The organic phase was collected, dried over anhydrous sodium sulfate, filtered, and concentrated to obtain compound **16-1.** LCMS m/z = 350.9/352.9 $[M+1]^+$.

Step 2: Synthesis of compound **16-2**

Compound **16-1**(5.68 g, 16.15 mmol, 1 *eq*) was dissolved in dichloromethane (60 mL). Triethylamine (4.90 g, 48.46 mmol, 3 *eq*) and p-toluenesulfonyl chloride (2.28 g, 32.31 mmol, 2 *eq*) were added at 0°C, and the mixture was stirred at 25 °C for 16 hours. The reaction solution was washed once with 30 mL of saturated brine. The organic phase was dried over anhydrous sodium sulfate, and concentrated. 30 mL of methanol was added, and the mixture was stirred for 1 hour. A solid was precipitated. The mixture was filtered to obtain compound **16-2.** LCMS m/z = 504.9/506.9 $[M+1]^+$.

Step 3: Synthesis of compound **16-3**

Compound **16-2** (5.7 g, 11.27 mmol, 1 *eq*) and compound **A-1** (3.62 g, 14.65 mmol, 1.3 *eq*) were dissolved in *N,N*-dimethylformamide (60 mL). Potassium carbonate (4.67 g, 33.81 mmol, *3 eq*) was added, and the mixture was stirred at 60 °C for 12 hours. Then, the reaction solution was extracted with 100 mL of water and 100 mL of ethyl acetate. The aqueous layer was further extracted with 60 mL of ethyl acetate. The organic phases were combined, washed twice with saturated brine, dried over anhydrous sodium sulfate, filtered and concentrated. The crude product was separated

by silica gel column chromatography to obtain compound **16-3.** LCMS m/z = 580.0/582.0 $[M+1]^+$.

Step 4: Synthesis of compound **16-4**

Compound **16-3** (3 g, 5.16 mmol, 1 *eq*), ferric chloride hexahydrate (209.40 mg, 774.72 μmol, 0.15 *eq*), and activated carbon (371.86 mg, 30.99 mmol, 6 *eq*) were dissolved in THF (30 mL). Hydrazine hydrate (6.58 g, 131.44 mmol, 25.45 *eq*) was added, and the mixture was stirred at 50°C under nitrogen for 2.5 hours. Then 60 mL of ethyl acetate was added. The mixture was filtered through diatomaceous earth, and the filtrate was collected. The filtrate was washed once with saturated brine, dried over anhydrous sodium sulfate, filtered and concentrated. The crude product was separated by silica gel column chromatography to obtain compound **16-4.** LCMS m/z = 550.0/552.0$[M+1]^+$.

Step 5: Synthesis of compound **16-5**

Compound **16-4** (4.42 g, 8.02 mmol, 1 *eq*) was dissolved in a mixed solution of dichloromethane (45 mL) and tert-butyl alcohol (9 mL). Cyanogen bromide (4.94 g, 46.64 mmol, 5.81 *eq)* was added, and the mixture was stirred at room temperature 25°C for 16 hours. 60 mL of dichloromethane was added to the reaction solution, and the mixture was washed once with 40 mL of saturated aqueous sodium bicarbonate solution, and once with saturated brine. The organic phase was dried over anhydrous sodium sulfate, filtered, and concentrated to obtain compound **16-5.** LCMS m/z = 575.0/577.0$[M+1]^+$.

Step 6: Synthesis of compound **16-6**

Compound **16-5** (744.53 mg, 1.29 mmol, 1 *eq*) was dissolved in a mixed solution of tetrahydrofuran (7 mL) and methanol (5 mL). An aqueous solution of sodium hydroxide (1 M, 5.17 mL, 4 *eq*) was added, and the mixture was stirred at 25°C for 2 hours. The reaction solution was concentrated, and 5mL of water was added. The mixture was adjusted to pH of 5-6 with 6 M hydrochloric acid, and a solid was precipitated. The mixture was filtered to obtain compound **16-6.** LCMS m/z = 561.0/563.0$[M+1]^+$.

Step 7: Synthesis of compound **16-7**

Compound **16-6** (0.6 g, 1.07 mmol, 1 *eq*) was dissolved in *N,N*-dimethylformamide (20 mL). Triethylamine (540.30 mg, 5.34 mmol, 743.19 μL, 5 *eq*) and O-benzotriazol-*N,N,N',N'*-tetramethyluronium tetrafluoroborate (1.03 g, 3.20 mmol, 3 *eq*) were added, and the mixture was stirred at 25°C for 1.5 hours. The mixture was extracted with 15 mL of saturated brine and 20 mL of ethyl acetate, then washed three times with saturated brine, dried over anhydrous sodium sulfate, filtered, and concentrated to obtain a crude product. The crude product was separated by silica gel column chromatography to obtain compound **16-7.** LCMS m/z = 543.0/545.0$[M+1]^+$.

Step 8: Synthesis of compound **WX-016**

Compound **16-7** (0.1 g, 183.88 μmol, 1 *eq*), S,S-dimethyl sulfoximine (17.13 mg, 183.88μmol, 1.0 *eq*) were dissolved in a mixed solution of 1,4-dioxane (2.5 mL) and dichloromethane (1 mL). 2-(Di-tert-butylphosphino)biphenyl (16.46 mg, 55.16 μmol, 0.3 eq), a solution of sodium tert-butoxide in tetrahydrofuran (2 M, 183.88 μL, 2 *eq*), and tris(dibenzylideneacetone)dipalladium(25.26 mg, 27.58 μmol, 0.15 *eq*) were added, and the mixture was stirred at 80 °C for 14 hours. The reaction solution was separated by preparative HPLC, column: Welch Xtimate C18 100*40mm*3μm; mobile phase: [water (trifluoroacetic acid)-acetonitrile]; acetonitrile%: 12%-42%,8 minutes. Then 30 mL of dichloromethane was added, and the mixture was washed once with saturated sodium bicarbonate solution and once with saturated sodium chloride solution, dried over anhydrous sodium sulfate, and concentrated to obtain compound **WX-016.** $^1$H NMR (400MHz, $CDCl_3$) δ = 12.09 - 11.62 (m, 1H), 8.39 (br s, 1H), 8.08 (br s, 1H), 7.58 (br s, 1H), 7.30 (br s, 1H), 7.14 (s, 1H), 4.39 (br d, J = 3.8 Hz, 1H), 4.23 (br d, J = 12.3 Hz, 1H), 3.81 (br s, 1H), 3.71 (s, 3H), 3.63 - 3.55 (m, 1H), 3.12 (br d, J = 17.3 Hz, 6H), 2.84 - 2.67 (m, 1H), 2.56 (br s, 3H), 2.25 - 2.10 (m, 1H), 2.09 - 1.96 (m, 1H), 1.92 - 1.81 (m, 1H), 1.43 - 1.36 (m, 1H), 0.83 (br d, J = 6.1 Hz, 3H). LCMS *m/z*:556.0 $[M+H]^+$.

**Example 17**

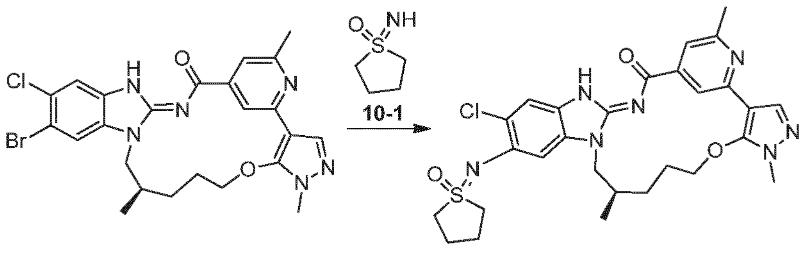


16-7 WX-017

Step 1: Synthesis of compound **WX-017**

Compound **16-7** (0.05 g, 91.94 μmol, 1 *eq*) and compound **10-1**(10.96 mg, 91.94 μmol, 1 *eq)* were dissolved in 1,4-dioxane (3 mL). 2-(Di-tert-butylphosphino)biphenyl(8.23 mg, 27.58 μmol, 0.3 *eq*), a solution of sodium tert-butoxide in tetrahydrofuran (2 M, 137.91 μL, 3 *eq*), tris(dibenzylideneacetone)dipalladium(12.63 mg, 13.79 μmol, 0.15 *eq*) were added, and the mixture was stirred at 80 °C for 14 hours. The reaction solution was separated by preparative HPLC, column: Welch Xtimate C18 100*40mm*3μm; mobile phase: [water (trifluoroacetic acid)-acetonitrile]; acetonitrile%: 18%-48%, 8 minutes. Then 30 mL of dichloromethane was added, and the mixture was washed once with saturated sodium bicarbonate solution and once with saturated sodium chloride solution, dried over anhydrous sodium sulfate, and concentrated to obtain compound **WX-017**. $^1H$ NMR (400MHz, $CDCl_3$) δ = 12.23 - 11.60 (m, 1H), 8.38 (s, 1H), 8.07 (s, 1H), 7.57 (s, 1H), 7.29 (s, 1H), 7.14 (s, 1H), 4.42 - 4.32 (m, 1H), 4.22 (br dd, J = 2.6, 13.4 Hz, 1H), 3.85 - 3.77 (m, 1H), 3.71 (s, 3H), 3.57 (br dd, J = 9.9, 13.4 Hz, 1H), 3.39 - 3.29 (m, 2H), 3.16 - 2.99 (m, 2H), 2.80 - 2.65 (m, 1H), 2.54 (s, 3H), 2.32 - 2.22 (m, 4H), 2.20 - 2.11 (m, 1H), 2.07 - 1.95 (m, 1H), 1.86 (ddd, J = 4.4, 9.5, 13.9 Hz, 1H), 1.49 - 1.37 (m, 1H), 0.82 (d, J = 6.5 Hz, 3H). LCMS *m/z*: 582.0 $[M+H]^+$.

**Example 18**

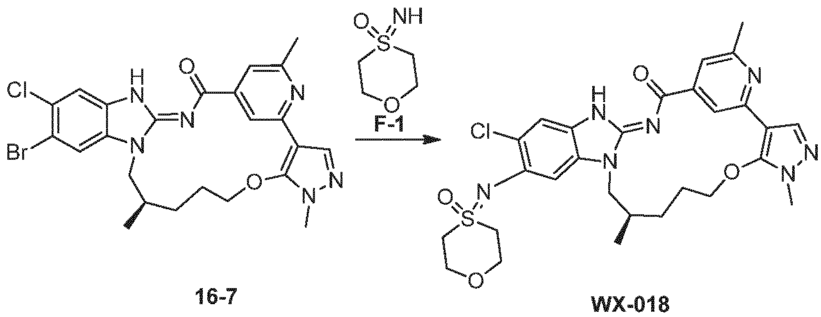


16-7 WX-018

Step 1: Synthesis of compound **WX-018**

Compound **16-7** (0.05 g, 91.94 μmol, 1 *eq*) and compound **F-1**(12.43 mg, 91.94 μmol, 1 *eq)* were dissolved in 1,4-dioxane (3 mL). 2-(Di-tert-butylphosphino)biphenyl(8.23 mg, 27.58 μmol, 0.3 *eq*), a solution of sodium tert-butoxide in tetrahydrofuran (2 M, 183.88 μL, 4 *eq*), tris(dibenzylideneacetone)dipalladium(12.63 mg, 13.79 μmol, 0.15 *eq*) were added, and the mixture was stirred at 80 °C for 14 hours. The reaction solution was filtered through diatomaceous earth, concentrated, and purified by silica gel column chromatography to obtain compound **WX-018.** $^1H$ NMR (400MHz, $CDCl_3$) δ = 12.31 - 11.31 (m, 1H), 8.39 (s, 1H), 8.08 (s, 1H), 7.57 (s, 1H), 7.30 (s, 1H), 7.20 (s, 1H), 4.39 (dt, J = 5.1, 9.1 Hz, 1H), 4.29 - 4.06 (m, 5H), 3.85 - 3.75 (m, 1H), 3.71 (s, 3H), 3.69 - 3.54 (m, 2H), 3.36 (br s, 1H), 3.26 - 3.12 (m, 2H), 2.75 (br d, J = 3.4 Hz, 1H), 2.56 (s, 3H), 2.29 - 2.11 (m, 1H), 2.08 - 1.97 (m, 1H), 1.87 (ddd, J = 4.4, 9.5, 14.1 Hz, 1H), 1.47 - 1.35 (m, 1H), 0.82 (d, J = 6.5 Hz, 3H). LCMS *m/z*: 598.1 $[M+H]^+$.

**Example 19**

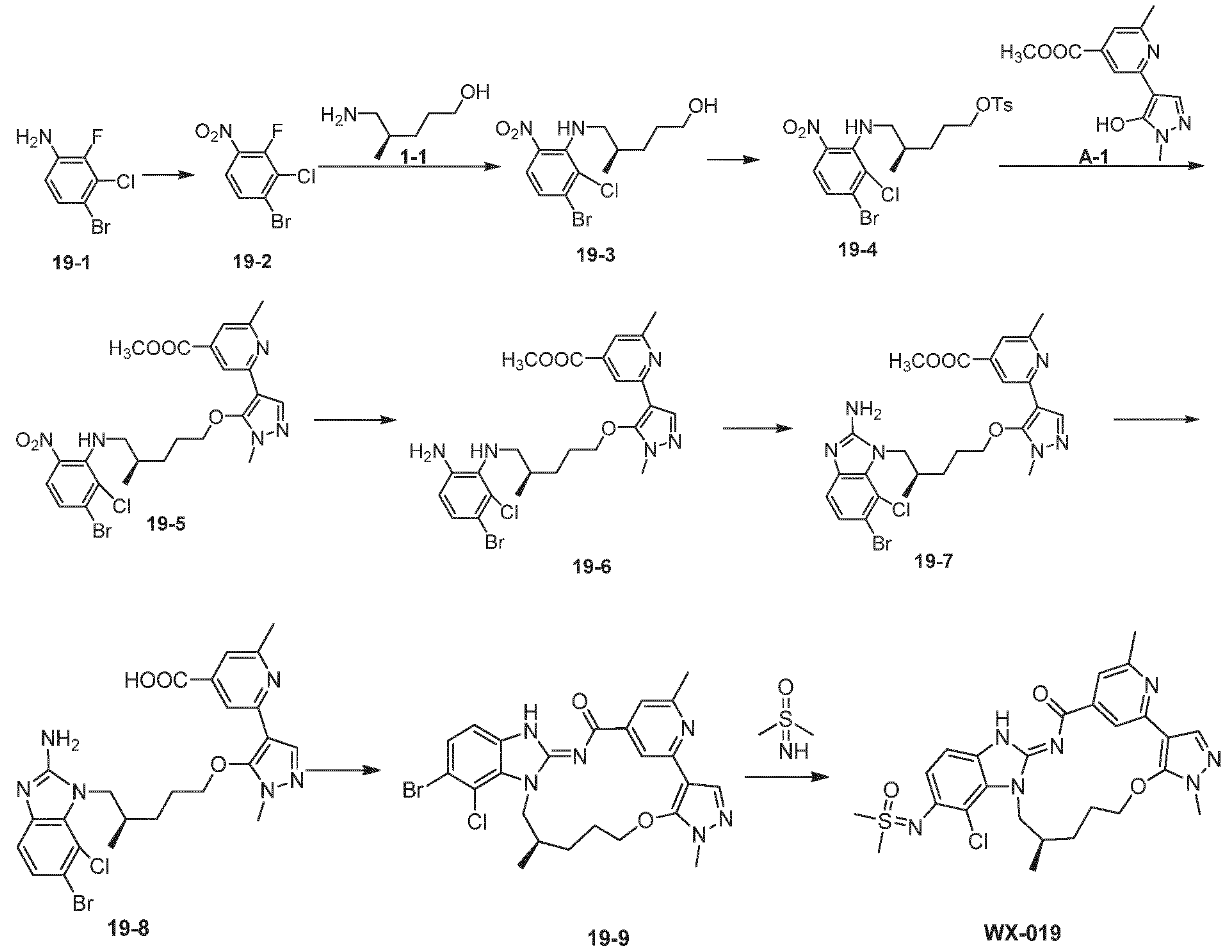


Step 1: Synthesis of compound **19-2**

Compound **19-1** (1 g, 4.46 mmol, 1 *eq*) was dissolved in toluene (15 mL). m-Chloroperoxybenzoic acid (3.08 g, 17.82 mmol, 4 *eq*) was added, and the mixture was stirred at 50 °C for 12 hours. Then 20 mL of ethyl acetate was added, and the mixture was filtered. The filtrate was washed once with 20 mL of 10% aqueous sodium thiosulfate solution, twice with saturated brine, and concentrated. The crude product was purified by silica gel column chromatography (ethyl acetate: petroleum ether = 0-30%) to obtain compound **19-2.** $^1H$ NMR (400MHz, $CDCl_3$) $\delta$ = 7.81 (dd, J = 7.2, 8.9 Hz, 1H), 7.55 (dd, J = 2.0, 9.0 Hz, 1H).

Step 2: Synthesis of compound **19-3**

Compound **19-2** (1.6 g, 6.29 mmol, 1 *eq*) was dissolved in absolute ethanol (30 mL). Compound **1-1**(1.16 g, 7.55 mmol, 1.2 *eq*) and N,N-diisopropylethylamine (2.44 g, 18.86 mmol, 3.29 mL, 3 *eq*) were added at 25°C, and the mixture was stirred at 60°C for 12 hours. The reaction solution was concentrated under reduced pressure, then dissolved in 40 mL of dichloromethane. The solution was washed twice with saturated brine. The organic phase was collected, dried over anhydrous sodium sulfate, filtered, and concentrated to obtain compound **19-3.** LCMS m/z = 350.9/352.9 $[M+1]^+$.

Step 3: Synthesis of compound **19-4**

Compound **19-3**(0.82 g, 2.33 mmol, 1 *eq*) was dissolved in dichloromethane (10 mL). Triethylamine (707.94 mg, 7.00 mmol, 3 *eq*) and p-toluenesulfonyl chloride (411.20 mg, 5.83 mmol, 2.5 *eq*) were added at 0°C, and the mixture was stirred at 25 °C for 16 hours. The reaction solution was washed once with saturated brine. The organic phase was dried over anhydrous sodium sulfate. The crude product was purified by silica gel column chromatography (ethyl acetate : petroleum ether = 10-50%) to obtain compound **19-4.** LCMS m/z = 504.9/506.9 $[M+1]^+$.

Step 4: Synthesis of compound **19-5**

Compound **19-4** (0.8 g, 1.58 mmol, 1 *eq*) and compound **A-1**(508.37 mg, 2.06 mmol, 1.3 *eq*) were dissolved in *N,N*-dimethylformamide (15 mL). Potassium carbonate (655.79 mg, 4.74 mmol, 3 *eq*) was added, and the mixture was stirred at 60 °C for 12 hours. The reaction solution was extracted with 20 mL of water and 20 mL of ethyl acetate. The organic phase was washed once with saturated brine, dried over anhydrous sodium sulfate, filtered and concentrated. The crude product was separated and purified by silica gel column chromatography (ethyl acetate : petroleum ether = 0~50%), to obtain compound **19-5.** LCMS m/z = 580.0/582.0 $[M+1]^+$.

Step 5: Synthesis of compound **19-6**

Compound **19-5** (1.6 g, 2.75 mmol, 1 *eq*), ferric chloride hexahydrate (111.68 mg, 413.18 μmol, 0.15 *eq*), and activated carbon (397.01 mg, 33.05 mmol, 12 *eq)* were dissolved in tetrahydrofuran (25 mL). Hydrazine hydrate (8.24 g, 164.60 mmol, 59.76 eq) was added, and the mixture was stirred at 50°C under nitrogen for 2.5 hours. Then, 40 mL of ethyl acetate was added, the mixture was filtered through diatomaceous earth, and the filtrate was collected. The filtrate was washed three times with saturated brine, dried over anhydrous sodium sulfate, and concentrated. The crude product was separated and purified by silica gel column chromatography (ethyl acetate : petroleum ether = 0~70%), to obtain compound **19-6.** LCMS m/z = 550.0/552.0 $[M+1]^+$.

Step 6: Synthesis of compound **19-7**

Compound **19-6** (1.5 g, 2.72 mmol, 1 *eq*) was dissolved in a mixed solution of dichloromethane (15 mL) and tert-butyl alcohol (3 mL), and the mixture was stirred at 25°C. Cyanogen bromide (1.29 g, 12.18 mmol, 4.47 *eq*) was added, and the mixture was stirred at 25°C for 16 hours. The reaction solution was extracted with 30 mL of dichloromethane and 20 mL of saturated sodium bicarbonate aqueous solution, washed once with saturated brine, dried over anhydrous sodium sulfate, filtered, and concentrated to obtain **19-7.** LCMS m/z = 575.0/577.0$[M+1]^+$.

Step 7: Synthesis of compound **19-8**

Compound **19-7** (0.6 g, 1.04 mmol, 1 *eq*) was dissolved in a mixed solution of tetrahydrofuran (7 mL) and methanol (5 mL). An aqueous solution of sodium hydroxide (1 M, 4.17 mL, 4 *eq*) was added, and the mixture was stirred at 25°C for 2 hours. The reaction solution was concentrated, and 5mL of water was added. The mixture was adjusted to pH of 5-6 with 6 M hydrochloric acid, and a solid was precipitated. The mixture was filtered to obtain compound **19-8.** LCMS m/z = 561.0/563.0$[M+1]^+$.

Step 8: Synthesis of compound **19-9**

Compound **19-8** (160.10 mg, 284.94 μmol, 1 *eq*) was dissolved in dichloromethane (20 mL). N,N-diisopropylethylamine (184.13 mg, 1.42 mmol, 5 *eq*) and O-benzotriazol-N,N,N',N'-tetramethyluronium tetrafluoroborate(274.47 mg, 854.82 μmol, 3 *eq*) were added, and the mixture was stirred at 25°C for 1.5 hours. The mixture was extracted with 15 mL of saturated brine and 20 mL of ethyl acetate, then washed three times with saturated brine, dried over anhydrous sodium sulfate, filtered, and concentrated to obtain a crude product. The crude product was separated by silica gel column chromatography (methanol: dichloromethane = 0~3%) to obtain compound **19-9.** LCMS m/z = 543.0/545.0$[M+1]^+$.

Step 9: Synthesis of compound **WX-019**

Compound **19-9** (42.79 mg, 78.68 μmol, 1 *eq*), compound S,S-dimethyl sulfoximine (10.99 mg, 118.01 μmol, 1.5 *eq*) were dissolved in a mixed solution of 1,4-dioxane (2.5 mL) and dichloromethane (1 mL). 2-(Di-tert-butylphosphino)biphenyl (7.04 mg, 23.60 μmol, 0.3 *eq*), a solution of sodium tert-butoxide in tetrahydrofuran (2 M, 183.88 μL, 2 *eq*), tris(dibenzylideneacetone)dipalladium(10.81 mg, 11.80 μmol, 0.15 *eq*) were added, and the mixture was stirred at 80 °C for 14 hours. The reaction solution was filtered through diatomaceous earth and concentrated. The crude product was separated and purified by silica gel column chromatography (methanol:dichloromethane = 0~3%), to obtain compound **WX-019.** $^1$H NMR (400MHz, DMSO-$d_6$) δ = 12.31 - 11.90 (m, 1H), 8.38 (s, 1H), 8.09 (s, 1H), 7.58 (s, 1H), 7.18 - 7.13 (m, 1H), 7.11 - 7.05 (m, 1H), 4.76 (dd, J = 10.3, 13.6 Hz, 1H), 4.38 (dt, J = 4.0, 9.8 Hz, 1H), 4.28 (dd, J = 3.8, 13.6 Hz, 1H), 3.83 - 3.77 (m, 1H), 3.71 (s, 3H), 3.13 (d, J = 10.3 Hz, 6H), 2.89 - 2.78 (m, 1H), 2.56 (s, 3H), 2.28 - 2.17 (m, 1H), 2.10 - 1.99 (m, 1H), 1.84 - 1.76 (m, 1H), 1.51 - 1.41 (m, 1H), 0.89 (d, J = 6.5 Hz, 3H). LCMS *m/z*:556.0 $[M+H]^+$. SFC (column: Chiralpak AS-3, 3 μm, 0.46 cm id × 10cm L; mobile phase: A ($CO_2$) and B (ethanol, containing 0.05% diethylamine); gradient: B% = 5-40%; flow rate: 2.8mL/min; column temperature: 35°C; wavelength: 220 nm; pressure:

1500 psi), retention time = 3.956 minutes, chiral isomer excess 98.8%.

**Example 20**

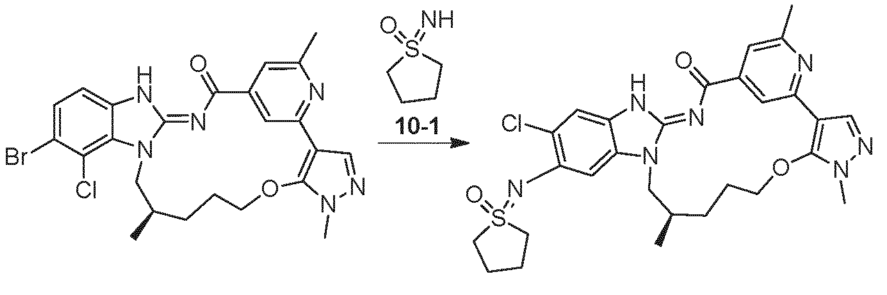


19-9 WX-020

Step 1: Synthesis of compound **WX-020**

Compound **19-9** (0.06 g, 110.33 μmol, 1 *eq*) and compound **10-1** (19.72 mg, 165.49 μmol, 1.5 *eq)* were dissolved in a mixed solution of 1,4-dioxane (3 mL) and dichloromethane (3 mL). 2-(Di-tert-butylphosphino)biphenyl (15.15 mg, 16.55 μmol, 0.15 *eq*), a solution of sodium tert-butoxide in tetrahydrofuran (2 M, 165.49 μL, 3 *eq*), tris(dibenzylideneacetone)dipalladium(9.88 mg, 33.10 μmol, 0.3 *eq*) were added, and the mixture was stirred at 80 °C for 14 hours. The reaction solution was separated by preparative HPLC, column: Welch Xtimate C18 100*40mm*3μm; mobile phase: [water (trifluoroacetic acid)-acetonitrile]; acetonitrile%: 17%-47%,8 minutes. Then 30 mL of dichloromethane was added, and the mixture was washed once with saturated sodium bicarbonate solution and once with saturated sodium chloride solution, dried over anhydrous sodium sulfate, and concentrated to obtain compound **WX-020**. $^1$H NMR (400MHz, $CDCl_3$) δ = 12.13 (br s, 1H), 8.37 (s, 1H), 8.07 (s, 1H), 7.57 (s, 1H), 7.17 - 6.96 (m, 2H), 4.75 (br dd, J = 10.4, 13.4 Hz, 1H), 4.37 (dt, J = 3.4, 9.5 Hz, 1H), 4.26 (br dd, J = 3.4, 13.6 Hz, 1H), 3.84 - 3.75 (m, 1H), 3.70 (s, 3H), 3.45 - 3.25 (m, 2H), 3.18 - 3.02 (m, 2H), 2.81 (br d, J = 5.8 Hz, 1H), 2.55 (s, 3H), 2.35 - 2.12 (m, 5H), 2.11 - 1.97 (m, 1H), 1.86 - 1.70 (m, 1H), 1.44 (br d, J = 3.8 Hz, 1H), 0.88 (br d, J = 6.5 Hz, 3H). LCMS *m/z*: 582.2 $[M+H]^+$.

**Example 21**

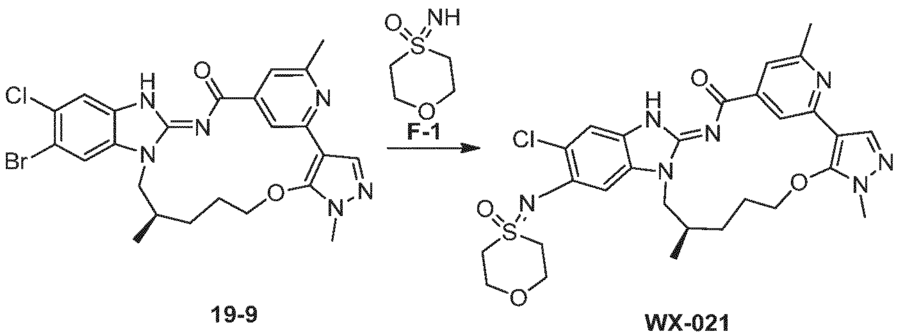


19-9 WX-021

Step 1: Synthesis of compound **WX-021**

Compound **19-9** (60.00 mg, 110.33 μmol, 1 *eq*) and compound **F-1**(22.37 mg, 165.49 μmol, 1.5 *eq)* were dissolved in a mixed solution of 1,4-dioxane (4 mL) and dichloromethane (4 mL). 2-(Di-tert-butylphosphino)biphenyl (9.88 mg, 33.10 μmol, 0.3 *eq*), a solution of sodium tert-butoxide in tetrahydrofuran (2 M, 220.65 μL, 4 eq), tris(dibenzylideneacetone)dipalladium(15.15 mg, 16.55 μmol, 0.15 *eq*) were added, and the mixture was stirred at 80 °C for 14 hours. The reaction solution was separated by preparative HPLC, column: Welch Xtimate C18 100*40mm*3μm; mobile phase: [water (trifluoroacetic acid)-acetonitrile]; acetonitrile%: 17%-47%,8 minutes. Then 30 mL of dichloromethane was added, and the mixture was washed once with saturated sodium bicarbonate solution and once with saturated sodium

chloride solution, dried over anhydrous sodium sulfate, and concentrated to obtain compound **WX-021.** $^1$H NMR (400MHz, $CDCl_3$) δ = 12.46 - 11.77 (m, 1H), 8.37 (s, 1H), 8.06 (s, 1H), 7.57 (s, 1H), 7.23 - 7.16 (m, 1H), 7.05 (d, J = 8.4 Hz, 1H), 4.76 (br dd, J = 10.2, 13.6 Hz, 1H), 4.38 (dt, J = 3.9, 9.8 Hz, 1H), 4.27 (br dd, J = 3.6, 13.6 Hz, 1H), 4.20 - 4.08 (m, 4H), 3.86 - 3.77 (m, 1H), 3.70 (s, 3H), 3.36 (br dd, J = 2.7, 13.8 Hz, 2H), 3.29 - 3.17 (m, 2H), 2.82 (br dd, J = 3.1, 6.3 Hz, 1H), 2.55 (s, 3H), 2.28 - 2.13 (m, 1H), 2.11 - 1.97 (m, 1H), 1.80 (ddd, J = 3.5, 10.6, 14.1 Hz, 1H), 1.52 - 1.39 (m, 1H), 0.90 (d, J = 6.6 Hz, 3H). LCMS m/z: 598.1 $[M+H]^+$.

**Example 22**

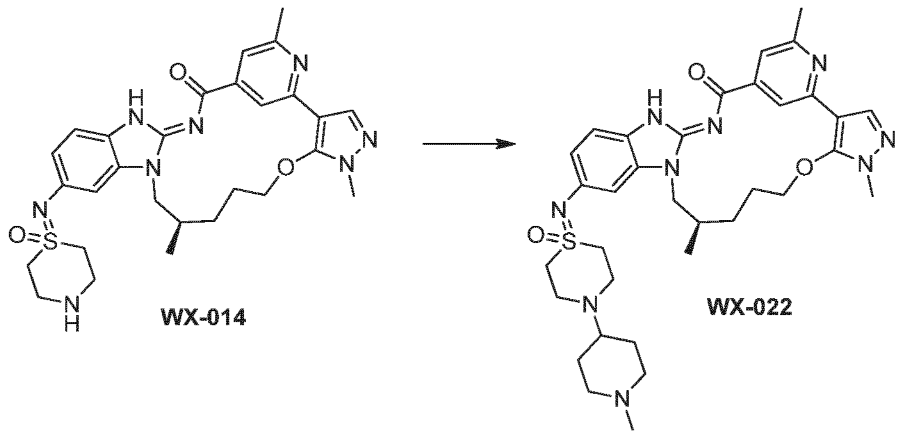

Step 1: Synthesis of compound **WX-022**

**WX-014** (87.13 mg, 154.84 μmol, 1 *eq*) was dissolved in methanol (2 mL). Acetic acid (0.065 mL), sodium cyanoborohydride (29.19 mg, 464.53 μmol, 3 *eq*), 1-methyl-4-piperidone (52.56 mg, 464.53 μmol, 54.02 μL, 3 *eq*) were added, and the mixture was stirred at 25 °C for 2 hours. 10 mL of water was added. The mixture was extracted with 50 mL of dichloromethane, and then washed once with 10 mL of saturated sodium chloride solution. The organic phase was dried over anhydrous sodium sulfate, filtered, and concentrated in vacuo to obtain a crude product. The crude product was separated and purified by silica gel column chromatography to obtain **WX-022.** LCMS *m*/*z*: 660.3 $[M+H]^+$.

**Example 23**

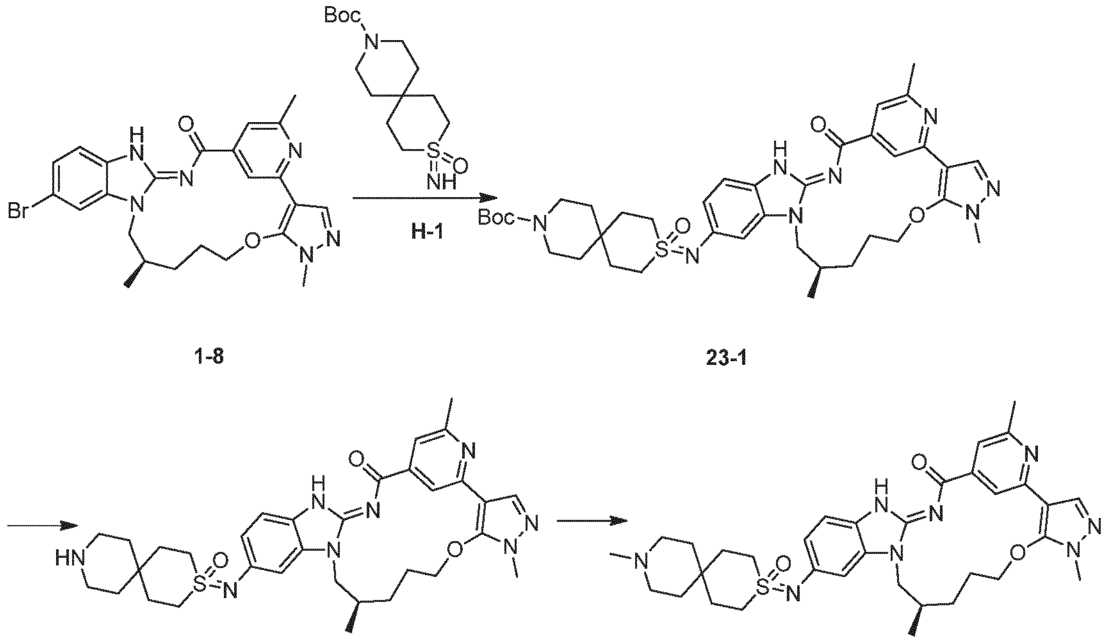


1-8

23-1

23-2

WX-023

Step 1: Synthesis of compound **23-1**

Compound **H-1**(106.66 mg, 352.66 µmol, 1.5 *eq*), **1-8** (119.76 mg, 235.11 µmol, 1 *eq*) were dissolved in 1,4-dioxane (3 mL) and dichloromethane (2 mL). Tris(dibenzylideneacetone)dipalladium (32.29 mg, 35.27 µmol, 0.15 *eq*), 2-(di-tert-butylphosphino)biphenyl (21.05 mg, 70.53 µmol, 0.3 *eq*), a solution of sodium tert-butoxide in tetrahydrofuran (2 M, 352.66 µL, 3 *eq*) were added, and the mixture was reacted at 80 °C under nitrogen for 12 hours. The reaction solution was filtered through diatomite, and the filtrate was collected. The crude product was separated and purified by silica gel column chromatography to obtain compound **23-1.** LCMS m/z = 731.2$[M+1]^+$.

Step 2: Synthesis of compound **23-2**

Compound **23-1**(70 mg, 95.77 µmol, 1 *eq*) was dissolved in dichloromethane (3 mL). Trifluoroacetic acid (1 mL) was added, and the mixture was stirred at 25 °C for 2 hours. After the reaction was completed, the reaction solution was concentrated. The reaction solution was separated by preparative HPLC, column: Welch Xtimate C18 100*40mm*3µm; mobile phase: [water (trifluoroacetic acid)-acetonitrile]; gradient: acetonitrile%: 4%-34%,8 minutes. Then 30 mL of dichloromethane was added. The mixture was washed once with saturated sodium bicarbonate solution and once with saturated sodium chloride solution, dried over anhydrous sodium sulfate, and concentrated to obtain compound **23-2.** LCMS m/z = 631.1$[M+1]^+$.

Step 3: Synthesis of compound **WX-023**

Compound **23-2** (30 mg, 47.56 µmol, 1 *eq*) was dissolved in a mixed solution of methanol (2 mL) and acetic acid (0.065 mL). Aqueous formaldehyde solution (7.14 mg, 95.12 µmol, 2 *eq*) and sodium cyanoborohydride (7.47 mg, 118.90 µmol, 2.5 *eq*) were added, and the mixture was stirred at 25 °C for 12 hours. The reaction solution was separated by preparative HPLC, column: Welch Xtimate C18 100*40mm*3µm; mobile phase: [water (trifluoroacetic acid)-acetonitrile]; acetonitrile%: 3%-33%,8 minutes. Then 30 mL of dichloromethane was added, and the mixture was washed once with saturated sodium bicarbonate solution and once with saturated sodium chloride solution, dried over anhydrous sodium sulfate, and concentrated to obtain compound **WX-023.** $^1H$ NMR (400MHz, $CDCl_3$) δ = 12.19 - 11.60 (m, 1H), 8.40 (s, 1H), 8.08 (s, 1H), 7.58 (s, 1H), 7.12 (d, J = 8.5 Hz, 1H), 6.99 (d, J = 1.5 Hz, 1H), 6.93 (dd, J = 1.6, 8.4 Hz, 1H), 4.39 (td, J = 4.5, 9.0 Hz, 1H), 4.24 (br dd, J = 2.8, 13.6 Hz, 1H), 3.88 - 3.77 (m, 1H), 3.71 (s, 3H), 3.59 (br dd, J = 9.8, 13.6 Hz, 1H), 3.28 - 3.15 (m, 2H), 3.12 - 3.03 (m, 2H), 2.83 - 2.69 (m, 1H), 2.56 (s, 3H), 2.36 (br s, 4H), 2.28 - 2.14 (m, 4H), 2.11 - 1.99 (m, 1H), 1.96 - 1.81 (m, 5H), 1.69 - 1.51 (m, 4H), 1.49 - 1.38 (m, 1H), 0.83 (d, J = 6.5 Hz, 3H). LCMS m/z = 645.1$[M+1]^+$.

**Example 24**

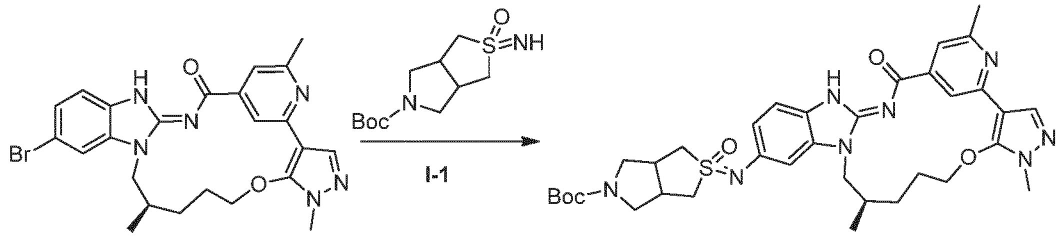


1-8 24-1

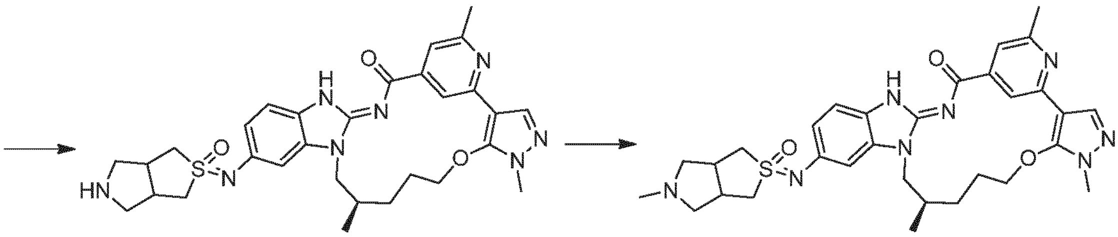

24-2 WX-024

Step 1: Synthesis of compound **24-1**

Compound **I-1**(115 mg, 441.70 μmol, 1.5 *eq*), **1-8** (150 mg, 294.47 μmol, 1 *eq*) were dissolved in 1,4-dioxane (3 mL). Tris(dibenzylideneacetone)dipalladium (40.45 mg, 44.17 μmol, 0.15 *eq*), 2-(di-tert-butylphosphino)biphenyl (26.36 mg, 88.34 μmol, 0.3 *eq*), a solution of sodium tert-butoxide in tetrahydrofuran (2 M, 441.70 μL, 3 *eq*) were added, and the mixture was reacted at 80 °C under nitrogen for 12 hours. The reaction solution was filtered through diatomite, and the filtrate was collected. The crude product was separated and purified by silica gel column chromatography to obtain compound **24-1.** LCMS m/z = 689.1 $[M+1]^+$.

Step 2: Synthesis of compound **24-2**

Compound **24-1**(120 mg, 174.21 μmol, 1 *eq*) was dissolved in dichloromethane (3 mL). Trifluoroacetic acid (1 mL) was added, and the mixture was stirred at 25 °C for 2 hours. After the reaction was completed, the reaction solution was concentrated. The reaction solution was separated by preparative HPLC, column: Welch Xtimate C18 100*40mm*3μm; mobile phase: [water (trifluoroacetic acid)-acetonitrile]; gradient: acetonitrile%: 2%-32%,8 minutes. Then 30 mL of dichloromethane was added. The mixture was washed once with saturated sodium bicarbonate solution and once with saturated sodium chloride solution, dried over anhydrous sodium sulfate, and concentrated to obtain compound **24-2.** LCMS m/z = 589.1 $[M+1]^+$.

Step 3: Synthesis of compound **WX-024**

Compound **24-2** (40 mg, 67.94μmol, 1 *eq*) was dissolved in a mixed solution of methanol (2 mL) and acetic acid (0.065 mL). Aqueous formaldehyde solution (10.2 mg, 135.89 μmol, 2 *eq*) and sodium cyanoborohydride (10.67 mg, 169.86 μmol, 2.5 *eq*) were added, and the mixture was stirred at 25 °C for 12 hours. The reaction solution was separated by preparative HPLC, column: Welch Xtimate C18 100*40mm*3μm; mobile phase: [water (trifluoroacetic acid)-acetonitrile]; acetonitrile%: 3%-33%,8 minutes. Then 30 mL of dichloromethane was added. The mixture was washed once with saturated sodium bicarbonate solution and once with saturated sodium chloride solution, dried over anhydrous sodium sulfate, and concentrated to obtain **WX-024**. LCMS m/z = 603.1 $[M+1]^+$.

**Example 25**

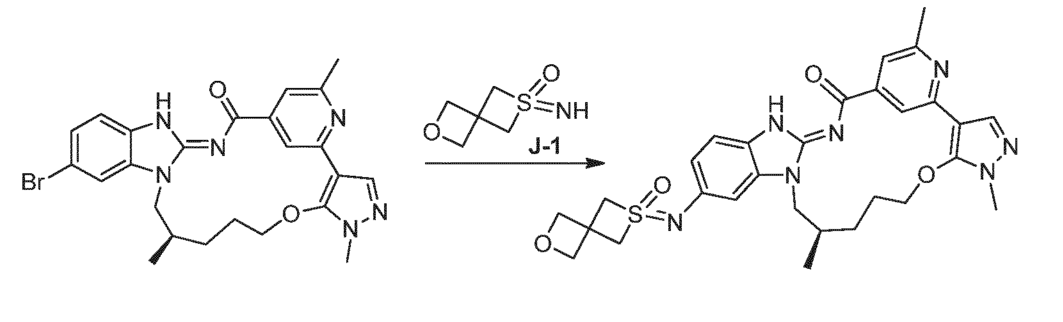


1-8 WX-025

Step 1: Synthesis of compound **WX-025**

To a solution of compound **1-8** (50 mg, 98.16 μmol, 1 *eq*), **J-1** (28.9 mg, 196.31 μmol, 2 *eq*) in 1,4-dioxane (3 mL) were added 2-(di-tert-butylphosphino)biphenyl (8.79 mg, 29.45 μmol, 0.3 *eq*), a solution of sodium tert-butoxide in tetrahydrofuran (2 M, 147.24 μmol, 73.62 μl, 1.5 *eq*). The atmosphere was replaced with nitrogen, and tris(dibenzylideneacetone)dipalladium (26.96 mg, 29.45 μmol, 0.3 *eq*) was added. The mixture was stirred at 100 °C under nitrogen for 12 hours. The mixture was cooled to room temperature, extracted with 50 mL of ethyl acetate and 20 mL of water, washed with aqueous saturated sodium chloride solution, dried over anhydrous sodium sulfate, filtered and concentrated. The crude product was separated and purified by silica gel column chromatography to obtain **WX-025.** $^1$H NMR (400MHz, $CDCl_3$) δ = 11.85 (br s, 1H), 8.40 (s, 1H), 8.09 (s, 1H), 7.59 (s, 1H), 7.14 (br d, J = 8.3 Hz, 1H), 6.87 (s, 1H), 6.82 (br d, J = 8.4 Hz, 1H), 4.82 (d, J = 12.5 Hz, 4H), 4.51 - 4.20 (m, 6H), 3.87 - 3.80 (m, 1H), 3.72 (s, 3H), 3.58 (br dd, J = 9.9, 13.5 Hz, 1H), 2.75 (br d, J = 13.9 Hz, 1H), 2.57 (s, 3H), 2.25 - 2.04 (m, 2H), 1.95 - 1.80 (m, 1H), 1.46 - 1.35 (m, 1H), 0.84 (br d, J = 6.6 Hz, 3H). LCMS m/z = 576.2 $[M+1]^+$.

**Example 26**

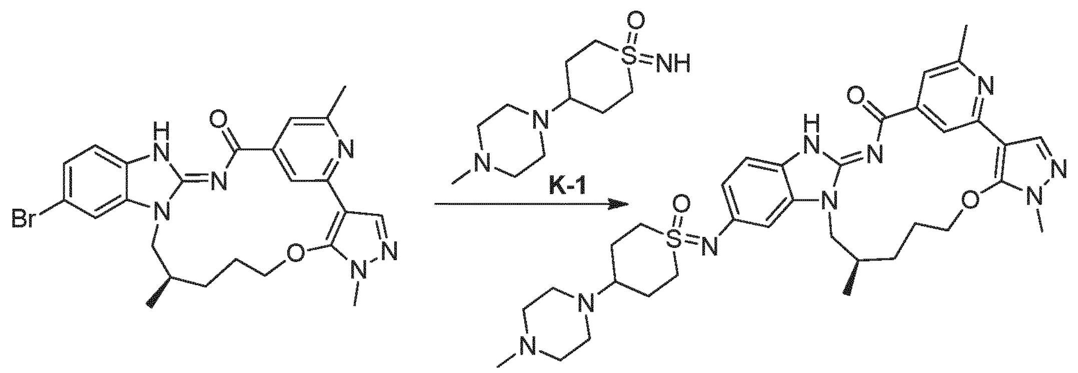


1-8 WX-026

Step 1: Synthesis of compound **WX-026**

To a solution of compound **1-8** (10 mg, 19.63 μmol, 1 *eq*), **K-1** (7.48 mg, 23.56 μmol, 1.2 *eq*) in 1,4-dioxane (3 mL) were added 2-(di-tert-butylphosphino)biphenyl (2.34 mg, 7.85 μmol, 0.4 *eq*), a solution of sodium tert-butoxide in tetrahydrofuran (2 M, 29.45 μmol, 73.62 μl, 1.5 *eq*). The atmosphere was replaced with nitrogen, and tris(dibenzylideneacetone)dipalladium (3.6 mg, 3.93 μmol, 0.2 *eq*) was added. The mixture was stirred at 100 °C under nitrogen for 12 hours. The mixture was cooled to room temperature, extracted with 30 mL of ethyl acetate and 10 mL of water, washed with aqueous saturated sodium chloride solution, dried over anhydrous sodium sulfate, filtered and concentrated. The crude product was separated and purified by silica gel column chromatography to obtain **WX-026.** $^1$H NMR (400MHz, $CDCl_3$) δ = 11.83 (br s, 1H), 8.41 (s, 1H), 8.09 (s, 1H), 7.65 - 7.57 (m, 1H), 7.13 (d, J = 8.3 Hz, 1H), 7.04 - 6.93 (m, 2H), 4.40 (br d, J = 5.0 Hz, 1H), 4.25 (br d, J = 11.0 Hz, 1H), 3.88 - 3.78 (m, 1H), 3.76 - 3.69 (m, 3H), 3.61 (br dd, J = 9.9, 13.4 Hz, 1H), 3.44 (br s, 2H), 3.03 (br t, J = 11.7 Hz, 2H), 2.76 (br s, 1H), 2.67 - 2.45 (m, 11H), 2.31 (br s, 3H), 2.24 - 1.98 (m, 7H), 1.87 (br s, 1H), 1.44 (br s, 1H), 0.84 (br d, J = 6.3 Hz, 3H). LCMS m/z: 660.3 $[M+1]^+$.

**Example 27**

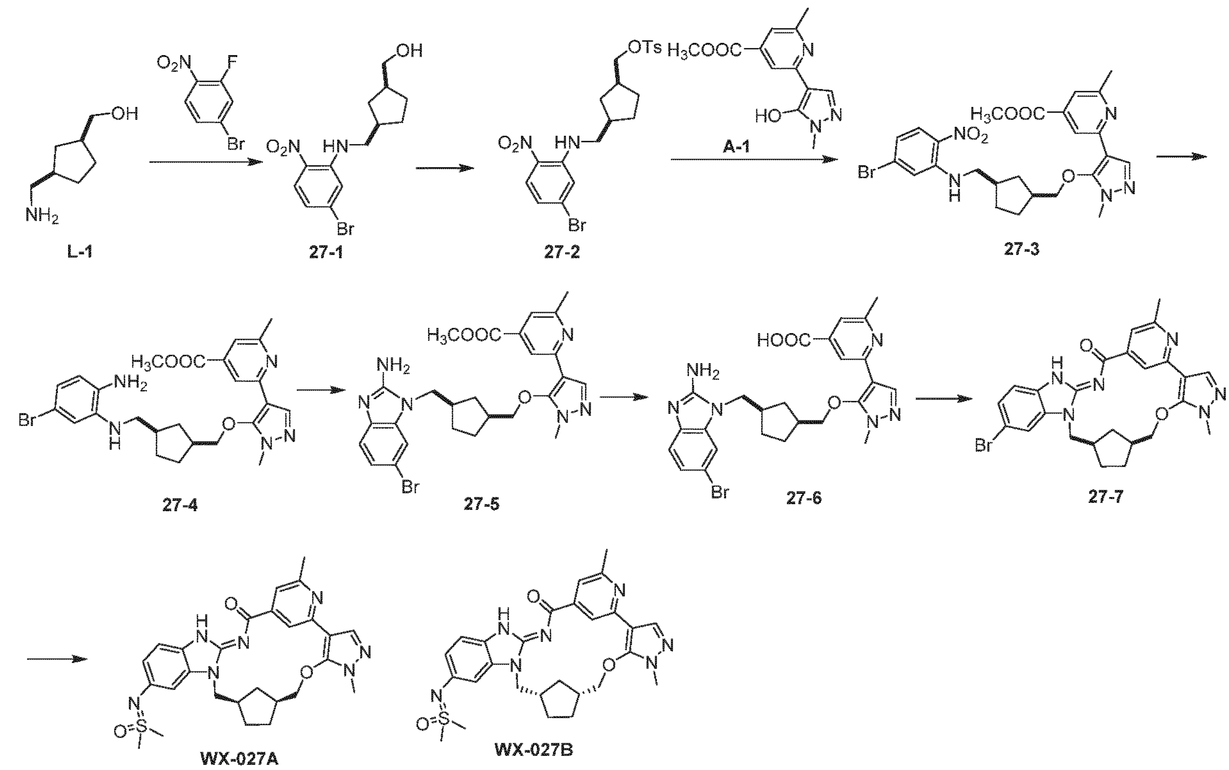


L-1, 27-1, 27-2, 27-3, 27-4, 27-5, 27-6, 27-7, WX-027A, WX-027B

Step 1: Synthesis of compound **27-1**

Compound **L-1**(16.5 g, 127.71 mmol, 1 *eq*), 2-fluoro-4-bromonitrobenzene (28.10 g, 127.71 mmol, 1 *eq*) were dissolved in ethanol (200 mL). N,N-diisopropylethylamine (49.52 g, 383.13 mmol, 3 *eq*) was added, and the mixture was stirred at 60 °C for 12 hours. The reaction solution was concentrated, and the residue was dissolved in 300 mL of ethyl acetate. The solution was washed once with saturated brine, dried over anhydrous sodium sulfate, and concentrated. The crude product was purified by silica gel column chromatography (ethyl acetate : petroleum ether = 0~50%), to obtain compound **27-1.** LCMS m/z: 328.9/330.9 $[M+1]^+$.

Step 2: Synthesis of compound **27-2**

Compound **27-1** (9.66 g, 29.34 mmol, 1 *eq*), triethylamine (8.91 g, 88.03 mmol, 12.25 mL, 3 *eq*) were dissolved in dichloromethane (200 mL), and the solution was cooled to 0°C. Paratoluensulfonyl chloride (16.78 g, 88.03 mmol, 3 *eq*) was added, and the mixture was stirred at 25 °C for 16 hours. The reaction solution was poured into 100 mL of water to quench, and the organic phase was separated. The aqueous phase was extracted with dichloromethane (100 mL × 3). The combined organic phase was washed once with 100 mL of saturated brine, dried over anhydrous sodium sulfate, filtered, and concentrated to obtain compound **27-2.** LCMS m/z = 483.0/485.0 $[M+1]^+$.

Step 3: Synthesis of compound **27-3**

Compound **27-2**(23.36 g, 29.00 mmol, 1 *eq*) and compound **A-1**(10.75 g, 43.49 mmol, 1.5 *eq*) were dissolved in *N,N*-dimethylformamide (200 mL). Potassium carbonate (12.02 g, 86.99 mmol, 3 *eq*) was added, and the mixture was stirred at 80 °C for 16 hours. The mixture was cooled to room temperature, quenched with water (400 mL), and extracted with ethyl acetate (400 mL*3). The organic phase was washed with saturated brine (400 mL*3) and concentrated. The crude product was separated and purified by silica gel column chromatography (ethyl acetate : petroleum ether = 0~50%), to obtain compound **27-3.** LCMS m/z = 558.1/560.1 $[M+1]^+$.

Step 4: Synthesis of compound **27-4**

Compound **27-3**(6.6 g, 11.82 mmol, 1 *eq*) was dissolved in a mixed solution of tetrahydrofuran (50 mL) and *N,N*-dimethylformamide (5 mL). Ferric chloride hexahydrate (3.19 g, 11.82 mmol, 1 *eq*), activated carbon (2.84 g, 236.38 mmol, 20 eq) were added, then hydrazine hydrate (18.340 g, 366.36 mmol, 31.00 *eq*) was added, and the mixture was stirred at 55 °C for 3 hours. The reaction solution was filtered through diatomaceous earth, and the filter cake was washed with ethyl acetate. The filtrate was collected, washed once with saturated brine, and then dried over anhydrous sodium sulfate and concentrated. The crude product was subjected to silica gel column chromatography (methanol:dichloromethane = 0-10%) to obtain compound **27-4.** $^1$H NMR (400MHz, $CDCl_3$) δ = 7.89 (d, J = 1.3 Hz, 2H), 7.48 (s, 1H), 6.84 - 6.65 (m, 2H), 6.56 (d, J = 8.0 Hz, 1H), 4.07 - 3.97 (m, 2H), 3.96 - 3.88 (m, 3H), 3.75 (s, 3H), 3.05 (dq, J = 7.2, 11.4 Hz, 2H), 2.62 (s, 3H), 2.56 - 2.42 (m, 1H), 2.38 - 2.16 (m, 2H), 1.98 - 1.84 (m, 2H), 1.64 - 1.51 (m, 1H), 1.51 - 1.37 (m, 1H), 1.20 - 1.09 (m, 1H). LCMS m/z = 528.1/530.1 $[M+1]^+$.

Step 5: Synthesis of compound **27-5**

Compound **27-4**(10.7 g, 20.25 mmol, 1 *eq*) was dissolved in a mixed solvent of tert-butyl alcohol (20 mL) and dichloromethane (200 mL). Cyanogen bromide (10.94 g, 103.28 mmol, 5.10 *eq*) was added, and the mixture was stirred at 25 °C for 16 hours. The mixture was washed once with aqueous saturated sodium bicarbonate solution, and dried over anhydrous sodium sulfate. The organic phase was concentrated and purified by silica gel column chromatography (methanol:dichloromethane = 0-3%) to obtain compound **27-5.** LCMS m/z = 553.0/555.0 $[M+1]^+$.

Step 6: Synthesis of compound **27-6**

Compound **27-5**(5.36 g, 9.68 mmol, 1 *eq*) was dissolved in tetrahydrofuran(40 mL). Aqueous sodium hydroxide solution (1 M, 38.74 mL, 4 *eq*) was added, and the mixture was reacted at 25°C for 2 hours. The reaction solution was concentrated, and adjusted to pH of 6 with 2M hydrochloric acid aqueous solution, and then filtered to obtain compound **27-6.** LCMS m/z = 539.0/541.0 $[M+1]^+$.

Step 7: Synthesis of compound **27-7**

Compound **27-6** (9.44 g, 17.50 mmol, 1 *eq*) was dissolved in dichloromethane (200 mL). 2-(1H-benzotriazol-1-yl)-1,1,3,3-tetramethyluronium tetrafluoroborate (8.43 g, 26.25 mmol, 1.5 *eq*) and triethylamine (8.85 g, 87.50 mmol, 12.18 mL, 5 *eq*) were added, and the mixture was reacted at 25 °C for 2 hours. The reaction solution was filtered to directly obtain compound 27-7. LCMS m/z = 521.0/523.0 $[M+1]^+$.

Step 8: Synthesis of compound **WX-027A** and **WX-027B**

To a solution of compound **27-7** (5.1 g, 9.78 mmol, 1 *eq),* S,S-dimethyl sulfoximine (5.47 g, 58.69 mmol, 6 *eq*) in 1,4-dioxane (50 mL) were added 2-(di-tert-butylphosphino)biphenyl(1.17 g, 3.91 mmol, 0.4 *eq*), a solution of sodium tert-butoxide in tetrahydrofuran (2 M, 29.34 mmol, 3 *eq*). The atmosphere was replaced with nitrogen, and tris(dibenzylideneacetone)dipalladium(1.79 g, 1.96 mmol, 0.2 *eq*) was added. The mixture was stirred at 80 °C under nitrogen for 16 hours. The reaction solution was concentrated, and 150 mL of dichloromethane and 150 mL of water were added. The mixture was adjusted to pH of 6 with 6M hydrochloric acid aqueous solution with stirring. The organic phase was separated, washed once with saturated brine, dried over anhydrous sodium sulfate, and concentrated. The residue was purified by silica gel column chromatography to obtain the crude racemate (methanol:dichloromethane = 0~3%). The racemate was separated by SFC, column: DAICEL CHIRALCEL OD (250mm*30mm,10um); mobile phase: A ($CO_2$) and B (isopropyl alcohol, containing 0. 1 % ammonia); gradient: B% = 45% to obtain **WX-027A** and **WX-027B.**

**WX-027A:** $^1$H NMR (400MHz, $CDCl_3$) δ = 11.68 (br s, 1H), 8.50 (s, 1H), 8.08 (br s, 1H), 7.47 (s, 1H), 7.15 (d, J = 8.3 Hz, 1H), 7.00 - 6.89 (m, 2H), 4.41 (br t, J = 12.8 Hz, 1H), 4.22 - 4.06 (m, 1H), 3.97 (dd, J = 3.3, 8.5 Hz, 1H), 3.86 - 3.75 (m, 1H), 3.72 (s, 3H), 3.12 (d, J = 2.3 Hz, 6H), 2.91 - 2.69 (m, 1H), 2.65 - 2.44 (m, 5H), 2.01 - 1.93 (m, 1H), 1.90 - 1.80 (m, 2H), 1.75 - 1.62 (m, 2H). LCMS m/z = 534.2 $[M+1]^+$. SFC (column: Chiralcel AD-3, 3 μm, 0.46 cm id × 15cm L; mobile phase: A ($CO_2$) and B (ethanol, containing 0.05% diethylamine); gradient: B% = 50%; flow rate: 2.2 mL/min; column temperature: 35°C; wavelength: 220 nm; pressure: 1500 psi), retention time = 2.925 minutes, chiral isomer excess 100.00%

**WX-027B:** $^1$H NMR (400MHz, $CDCl_3$) δ = 11.77 (br s, 1H), 8.55 (s, 1H), 8.13 (s, 1H), 7.53 (s, 1H), 7.20 (br d, J = 8.0 Hz, 1H), 7.07 - 6.97 (m, 2H), 4.47 (br t, J = 12.2 Hz, 1H), 4.26 - 4.17 (m, 1H), 4.03 (br d, J = 5.0 Hz, 1H), 3.86 (br d, J = 12.0 Hz, 1H), 3.78 (s, 3H), 3.18 (d, J = 1.8 Hz, 6H), 2.87 (br s, 1H), 2.62 (m, 5H), 2.09 - 1.97 (m, 1H), 1.96 - 1.85 (m, 2H), 1.79 - 1.71 (m, 2H). LCMS m/z = 534.2 $[M+1]^+$. SFC (column: Chiralcel AD-3, 3 μm, 0.46 cm id × 15cm

L; mobile phase: A ($CO_2$) and B (ethanol, containing 0.05% diethylamine); gradient: B% = 50%; flow rate: 2.2 mL/min; column temperature: 35°C; wavelength: 220 nm; pressure: 1500 psi), retention time = 3.474 minutes, chiral isomer excess 99.66%.

**Example 28**

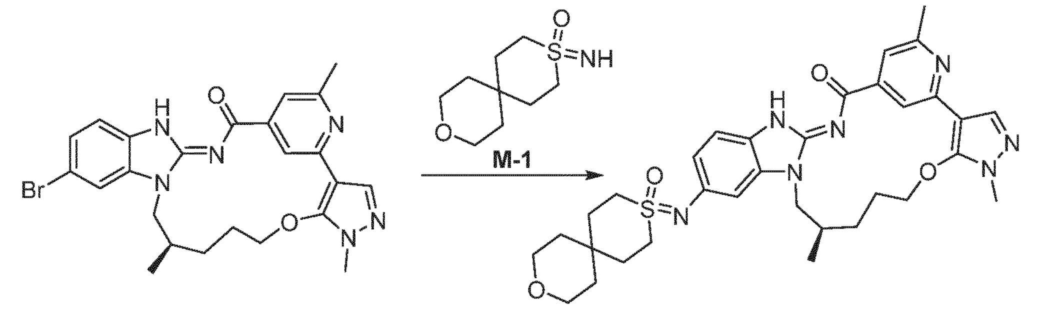


**1-8** **WX-028**

Step 1: Synthesis of compound **WX-028**

To a solution of compound **1-8** (20 mg, 39.26 μmol, 1 *eq*), **M-1**(15.96 mg, 78.52 μmol, 2 *eq*) in 1,4-dioxane (50 mL) were added 2-(di-tert-butylphosphino)biphenyl(1.17 g, 3.91 μmol, 0.4 *eq*), a solution of sodium tert-butoxide in tetrahydrofuran (2 M, 117.79 μmol, 3 *eq*). The atmosphere was replaced with nitrogen, and tris(dibenzylideneacetone)dipalladium (7.19 mg, 7.85 μmol, 0.2 *eq*) was added. The mixture was stirred at 100 °C under nitrogen for 12 hours. After the reaction was completed, 50 mL of ethyl acetate and 20 mL of water were added. The organic phase was separated, washed once with saturated brine, dried over anhydrous sodium sulfate, and concentrated. The residue was purified by silica gel column chromatography to obtain compound **WX-028.** $^1H$ NMR (400MHz, $CDCl_3$) δ = 11.88 (br s, 1H), 8.40 (s, 1H), 8.09 (s, 1H), 7.59 (s, 1H), 7.12 (br d, J = 8.3 Hz, 1H), 6.99 (s, 1H), 6.93 (br d, J = 8.3 Hz, 1H), 4.45 - 4.33 (m, 1H), 4.24 (br d, J = 11.3 Hz, 1H), 3.85 - 3.77 (m, 1H), 3.71 (s, 3H), 3.61 (br s, 4H), 3.21 (br d, J = 3.8 Hz, 2H), 3.14 - 3.05 (m, 2H), 2.98 (br s, 1H), 2.76 (br s, 1H), 2.56 (s, 3H), 2.16 (br s, 1H), 2.02 - 1.97 (m, 5H), 2.05 (br d, J = 17.1 Hz, 1H), 1.92 - 1.86 (m, 1H), 1.63 - 1.47 (m, 4H), 0.86 - 0.79 (m, 3H). LCMS m/z = 632.0 $[M+1]^+$.

**Example 29**

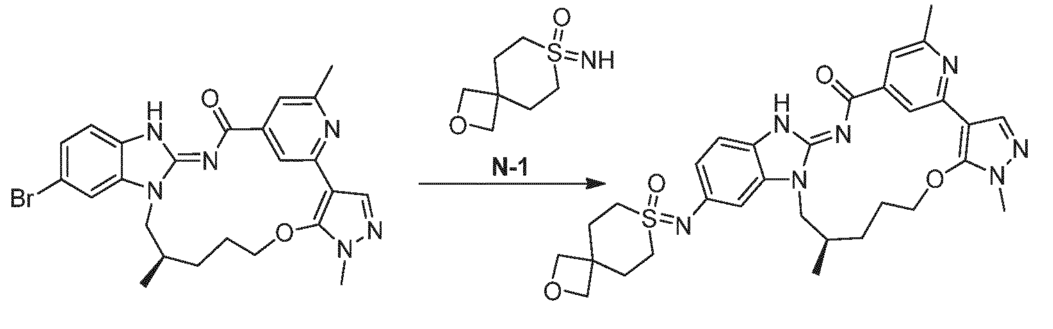


**1-8** **WX-029**

Step 1: Synthesis of compound **WX-029**

To a solution of compound **1-8** (110 mg, 215.94 μmol, 1 *eq*), **N-1** (56.76 mg, 323.91 μmol, 1.5 *eq*) in 1,4-dioxane (2.5 mL) and dichloromethane (1 mL) were added 2-(di-tert-butylphosphino)biphenyl (19.33 mg, 64.78 μmol, 0.3 *eq*), a solution of sodium tert-butoxide in tetrahydrofuran (2 M, 647.8 μmol, 323.91 μl, 3 *eq*). The atmosphere was

replaced with nitrogen, and tris(dibenzylideneacetone)dipalladium (29.66 mg, 32.39 μmol, 0.15 *eq*) was added. The mixture was stirred at 80 °C under nitrogen for 14 hours. The reaction solution was cooled to room temperature, filtered and concentrated. The crude product was separated and purified by silica gel column chromatography to obtain **WX-029.** $^1$H NMR (400MHz, $CDCl_3$) δ = 12.41 - 11.62 (m, 1H), 8.48 (s, 1H), 8.17 (s, 1H), 7.67 (s, 1H), 7.20 (d, J = 8.5 Hz, 1H), 7.05 (d, J = 1.8 Hz, 1H), 6.99 (dd, J = 1.8, 8.5 Hz, 1H), 4.55 (s, 2H), 4.49 (s, 2H), 4.51 - 4.44 (m, 1H), 4.32 (dd, J = 3.0, 13.6 Hz, 1H), 3.94 - 3.85 (m, 1H), 3.79 (s, 3H), 3.66 (dd, J = 9.8, 13.6 Hz, 1H), 3.40 - 3.27 (m, 2H), 3.12 (ddd, J = 3.9, 9.8, 13.9 Hz, 2H), 2.93 - 2.78 (m, 1H), 2.64 (s, 3H), 2.53 - 2.37 (m, 4H), 2.33 - 2.20 (m, 1H), 2.18 - 2.07 (m, 1H), 1.95 (ddd, J = 4.5, 9.7, 14.2 Hz, 1H), 1.52 - 1.46 (m, 1H), 0.92 (d, J = 6.5 Hz, 3H). LCMS m/z: 604.2 $[M+1]^+$.

**Example 30**

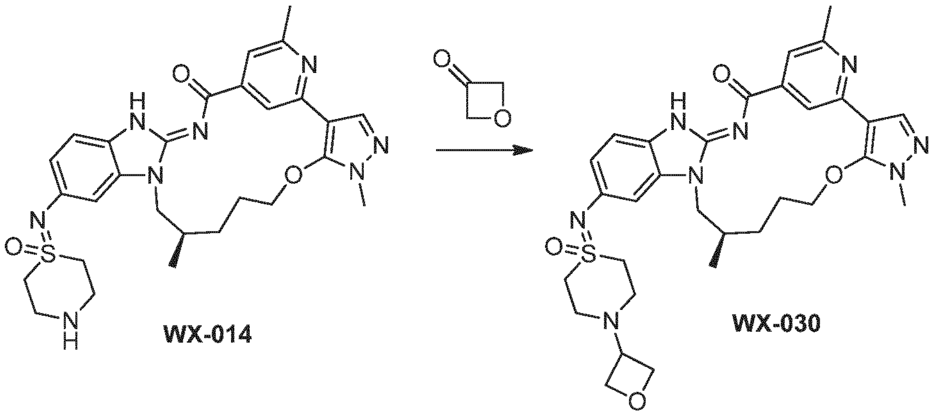

WX-014

WX-030

Step 1: Synthesis of compound **WX-030**

**WX-014** (20 mg, 35.54 μmol, 1 *eq*) was dissolved in methanol (2 mL). Acetic acid (0.065 mL), sodium cyanoborohydride (6.70 mg, 106.63 μmol, 3 *eq*), 3-oxetanone (5.12 mg, 71.09 μmol, 54.02 μL, 3 *eq*) were added, and the mixture was stirred at 25 °C for 2 hours. The reaction solution was separated by preparative HPLC, column: Welch Xtimate C18 100*40mm*3μm; mobile phase: [water (trifluoroacetic acid)-acetonitrile]; acetonitrile %: 7%-37%,8 minutes. To the isolate was added 10 mL of saturated sodium bicarbonate solution, and the mixture was extracted with 30 mL of dichloromethane. The organic phase was washed once with saturated sodium chloride solution, dried over anhydrous sodium sulfate, and concentrated to obtain compound **WX-030.** $^1$H NMR (400MHz, $CDCl_3$) δ = 11.92 (br s, 1H), 8.85 (br s, 1H), 8.55 (s, 1H), 7.90 (br s, 1H), 7.31 (br s, 1H), 7.20 - 7.01 (m, 2H), 4.73 (br t, J = 6.1 Hz, 2H), 4.68 - 4.57 (m, 2H), 4.45 (br d, J = 4.0 Hz, 1H), 4.30 (br d, J = 13.3 Hz, 1H), 3.97 (br s, 1H), 3.81 (s, 4H), 3.76 - 3.68 (m, 1H), 3.51 - 3.29 (m, 4H), 3.06 - 2.66 (m, 8H), 2.28 (br s, 1H), 2.16 - 1.94 (m, 2H), 1.56 (br s, 1H), 0.94 (br d, J = 6.0 Hz, 3H). LCMS m/z: 619.1 $[M+1]^+$.

**Example 31**

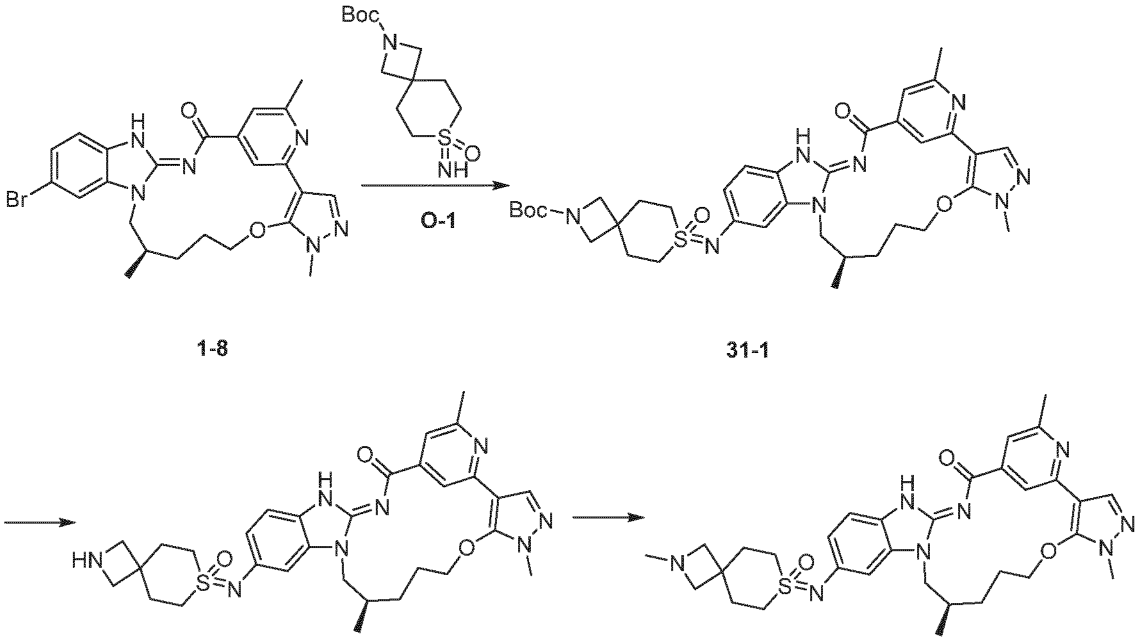


1-8

31-1

31-2

WX-031

Step 1: Synthesis of compound **31-1**

To a solution of compound **1-8** (0.4 g, 785.24 μmol, 1 *eq*), **O-1** (323.18 mg, 1.18 mmol, 1.5 *eq*) in 1,4-dioxane (20 mL) and dichloromethane (5 mL) were added 2-(di-tert-butylphosphino)biphenyl (70.29 mg, 235.57 μmol, 0.3 *eq*), a solution of sodium tert-butoxide in tetrahydrofuran (2 M, 3.93 mmol, 1.96 mL, 5 *eq*). The atmosphere was replaced with nitrogen, and tris(dibenzylideneacetone)dipalladium (107.86 mg, 117.79 μmol, 0.15 *eq*) was added. The mixture was stirred at 80 °C under nitrogen for 14 hours. The reaction solution was cooled to room temperature, filtered and concentrated. The crude product was separated and purified by silica gel column chromatography to obtain **31-1.** LCMS m/z: 703.3 $[M+1]^+$.

Step 2: Synthesis of compound **31-2**

Compound **31-1** (0.35 g, 497.96 μmol, 1 *eq*) was dissolved in dichloromethane (4 mL). Trifluoroacetic acid (4 mL) was added, and the mixture was stirred at 25 °C for 2 hours. The reaction solution was concentrated to obtain compound **31-2** trifluoroacetate. LCMS m/z = 603.2$[M+1]^+$.

Step 3: Synthesis of compound **WX-031**

Compound **31-2** trifluoroacetate (0.1 g) was dissolved in a mixed solution of methanol (3 mL) and acetic acid (0.1 mL). Aqueous formaldehyde solution (26.9 mg) was added, and the mixture was stirred at 25 °C for 0.5 hours. Sodium cyanoborohydride (26.06 mg, 414.77μmol, 2.5 *eq)* was then added, and the mixture was stirred at 25 °C for 12 hours. The reaction solution was purified by silica gel column chromatography to obtain compound **WX-031**. $^1H$ NMR (400MHz, $CD_3OD$) δ = 8.86 (s, 1H), 8.25 (s, 1H), 7.99 (s, 1H), 7.39 (d, J = 8.5 Hz, 1H), 7.18 (d, J = 1.8 Hz, 1H), 7.06 (dd, J = 1.8, 8.5 Hz, 1H), 4.53 (dt, J = 4.1, 9.1 Hz, 1H), 4.33 (br d, J = 11.3 Hz, 2H), 4.28 - 4.18 (m, 1H), 4.16 - 4.10 (m, 1H), 4.08 - 3.94 (m, 2H), 3.88 - 3.78 (m, 4H), 3.53 - 3.37 (m, 4H), 3.00 (s, 3H), 2.81 (s, 4H), 2.52 - 2.27 (m, 5H), 2.14 - 1.97 (m, 2H), 1.67 - 1.51 (m, 1H), 0.91 (d, J = 6.5 Hz, 3H). LCMS m/z = 617.3$[M+1]^+$.

## Biological assay

### Assay example 1: Inhibitory activity of compounds against EGFR (L858R/T790M/C797S), EGFR (del19-E746-A750/T790M/C797S), and EGFR (WT) kinases

Compound assay for kinase inhibitory activity against EGFR (L858R/T790M/C797S), EGFR (del19-E746-A750/T790M/C797S), and EGFR (WT) was done at Reaction Biology Corp. In reaction buffer (20 mM Hepes (pH 7.5), 10 mM magnesium chloride ($MgCl_2$), 1 mM ethylenebis(oxyethylenenitrilo)tetraacetic acid (EGTA), 0.02% polyethylene

glycol monododecyl ether (Brij35), 0.02 mg/ml BSA, 0.1 mM sodium vanadate ($Na_3VO_4$), 2 mM dithiothreitol (DTT), 1% DMSO), a certain concentration of substances, coenzyme factors, kinases and test compounds (10 doses, 3-fold serial dilutions, 2% DMSO final concentration) were added in sequence, and mixed well. The mixture was incubated at room temperature for 20 minutes. A certain concentration of $^{33}$P-ATP was added to the reaction mixture to initiate reaction, followed by incubation at room temperature for 120 minutes. Finally, the radioactivity of the reactants was detected by filtration-binding method. The final kinase activity was expressed as the ratio of the remaining kinase activity of the test sample to the kinase activity of the DMSO control group. The dose-response relationship curve was fitted and $IC_{50}$ was calculated using GraphPad software. The results are shown in Table 1 and Table 2:

Table 1. Kinase half-maximal inhibition concentration $IC_{50}$ (nM)

| | Compound | ATP (μM) | EGFR(L858R/T790M/C797S) |
|---|---|---|---|
| 1 | **WX-001 (trifluoroacetate)** | 10 | 0.159 |
| 2 | **WX-002 (formate)** | 10 | 0.468 |
| 3 | **WX-001 (trifluoroacetate)** | 100 | 1.17 |
| 4 | **WX-004 (trifluoroacetate)** | 100 | 1.75 |
| 5 | **WX-005 (trifluoroacetate)** | 100 | 1.63 |
| 6 | **WX-006 (trifluoroacetate)** | 100 | 0.831 |
| 7 | **WX-019** | 100 | 3.55 |
| 8 | **WX-027A** | 100 | 1.82 |

Table 2. Kinase half-maximal inhibition concentration $IC_{50}$ (nM)

| | Compound | ATP (μM) | EGFR(del19-E746-A750/T790M/C797S) | EGFR(WT) |
|---|---|---|---|---|
| 1 | **WX-019** | 100 | 0.139 | 212 |
| 2 | **WX-027A** | 100 | 0.093 | 55.8 |

**The results show that:** the compounds of the present disclosure exhibited high inhibitory activity against kinases carrying triple mutations of EGFR (L858R/T790M/C797S) and EGFR (del19-E746-A750/T790M/C797S), and had lower inhibitory activity against EGFR (WT) kinase, showing good selectivity.

**Assay example 2: Inhibitory activity of compounds on the proliferation of Ba/F3-EGFR-associated mutant cells**

Adenosine Tri-Phosphate (ATP) is an energy carrier shared by various life activities in nature, and is the smallest unit of energy storage and transfer. The CellTiter-Glo™ living cell assay kit uses luciferase as a detector, and luciferase requires the participation of ATP in the process of luminescence. CellTiter-Glo™ reagent is added to a cell culture medium, and the luminescence value is measured. The light signal is directly proportional to the amount of ATP in the system, and ATP is positively correlated with the number of living cells. Therefore, cell proliferation can be detected by detecting ATP content with the CellTiter-Glo kit. In this assay, the cell lines were Ba/F3-FL-EGFR (L858R/T790M/C797S), Ba/F3-FL-EGFR (WT), Ba/F3-FL-EGFR (L858R), Ba/F3-FL-EGFR (del19-S752-I759), Ba/F3-FL-EGFR (del19-E746-A750/T790M/C797S), Ba/F3-FL-EGFR (L858R/T790M), Ba/F3-FL-EGFR (del19-E746-A750/T790M), Ba/F3-TEL-EGFR (L858R/C797S), Ba/F3-FL-EGFR (del19-E746-A750/C797S) stably transfected cell lines.

**$IC_{50}$ assay process:**

**1) Cell culture**

The cell lines were cultured in an incubator at 37°C and 5% $CO_2$. The cells were periodically passaged, and cells in logarithmic growth phase were taken for plating.

**2) Preparation of compound storage plates**

a) DMSO was used to prepare a 10 mM solution of the compound to be tested, and then the compound to be tested was diluted to 0.3 or 1 mM with DMSO.
b) Preparation of 1000× compound storage plate (tube): DMSO was used to dilute by 3-fold gradient from the highest concentration to the lowest concentration, with a total of 9 concentrations.
c) Preparation of 20× compound working solution: 49 μL of cell culture medium was added to a flat-bottomed 96-well transparent drug plate, and 1 μL of compound was pipetted from the 1000x compound storage plate to the cell culture medium of the 96-well transparent drug plate. 1 μL of DMSO was added to the vehicle control. After adding compound or DMSO, the well content was mixed to uniform by aspiration and expelling with pipette.

**3) Cell plating and drug administration**

a) Cells were stained with trypan blue and viable cells were counted. The cell viability rate was required to be above 90%.
b) 95 μL of cell suspension (2000 cells/well) was added to each well of the compound detection cell plate, and culture medium without cells (containing 0.1% DMSO) was added to the Min control well.
c) Dosing in the compound detection cell plate: 5 μL of 20× compound working solution was added to the cell culture plate. 5 μL of DMSO-cell culture mixture was added to the Max control. The final concentration of DMSO was 0.1%.
d) The culture plate was incubated in an incubator at 37 °C, 5% $CO_2$ for 72 hours.

**4) CellTiter-Glo luminescence cell viability detection**

The following steps were performed according to the instruction of Promega CellTiter-Glo luminescence cell viability detection kit (Promega-G7573).

a) The CellTiter-Glo buffer was thawed and allowed to stand to reach room temperature;
b) CellTiter-Glo substrate was allowed to stand to reach room temperature;
c) CellTiter-Glo buffer was added to CellTiter-Glo substrate in a bottle to dissolve the substrate to formulate CellTiter-Glo working solution;
d) The working solution was vortexed slowly for fully dissolution;
e) The cell culture plates were taken out and allowed to stand for 10 minutes to equilibrate to room temperature;
f) 50 μL (equal to half the volume of cell culture solution in each well) of Cell Titer-Glo working solution was added into each well.
g) The culture plates were shaken on an orbital shaker for 2 minutes to induce cell lysis;
h) The culture plates were left at room temperature for 10 minutes to stabilize the luminescence signal;
i) The luminous signal was detected on the PerkinElmer EnVision®2105 plate reader.

**5) Data processing**

By PerkinElmer EnVision®2105 reading, the corresponding fluorescence value RLU for each well was yielded.
Cell proliferation inhibition rate (Inhibition Rate) data were processed using the following formula:

$$\text{Inhibition Rate (Inh\%)} = 100\text{-(RLUDrug-RLUMin)/(RLUMax-RLUMin)*}100\%.$$

The inhibition rates corresponding to different concentrations of compounds were calculated, and then GraphPad Prism software was used to draw the inhibition rate curve and the $IC_{50}$ value was calculated. The data are shown in Table 3 and Table 4.

Table 3. Cell half-maximal inhibition concentration $IC_{50}$ (nM)

| | Compound | Ba/F3-FL-EGFR (L858R/T790M/C797S) |
|---|---|---|
| 1 | WX-005 (trifluoroacetate) | 7.2 |
| 2 | WX-006 (trifluoroacetate) | 2.3 |
| 3 | WX-010 (trifluoroacetate) | 2.8 |
| 4 | WX-011 | 4.4 |
| 5 | WX-012 (hydrochloride) | 7.3 |
| 6 | WX-013 | 4.3 |
| 7 | WX-014 | 14.3 |
| 8 | WX-015 | 10.6 |
| 9 | WX-016 | 13.4 |
| 10 | WX-017 | 6.4 |
| 11 | WX-018 | 6.5 |
| 12 | WX-019 | 5.7 |
| 13 | WX-020 | 4.6 |
| 14 | WX-021 | 8.6 |
| 15 | WX-022 | 12.7 |
| 16 | WX-023 | 2.7 |
| 17 | WX-024 | 1.4 |
| 18 | WX-025 | 5.7 |
| 19 | WX-026 | 11.6 |
| 20 | WX-027A | 8.6 |
| 21 | WX-028 | 3.3 |
| 22 | WX-029 | 5.5 |
| 23 | WX-030 | 13 |
| 24 | WX-031 | 2.1 |

Table 4. Cell half-maximal inhibition concentration $IC_{50}$ (nM)

| | Ba/F3-FL-EGFR (WT) | Ba/F3-FL-EGFR (L858R ) | Ba/F3-FL-EGFR (del19-S752-1759) | Ba/F3-FL-EGFR (L858R /T790M ) | Ba/F3-FL-EGFR (del19-E746-A750/T 790M) | Ba/F3-TEL-EGFR (L858R /C797S) | Ba/F3-FL-EGFR (del19-E746-A750/C 797S) | Ba/F3-FL-EGFR (del19-E746-A750/T 790M/ C797S) |
|---|---|---|---|---|---|---|---|---|
| WX-006 | 111.0 | 18.6 | 15.4 | 12.9 | 0.8 | 3.1 | / | 0.6 |
| WX-010 trifluoro acetate | 86.0 | 14.0 | 11.2 | 10.5 | 0.4 | 3.0 | / | 0.8 |
| WX-013 | 122.9 | / | / | 12.1 | 0.6 | 3.1 | / | / |
| WX-014 | 420.9 | 53.9 | 38.1 | / | / | / | / | 2.9 |
| WX-019 | 302.5 | 42.2 | 15.6 | 16.8 | 2.9 | 7.3 | 6.5 | 1.3 |

(continued)

| | Ba/F3-FL-EGFR (WT) | Ba/F3-FL-EGFR (L858R ) | Ba/F3-FL-EGFR (del19-S752-1759) | Ba/F3-FL-EGFR (L858R /T790M ) | Ba/F3-FL-EGFR (del19-E746-A750/T 790M) | Ba/F3-TEL-EGFR (L858R /C797S) | Ba/F3-FL-EGFR (del19-E746-A750/C 797S) | Ba/F3-FL-EGFR (del19-E746-A750/T 790M/ C797S) |
|---|---|---|---|---|---|---|---|---|
| **WX-027A** | 295.2 | 39.6 | 12.9 | 14.3 | 1.9 | 5.2 | 4.7 | 1.2 |
| "/": Not tested. | | | | | | | | |

**The results show that:** the compounds of the present disclosure exhibited extremely strong cell proliferation inhibitory activity on cell lines carrying triple mutations of EGFR(L858R/T790M/C797S), EGFR(del19-E746-A750/T790M/C797S), and also showed high cell proliferation inhibitory activity on cell lines carrying double mutations of EGFR(L858R/T790M), EGFR(del19-E746-A750/T790M), EGFR(del19-E746-A750/C797S) and EGFR(L858R/C797S); In addition, the inhibition of EGFR (WT) cell line was weak, showing good selectivity.

**Assay example 3: Compound inhibition assay on phosphorylation of Ba/F3-FL-EGFR(L858R/T790M/C797S) and Ba/F3-FL-EGFR (dell9-E746-A750/T790M/C797S) cells**

Autophosphorylation of EGFR can activate its intracellular kinase pathway. In this assay, EGFR phosphorylation was quantified in order to detect the inhibitory effect of compounds on Ba/F3-FL-EGFR(L858R/T790M/C797S) and Ba/F3-FL-EGFR (del19-E746-A750/T790M/C797S) phosphorylation.

**$IC_{50}$ assay process:**

1) Cell plating

The cells in the logarithmic growth phase were taken and counted with a cell counter. The cell viability rate was required to be >90%. Cells were inoculated into a sterile 6-well plate with $2\times10^6$ cells per well, replenished with 1640 complete medium to 1998 μL, and the 6-well plate was incubated in a constant temperature incubator at 37°C and 5% $CO_2$.

2) Compound administration

The 6-well plate was taken out, and the cells were normal under microscope. In an ultraclean bench, 2 μL of each of the prepared gradient diluted 1000× stock solution was added to the 6-well plate inoculated with cells, and the 6-well plate was gently shaken to distribute the compounds evenly.

3) Incubation

The 6-well plate was incubated statically in a constant temperature incubator at 37°C, 5% $CO_2$ for 4 h;

4) Sample collection

After the cells were cultured statically for 4 hours, the culture medium was collected, and centrifuged at 8000 rpm for 3 minutes. The cells were collected in a 1.5 mL EP tube. The supernatant was discarded, and the total protein was extracted;

5) Cell lysis

Cells from each group were collected into 1.5 ml EP tubes and a certain volume of Lysis Buffer (RIPA: protease/phosphatase inhibitor = 100:1) was added (-66 μL per $2\times10^6$ cells, depending on the cell amount). After adding Lysis Buffer, the EP tubes were placed on ice quickly and vortexed using a vortexer to fully lyse the cells. Be careful not to vortex for too long, and quickly place the sample tube on ice to lysis, shaking every 10 min for three times. At the end

of the lysis time, the EP tubes were centrifuged at 12000 rpm for 10 min in a pre-cooled centrifuge at 4°C. After centrifugation, the supernatant was transferred to new 1.5 ml EP tubes which were labeled.

6) Determination of BCA protein concentration

Using the BCA Protein Concentration Assay Kit, protein quantification of the sample lysis supernatant was performed according to the kit instructions. After the protein concentration was determined, the lysis supernatant with the lowest concentration was used as the reference, and the rest of the lysis supernatants were diluted to that concentration using Lysis Buffer in a volume calculated according to the BCA standard curve. To the diluted lysis supernatant, the corresponding volume of 5× loading Buffer was added. The diluted solution was heated in a metal bath at 95 °C for 10 min, then placed on ice to be cooled, and stored in a refrigerator at -20 °C.

7) Glue making

The glass plates used for making glue were cleaned, and two glass plates were aligned the bottoms, put into the bracket, and fixed with clips. The separation gel according to the 10% separation gel formula was prepared, mixed well and then added to the glass plate. The addition was stopped until the separation gel reached about 3/4 of the glass plate and the glue was pressed with 75% ethanol. After 90 minutes, the separation gel was solidified. The 75% ethanol was poured out. Filter paper was used to absorb the remaining ethanol solution on the glass plate. A 5% stacking gel was prepared, mixed evenly and added to the glass plate. Then a spiking comb was inserted. After 60 minutes, the glue was made.

8) Protein loading and electrophoresis

The gel plate was fixed in an electrophoresis tank, and the inner tank was filled with 1 × electrophoresis solution, while in the outer tank the electrophoresis solution was over the metal wire. The spiking comb was removed, the protein samples of Ba/F3-FL-EGFR (L858R/T790M/C797S) and Ba/F3-FL-EGFR (del19-E746-A750/T790M/C797S) cells were sampled 25 μg, and Protein Ladder was then added. Turning on the power supply, electrophoresis with a constant voltage of 70 V lasted for 30 min, electrophoresis was continued at constant voltage 86 V for 1 h 20 min until the bromophenol blue indicator band reached the bottom and electrophoresis was stopped.

9) Protein electroporation

The PVDF membrane was soaked in methanol for 30 s to wake up the membrane, and then put into a membrane-transfer cassette together with 4 pieces of filter paper and 2 sponges. 1× electroporation solution prepared in advance was poured into the membrane-transfer cassette. The gel was taken out, and cut into the required size. Sponge, filter paper, gel, PVDF membrane, filter paper, and sponge were put in sequence to the membrane-transfer cartridge, and the air bubbles were removed. The membrane was put on the anode side and the gel was put on the cathode side (black gel and white membrane), and inserted into the electrophoresis tank. The membrane-transfer buffer was added, and the ice bag was added. Ice bags were also put around the electrophoresis tank to cool. The electrophoretic transfer was carried out at a constant pressure of 100 V for 90 min.

10) Blocking and incubation of primary antibody

After electroporation was completed, the PVDF membrane was placed in a 5% skimmed milk blocking solution, shaken slowly on a shaker and blocked at room temperature for 1 h. The blocked PVDF membrane was washed slowly in TBST three times for 10 min each time. After the washing was completed, the PVDF membrane was put into an antibody cassette with primary antibody, which was diluted with primary antibody diluent according to the ratio of 1:1000 and the antibody cassette was incubated in a gentle shaker at 4°C (10-16 h).

11) Incubation of secondary antibody and developing color

The overnight incubated PVDF membrane was removed from the antibody cassette and the primary antibody was recovered. The PVDF membrane was washed 3 times slowly in TBST for 10 min each time. The washed PVDF membrane was put into the secondary antibody (1:3000) dilution (containing 5% skimmed milk) of the corresponding genus and incubated in a gentle shaker at room temperature for 1 h. After the secondary antibody incubation was completed, the PVDF membrane was taken out, and washed 3 times slowly in TBST for 10 min each time. The equal volume of liquid A and liquid B in ECL kit were mixed, added evenly on the surface of the membrane (protein side), and

put into Tanon 5200 luminescence imager (turned on pre-cool for 5 min in advance) for exposure and development. Pictures were taken to save the images.

12) Western Blot band gray value analysis

The bands of p-EGFR and GAPDH in the Western Blot result graphs were analyzed for gray values using Image J2X software, and the results of the gray values were presented as a graph with the horizontal coordinate as the compound concentration and the value of the vertical coordinate as the value of the inhibition rate of the drug concentration. The value of the vertical coordinate was calculated using the following formula: Inhibition (%) = 100% - $Gray_{compound}$ / $Gray_{dmso}$ *100%, which was fitted using GraphPad Prism software with log(inhibitor) vs. response -- Variable slope (four parameters). The assay results are shown in Table 5:

Table 5. Cell half-maximal inhibition concentration $IC_{50}$ (nM)

| **Compound** | **WX-006** | **WX-010 (trifluoroace tate)** | **WX-013** | **WX-027A** |
|---|---|---|---|---|
| **Ba/F3-FL-EGFR(L858R/T790M/C797S)** | 3.4 | 2.3 | 2.9 | 2.1 |
| **Ba/F3-FL-EGFR (de119-E746-A750/T790M/C797S)** | 0.6 | 0.8 | 1.3 | 1.9 |

**The results show that:** the compounds of the present disclosure had a good inhibitory effect on EGFR phosphorylation in cell lines carrying triple mutations of EGFR (L858R/T790M/C797S) and EGFR (dell 9-E746-A750/T790M/C797S).

**Assay example 4: Assay of cytochrome P450 isoenzyme inhibition:**

The purpose of this study was to determine the inhibitory effect of test compounds on the activity of human liver microsomal cytochrome P450 isoenzymes (CYP1A2, CYP2C9, CYP2C19, CYP2D6 and CYP3A4).

**$IC_{50}$ assay process:**

Working solutions (100×final concentration) with concentrations of 5.00, 1.50, 0.500, 0.150, 0.0500, 0.0150, and 0.00500 mM were prepared by gradient dilution of the test compound (10.0 mM), while working solutions of each positive inhibitor and its specific substrate mixtures of P450 isozymes (CYP1A2, CYP2C9, CYP2C19, CYP2D6, and CYP3A4) were prepared; human liver microsomes stored in a refrigerator below -60°C were thawed on ice; after all the human liver microsomes were thawed, the human liver microsomes were diluted with Potassium phosphate buffer (PB) to prepare a certain concentration of working solution (0.253 mg/ml).

First, 20.0 μL of the substrate mixture was added to a reaction plate (20.0 μL of PB was added to the Blank well), then 158 μL of human liver microsomal working solution was added to the reaction plate, and the reaction plate was placed on ice for use; at this time, 2.00 μL of each concentration of the test compound (N = 1) and specific inhibitor (N = 2) were added to the corresponding wells, and to the group without inhibitor (test compound or positive inhibitor) was added the corresponding organic solvent as control samples (1:1 DMSO:MeOH for the control sample of the test compound, and 1:9 DMSO:MeOH for all the positive control samples); after pre-incubation in a water bath at 37 °C for 10 min, 20.0 μL of cofactor (NADPH) solution was added to the reaction plate, and the reaction plate was incubated in a water bath at 37 °C for 10 min; The reaction was terminated by adding 400 μL of pre-cooled acetonitrile solution (containing 200 ng/mL Tolbutamide and Labetalol internal standard); the reaction plate was shaken on a shaker for 10 min; then centrifuged at 4000 rpm at 4 °C for 20 min; 200 μL of the supernatant was added to 100 μL of water to dilute the samples; and the plate was sealed, shaken for 10 min to mix well, and then analyzed by LC/MS/MS.

The rate of production of metabolites of each probe substrate was used to reflect the activity of each CYP isozyme. The activity of each isozyme in the solvent control incubation system without test article or positive inhibitor was set to be 100%, and the rate of production of metabolites of the probe substrate with different concentrations of test article or positive inhibitor was divided by the rate of production of metabolites of the solvent control samples, and then multiplied by 100% as the percentage of residual activity of each isozyme. The $IC_{50}$ values of the test article and each positive inhibitor were calculated by nonlinear regression analysis using three or four parameters in XL fit software with the percentage of residual activity as the vertical coordinate and the inhibitor concentration as the horizontal coordinate. The $IC_{50}$ values were labeled as ">50 μM" when the $IC_{50}$ fitted by XL fit software was greater than the highest administered concentration (50 μM) or when the $IC_{50}$ could not be fitted. The assay results are shown in Table 6.

Table 6. Cytochrome P450 isoenzyme inhibitory concentration (μM)

| Compound | WX-027A | WX-019 |
|---|---|---|
| CYP1A2 | >50 | >50 |
| CYP2C9 | 14 | 31 |
| CYP2C19 | 9.8 | 3.4 |
| CYP2D6 | >50 | >50 |
| CYP3A4 | 20 | >50 |

Assay conclusion: The compounds of the present disclosure had weak inhibitory effect on P450 isoenzyme.

**Assay example 5: Pharmacokinetic assay of compounds in mice**

Assay purpose: 7-9-week-old male ICR mice were used as test animals. LC/MS/MS method was used to determine the drug concentration of the compound in the plasma at different times after a single intravenous injection (IV) and oral administration (PO) of the compound, to study the pharmacokinetic behavior of the compounds of the present disclosure in mice, and to evaluate their pharmacokinetic characteristics.

Formulation of drug: The compounds were formulated as solutions in the respective solvents for administration in IV (intravenous injection) and PO (oral administration) groups. The dose of each compound was 3 mg/kg for IV and 10 mg/kg for PO, and the vehicle was 10% DMSO + 10% solutol + 80% (10% HP-β-CD). The results of pharmacokinetic parameters are shown in Table 7:

Table 7. In vivo pharmacokinetic test results in mice

| | | WX-006 | WX-010 (trifluoroacetate) | WX-013 | WX-019 | WX-027A |
|---|---|---|---|---|---|---|
| IV | Starting concentration $C_0$ (nM) | 8144 | 8138 | 12124 | 5733 | 6849 |
| | Half-life $T_{1/2}$ (h) | 3.6 | 2.8 | 3.3 | 2.5 | 1.1 |
| | Apparent volume of distribution Vd (L/kg) | 1.9 | 1.0 | 1.0 | 1.6 | 1.0 |
| | Apparent clearance Cl (mL/Kg/min) | 14.6 | 5.7 | 4.7 | 8.6 | 14.2 |
| | Area under the curve $AUC_{0\text{-last}}$ (nM.hr) | 6536 | 16112 | 18870 | 10569 | 7702 |
| PO | Peak concentration $C_{max}$ (nM) | 3594 | 4492 | 3938 | 3921 | 5908 |
| | Time to peak $T_{max}$ (h) | 1.5 | 1.0 | 1.0 | 1.0 | 0.8 |
| | Area under the curve $AUC_{0\text{-last}}$ (nM.hr) | 18117 | 30054 | 21521 | 23165 | 15682 |
| | Bioavailability F% | 83% | 56% | 34% | 65% | 64% |

**The results show that:** the compounds of the present disclosure exhibited excellent pharmacokinetic properties after administration to mice.

**Assay example 6: In vivo efficacy assay of compounds in mice**

**1. Assay method**

1.1 Cell culture and inoculation

Ba/F3-FL-EGFR (del19-T790M-C797S) cell line was cultured in RPMI1640 medium (Viva Cell) + 10% fetal

bovine serum (Viva Cell) + 1% double-antibody (Penicillin Streptomycin solution, Gibco, USA) at 37°C, 5 % $CO_2$, and passaged 2-3 times a week. When the cell saturation was 80%-90%, cells were collected, counted and inoculated. When the cells in the logarithmic growth phase reached the required number for the assay, the cells were collected, and centrifuged at 1000 rpm for 5 min to remove the supernatant. The cells were resuspended with medium, and counted using a cell counter. According to the counting results, the original solution was diluted into a cell suspension with a concentration of $1*10^7$ viable cells/mL, and the cell viability was 98.77%, P17 generation. The diluted cell suspension and matrix gel were diluted in a 1:1 ratio. After mixing well and placing on ice, the suspension was aspirated with a 1 mL sterile insulin syringe, and each mouse was subcutaneously inoculated with 0.2 mL of cell suspension in the right axilla. That is, each mouse was inoculated with $1*10^6$ Ba/F3-FL-EGFR (del19-T790M-C797S) cells.

1.2 Grouping method

After the inoculation was completed, the tumor growth status was observed daily. When the average tumor volume reached approximately 100~150 $mm^3$, the mice were randomly divided into groups according to tumor volume.

1.3 Type of administration

Dosage according to mouse body weight: 10 μL/g. If the body weight decreases by more than 20%, the dosing regimen was adjusted accordingly.

1.4 Observation

The formulation and any modifications of this assay protocol were evaluated and approved by the Laboratory Animal Welfare Ethics Committee of the testing institution. The health status and death of animals were checked every day. Routine inspections included tumor growth, activity, diet, body weight, eyes, hair and other abnormal behaviors of animals. Tumor volume and body weight were measured three times a week (Monday, Wednesday and Friday). The tumor volume was measured with a vernier caliper, and the formula was $TV = 0.5\ a \times b^2$, where a is the long diameter of a tumor and b is the short diameter of a tumor.

1.5 Statistical analysis of data

Statistical analysis included the mean (Mean) and standard error (SEM) of tumor volume at each time point for each group. Statistical analysis was performed to evaluate differences between groups based on the data on the 10th day after administration. One way ANOVA post hoc test and Tamhane test were used to compare whether there was a significant difference in tumor volume between the treatment group and the control group. $p < 0.05$ meaned a significant difference. Drug efficacy evaluation indicators included tumor volume and tumor weight to examine whether tumor growth was delayed, inhibited or cured. The anti-tumor efficacy of compounds was evaluated using the relative tumor proliferation rate T/C (%) or the tumor growth inhibition rate TGI (%). Tumor growth inhibition was calculated by T/C and TGI (TGI (%) = $[1-(T_{10}-T_0)/(V_{10}-V_0)] \times 100\%$). In principle, the evaluation criteria were: T/C (%) > 40% or TGI (%) < 40% represents ineffective; T/C (%) ≤ 40% and $p < 0.05$, or TGI (%) ≥ 40% and $p < 0.05$ represents effective.

**2. Assay results**

2.1 Body weight

The body weight of assay animals was used as a reference index to indirectly determine drug toxicity. The results are shown in Table 8.

Table 8. Average body weight of animals in each group after administration

| Days After administration | Body weight (g) | | | | |
|---|---|---|---|---|---|
| | Control group | WX- 027A 10 mg/kg/bid | WX-027A 25 mg/kg/bid | WX-019 10 mg/kg/bid | WX-019 25 mg/kg/bid |
| 0 | 21.6 | 21.7 | 21.0 | 20.6 | 22.5 |
| 3 | 21.9 | 21.9 | 21.5 | 20.7 | 23.4 |
| 5 | 22.4 | 22.1 | 21.6 | 20.8 | 23.0 |
| 7 | 23.1 | 22.1 | 21.4 | 20.7 | 22.8 |

(continued)

| Days After administration | Body weight (g) | | | | |
|---|---|---|---|---|---|
| | Control group | WX- 027A 10 mg/kg/bi d | WX-027A 25 mg/kg/bid | WX-019 10 mg/kg/bi d | WX-019 25 mg/kg/bi d |
| 10 | 23.9 | 22.2 | 21.8 | 20.9 | 23.3 |

2.2 Tumor volume

The results of tumor volume changes are shown in Table 9.

Table 9. The average tumor volume of animals in each group after administration

| Days After administration | Tumor volume ($mm^3$) | | | | |
|---|---|---|---|---|---|
| | Control group | WX-027A 10 mg/kg/bi d | WX-027A 25 mg/kg/bid | WX-019 10 mg/kg/bi d | WX-019 25 mg/kg/bi d |
| 0 | 113 | 113 | 112 | 113 | 113 |
| 3 | 349 | 123 | 105 | 133 | 120 |
| 5 | 630 | 111 | 96 | 140 | 94 |
| 7 | 941 | 183 | 108 | 148 | 130 |
| 10 | 1256 | 179 | 86 | 106 | 82 |

2.3 Anti-tumor efficacy evaluation indicators

The results of anti-tumor efficacy evaluation indicators are shown in Table 10.

Table 10. Evaluation of anti-tumor efficacy of test compounds in Ba/F3-FL-EGFR-Del19-T790M-C797S cell homograft tumor model

| Group | Tumor volume ($mm^3$) (Day 10) | T/C (%) | TGI (%) | *p* value |
|---|---|---|---|---|
| **Control group** | 1256 | -- | -- | -- |
| **WX-027 (10 mg/kg/bid)** | 179 | 14 | 94 | < 0.01 |
| **WX-027 (25 mg/kg/bid)** | 86 | 7 | 102 | < 0.01 |
| **WX-019 (10 mg/kg/bid)** | 106 | 8 | 101 | < 0.01 |
| **WX-019 (25 mg/kg/bid)** | 82 | 7 | 103 | < 0.01 |

**3. Assay results**

At the end of the assay, compounds WX-027A and WX -019 showed a sustained increase in body weight of the mice after 10 days of continuous administration at the administered doses of 10 mg/kg and 25 mg/kg, respectively, with no significant difference as compared to that in the control group, showing well tolerance in animals.

Meanwhile, the tumor inhibition rate of WX-027A was 94% and 102% in the 10 mg/kg and 25 mg/kg dose groups, respectively, and the P-value was <0.01; and the tumor inhibition rate of WX-019 was 101% and 103% in the 10 mg/kg and 25 mg/kg dose groups, respectively, and the P-value was <0.01. The compounds of the present disclosure significantly inhibited the growth of transplanted tumors.

**Claims**

**1.** A compound of formula (V) or a pharmaceutically acceptable salt thereof,

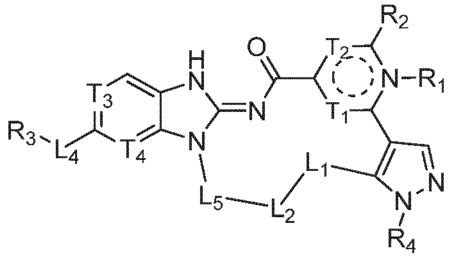

( V )

wherein

$T_1$ and $T_2$ are each independently selected from N and CH;
$T_3$ is selected from N and $CR_e$;
$T_4$ is selected from N and CRr;
$L_1$ is selected from -O- and -NH-;
$L_2$ is selected from $-CH_2-$ and -C(=O)-;
$L_3$ is selected from -O-, -NH- and $-CH_2-$;
$L_4$ is selected from $-C_{1-3}$ alkyl-, $-P(=O)(R_a)-$, $-S(=O)_2-$, $-S(=O)(=NR_c)-$ and $-N=S(=O)(R_b)-$;
$L_5$ is selected from

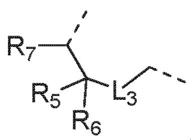

,

$C_{3-5}$ cycloalkyl, 4- to 5-membered heterocycloalkyl, $-CH_2-C_{5-6}$ cycloalkyl-, $-CH_2-C_{5-6}$ cycloalkyl-$CH_2$-, $-CH_2$-5- to 6-membered heterocycloalkyl and $-CH_2$-5- to 6-membered heterocycloalkyl-$CH_2$-;
$R_1$ is absent, $R_2$ is selected from $CH_3$ and cyclopropyl;
alternatively, $R_1$ and $R_2$ are taken together with the atoms to which they are attached to form a 5-membered heteroaryl;
$R_3$ is selected from $C_{1-3}$ alkyl, $C_{3-5}$ cycloalkyl and -piperazinyl-methyl;
$R_4$ is selected from $C_{1-3}$ alkyl, wherein the $C_{1-3}$ alkyl is optionally substituted with 1, 2 or 3 $R_d$;
$R_5$ is selected from $CH_3$, wherein the $CH_3$ is optionally substituted with 1, 2 or 3 halogens;
$R_6$ is selected from H and $CH_3$, wherein the $CH_3$ is optionally substituted with 1, 2 or 3 halogens;
$R_7$ is selected from Hand $CH_3$, wherein the $CH_3$ is optionally substituted with 1, 2 or 3 halogens; alternatively, $R_5$ and $R_6$ are taken together with the atom to which they are attached to form a cyclopropyl;
$R_a$ is selected from $C_{1-3}$ alkyl and $C_{3-5}$ cycloalkyl;
$R_b$ is selected from $C_{1-3}$ alkyl;
$R_c$ is selected from H and $C_{1-3}$ alkyl;
alternatively, $R_a$ and $R_3$ are taken together with the atom to which they are attached to form a 5- to 6-membered heterocycloalkyl, wherein the 5- to 6-membered heterocycloalkyl is optionally substituted with 1 or 2 $CH_3$;
alternatively, $R_b$ and $R_3$ are taken together with the atoms to which they are attached to form a 5- to 11-membered heterocycloalkyl, wherein the 5- to 11-membered heterocycloalkyl is optionally substituted with 1 or 2 $R_g$;
alternatively, $R_c$ and $R_3$ are taken together with the atoms to which they are attached to form a 5- to 6-membered heterocycloalkyl, wherein the 5- to 6-membered heterocycloalkyl is optionally substituted with 1 or 2 $CH_3$;
$R_d$ is selected from halogen and $C_{1-3}$ alkylamino;
$R_e$ is selected from H, F, Cl, Br, I and CN;
$R_f$ is selected from H, F, Cl, Br, I and CN;
$R_g$ is selected from $CH_3$ and 4- to 6-membered heterocycloalkyl, wherein the 4- to 6-membered heterocycloalkyl is optionally substituted with 1 or 2 $CH_3$;
provided that, when $L_1$ is selected from -O-, $L_2$ is selected from $-CH_2-$, $L_3$ is selected from - $CH_2$-, $L_4$ is selected from $-CH_2-$, and $R_3$ is selected from -piperazinyl-methyl, then at least one of $T_1$ and $T_2$ is selected from N, alternatively, $R_1$ and $R_2$ are taken together with the atoms to which they are attached to form a 5-membered heteroaryl group.

**2.** The compound according to claim 1 or a pharmaceutically acceptable salt thereof, wherein $R_g$ is selected from $CH_3$, oxetanyl, azetidinyl, tetrahydropyranyl, morpholinyl, piperidinyl, piperazinyl and N-methylpiperidinyl.

**3.** The compound according to claim 1 or a pharmaceutically acceptable salt thereof, wherein $R_a$ is selected from $CH_3$, $CH_2CH_3$, $CH(CH_3)_2$ and cyclopropyl.

**4.** The compound according to claim 1 or a pharmaceutically acceptable salt thereof, wherein $R_a$ and $R_3$ are taken together with the atom to which they are attached to form a

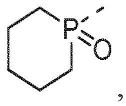,

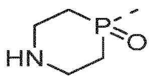 and 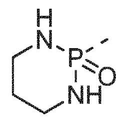,

wherein the

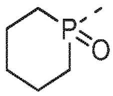, 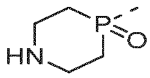 and 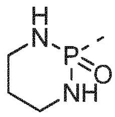

are optionally substituted with 1 or 2 $CH_3$.

**5.** The compound according to claim 1 or a pharmaceutically acceptable salt thereof, wherein $R_b$ and $R_3$ are taken together with the atoms to which they are attached to form a 5- to 6-membered monocyclic heterocycloalkyl and 7- to 11-membered bicyclic heterocycloalkyl, wherein the 5- to 6-membered monocyclic heterocycloalkyl and 7- to 11-membered bicyclic heterocycloalkyl are optionally substituted with 1 or 2 $R_g$.

**6.** The compound according to claim 1 or 5 or a pharmaceutically acceptable salt thereof, wherein $R_b$ and $R_3$ are taken together with the atoms to which they are attached to form a

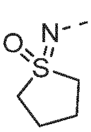, 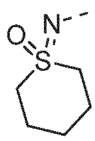, 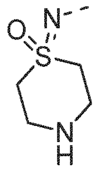, 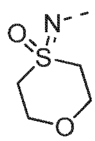, 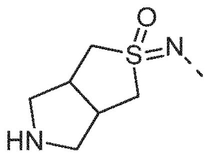, 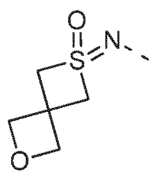, 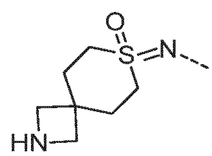,

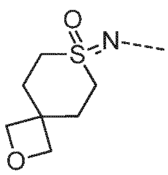, 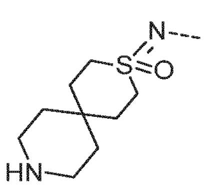 and 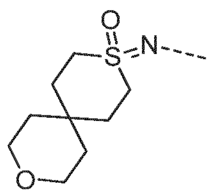,

wherein the

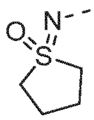, 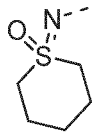,

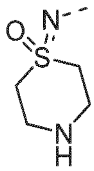, 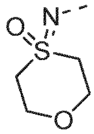, 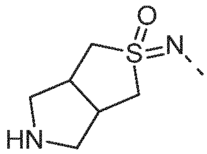, 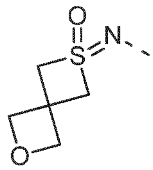, 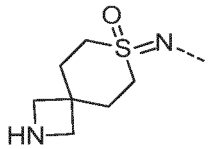, 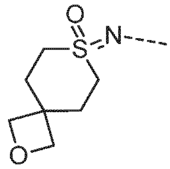,

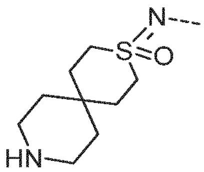 and 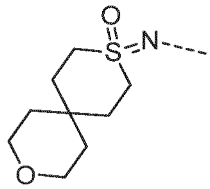

are optionally substituted with 1 or 2 $R_g$.

7. The compound according to claim 1 or a pharmaceutically acceptable salt thereof, wherein $R_c$ and $R_3$ are taken together with the atoms to which they are attached to form

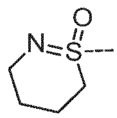,

wherein the

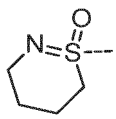

is optionally substituted with 1 or 2 $CH_3$.

8. The compound according to claim 1 or a pharmaceutically acceptable salt thereof, wherein $R_b$ is selected from $CH_3$, $CH_2CH_3$ and $CH(CH_3)_2$.

9. The compound according to claim 1 or a pharmaceutically acceptable salt thereof, wherein $L_4$ is selected from $-CH_2-$, $-P(=O)(CH_3)-$, $-P(=O)(CH_2CH_3)-$,

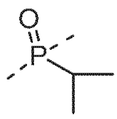, 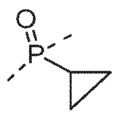,

$-S(=O)_2-$, $-S(=O)(=NH)-$, $-S(=O)(=NCH_3)-$, $-S(=O)(=NCH_2CH_3)-$, $-N=S(=O)(CH_3)-$, and $-N=S(=O)(CH_2CH_3)-$.

10. The compound according to claim 1 or a pharmaceutically acceptable salt thereof, wherein $R_3$ is selected from $CH_3$, $CH_2CH_3$, $CH(CH_3)_2$, cyclopropyl and

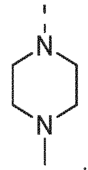.

**11.** The compound according to claim 1 or a pharmaceutically acceptable salt thereof, wherein $R_4$ is selected from $CH_3$, $CH_2CH_2N(CH_3)_2$ and $CH_2CH_2NHCH(CH_3)_2$.

**12.** The compound according to claim 1 or a pharmaceutically acceptable salt thereof, wherein the structural moiety $-L_4-R_3$ is selected from

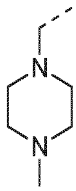, 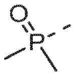, 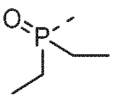, 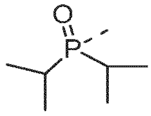,

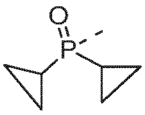, 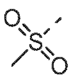, 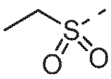, 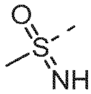, 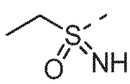, 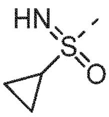, 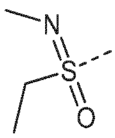, 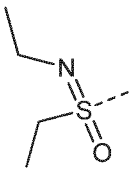,

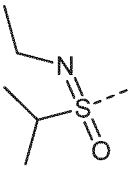, 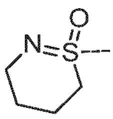, 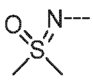, 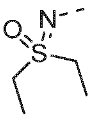, 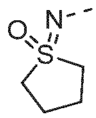, 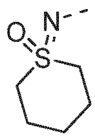, 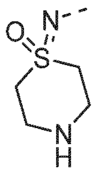, 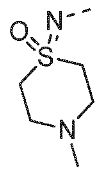,

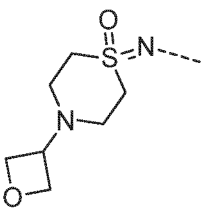, 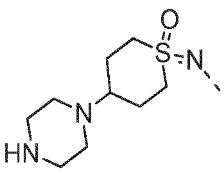, 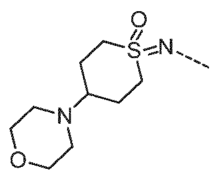, 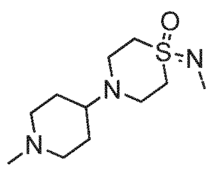,

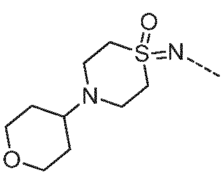, 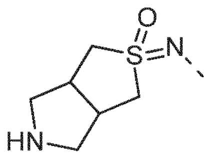, 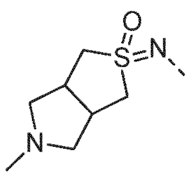, 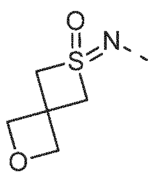, 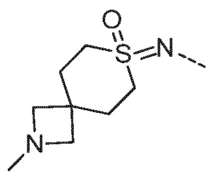,

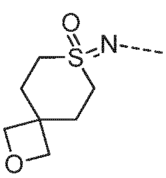, 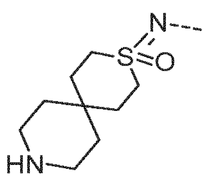, 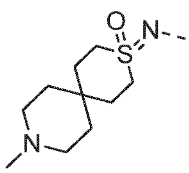, 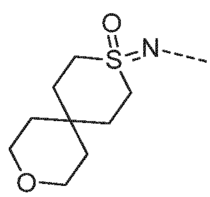, 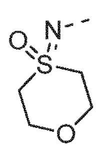, 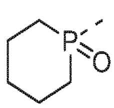,

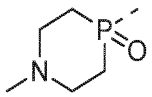 and 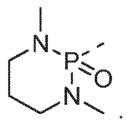.

**13.** The compound according to claim 1 or a pharmaceutically acceptable salt thereof, wherein $L_5$ is selected from $-CH_2CH(CH_3)CH_2CH_2-$, $-CH_2CH(CH_3)OCH_2-$, $-CH_2C(CH_3)_2CH_2CH_2-$, $-CH(CH_3)CH(CH_3)CH_2CH_2-$, $-CH_2CH(CF_3)CH_2CH_2-$, cyclopentyl, pyrrolidinyl, $-CH_2$-cyclopentyl-, $-CH_2$-cyclopentyl-$CH_2$-, $-CH_2$-cyclohexyl-, $-CH_2$-pyrrolidinyl-, $-CH_2$-tetrahydrofuranyl- and

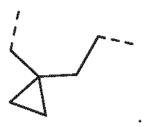.

**14.** The compound according to claim 1 or 13 or a pharmaceutically acceptable salt thereof, wherein $L_5$ is selected from

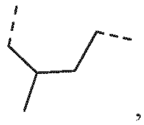, 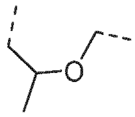, 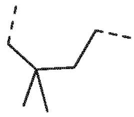, 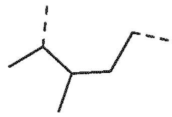, 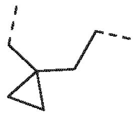,

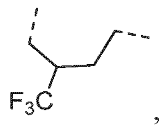, 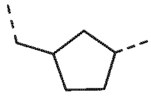, 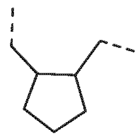, 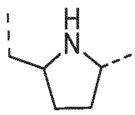, 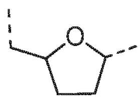 and 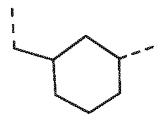.

**15.** The compound according to claim 1 or a pharmaceutically acceptable salt thereof, wherein the structural moiety

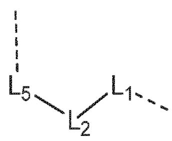

is selected from

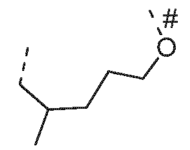, 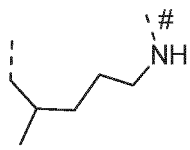,

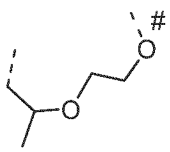, 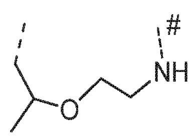, 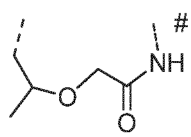, 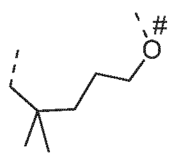, 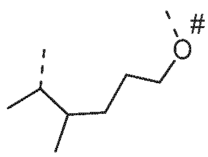,

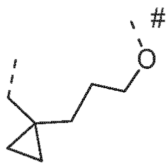 , 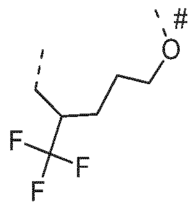 , 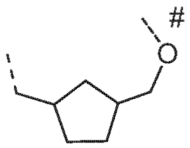 , 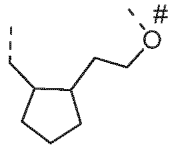 , 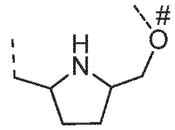 ,

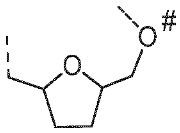 and 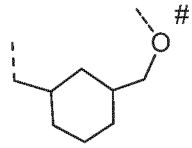 ,

wherein the "#" end is connected to the pyrazolyl group.

**16.** The compound according to claim 1 or 15 or a pharmaceutically acceptable salt thereof, wherein the structural moiety

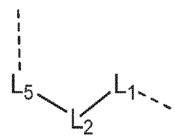

is selected from

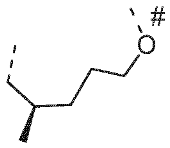 , 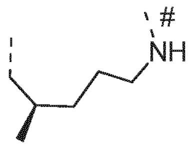 ,

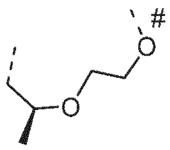 , 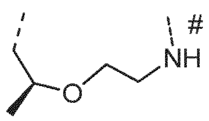 , 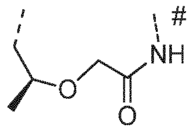 , 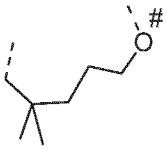 , 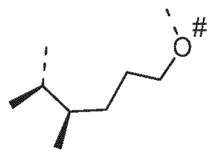 ,

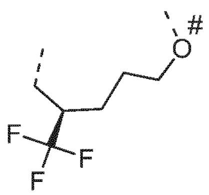 , 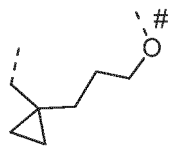 , 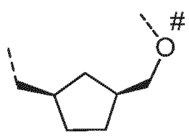 , 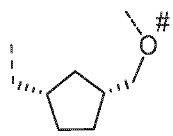 , 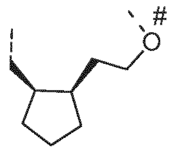 ,

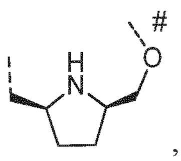 , 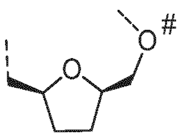 and 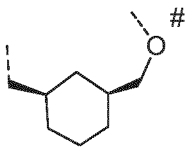 ,

wherein the "#" end is connected to the pyrazolyl group.

**17.** The compound according to claim 1 or a pharmaceutically acceptable salt thereof, wherein the structural moiety

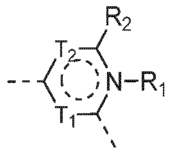

is selected from

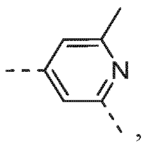, 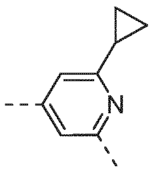, 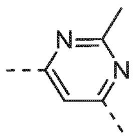,

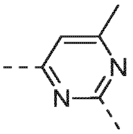, 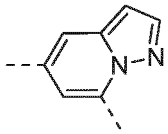, 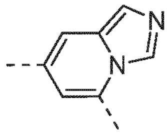, 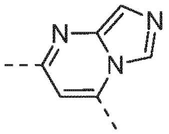 and 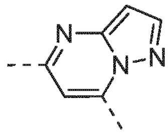.

**18.** The compound according to any one of claims 1 to 17 or a pharmaceutically acceptable salt thereof, wherein the compound is selected from

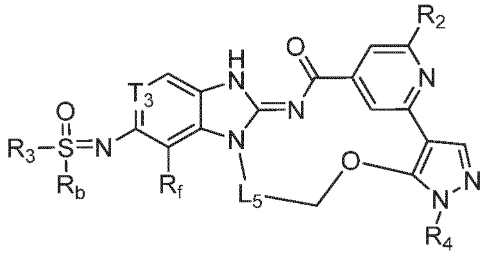

( VI-1 ) ,

wherein $T_3$, $L_5$, $R_2$, $R_3$, $R_4$, $R_b$ and $R_f$ are as defined in any one of claims 1 to 17.

**19.** A compound which is selected from the following compounds, or a pharmaceutically acceptable salt thereof,

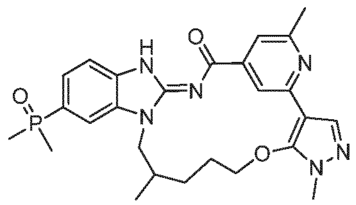 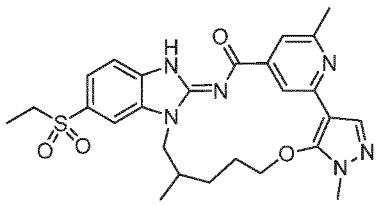 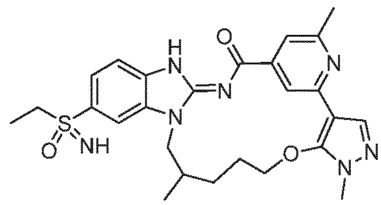

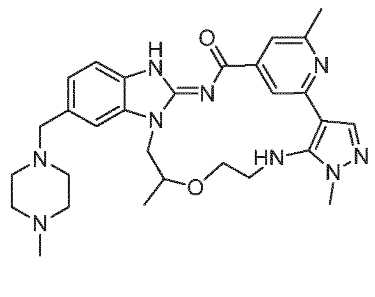 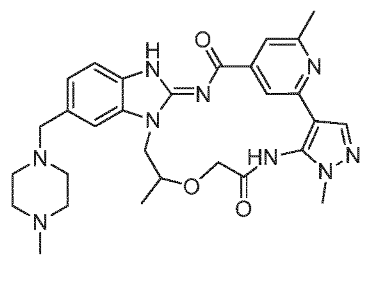 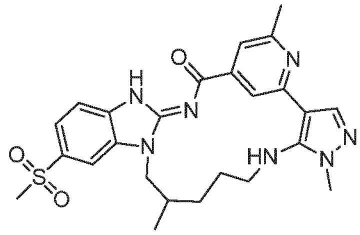

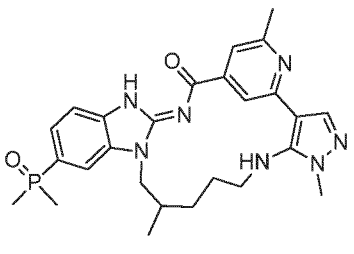
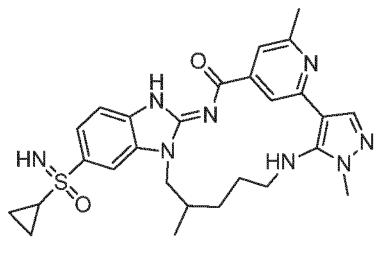
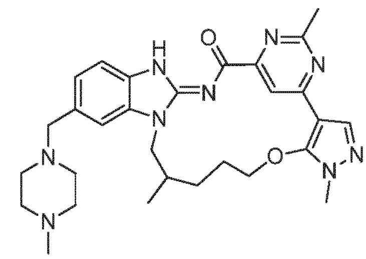

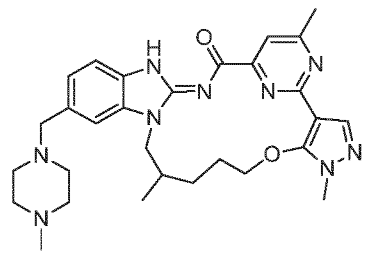
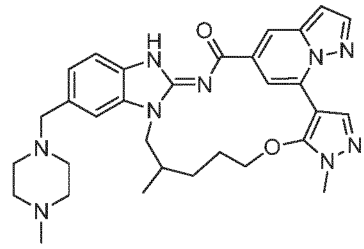
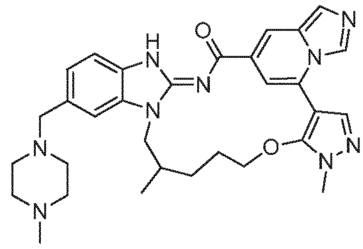

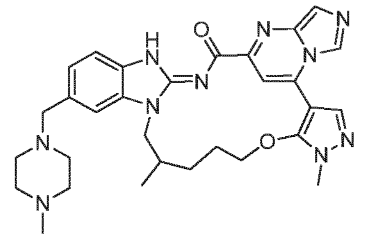
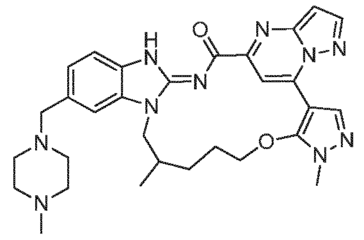
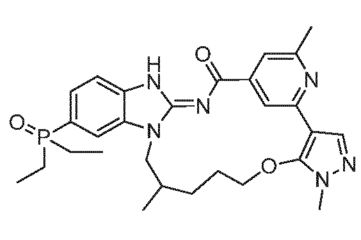

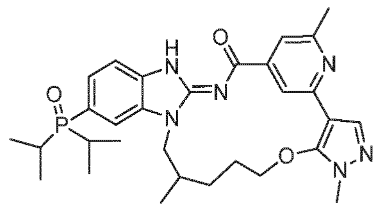
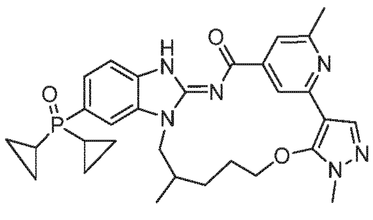
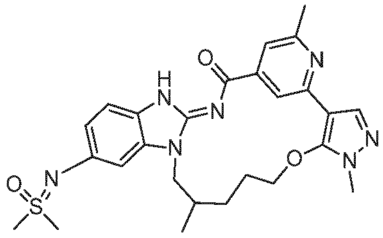

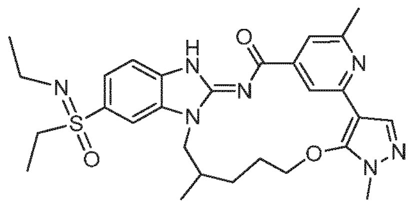
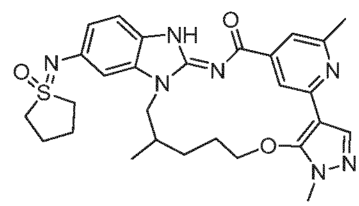
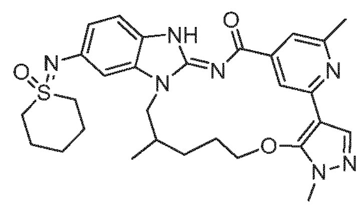

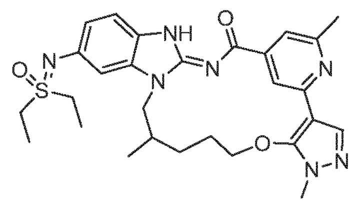
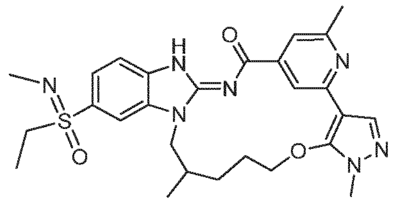
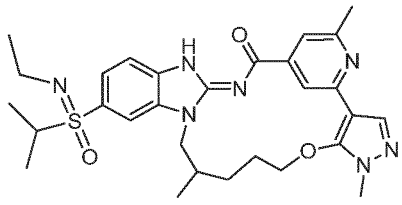

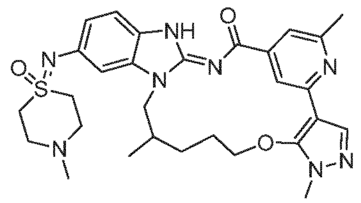
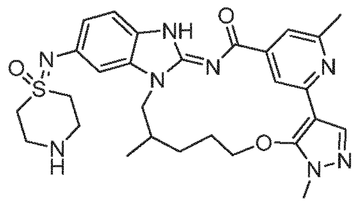
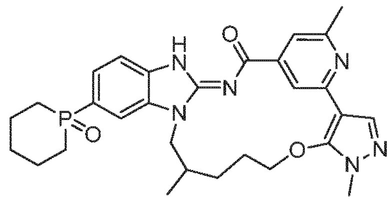

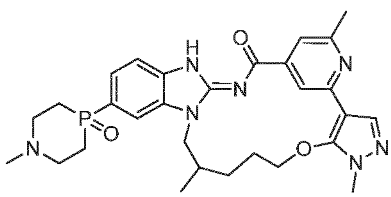
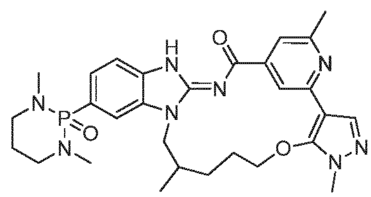
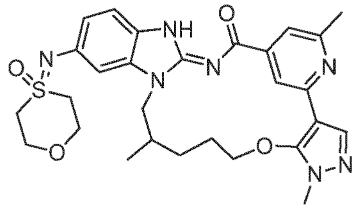

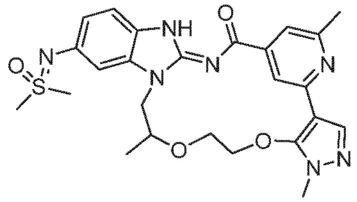
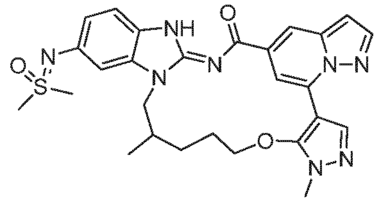
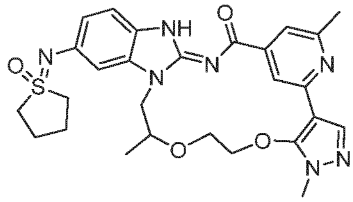

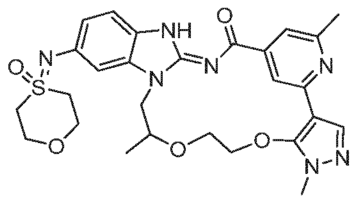
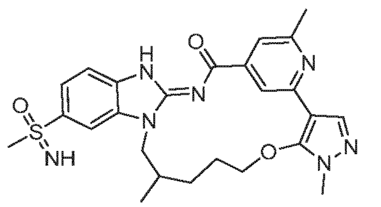
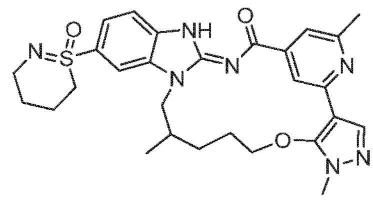

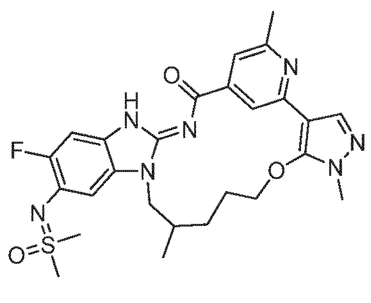
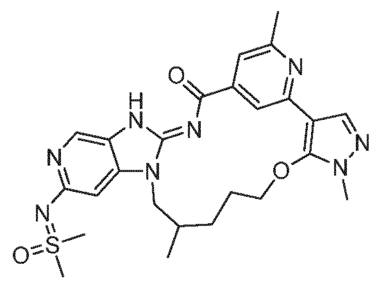
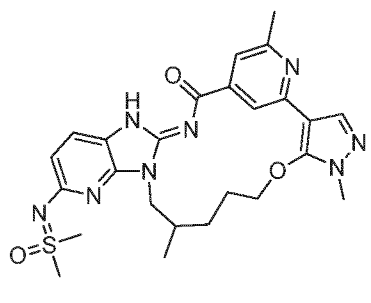

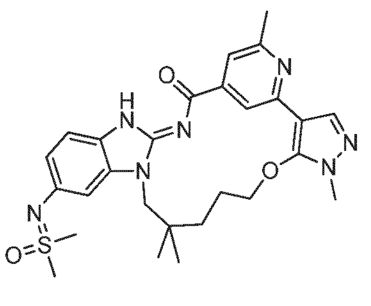
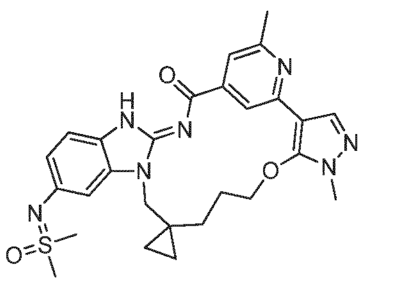
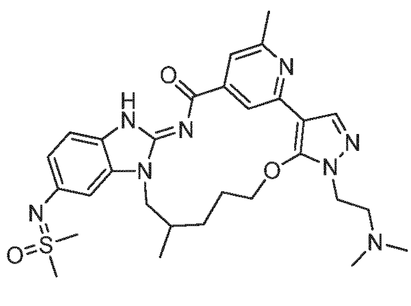

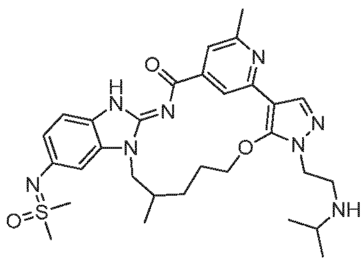
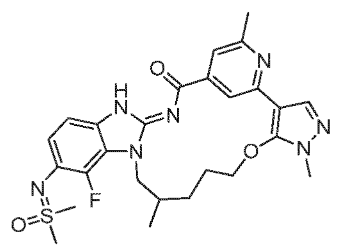
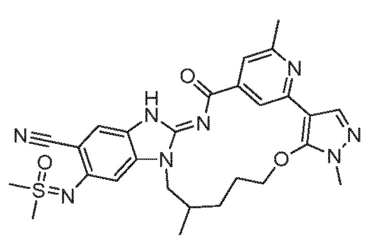

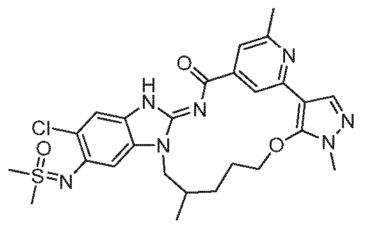
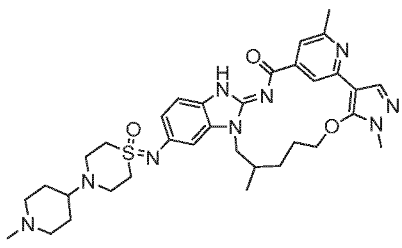
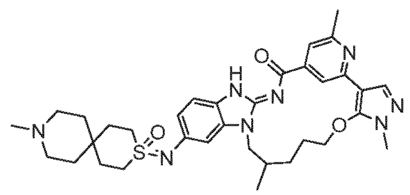

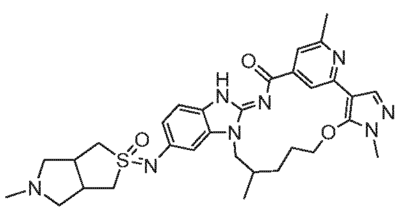
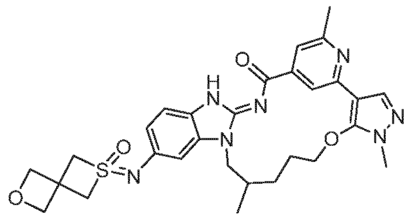
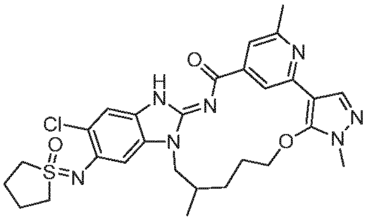

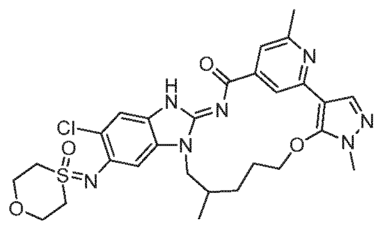
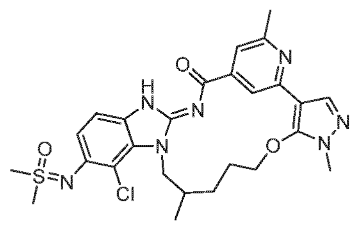
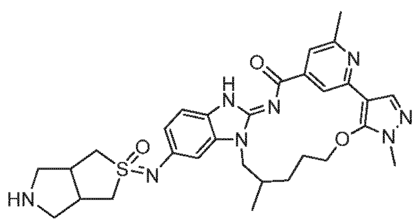

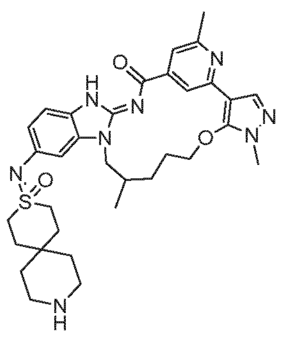
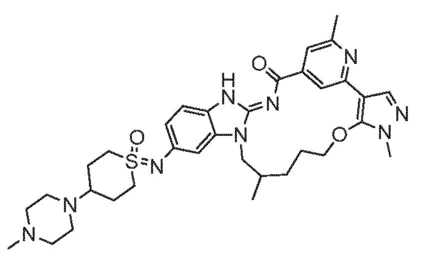
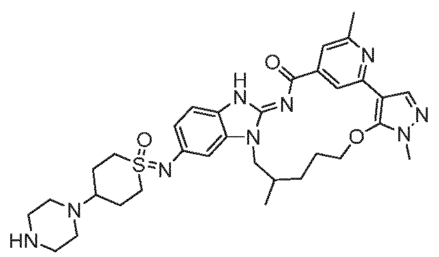

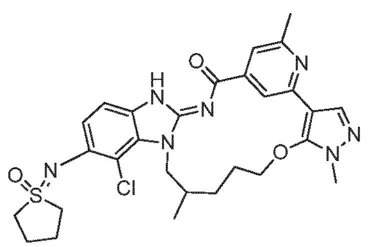
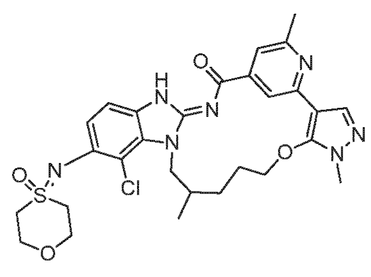
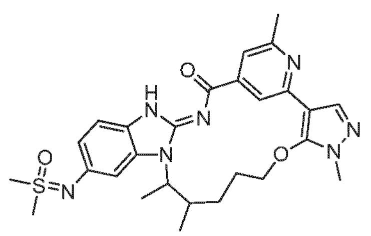

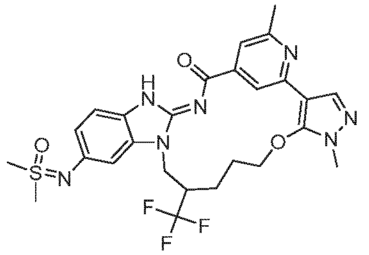
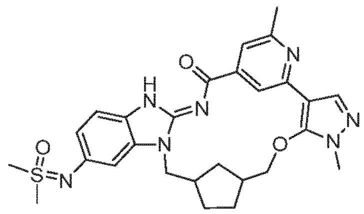
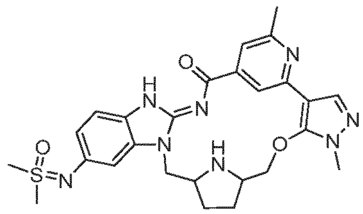
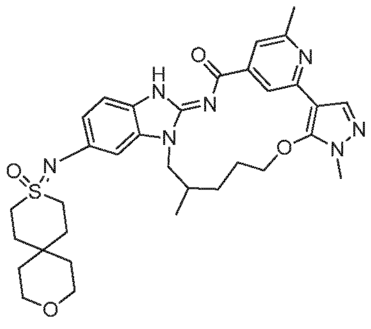
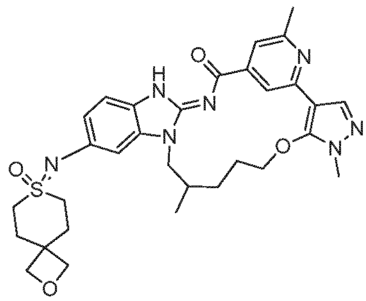
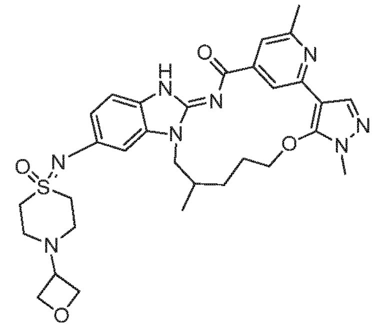
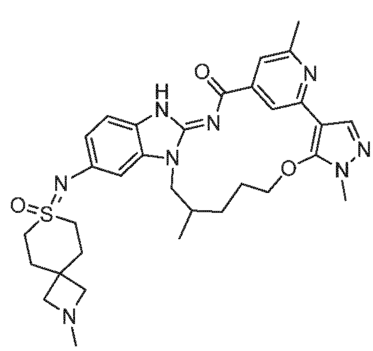
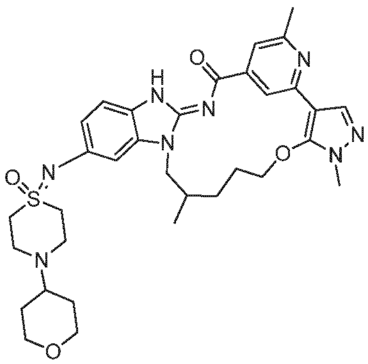
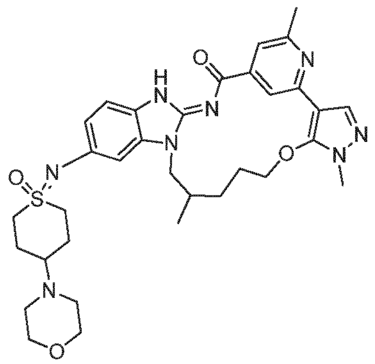
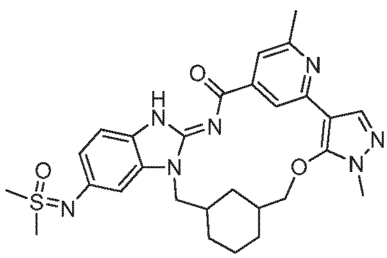
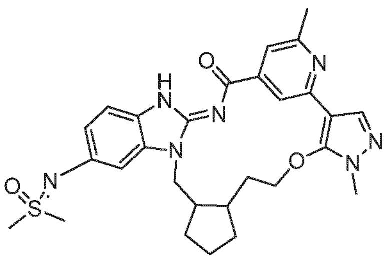
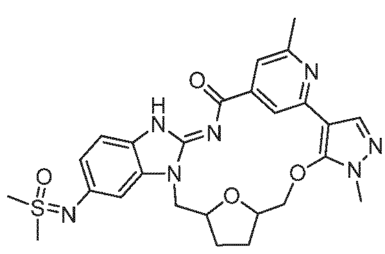
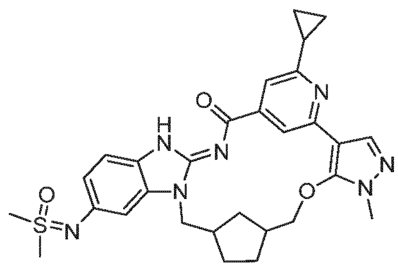

.

**20.** The compound according to claim 1 or a pharmaceutically acceptable salt thereof, wherein the compound is selected from,

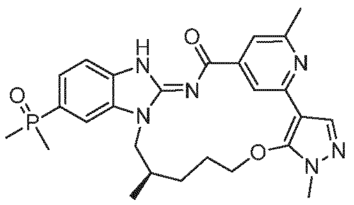
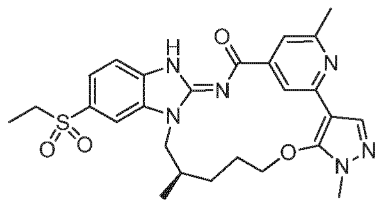
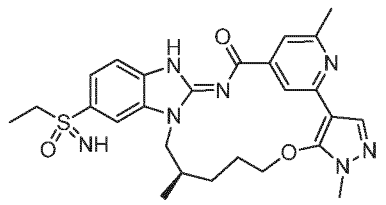

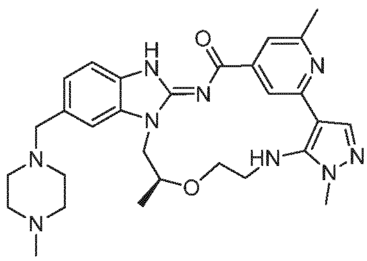
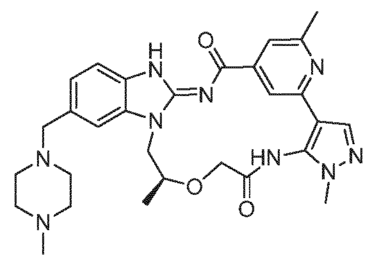
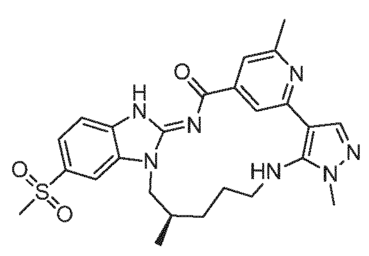

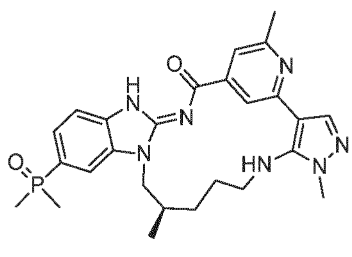
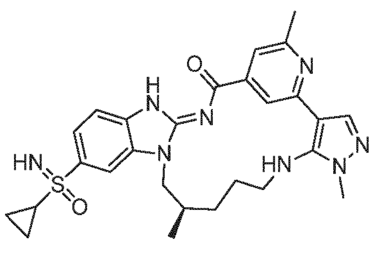
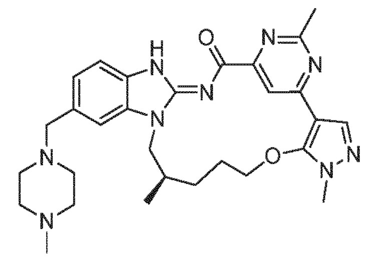

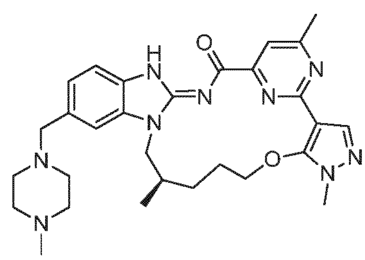
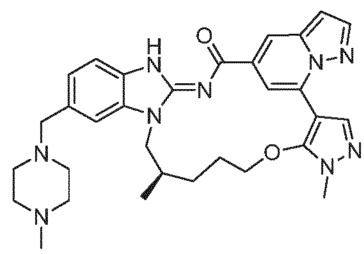
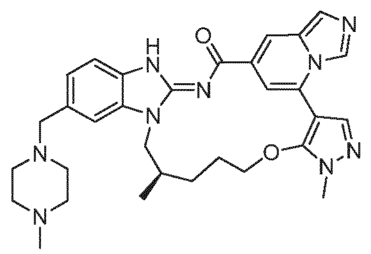

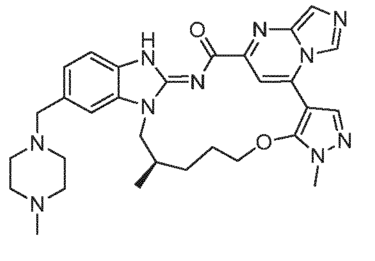
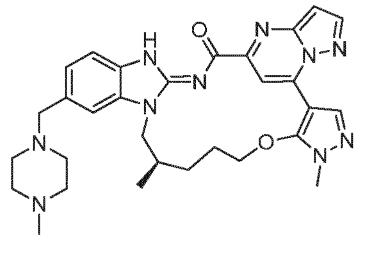
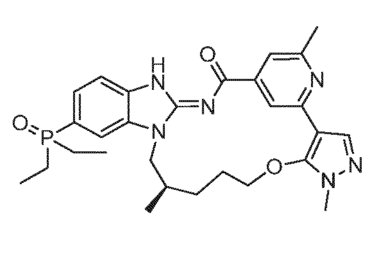

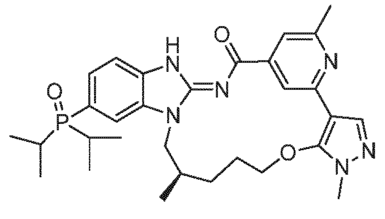
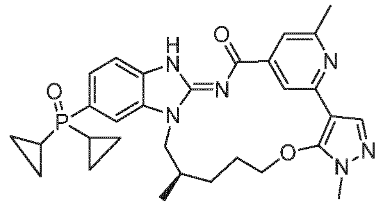
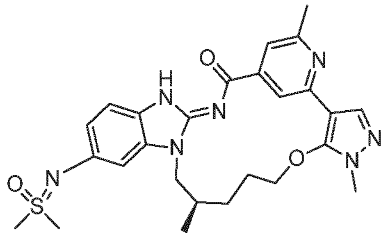

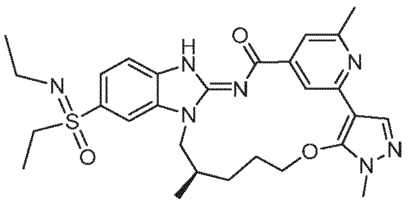
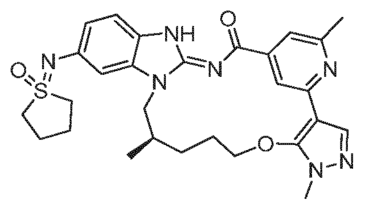
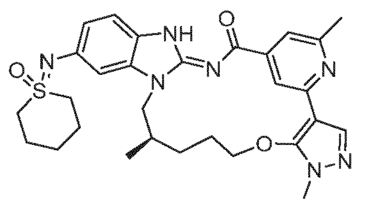

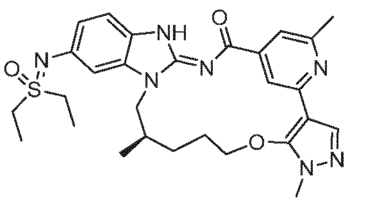
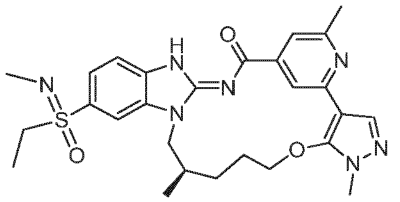
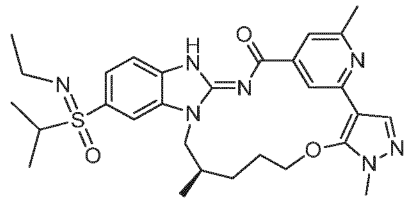

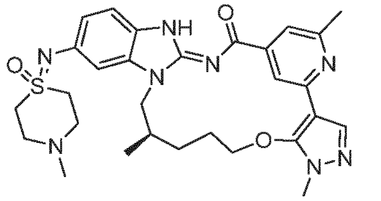
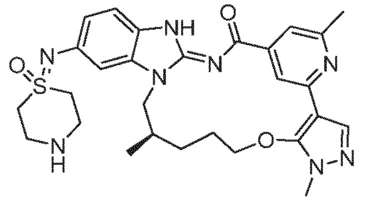
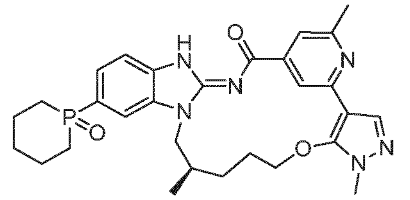

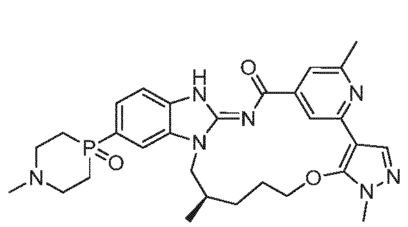
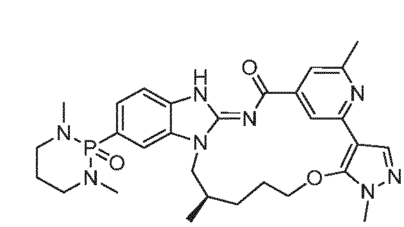
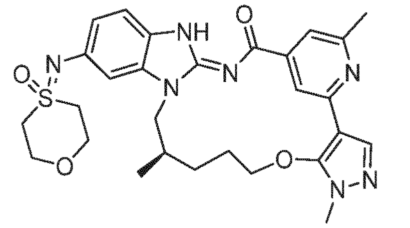

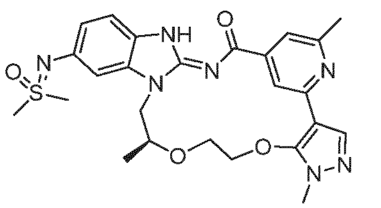
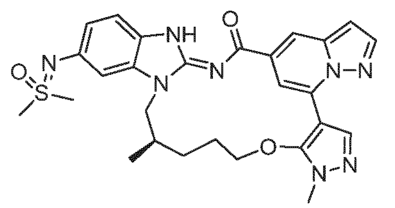
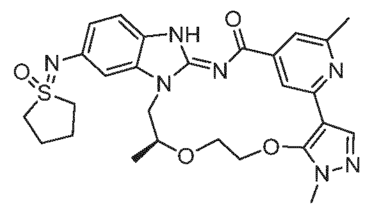

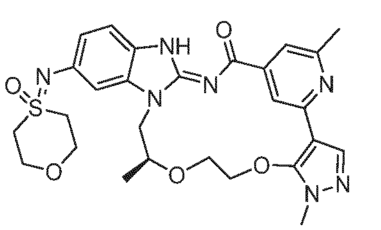
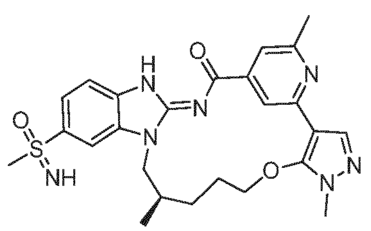
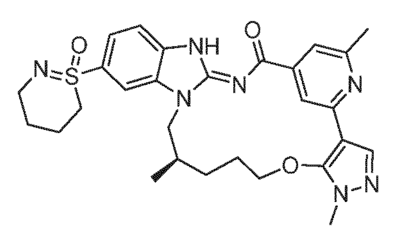

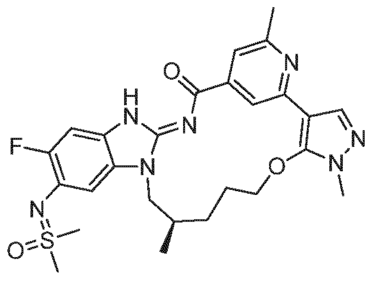

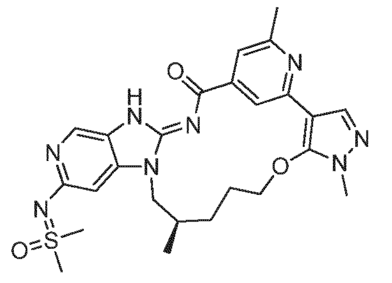

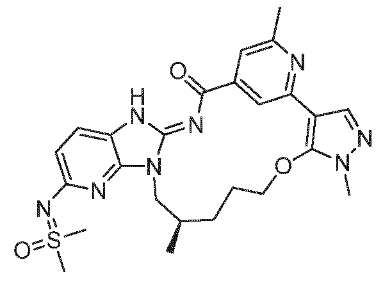

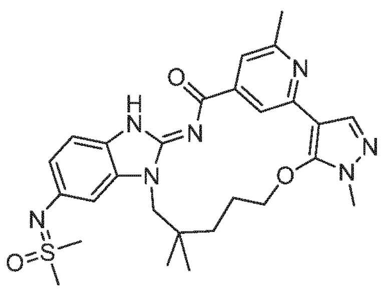

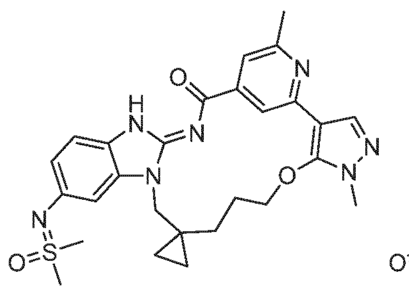

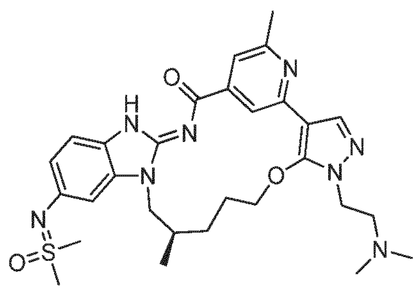

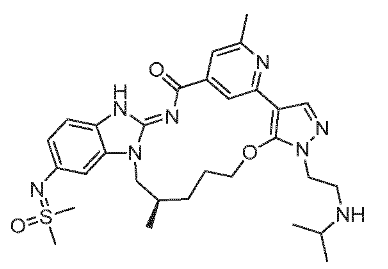

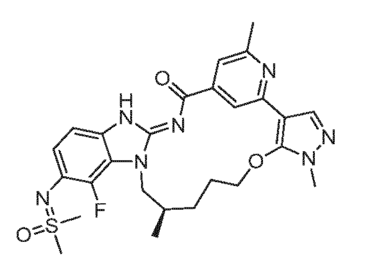

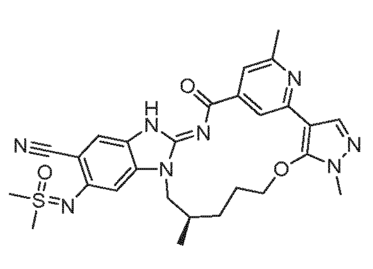

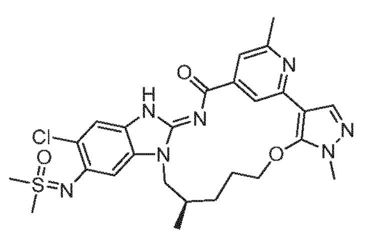

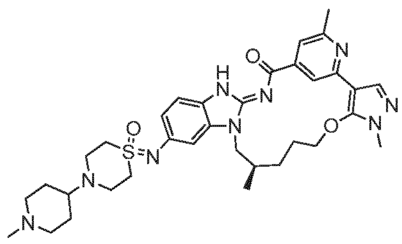

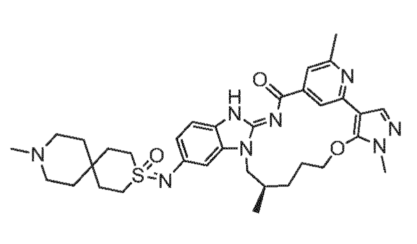

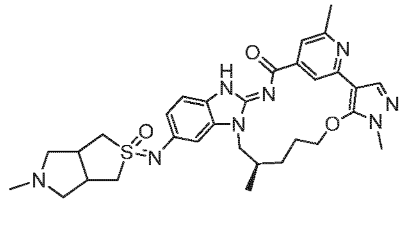

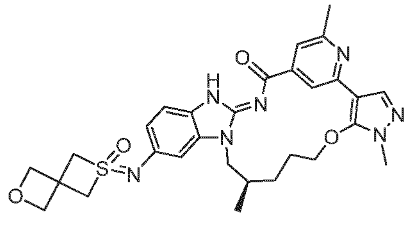

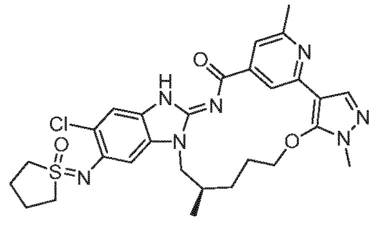

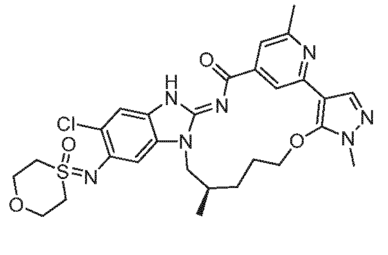

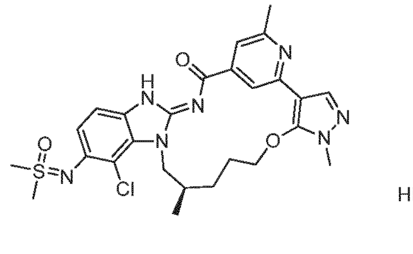

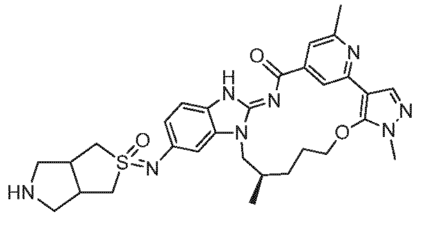

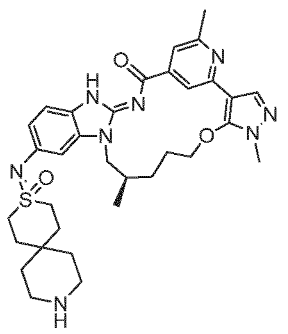

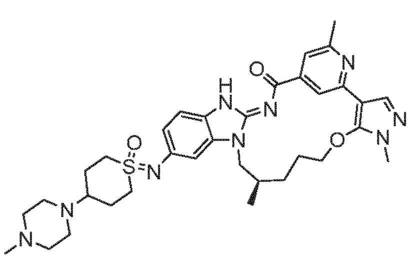

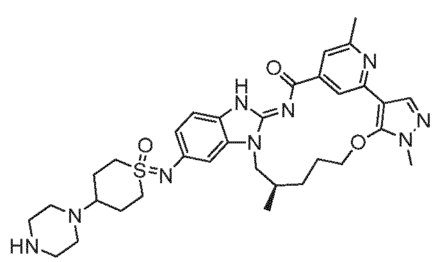

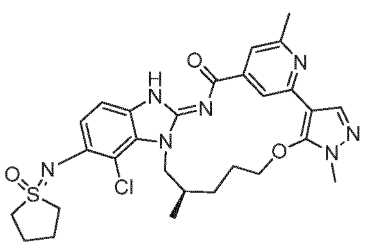

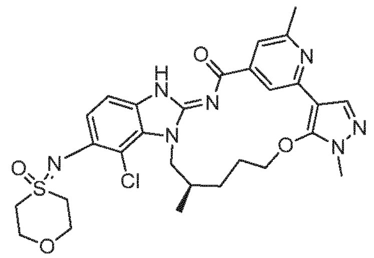

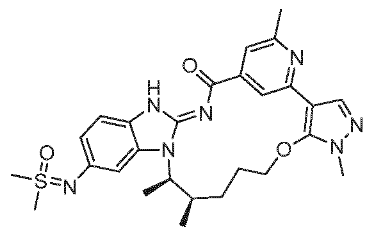

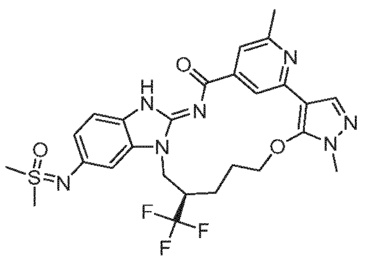

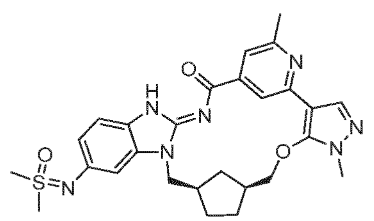

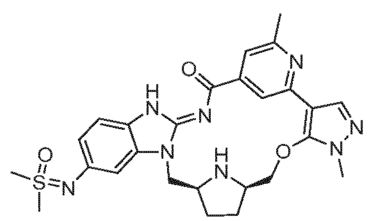

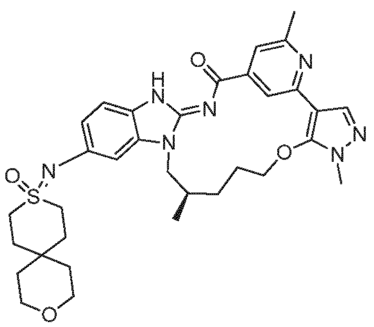

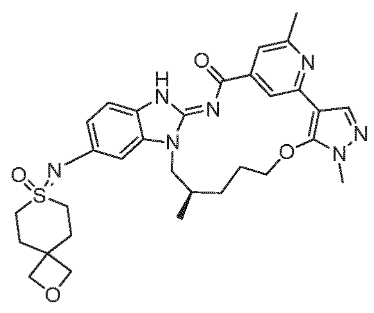

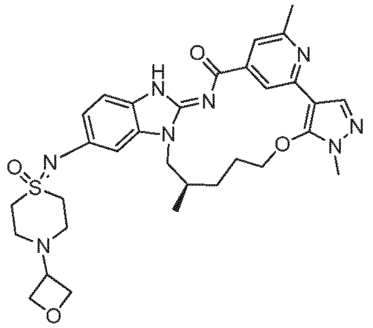

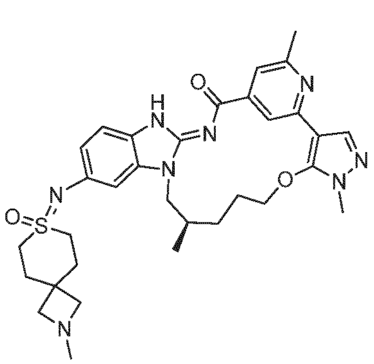

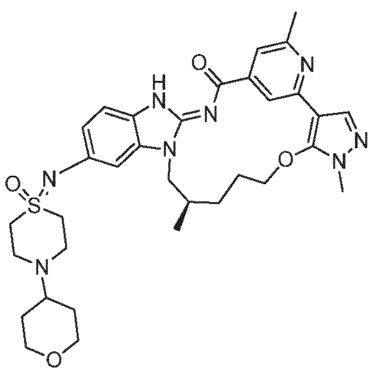

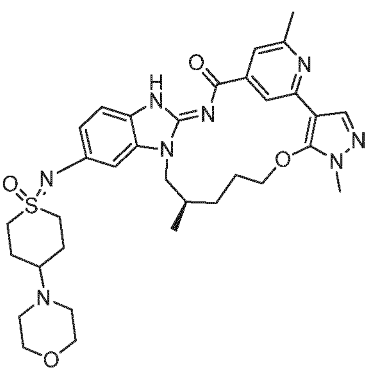

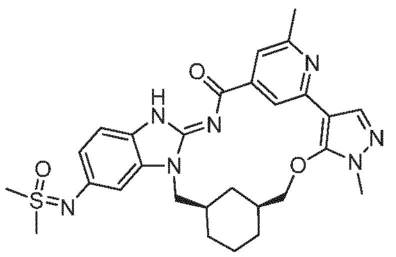
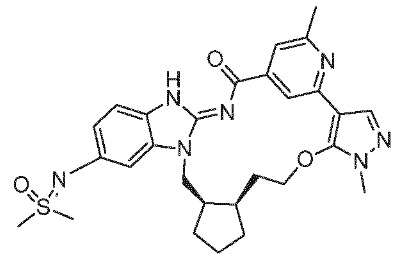
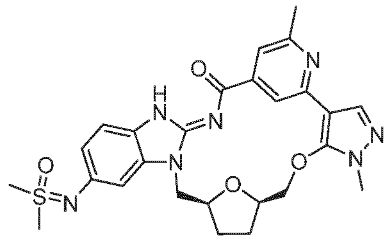

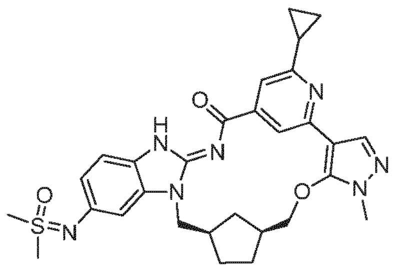
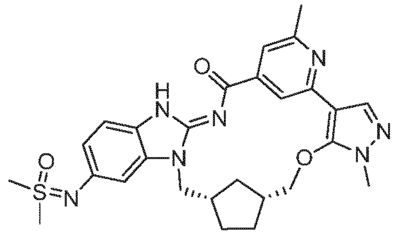
.

**INTERNATIONAL SEARCH REPORT**

International application No. **PCT/CN2022/105959**

**A. CLASSIFICATION OF SUBJECT MATTER**

C07D 498/22(2006.01)i； A61K 31/496(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D498/-； A61K31/-； A61P35/-

Documentation searched other than minimum documentation to the extent that such documents are included in the fields searched

Electronic data base consulted during the international search (name of data base and, where practicable, search terms used)

CNPAT, WPI, EPODOC, STN: 结构式, EGFR, 抑制剂, 大环酰胺, BI-4020, inhibitor, macrocyclic amide

**C. DOCUMENTS CONSIDERED TO BE RELEVANT**

| Category* | Citation of document, with indication, where appropriate, of the relevant passages | Relevant to claim No. |
|---|---|---|
| X | WO 2020260252 A1 (BOEHRINGER INGELHEIM INTERNATIONAL GMBH) 30 December 2020 (2020-12-30)<br>claims 1-18, and description, table 14 | 1-2, 9-17, 19-20 |
| PX | CN 114656482 A (NANJING SANHOME PHARMACEUTICAL CO., LTD.) 24 June 2022 (2022-06-24)<br>claims 1-8 and 10 | 1-2, 9-17, 19-20 |
| PX | CN 114163454 A (SHANGHAI HANSOH BIOMEDICAL CO., LTD. et al.) 11 March 2022 (2022-03-11)<br>1-7, 9-10 | 1-2, 9-17, 19-20 |
| PX | CN 113527335 A (NANJING SANHOME PHARMACEUTICAL CO., LTD.) 22 October 2021 (2021-10-22)<br>claims 1-8 and 10 | 1-2, 9-17, 19-20 |
| A | WO 2021121261 A1 (BEIJING TIDE PHARMACEUTICAL CO., LTD.) 24 June 2021 (2021-06-24)<br>claims 1-24 | 1-20 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 September 2022** | **10 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

INTERNATIONAL SEARCH REPORT

International application No.
**PCT/CN2022/105959**

**C. DOCUMENTS CONSIDERED TO BE RELEVANT**

| Category* | Citation of document, with indication, where appropriate, of the relevant passages | Relevant to claim No. |
|---|---|---|
| A | ENGELHARDT, Harald et al. "Start Selective and Rigidify: The Discovery Path toward a Next Generation of EGFR Tyrosine Kinase Inhibitors" *Journal of Medicinal Chemistry,* Vol. 62, 05 November 2019 (2019-11-05), pp. 10272-10293 | 1-20 |

Form PCT/ISA/210 (second sheet) (January 2015)

**INTERNATIONAL SEARCH REPORT**
**Information on patent family members**

International application No.
**PCT/CN2022/105959**

| Patent document cited in search report | | | Publication date (day/month/year) | Patent family member(s) | | | Publication date (day/month/year) |
|---|---|---|---|---|---|---|---|
| WO | 2020260252 | A1 | 30 December 2020 | JP | 2022538228 | A | 01 September 2022 |
| | | | | CN | 114007698 | A | 01 February 2022 |
| | | | | EP | 3986564 | A1 | 27 April 2022 |
| CN | 114656482 | A | 24 June 2022 | WO | 2022135432 | A1 | 30 June 2022 |
| CN | 114163454 | A | 11 March 2022 | | None | | |
| CN | 113527335 | A | 22 October 2021 | | None | | |
| WO | 2021121261 | A1 | 24 June 2021 | KR | 20220114615 | A | 17 August 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110839425
- CN 202110960699
- CN 202111127366
- CN 202111187955
- CN 202210093818
- CN 202210731479


### Non-patent literature cited in the description


- *J. Med. Chem.,* 2019, vol. 62 (22), 10272-10293